# EUROPEAN PATENT SPECIFICATION

(11) **EP 1 444 210 B1**
(45) Date of publication and mention of the grant of the patent: **18.02.2009**
(21) Application number: 02774960.5
(22) Date of filing: 31.10.2002
(51) Int. Cl.: C07D 239/94, C07D 401/14, C07D 401/12, C07D 409/12, C07D 403/12, C07D 417/14, C07D 413/14, C07D 409/14, A61K 31/505, A61P 35/00

(54) **QUNAZOLINE DERIVATIVES AS ANTITUMOR AGENTS**
QUINAZOLIN DERIVATE ALS ANTITUMOR-MITTEL
DERIVES QUINAZOLINE UTILISES EN TANT QU'AGENTS ANTITUMORAUX

(30) Priority: 03.11.2001 GB 0126433; 05.12.2001 GB 0129059
(43) Date of publication of application: 11.08.2004
(73) Proprietor: AstraZeneca AB, 151 85 Södertälje (SE)
(72) Inventor: HENNEQUIN, Laurent, Francois, Andre, Macclesfield, Cheshire SK10 4TG (GB); KETTLE, Jason, Grant, Macclesfield, Cheshire SK10 4TG (GB); PASS, Martin, Macclesfield, Cheshire SK10 4TG (GB); BRADBURY, Robert, Hugh, Macclesfield, Cheshire SK10 4TG (GB)
(86) International application number: PCT/GB2002/004931
(87) International publication number: WO 2003/040108

(56) References cited:
- WO-A-96/09294
- WO-A-96/15118
- WO-A-97/03069
- WO-A-97/30034
- WO-A-98/02434

## Description

The invention concerns certain novel quinazoline derivatives, or pharmaceutically-acceptable salts thereof, which possess anti-tumour activity and are accordingly useful in methods of treatment of the human or animal body. The invention also concerns processes for the manufacture of said quinazoline derivatives, to pharmaceutical compositions containing them and to their use in therapeutic methods, for example in the manufacture of medicaments for use in the prevention or treatment of solid tumour disease in a warm-blooded animal such as man.

Many of the current treatment regimes for diseases resulting from the abnormal regulation of cellular proliferation such as psoriasis and cancer, utilise compounds that inhibit DNA synthesis and cellular proliferation. To date, compounds used in such treatments are generally toxic to cells however their enhanced effects on rapidly dividing cells such as tumour cells can be beneficial. Alternative approaches to these cytotoxic anti-tumour agents are currently being developed, for example selective inhibitors of cell signalling pathways. These types of inhibitors are likely to have the potential to display an enhanced selectivity of action against tumour cells and so are likely to reduce the probability of the therapy possessing unwanted side effects.

Eukaryotic cells are continually responding to many diverse extracellular signals that enable communication between cells within an organism. These signals regulate a wide variety of physical responses in the cell including proliferation, differentiation, apoptosis and motility. The extracellular signals take the form of a diverse variety of soluble factors including growth factors as well as paracrine and endocrine factors. By binding to specific transmembrane receptors, these ligands integrate the extracellular signal to the intracellular signalling pathways, therefore transducing the signal across the plasma membrane and allowing the individual cell to respond to its extracellular signals. Many of these signal transduction processes utilise the reversible process of the phosphorylation of proteins that are involved in the promotion of these diverse cellular responses. The phosphorylation status of target proteins is regulated by specific kinases and phosphatases that are responsible for the regulation of about one third of all proteins encoded by the mammalian genome. As phosphorylation is such an important regulatory mechanism in the signal transduction process, it is therefore not surprising that aberrations in these intracellular pathways result in abnormal cell growth and differentiation and so promote cellular transformation (reviewed in Cohen et al, Curr Opin Chem Biol, 1999, 3, 459-465).

It has been widely shown that a number of these tyrosine kinases are mutated to constitutively active forms and/or when over-expressed result in the transformation of a variety of human cells. These mutated and over-expressed forms of the kinase are present in a large proportion of human tumours (reviewed in Kolibaba et al, Biochimica et Biophysica Acta,1997, 133, F217-F248). As tyrosine kinases play fundamental roles in the proliferation and differentiation of a variety of tissues, much focus has centred on these enzymes in the development of novel anti-cancer therapies. This family of enzymes is divided into two groups - receptor and non-receptor tyrosine kinases e.g. EGF Receptors and the SRC family respectively. From the results of a large number of studies including the Human Genome Project, about 90 tyrosine kinase have been identified in the human genome, of this 58 are of the receptor type and 32 are of the non-receptor type. These can be compartmentalised in to 20 receptor tyrosine kinase and 10 non-receptor tyrosine kinase sub-families (Robinson et al, Oncogene, 2000, 19, 5548-5557).

The receptor tyrosine kinases are of particular importance in the transmission of mitogenic signals that initiate cellular replication. These large glycoproteins, which span the plasma membrane of the cell possess an extracellular binding domain for their specific ligands . (such as Epidermal Growth Factor (EGF) for the EGF Receptor). Binding of ligand results in the activation of the receptor's kinase enzymatic activity that is encoded by the intracellular portion of the receptor. This activity phosphorylates key tyrosine amino acids in target proteins, resulting in the transduction of proliferative signals across the plasma membrane of the cell.

It is known that the erbB family of receptor tyrosine kinases, which include EGFR, erbB2, erbB3 and erbB4, are frequently involved in driving the proliferation and survival of tumour cells (reviewed in Olayioye et al., EMBO J., 2000, 19, 3159). One mechanism in which this can be accomplished is by overexpression of the receptor at the protein level, generally as a result of gene amplification. This has been observed in many common human cancers (reviewed in Klapper et al., Adv. Cancer Res., 2000, 77, 25) such as breast cancer (Sainsbury et al., Brit. J. Cancer, 1988, 58, 458; Guerin et al., Oncogene Res., 1988, 3, 21; Slamon et al., Science, 1989, 244, 707; Klijn et al., Breast Cancer Res. Treat., 1994, 29, 73 and reviewed in Salomon et al., Crit. Rev. Oncol. Hematol., 1995, 19, 183), non-small cell lung cancers (NSCLCs) including adenocarcinomas (Cerny et al., Brit. J. Cancer, 1986, 54, 265; Reubi et al., Int. J. Cancer, 1990, 45, 269; Rusch et al., Cancer Research, 1993, 53, 2379; Brabender et al, Clin. Cancer Res., 2001, 7, 1850) as well as other cancers of the lung (Hendler et al., Cancer Cells, 1989, 7, 347; Ohsaki et al., Oncol. Rep., 2000, 7, 603), bladder cancer (Neal et al., Lancet, 1985, 366; Chow et al., Clin. Cancer Res., 2001, 7, 1957, Zhau et al., Mol Carcinog., 3, 254), oesophageal cancer (Mukaida et al., Cancer, 1991, 68, 142), gastrointestinal cancer such as colon, rectal or stomach cancer (Bolen et al., Oncogene Res., 1987, 1, 149; Kapitanovic et al., Gastroenterology, 2000, 112,1103; Ross et al., Cancer Invest., 2001, 19, 554), cancer of the prostate (Visakorpi et al., Histochem. J., 1992, 24, 481; Kumar et al., 2000, 32, 73; Scher et al., J. Natl. Cancer Inst., 2000, 92, 1866), leukaemia (Konaka et al., Cell, 1984, 37, 1035, Martin-Subero et al., Cancer Genet Cytogenet., 2001, 127, 174), ovarian (Hellstrom et al., Cancer Res., 2001, 61, 2420), head and neck (Shiga et al., Head Neck, 2000, 22, 599) or pancreatic cancer (Ovotny et al., Neoplasma, 2001, 48, 188). As more human tumour tissues are tested for expression of the erbB family of receptor tyrosine kinases it is expected that their widespread prevalence and importance will be further enhanced in the future.

As a consequence of the mis-regulation of one or more of these receptors (in particular erbB2), it is widely believed that many tumours become clinically more aggressive and so correlate with a poorer prognosis for the patient (Brabender et al, Clin. Cancer Res., 2001, 7, 1850; Ross et al, Cancer Investigation, 2001, 19, 554, Yu et al., Bioessays, 2000, 22.7, 673). In addition to these clinical findings, a wealth of pre-clinical information suggests that the erbB family of receptor tyrosine kinases are involved in cellular transformation. This includes the observations that many tumour cell lines overexpress one or more of the erbB receptors and that EGFR or erbB2 when transfected into non-tumour cells have the ability to transform these cells. This tumourigenic potential has been further verified as transgenic mice that overexpress erbB2 spontaneously develop tumours in the mammary gland. In addition to this, a number of pre-clinical studies have demonstrated that anti-proliferative effects can be induced by knocking out one or more erbB activities by small molecule inhibitors, dominant negatives or inhibitory antibodies (reviewed in Mendelsohn et al., Oncogene, 2000, 19, 6550). Thus it has been recognised that inhibitors of these receptor tyrosine kinases should be of value as a selective inhibitor of the proliferation of mammalian cancer cells (Yaish et al. Science, 1988, 242, 933, Kolibaba et al, Biochimica et Biophysica Acta, 1997, 133, F217-F248; Al-Obeidi et al, 2000, Oncogene, 19, 5690-5701; Mendelsohn et al, 2000, Oncogene, 19, 6550-6565). In addition to this pre-clinical data, findings using inhibitory antibodies against EGFR and erbB2 (c-225 and trastuzumab respectively) have proven to be beneficial in the clinic for the treatment of selected solid tumours (reviewed in Mendelsohn et al, 2000, Oncogene, 19, 6550-6565).

Amplification and/or activity of members of the ErbB type receptor tyrosine kinases have been detected and so have been implicated to play a role in a number of non-malignant proliferative disorders such as psoriasis (Ben-Bassat, Curr. Pharm. Des., 2000, 6, 933; Elder et al., Science, 1989, 243, 811), benign prostatic hyperplasia (BPH) (Kumar et al., Int. Urol. Nephrol., 2000, 32,73), atherosclerosis and restenosis (Bokemeyer et al., Kidney Int., 2000, 58, 549). It is therefore expected that inhibitors of erbB type receptor tyrosine kinases will be useful in the treatment of these and other non-malignant disorders of excessive cellular proliferation.

International Patent Applications WO 96/33977, WO 96/33978, WO 96/33979, WO 96/33980 and WO 96/33981 disclose that certain quinazoline derivatives which bear an anilino substituent at the 4-position possess receptor tyrosine kinase inhibitory activity.

A review of the structure activity relationship of various quinazoline derivatives is disclosed by G. W. Rewcastle et al (J. Med. Chem. 1995, 38, 3428-3487), including a number of 5-substituted compounds. However, such 5-substituted compounds are stated to have low in-vitro activity as EGFR tyrosine kinase inhibitors compared to quinazolines substituted at the 6- and 7- positions

WO 96/09294 discloses 4-anilinoquinazoline derivatives, including 5-chloro and 5-methoxy substituted quinazoline derivatives as protein tyrosine kinase inhibitors.

WO 06/15118 discloses aniline derivatives, which possess anti-proliferative activity, such as anti-cancer activity.

WO 97/30034 discloses quinazoline derivatives, which possess anti-proliferative activity such as anti-cancer activity.

WO 98/02434 discloses quinoline and quinazoline derivatives, which exhibit protein tyrosine kinase inhibition.

WO 97/03069 discloses quinoline and quinazoline derivatives, which exhibit protein tyrosine kinase inhibition.

WO 01/94341 (Co-pending International Patent Application PCT/GB01/02424) discloses that certain quinazoline derivatives which carry a 5-substituent are inhibitors of the Src family of non-receptor tyrosine kinases, such as c-Src, c-Yes and c-Fyn.

We have now found that -surprisingly certain 5-substituted quinazoline derivatives possess potent anti-tumour activity. Without wishing to imply that the compounds disclosed in the present invention possess pharmacological activity only by virtue of an effect on a single biological process, it is believed that the compounds provide an anti-tumour effect by way of inhibition of one or more of the erbB family of receptor tyrosine kinases that are involved in the signal transduction steps which lead to the proliferation of tumour cells. In particular, it is believed that the compounds of the present invention provide an anti-tumour effect by way of inhibition of EGFR and/or erbB2 receptor tyrosine kinases.

Generally the compounds of the present invention possess potent inhibitory activity against the erbB receptor tyrosine kinase family, for example by inhibition of EGFR and/or erbB2 and/or erbB4 receptor tyrosine kinases, whilst possessing less potent inhibitory activity against other kinases. Furthermore, generally the compounds of the present invention possess substantially better potency against the erbB2 over that of the EGFR tyrosine kinase, thus potentially providing effective treatment for erbB2 driven tumours. The invention also includes compounds that are active against all or a combination of EGFR, erbB2 and erbB4 receptor tyrosine kinases, thus potentially providing treatments for conditions mediated by one or more of these receptor tyrosine kinases.

According to a first aspect of the invention there is provided a quinazoline derivative of the Formula I 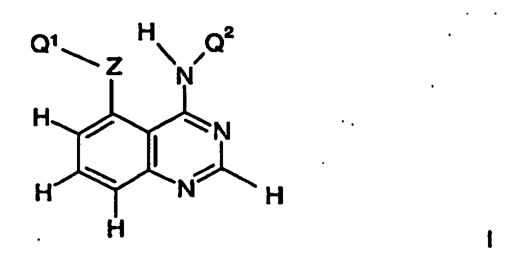 wherein
the **Q**¹-**Z**- group is selected from cyclopentyloxy, cyclohexyloxy, tetrahydrofuran-3-yloxy, tetrahydropyran-3-yloxy, tetrahydropyran-4-yloxy, tetrahydrothiopyran-3-yloxy, tetrahydrothiopyran-4-yloxy, 1,1-dioxotetrahydrothiopyran-3-yloxy, 1,1-dioxotetrahydrothiopyran-4-yloxy, 1-oxotetrahydrothiopyran-3-yloxy, 1-oxotetrahydrothiopyran-4-yloxy, tetrahydrothien-3-yloxy, 1,1-dioxodotetrahydrothien-3-yloxy, 1-oxotetrahydrothien-3-yloxy, pyrrolidin-3-yloxy, pyrrolidin-2-yloxy, piperidin-3-yloxy, piperidin-4-yloxy, homopiperidin-3-yloxy, homopiperidin-4-yloxy and azetidin-3-yloxy,
and wherein any azetidinyl, pyrrolidinyl, piperidinyl or homopiperidinyl group within the Q¹-Z- group is optionally N- substituted by a substituent T¹ selected from (1-4C)alkyl, (2-4C)alkenyl, (2-4C)alkynyl, (2-4C)alkanoyl, (1-4C)alkoxycarbonyl, carbamoyl, N-(1-4C)alkylcarbamoyl, N,N-di-(1-4C)alkylcarbamoyl and (1-4C)alkylsulphonyl,
and wherein adjacent carbon atoms in any (2-4C)alkylene chain within the N-substituent T¹ are optionally separated by the insertion into the chain of a group selected from O, NH and CO,
and wherein any CH₂ or CH₃ group within the N- substituent T¹ optionally bears on each said CH₂ or CH₃ group a substituent selected from hydroxy, amino, methylamino, di-methylamino, ethylamino, diethylamino, carbamoyl, N-methylcarbamoyl, N,N-dimethylcarbamoyl, acetyl, methoxycarbonyl and ethoxycarbonyl,
and wherein any heterocyclyl group within the Q¹-Z- group optionally bears 1 or 2 oxo substituents;
**R**¹ is hydrogen;
**Q**² is selected from a group of formula Ia, Ib and Id 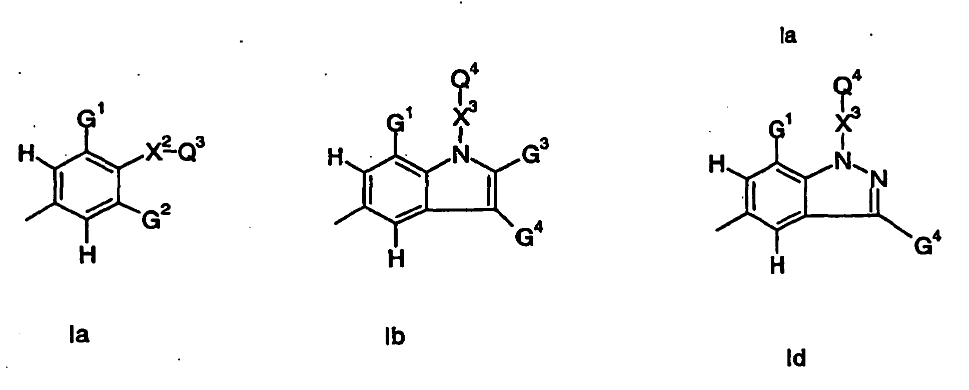 wherein G² and G³ are hydrogen,
G¹ is selected from hydrogen, fluoro, chloro, bromo, cyano, (1-4C)alkyl and (2-3C)alkynyl,
G⁴ is selected from hydrogen, fluoro, chloro, bromo, cyano, (1-4C)alkyl and ethynyl,
X² is selected from O, S, NH, N(CH₃), OCH₂, CO and NHCH₂,
and Q³ is a group selected from phenyl, 2-furyl, 2- or 3-thienyl, 2-,4- or 5-oxazolyl, 3-,4- or 5-isoxazolyl, 2-,4-or 5-1H-imidazolyl, 2-,4-or 5-thiazolyl, 3- or 4-(1H-1,2,4-triazolyl), 3- or 5-(1,2,5-thiadiazolyl), 2- 3- or 4-pyridyl, 2-, 4- or 5-pyrimidinyl, 1,3-benzodioxol-4-yl, 1,3-benzadioxol-5-yl, 2-, 3-, 4-, 5-, 6-, 7- or 8-quinolinyl, 1-, 3-,4-, 5-, 6-, 7- or 8-isoquinolinyl and 2-, 3-, 4-, 5-, 6-, 7- or 8-quinazolinyl, which group is optionally substituted with one or two substituents, which may be the same or different, selected from nitro, fluoro, chloro, bromo, (1-4C)alkyl, trifluoromethyl, (1-4C)alkoxy, (2-3C)alkynyl and cyano,
or X² is CO and Q³ is a group selected from pyrrolidin-1yl, piperidino, homopiperidino, morpholino, piperazin-1-yl, homopiperazin-1-yl, decahydroquinolin-1-yl, and decahydroisoquinolin-2-yl, which group optionally bears 1 or 2 substituents which may be the same or different selected from fluoro, chloro, bromo, cyano, hydroxy and (1-4C)alkyl, and any heterocyclyl group represented by Q³ optionally bears 1 or 2 oxo substituents,
X³ is selected from SO₂ and CH₂,
Q⁴ is a group selected from phenyl, phenyl, 2-furyl, 3-furyl, 2-(1,3-oxazolyl), 4-(1,3-oxazolyl), 3-, 4- or 5-isoxazolyl, 2-imidazolyl, 4-imidazolyl, 2-, 3-or 4-pyridyl, 2-, 4- or 5-pyrimidinyl, 1,2,4-triazol-3-yl, 2-thienyl, 3-thienyl, 2-(1,3-thiazolyl), 4-(1,3-thiazolyl), 3-, 4-or 5- isothiazolyl and 1,2,5-thiadiazol-3-yl group which group optionally bears 1 or 2 substituents, which may be the same or different, selected from nitro, fluoro, chloro, bromo, cyano, (1-4C)alkyl, (1-4C)alkoxy, trifluoromethyl and (2-3C)alkynyl;
or a pharmaceutically acceptable salt thereof.

In this specification the generic term "alkyl" includes both straight-chain and branched-chain alkyl groups such as propyl, isopropyl and tert-butyl, and (3-7C)cycloalkyl groups such as cyclopropyl, cyclobutyl, cyclopentyl, cyclohexyl and cycloheptyl. However references to individual alkyl groups such as "propyl" are specific for the straight-chain version only, references to individual branched-chain alkyl groups such as "isopropyl" are specific for the branched-chain version only and references to individual cycloalkyl groups such as "cyclopentyl" are specific for that 5-membered ring only. An analogous convention applies to other generic terms, for example (1-6C)alkoxy includes methoxy, ethoxy, cyclopropyloxy and cyclopentyloxy, (1-6C)alkylamino includes methylamino, ethylamino, cyclobutylamino and cyclohexylamino, and di-[(1-6Calkyl]amino includes dimethylamino, diethylamino, N-cyclobutyl-N-methylamino and N-cyclohexyl-N-ethylamino.

It is to be understood that, insofar as certain of the compounds of Formula I defined above may exist in optically active or racemic forms by virtue of one or more asymmetric carbon atoms, the invention includes in its definition any such optically active or racemic form which possesses the above-mentioned activity. The synthesis of optically active forms may be " carried out by standard techniques of organic chemistry well known in the art, for example by synthesis from optically active starting materials or by resolution of a racemic form. Similarly, the above-mentioned activity may be evaluated using the standard laboratory techniques referred to hereinafter.

It is to be understood that the present invention includes in its definition any and all tautomeric forms of the compounds of the formula I which possess the above mentioned activity.

It is also to be understood that in so far as certain compounds of the formula 1 may exist in solvated forms as well as unsolvated forms, for example, hydrated forms, the present invention includes any and all such solvated forms, which possess the above mentioned activity.

Suitable values for the generic radicals referred to above include those set out below.

A suitable value for any one of the 'Q' groups (Q¹, Q³ to Q⁴) when it is aryl or for the aryl group within a 'Q' group is, for example, phenyl or naphthyl, preferably phenyl.

A suitable value for any one of the 'Q' groups (Q¹) when it is (3-7C)cycloalkyl or for the (3-7C)cycloalkyl group within a 'Q' group is, for example, cyclopropyl, cyclobutyl, cyclopentyl, cyclohexyl, cycloheptyl or bicyclo[2.2.1]heptyl.

A suitable value for any one of the 'Q' groups (Q³ to Q⁴) when it is heteroaryl or for the heteroaryl group within a 'Q' group is, for example, an aromatic 5- or 6-membered monocyclic ring or a 9- or 10-membered-bicyclic ring with up to five ring heteroatoms selected from oxygen, nitrogen and sulphur, which, unless specified otherwise, may be carbon or nitrogen linked. Examples of suitable values of "heteroaryl" include furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazenyl, 1,3-benzodioxolyl, benzofuranyl, indolyl, benzothienyl, benzoxazolyl, benzisoxazolyl, benzimidazolyl, benzothiazolyl, imidazopyridinyl, indazolyl, benzofurazanyl, quinolyl, isoquinolyl, quinazolinyl, quinoxalinyl, cinnolinyl or naphthyridinyl. Such as furyl, pyrrolyl, thienyl, oxazolyl, isoxazolyl, imidazolyl, pyrazolyl, thiazolyl, isothiazolyl, oxadiazolyl, thiadiazolyl, triazolyl, tetrazolyl, pyridyl, pyridazinyl, pyrimidinyl, pyrazinyl, 1,3,5-triazinyl, 1,3-benzodioxolyl, benzofuranyl, indolyl, benzothienyl, benzoxazolyl, benzimidazolyl, benzothiazolyl, indazolyl, benzofurazanyl, quinolyl, isoquinolyl, quinazolinyl, quinoxalinyl, cinnolinyl or naphthyridinyl.

A suitable value for any one of the 'Q' groups (Q¹ or Q³) when it is heterocyclyl or for the heterocyclyl group within a 'Q' group is, for example, a non-aromatic saturated or partially saturated 3 to 10 membered monocyclic or bicyclic ring with up to five heteroatoms selected from oxygen, nitrogen and sulphur, which, unless specified otherwise, may be carbon or nitrogen linked. Examples of suitable values of "heterocylcyl" include oxiranyl, oxetanyl, azetidinyl, tetrahydrofuranyl, tetrahydropyranyl, oxepanyl, pyrrolinyl, pyrrolidinyl, morpholinyl, tetrahydro-1,4-thiazinyl, 1,1-dioxotetrahydro-1,4-thiazinyl, piperidinyl, homopiperidinyl, piperazinyl, homopiperazinyl, dihydropyridinyl, tetrahydropyridinyl, dihydropyrimidinyl, tetrahydropyrimidinyl, tetrahydrothienyl, tetrahydrothiopyranyl, decahydroisoquinolinyl or decahydroquinolinyl, preferably tetrahydrofuranyl, tetrahydropyranyl, pyrrolidinyl, morpholinyl, 1,4-oxazepanyl, 1,1-dioxotetrahydro-4H-1,4-thiazinyl, piperidinyl or piperazinyl, more preferably tetrahydrofuran-3-yl, tetrahydropyran-4-yl, tetrahydrothien -3-yl, tetrahydrothiopyran-4-yl, pyrrolidin-3-yl, morpholino, 1,1-dioxotetrahydro-4H-1,4-thiazin-4-yl, piperidino, piperidin-4-yl, piperidin-3-yl or piperazin-1-yl. A nitrogen or sulphur atom within a heterocyclyl group may be oxidized to give the corresponding N or S oxide, for example 1,1-dioxotetrahydrothienyl, 1-oxotetrahydrothienyl, 1,1-dioxotetrahydrothiopyranyt or 1-oxotetrahydrothiopyranyl. A suitable value for such a group which bears 1 or 2 oxo or thioxo substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl.

A suitable value for any one of the 'Q' groups (such as Q³) when it is a nitrogen containing heterocyclyl group is, for example, a non-aromatic saturated or partially saturated 3 to 10 membered monocyclic or bicyclic ring with up to five heteroatoms selected from oxygen, nitrogen and sulphur, provided at lease one heteroatom is nitrogen. Suitable values include, for example, those heterocyclic groups mentioned above that contain at least one nitrogen atom, for example azetidinyl, pyrrolinyl, pyrrolidinyl, morpholinyl (including morpholino), tetrahydro-1,4-thiazinyl, 1,1-dioxotetrahydro-1,4-thiazinyl, piperidinyl (including piperidino), homopiperidinyl, piperazinyl, homopiperazinyl, dihydropyridinyl, tetrahydropyridinyl, dihydropyrimidinyl, tetrahydropyrimidinyl, decahydroisoquinolinyl or decahydroquinolinyl.

Suitable values for any of the 'G' groups (G¹ and G⁴) within Q², or for various groups within Q², or for Q¹ or for various groups within Q¹, or for various groups within the Q¹-Z- group include:-

| | |
|---|---|
| for halogeno | fluoro, chloro, bromo and iodo; |
| for (1-6C)alkyl: | methyl, ethyl, propyl, isopropyl and tert-butyl; |
| for (2-8C)alkenyl: | vinyl, isopropenyl, allyl and but-2-enyl; |
| for (2-8C)alkynyl: | ethynyl, 2-propynyl and but-2-ynyl; |
| for (1-6C)alkoxy: | methoxy, ethoxy, propoxy, isopropoxy and butoxy; |
| for (2-6C)alkenyloxy: | vinyloxy and allyloxy; |
| for (2-6C)alkynyloxy: | ethynyloxy and 2-piopynyloxy; |
| for (1-6C)alkylthio: | methylthio, ethylthio and propylthio; |
| for (1-6C)alkylsulphinyl: | methylsulphinyl and ethylsulphinyl; |
| for (1-6C)alkylsulphonyl: | methylsulphonyl and ethylsulphonyl; |
| for (1-6C)alkylamino: | methylamino, ethylamino, propylamino, |
| | isopropylamino and butylamino; |
| for di-[(1-6C)alkyl]amino: | dimethylamino, diethylamino, N-ethyl-N-methylamino and diisopropylamino; |
| for (1-6C)alkoxycarbonyl: | methoxycarbonyl, ethoxycarbonyl, propoxycarbonyl and tert-butoxycarbonyl; |
| for N-(1-6C)alkylcarbamoyl: | N-methylcarbamoyl, N-ethylcarbamoyl and N-propylcarbamoyl; |
| for N,N-di-[(1-6C)alkyl]carbamoyl: | N,N-dimethylcarbamoyl, N-ethyl-N-methylcarbamoyl and N,N-diethylcarbamoyl; |
| for (2-6C)alkanoyl: | acetyl and propionyl; |
| for (2-6C)alkanoyloxy: | acetoxy and propionyloxy; |
| for (2-6C)alkanoylamino: | acetamido and propionamido; |
| for amino(2-6C)alkanoyl: | aminoacetyl and 2-aminopropionyl; |
| for N-(1-6C)alkylamino(2-6C)alkanoyl: | N-methylaminoacetyl and 2-(N-methylaminopropionyl; |
| for N,N-di-[(1-6C)alkyl]amino(2-6C)alkanoyl: | N,N-di-methylaminoacetyl; |
| for N-(1-6C)alkyl-(2-6C)alkanoylamino: | N-methylacetamido and N-methylpropionamido; |
| for N-(1-6C)alkylsulphamoyl: | N-methylsulphamoyl and N-ethylsulphamoyl; |
| for N,N-di-[(1-6C)alkyl]sulphamoyl: | N,N-dimethylsulphamoyl; |
| for (1-6C)alkanesulphonylamino: | methanesulphonylamino and ethanesulphonylamino; |
| for N-(1-6C)alkyl-(1-6C)alkanesulphonylamino: | N-methylmethanesulphonylamino and N-methylethanesulphonylamino; |
| for (3-6C)alkenoylamino: | acrylamido, methacrylamido and crotonamido; |
| for N-(1-6C)alkyl-(3-6C)alkenoylamino: | N-methylacrylamido and N-methylcrotonamido; |
| for (3-6C)alkynoylamino: | propiolamido; |
| for N-(1-6C)alkyl-(3-6C)alkynoylamino: | N-methylpropiolamido; |
| for amino-(1-6C)alkyl: | aminomethyl, 2-aminoethyl, 1-aminoethyl and 3-aminopropyl; |
| for (1-6C)alkylamino-(1-6C)alkyl: | methylaminomethyl, ethylaminomethyl, |
| | 1-methylaminoethyl, 2-methylaminoethyl, |
| | 2-ethylaminoethyl and 3-methylaminopropyl; |
| for di-[(1-6C)alkyl]amino-(1-6C)alkyl: | dimethylaminomethyl, diethylaminomethyl, |
| | 1-dimethylaminoethyl, 2-dimethylaminoethyl and 3-dimethylaminopropyl; |
| for halogeno-(1-6C)alkyl: | chloromethyl, 2-chloroethyl, 1-chloroethyl and 3-chloropropyl; |
| for hydroxy-(1-6C)alkyl: | hydroxymethyl, 2-hydroxyethyl, 1-hydroxyethyl and 3-hydroxypropyl; |
| for (1-6C)alkoxy-(1-6C)alkyl: | methoxymethyl, ethoxymethyl, 1-methoxyethyl, |
| | 2-methoxyethyl, 2-ethoxyethyl and 3-methoxypropyl; |
| for cyano-(1-6C)alkyl: | cyanomethyl, 2-cyanoethyl, 1-cyanoethyl and 3-cyanopropyl; |
| for carboxy-(1-6C)alkyl: | carboxymethyl, 2-carboxyethyl, 1-carboxyethyl and 3-carboxypropyl; |
| for (1-6C)alkylthio-(1-6C)alkyl: | methylthiomethyl, ethylthiomethyl, |
| | 2-methylthioethyl, 1-methylthioethyl and 3-methylthiopropyl; |
| for (1-6C)alkylsulphinyl-(1-6C)alkyl: | methylsulphinylmethyl, ethylsulphinylmethyl, |
| | 2-methylsulphinylethyl, 1-methylsulphinylethyl and 3-methylsulphinylpropyl; |
| for (1-6C)alkylsulphonyl-(1-6C)alkyl: | methylsulphonylmethyl, ethylsulphonylmethyl, |
| | 2-methylsulphonylethyl, 1-methylsulphonylethyl and 3-methylsulphonylpropyl; |
| for (2-6C)alkanoylamino-(1-6C)alkyl: | acetamidomethyl, propionamidomethyl and 2-acetamidoethyl; |
| for (1-6C)alkoxycarbonyl-(1-6C)alkyl: | methoxycarbonylmethyl, 2-methoxycarbonylethyl and 2-ethoxycarbonylethyl; |
| for (1-6C)alkoxycarbonylamino-(1-6C)alkyl: | methoxycarbonylaminomethyl, ethoxycarbonylaminomethyl, tert-butoxycarbonylaminomethyl and 2-methoxycarbonylaminoethyl; |
| for (1-6C)alkoxycarbonylamino-(1-6C)alkyl: | methoxycarbonylaminomethyl, ethoxycarbonylaminomethyl, tert-butoxycarbonylaminomethyl and 2-methoxycarbonylaminoethyl; |
| for carbamoyl-(1-6C)alkyl: | carbamoylmethyl, 1-carbamoylethyl, |
| | 2-carbamoylethyl and 3-carbamoylpropyl; |
| for (2-6C)alkanoyl-(1-6C)alkyl: | acetylmethyl and 2-acetylethyl; |
| for N-(1-6C)alkylcarbamoyl-(1-6C)alkyl : | N-methylcarbamoylmethyl, |
| | N-ethylcarbamoylmethyl, N-propylcarbamoylmethyl, |
| | 1-(N-methylcarbamoyl)ethyl, |
| | 1-(N-ethylcarbamoyl)ethyl, |
| | 2-(N-methylcarbamoyl)ethyl, |
| | 2-(N-ethylcarbamoyl)ethyl and |
| | 3-(N-methylcarbamoyl)propyl; and |
| for N,N-di[(1-6C)alkyl]carbamoyl-(1-6C)alkyl: | N,N-dimethylcarbamoylmethyl, |
| | N,N-diethylcarbamoylmethyl, |
| | 2-(N,N-dimethylcarbamoyl)ethyl, and |
| | 3-(N,N-dimethylcarbamoyl)propyl. |

When in this specification reference is made to a (1-4C)alkyl group it is to be understood that such groups refer to alkyl groups containing up to 4 carbon atoms. A skilled person will realise that representative examples of such groups are those listed above under (1-6C)alkyl that contain up to 4 carbon atoms, such as methyl, ethyl, propyl and butyl. A similar convention is adopted for the other groups listed above such as (1-4C)alkoxy; (2-4C)alkenyl" (2-4C)alkynyl and (2-4C)alkanoyl.

When, as defined hereinbefore, in the group of the formula -X² -Q³, X² is, for example, a OC(H)₂ linking group, it is the oxygen atom, not the carbon atom, of the OC(H)₂ linking group which is attached to the phenyl ring in the formula Ia and the carbon atom is attached to the Q³ group.

As defined hereinbefore, adjacent carbon atoms in any (2-4C)alkylene chain within a Q¹-Z- group may be optionally separated by the insertion into the chain of a group such as O. For example, insertion of a C≡C group into the ethylene chain within a 2-morpholinoethoxy group gives rise to a 4-morpholinobut-2-ynyloxy group.

When, as defined hereinbefore, any CH₂ or CH₃ group within a Q¹-Z- group optionally bears on each said CH₂ or CH₃ group one or more halogeno or (1-6C)alkyl substituents, there are suitably 1 or 2 halogeno or (1-6C)alkyl substituents present on each said CH₂ group and there are suitably 1,2 or 3 such substituents present on each said CH₃ group.

When, as defined hereinbefore, any CH₂ or CH₃ group within a Q¹-Z- group optionally bears on each said CH₂ or CH₃ group a substituent as defined hereinbefore, suitable substituents so formed include, for example, hydroxy-substituted heterocyclyl-(1-6C)alkoxy groups such as 2-hydroxy-3-piperidinopropoxy and 2-hydroxy-3-morpholinopropoxy, hydroxy-substituted heterocyclyl-(1-6C)alkylamino groups such as 2-hydroxy-3-piperidinopropylamino and 2-hydroxy-3-motpholinopropylamino, heterocyclyl-substituted (1-6C)alkylamino-(1-6C)alkyl groups such as 2-morpholinoethylaminomethyl, 2-piperazin-1-ylethylaminomethyl and 3-morpholinopropylaminomethyl and halogen substituted alkyl groups, for example difluoromethyl and 2,2-difluoroethyl.

Similar considerations apply to the attachments and substitutions within the -X²-Q³ and -X³-Q⁴ groups present in Q².

It is to be understood that when, as defined hereinbefore, any CH₂ or CH₃ group within a Q¹-Z- group optionally bears on each said CH₂ or CH₃ group a substituent as defined hereinbefore, the optional substituent may be present on any CH₂ or CH₃ group within a Q¹-Z-group, including those on the hereinbefore defined substituents that may be present on an aryl, heteroaryl or heterocyclyl groups within Q¹-Z-. For example, if Q¹ is a 1-(1-6C)alkyl-piperidin-4-yl group, the (1-6C)alkyl group may be optionally substituted by, for example a (2-6C)alkanoyl group to give a 1-((2-6C)alkanoyl-(1-6C)alkyl)-piperidin-4-yl group such as 1-(acetylmethyl)piperidin-4-yl or 1-(2-acetylethyl)piperidin-4-yl. Other suitable groups that may be so formed by Q¹ include, (1-6C)alkoxycarbonyl-(1-6C)alkyl substituted heterocyclyl groups, such as 1-(methoxycarbonylmethyl)piperidin-4-yl or 1-(2-methoxycarbonylethyl)piperidin-4-yl, carbamoyl-(1-6C)alkyl substituted heterocyclyl groups such as 1-(carbamoylmethyl)piperidin-4-yl, or (1-6C)alkoxy-(1-6C)alkyl substituted heterocyclyl groups, such as 1-(2-methoxyethyl)piperidin-4-yl.

A suitable pharmaceutically-acceptable salt of a compound of the Formula I is, for example, an acid-addition salt of a compound of the Formula I, for example an acid-addition salt with an inorganic or organic acid such as hydrochloric, hydrobromic, sulphuric, trifluoroacetic, citric or maleic acid; or, for example, a salt of a compound of the Formula I which is sufficiently acidic, for example an alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine.

Particular novel compounds of the invention include, for example, quinazoline derivatives of the Formula I, or pharmaceutically-acceptable salts thereof, wherein, unless otherwise stated, each of Z, Q¹ and Q² has any of the meanings defined hereinbefore or in paragraphs (a) to (l) hereinafter :-.
(a) Z is O;
(b) the Q¹-Z- group is selected from cyclopentyloxy, cyclohexyloxy, tetrahydrofuran-3-yloxy, tetrahydropyran-3-yloxy, tetrahydropyran-4-yloxy, tetrahydrothiopyran-3-yloxy, tetrahydrothiopyran-4-yloxy, 1,1-dioxotetrahydrothiopyran-3-yloxy, 1,1-dioxotetrahydrothiopyran-4-yloxy, 1-oxotetrahydrothiopyran-3-yloxy, 1-oxotetrahydrothiopyran-4-yloxy, tetrahydrothien-3-yloxy,1,1-dioxotetrahydrothien-3-yloxy, 1-oxotetrahydrothien-3-yloxy, pyrrolidin-3-yloxy, pyrrolidin-2-yloxy, pipexidin-3-yloxy, piperidin-4-yloxy, homopiperidin-3-yloxy, homopiperidin-4-yloxy and azetidin-3-yloxy,
   and wherein any azetidinyl, pyrrolidinyl, piperidinyl or homopiperidinyl group within the Q¹-Z- group is optionally N- substituted by a substituent, T¹, selected from (1-4C)alkyl, (2-4C)alkenyl, (2-4C)alkynyl, (2-4C)alkanoyl, (1-4C)alkoxycarbonyl, carbamoyl, N-(1-4C)alkylcarbamoyl, N,N-di-(1-4C)alkylcarbamoyl and (1-4C)alkylsulphonyl,
   and wherein adjacent carbon atoms in any (2-4C)alkylene chain within a N-substituent, T¹, are optionally separated by the insertion into the chain of a group selected from O, NH and CO,
   and wherein any CH₂ or CH₃ group within the N- substituent, T¹, optionally bears on each said CH₂ or CH₃ group a substituent selected from hydroxy, amino, methylamino, di-methylamino, ethylamino, diethylamino, carbamoyl, N-methylcarbamoyl, N,N-dimethylcarbamoyl, acetyl, methoxycarbonyl and ethoxycarbonyl,
   and wherein any heterocyclyl group within the Q¹-Z- group optionally bears 1 or 2 oxo substituents;
(c) the Q¹-Z- group is selected from cyclopentyloxy, tetrahydrofuran-3-yloxy, tetrahydropyran-4-yloxy, tetrahydrothiopyran-4-yloxy. 1,1-dioxotetrahydrothiopyran-4-yloxy, 1-oxotetrahydrothiopyran-4-yloxy, tetrahydrothien-3-yloxy, 1,1-dioxotetrahydrothien-3-yloxy, 1-oxotetrahydrothien-3-yloxy, pyrrolidin-3-yloxy, pyrrolidin-2-yloxy, piperidin-3-yloxy, piperidin-4-yloxy, homopiperidin-3-yloxy, homopiperidin-4-yloxy and azetidin-3-yloxy,
   and wherein the azetidinyl, pyrrolidinyl, piperidinyl or homopiperidinyl group within the Q¹-Z- group is optionally N- substituted by a substituent selected from methyl, ethyl, n-propyl, iso-propyl, n-butyl, iso-butyl, tert-butyl, allyl, 2-propynyl, acetyl, propionyl, methoxycarbonyl, ethoxycarbonyl, propoxycarbonyl, tert-butoxycarbonyl, methylsulphonyl, ethylsulphonyl, 2-methoxyethyl, carbamoylmethyl, N-methylcarbamoylmethyl, N,N-di-methylcarbamoylmethyl, 2-carbamoylethyl, 2-(N-methylcarbamoyl)ethyl, 2-(N,N-di-methylcarbamoyl)ethyl, acetylmethyl, 2-acetylethyl, methoxycarbonylmethyl and 2-methoxycarbonylethyl,
   and wherein any heterocyclyl group within the Q¹-Z- group optionally bears 1 or 2 oxo substituents;
(d) the Q¹-Z- group is piperidin-4-yloxy which group is optionally N- substituted by a substituent selected from methyl, ethyl, n-propyl, iso-propyl, allyl, 2-propynyl, methoxycarbonyl, ethoxycarbonyl, propoxycarbonyl, tert-butoxycarbonyl, methylsulphonyl, 2-methoxyethyl, carbamoylmethyl, N-methylcarbamoylmethyl, N,N-di-methylcarbamoymethyl, acetylmethyl, 2-acetylethyl, methoxycarbonylmethyl and 2-methoxycarbonylethyl,
   and wherein any heterocyclyl group within the Q¹-Z- group optionally bears 1 or 2 oxo substituents;
(e) Q² is a group of the formula Ia as hereinbefore defined wherein
   G¹ is selected from fluoro, chloro, bromo and ethynyl,
   X² is CO and Q³ is selected from piperidino, homopiperidino, decahydroquinolin-1-yl, and decahydroisoquinolin-2-yl,
   and wherein Q³ optionally bears 1 or 2 substituents selected from fluoro, chloro, bromo, cyano, hydroxy, methyl and ethyl,
   and wherein Q³ optionally bears 1 or 2 oxo substituents;
(f) Q² is a group of the formula Ib as hereinbefore defined wherein G¹ is hydrogen,
   G⁴ is selected from hydrogen, fluoro, chloro, bromo, cyano, methyl and ethynyl,
   X³ is selected from SO₂ and CH₂, and Q⁴ is selected from phenyl and 2-, 3- or 4-pyridyl which optionally bears 1 or 2 substituents, which may be the same or different, selected from fluoro, chloro, bromo, nitro, cyano and methyl;
(g) Q² is a group of the formula Id as hereinbefore defined wherein G¹ is hydrogen,
   G⁴ is selected from hydrogen, fluoro, chloro, bromo, cyano, methyl and ethynyl,
   X³ is selected from SO₂ and CH₂, and Q⁴ is selected from phenyl and 2-, 3- or 4-pyridyl which optionally bears 1 or 2 substituents, which may be the same or different, selected from fluoro, chloro, bromo, nitro, cyano and methyl;
(h) the Q¹ -Z-group is selected from tetrahydrofuran-3-yloxy, tetrahydropyran-3-yloxy and tetrahydropyran-4-yloxy.
   and wherein Q¹ optionally bears an oxo substituent;
(i) the Q¹ -Z-group is piperidin-4-yloxy optionally substituted at the 1 position by a substituent selected from methyl, allyl, 2-methoxyethyl and carbamoylmethyl,
   and wherein the piperidin-4-yl group optionally bears an oxo substituent;
(j) Q¹Z is 1-methylpiperidin-4-yloxy;
(k) Q² is a group of the formula Ia as hereinbefore defined wherein G², G⁶ and G⁷ are hydrogen.
   G¹ is chloro,
   X² is OCH₂,
   Q³ is phenyl optionally substituted by 1 or 2 substituents selected from fluoro and cyano (for example Q³ is selected from phenyl, 2-fluorophenyl, 3-fluorophenyl, 2-cyanophenyl, 3-cyanophenyl, 2,5-difluorophenyl and 2,6-difluorophenyl); and
(l) Q² is a group of the formula Id as hereinbefore defined wherein G¹ and G⁴ are hydrogen,
   X³ is CH₂ and Q⁴ is phenyl optionally substituted by fluoro or chloro for example 3-fluorophenyl;

A further embodiment of the invention is a quinazoline derivative of the Formula I wherein:
the **Q¹-Z-** group is 1-methylpiperidin-4-yl,
**R¹** is hydrogen;
**Q²** is a group of the formula Ib 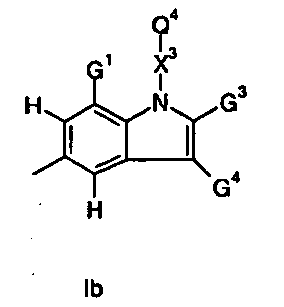 wherein G¹ and G³ are hydrogen,
G⁴ is selected from fluoro, chloro, bromo, cyano and methyl
X³ is selected from CH₂ and SO₂**,** and Q⁴ is phenyl or 2-pyridyl optionally bearing one substituent selected from fluoro, chloro and bromo;
or a pharmaceutically acceptable salt thereof.

Suitable values for Q² in this embodiment include, for example 1-benzenesulphonylindol-5-yl, 1-benzylindol-5-yl, 1-(3-fluorobenzyl)indol-5-yl and 1-(2-pyridylmethyl)indol-5-yl.

A further embodiment of the invention is a quinazoline derivative of the Formula I wherein:
the **Q¹-Z-** group is 1-methylpiperidin-4-yl,
**R¹** is hydrogen;
**Q²** is a group of the formula Id 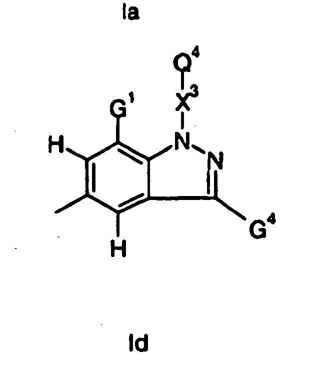 wherein G¹ is hydrogen,
**G⁴** is selected from fluoro, chloro, bromo, cyano and methyl
**X³** is selected from CH₂ and SO₂, and Q⁴ is selected from phenyl and 2-pyridyl optionally bearing one substituent selected from fluoro, chloro and bromo;
or a pharmaceutically acceptable salt thereof.

Suitable values for Q² in this embodiment include, for example 1-(2-pyridylmethyl)indazol-5-yl and 1-(3-fluorobenzyl)indazol-5-yl.

A further particular preferred compound of the invention is, for example, a quinazoline derivative of the Formula I selected from:
4-(3-Chloro-4-phenoxyanilino)-5-(1-methylpiperidin-4-yloxy)quinazoline;
4-(3-Chloro-4-((3-fluorobenzyloxy)anilino))-5-(1-methylpiperidin-4-yloxy)quinazoline;
4-(1-(3-Fluorobenzyl)indazol-5-ylamino)-5-(1-methylpiperidin-4-yloxy)quinazoline;
4-(3-Chloro-4-(decahydroquinolin-1-ylcarbonyl)anilino)-5-(1-methylpiperidin-4-yloxy)quinazoline;
4-(3-Chloro-4-(decahydroisoquinolin-2-ylcarbonyl)anilino)-5-(1-methylpiperidin-4-yloxy)quinazoline;
4-(3-Chloro-4-(3-methylpiperidin-1-ylcarbonyl)anilino-5-(1-methylpiperidin-4-yloxy)quinazoline;
4-(3-Ethynyl-4-(decahydroquinolin-1-ylcarbonyl)anilino)-5-(1-methylpiperidin-4-yloxy)quinazoline;
4-(3-Chloro-4-(5-methylisoxazol-3-ylmethoxy)anilino-5-(1-methylpiperidin-4-yloxy)quinazoline;
4-(3-Chloro-4-(2,6-difluorobenzyloxy)anilino)-5-(1-methylpiperidin-4-yloxy)quinazoline;
4-(3-Chloro-4-(2-fluorobenzyloxy)anilino)-5-(1-methylpiperidin-4-yloxy)quinazoline;
4-(3-Chloro-4-(2-cyanobenzyloxy)anilino-5-(1-methylpiperidin-4-yloxy)quinazoline; and
4-(3-Chloro-4-(thiazol-4-ylmethoxy)anilino)-5-(1-methylpiperidin-4-yloxy)quinazoline;
or a pharmaceutically acceptable acid addition salt thereof.

A further particular preferred compound of the invention is, for example, a quinazoline derivative of the Formula I selected from:
4-(1-Benzylindol-5-ylamino)-5-(1-methylpiperidin-4-yloxy)quinazoline;
4-(1-Benzenesulphonylindol-5-ylamino)-5-(1-methylpiperidin-4-yloxy)quinazoline;
4-(1-(2-Cyanobenzyl)indol-5-ylamino)-5-(1-methylpiperidin-4-yloxy)quinazoline;
4-(1-(2-Methoxybenzyl)indol-5-ylamino)-5-(1-methylpiperidin-4-yloxy)quinazoline;
4-(1-(2-Chlorobenzyl)indol-5-ylamino-5-(1-methylpiperidin-4-yloxy)quinazoline;
4-(1-(2,5-Dimethylbenzyl)indol-5-ylamino)-5(1-methylpiperidin-4-yloxy)quinazoline;
4-(1-(2 Fluorobenzyl)indol-5-ylamino)-5-(1-methylpiperidin-4-yloxy)quinazoline; and
4-(1-(3-Fluorobenzyl)indol-5-ylamino)-5-(1-methylpiperidin-4-yloxy)quinazoline
or a pharmaceutically acceptable acid addition salt thereof.

A further particular preferred compound of the invention is, for example, a quinazoline derivative of the Formula I selected from:
4-(3-Chloro-4-(3-fluorophenoxy)anilino-5-(1-methylpiperidin-4-yloxy)quinazoline; and
4-(3-Chloro-4-((1-methyl-1*H*-imidazol-2-yl)thio)anilino)-5-(1-methylpiperidin-4-yloxy)quinazoline;
or a pharmaceutically acceptable acid addition salt thereof.

A further particular preferred compound of the invention is, for example, a quinazoline derivative of the Formula I selected from:
4-(3-Chloro-4-(3-fluorobenzyloxy)anilino)-5-(tetrahydropyran-4-yloxy)quinazoline;
4-(4-Benzyloxy-3-methylanilino-5-(1-methylpiperidin-4-yloxy)quinazoline;
4-(3-Methyl-4-(5-methylisoxazol-3-ylmethoxy)anilino)-5-(1-methylpiperidin-4-yloxy)quinazoline;
4-(4-(3-Fluorobenzyloxy)-3-methylanilino)-5-(1-methylpiperidin-4-yloxy)quinazoline;
4-(3-Ethynyl 4-(3-fluorobenzyloxy)anilino)-5-(1-methylpiperidin-4-yloxy)quinazoline;
4-(3-Ethynyl 4-(2,6-difluorobenzyloxy)anilino)-5-(1-methylpiperidin-4-yloxy)quinazoline; and
4-(3-Chloro-4-(pyridin-2-ylmethoxy)anilino)-5-(1-methylpiperidin-4-yloxy)quinazoline;
or a pharmaceutically acceptable acid addition salt thereof.

A quinazoline derivative of the Formula I, or a pharmaceutically-acceptable salt thereof, may be prepared by any process known to be applicable to the preparation of chemically-related compounds. Such processes, when used to prepare a quinazoline derivative of the Formula I are provided as a further feature of the invention and are illustrated by the following representative process variants in which, unless otherwise stated, Q¹, Z, and Q² have any of the meanings defined hereinbefore. Necessary starting materials may be obtained by standard procedures of organic chemistry. The preparation of such starting materials is described in conjunction with the following representative process variants and within the accompanying Examples. Alternatively necessary starting materials are obtainable by analogous procedures to those illustrated which are within the ordinary skill of an organic chemist.
Process (a) The reaction, conveniently in the presence of a suitable base, of a quinazoline of the Formula II 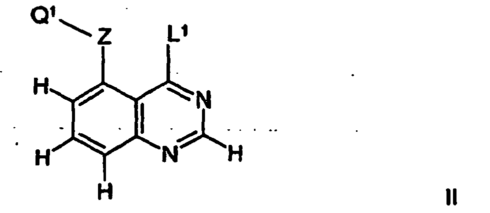 wherein L¹ is a displaceable group and Q¹ and Z have any of the meanings defined hereinbefore except that any functional group is protected if necessary, with an compound of the Formula

Q²NH₂

wherein Q² has any of the meanings defined hereinbefore except that any functional group is protected if necessary, whereafter any protecting group that is present is removed by conventional means.

A suitable base is, for example, an organic amine base such as, for example, pyridine, 2,6-lutidine, collidine, 4-dimethylaminopyridine, triethylamine, di-isopropylethylamine, N-methylmorpholine or diazabicyclo[5.4.0]undec-7-ene, or, for example, an alkali or alkaline earth metal carbonate, for example sodium carbonate, potassium carbonate, calcium carbonate, or, for example, an alkali metal hydride, for example sodium hydride.

A suitable displaceable group L¹ is, for example, a halogeno, alkoxy, aryloxy, mercapto, alkylthio, arylthio, alkylsulphinyl, arylsulphinyl, alkylsulphonyl, arylsulphonyl or sulphonyloxy group, for example a chloro, bromo, methoxy, phenoxy, pentafluorophenoxy, methylthio, methanesulphonyl, methanesulphonyloxy or toluene-4-sulphonyloxy group. The reaction is conveniently carried out in the presence of a suitable inert solvent or diluent, for example an alcohol or ester such as methanol, ethanol, isopropanol or ethyl acetate, a halogenated solvent such as ethylene chloride, chloroform or carbon tetrachloride, an ether such as tetrahydrofuran or 1,4-dioxan, an aromatic solvent such as toluene, or a dipolar aprotic solvent such as N,N-dimethylformamide, N,N-dimethylacetamide, N-methylpyrrolidin-2-one or dimethylsulphoxide. The reaction is conveniently carried out at a temperature in the range, for example, 10 to 250°C, conveniently in the range 40 to 80°C.

The quinazoline of the Formula II may also be reacted with a compound of the formula Q²NH₂ in the presence of a protic solvent such as isopropanol, conveniently in the presence of an acid, for example hydrogen chloride gas in diethyl ether or dioxane, or hydrochloric acid. Alternatively, this reaction may be conveniently carried out in an aprotic solvent, such as dioxane or a dipolar aprotic solvent such as N,N-dimethylacetamide in the presence of an acid, for example hydrogen chloride gas in diethyl ether or dioxane, or hydrochloric acid. The above reactions are conveniently carried out at a temperature in the range, for example, 0 to 150°C, preferably at or near the reflux temperature of the reaction solvent.

The quinazoline derivative of the Formula II, wherein L¹ is halogeno, may be reacted with a compound of the formula Q²NH₂ in the absence of an acid or a base. In this reaction dispalcement of the halogeno leaving group L¹ results in the formation of the acid HL¹ in-situ and the autocatalysis of the reaction. Conveniently the reaction is carried out in a suitable inert organic solvent, for example isopropanol, dioxane or N,N-dimethylacetamide. Suitable conditions for this reaction are as described above.

The quinazoline derivative of the Formula I may be obtained from this process in the form of the free base or alternatively it may be obtained in the form of a salt with the acid of the formula H-L¹ wherein L¹ has the meaning defined hereinbefore. When it is desired to obtain the free base from the salt, the salt may be treated with a suitable base, for example, an alkali or alkaline earth metal carbonate or hydroxide, for example sodium carbonate, potassium carbonate, calcium carbonate, sodium hydroxide or potassium hydroxide.

Protecting groups may in general be chosen from any of the groups described in the literature or known to the skilled chemist as appropriate for the protection of the group in question and may be introduced by conventional methods. Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower", as in, for example, lower alkyl, signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned are, of course, within the scope of the invention.

A carboxy protecting group may be the residue of an ester-forming aliphatic or arylaliphatic alcohol or of an ester-forming silanol (the said alcohol or silanol preferably containing 1-20 carbon atoms). Examples of carboxy protecting groups include straight or branched chain (1-12C)alkyl groups (for example isopropyl, and tert-butyl); lower alkoxy- lower alkyl groups (for example methoxymethyl, ethoxymethyl and isobutoxymethyl); lower acyloxy-lower alkyl groups, (for example acetoxymethyl, propionyloxymethyl, butyryloxymethyl and pivaloyloxymethyl); lower alkoxycarbonyloxy-lower alkyl groups (for example 1-methoxycarbonyloxyethyl and 1-ethoxycarbonyloxyethyl); aryl-lower alkyl groups (for example benzyl, 4-methoxybenzyl, 2-nitrobenzyl, 4-nitrobenzyl, benzhydryl and phthalidyl); tri(lower alkyl)silyl groups (for example trimethylsilyl and tert-butyldimethylsilyl); tri(lower alkyl)silyl-lower alkyl groups (for example trimethylsilylethyl); and (2-6C)alkenyl groups (for example allyl). Methods particularly appropriate for the removal of carboxyl protecting groups include for example acid-, base-, metal- or enzymically-catalysed cleavage.

Examples of hydroxy protecting groups include lower alkyl groups (for example tert-butyl), lower alkenyl groups (for example allyl); lower alkanoyl groups (for example acetyl); lower alkoxycarbonyl groups (for example tert-butoxycarbonyl); lower alkenyloxycarbonyl groups (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); tri(lower alkyl)silyl (for example trimethylsilyl and tert-butyldimethylsilyl) and aryl-lower alkyl (for example benzyl) groups.

Examples of amino protecting groups include formyl, aryl-lower alkyl groups (for example benzyl and substituted benzyl, 4-methoxybenzyl, 2-nitrobenzyl and 2,4-dimethoxybenzyl, and triphenylmethyl); di-4-anisylmethyl and furylmethyl groups; lower alkoxycarbonyl (for example tert-butoxycarbonyl); lower alkenyloxycarbonyl (for example allyloxycarbonyl); aryl-lower alkoxycarbonyl groups (for example benzyloxycarbonyl, 4-methoxybenzyloxycarbonyl, 2-nitrobenzyloxycarbonyl and 4-nitrobenzyloxycarbonyl); lower alkanoyloxyalkyl groups (for example pivaloyloxymethyl); trialkylsilyl (for example trimethylsilyl and tert-butyldimethylsilyl); alkylidene (for example methylidene) and benzylidene and substituted benzylidene groups.

Methods appropriate for removal of hydroxy and amino protecting groups include, for example, acid-, base-, metal- or enzymically-catalysed hydrolysis for groups such as 2-nitrobenzyloxycarbonyl, hydrogenation for groups such as benzyl and photolytically for groups such as 2-nitrobenzyloxycarbonyl. For example a tert butoxycarbonyl protecting group may be removed from an amino group by an acid catalysed hydrolysis using trifluoroacetic acid.

The reader is referred to Advanced Organic Chemistry, 4th Edition, by J. March, published by John Wiley & Sons 1992, for general guidance on reaction conditions and reagents and to Protective Groups in Organic Synthesis, 2nd Edition, by T. Green et al., also published by John Wiley & Son, for general guidance on protecting groups.

Quinazoline starting materials of the Formula II may be obtained by conventional procedures. For example, a 3,4-dihydroquinazolin-4-one of Formula III 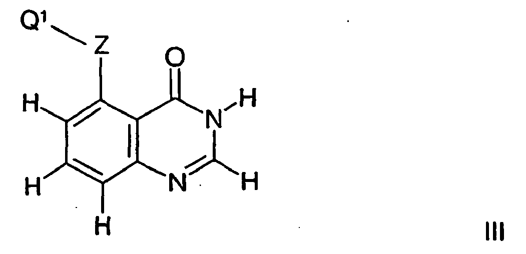 wherein Q¹ and Z have any of the meanings defined hereinbefore except that any functional group is protected if necessary, may be reacted with a halogenating agent such as thionyl chloride, phosphoryl chloride or a mixture of carbon tetrachloride and triphenylphosphine whereafter any protecting group that is present is removed by conventional means. The reaction is conveniently carried out in a suitable inert solvent, for example 1,2-dichloroethane or N,N-dimethylformamide conveniently in the presence of an base such as an organic base, for example di-isopropylethylamine. The reaction is conveniently carried out at a temperature in the range, for example, 0 to 150°C, preferably at or near the reflux temperature of the reaction solvent.

The 4-chloroquinazoline so obtained may be converted, if required, into a 4-pentafluorophenoxyquinazoline by reaction with pentafluorophenol in the presence of a suitable base such as potassium carbonate and in the presence of a suitable solvent such as N,N-dimethylformamide.

The compound of Formula I may be also be prepared using a telescoped process stating from the compound of Formula III, wherein the compound of the Formula Q²NH₂ is reacted with the compound of Formula II following halogenation of the compound of Formula III. The use of such a telescoped process avoids the need to isolate the compound of Formula II prior to reaction with the compound of formula Q²NH₂.

The 3,4-dihydroquinazolin-4-one of Formula III may be obtained using conventional procedures. For example when Z is an oxygen atom the compound of Formula III may be prepared as illustrated by Reaction Scheme 1 starting with the compound of Formula IV. 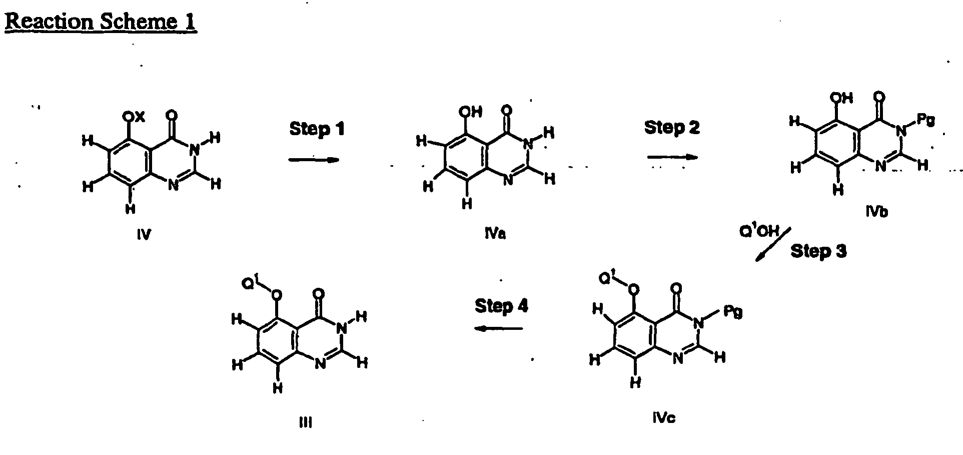 wherein Q¹ is as hereinbefore defined, X is (1-6C)alkyl (for example methyl) or benzyl, and Pg is a suitable amine protecting group.

### Notes:

### Step (1)

When X is (1-6C)alkyl, it may be may be cleaved from the compound of formula IV by conventional methods, such as by treatment of the compound of Formula IV with, for example:
(i) an alkali metal (1-6C)alkylsulphide such as sodium ethanethiolate;
(ii) an alkali metal diarylphosphide such as lithium diphenylphosphide;
(iii) a boron or aluminium trihalide such as boron tribromide;
(iv) magnesium bromide in pyridine; or
(v) pyridine hydrochloride in pyridine.
Generally the cleavage reaction is carried out at a temperature in the range of from, for example, 40 to 150°C.

When X is benzyl, it may be may be cleaved from the compound of formula IV by, for example, acid catalysed hydrolysis, for example by treatment of the compound of Formula IV with trifluoroacetic acid. Conveniently the reaction is carried out at a temperature in the range of 30 to 120°C, for example 70°C.

### Step (2)

The protecting group Pg is added to the 3,4-dihydro-5-hydroxyquinazolin-4-one of Formula IVa using conventional techniques. For example a suitable Pg is a pivaloyloxymethyl group that may be added to the compound of Formula IVa by reacting the compound of Formula IVa with chloromethylpivalate in the presence of a suitable base such as sodium hydride.

### Step 3

The Q¹O group may be introduced by coupling the compound of Formula IVb with a compound of the Formula Q¹OH in the presence of a suitable dehydrating agent. Suitable conditions for the coupling reaction are described below with reference to process (b).

### Step (4)

The protecting group Pg may be removed using conventional methods, for example when Pg is a pivaloyloxymethyl group it may be removed by treating the compound of Formula IVc with a methanolic ammonia solution.

The compound of formula IV may, for example, be prepared starting from an aniline of the Formula V as illustrated in Reaction Scheme 2. 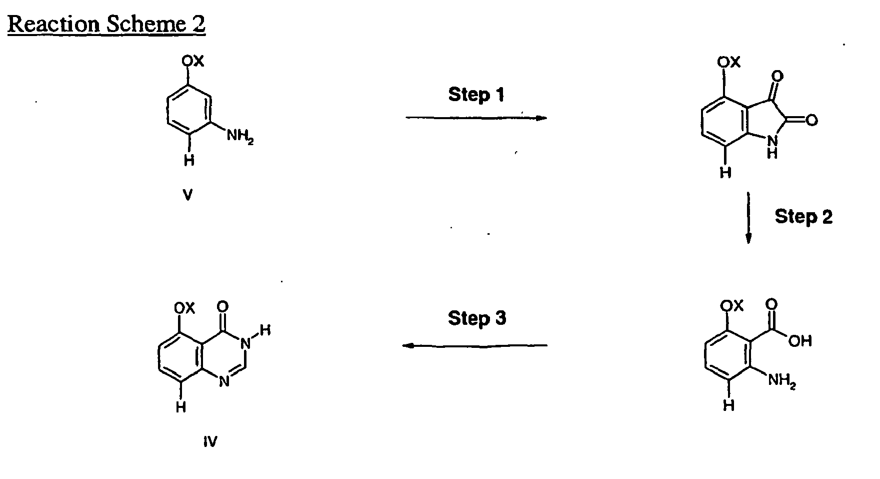 wherein X is as hereinbefore defined.

### Notes:

Steps 1 and 2 may be carried out using analogous conditions to the processes described in Organic Syntheses, Coll. Vol. 1, p 327-330; J. Org. Chem. 1969,34,3484-3489.

Step 3 may be carried out using analogous conditions to the process described in J. Org. Chem. 1952,17, 141-148; J. Med. Chem.1994, 37, 2106-2111.

Anilines of Formula V are commercially available compounds, or they are known in the literature, or can be prepared using well known processes in the art.

In an alternative process the compound of formula VIII may be obtained according to Reaction Scheme 1a: 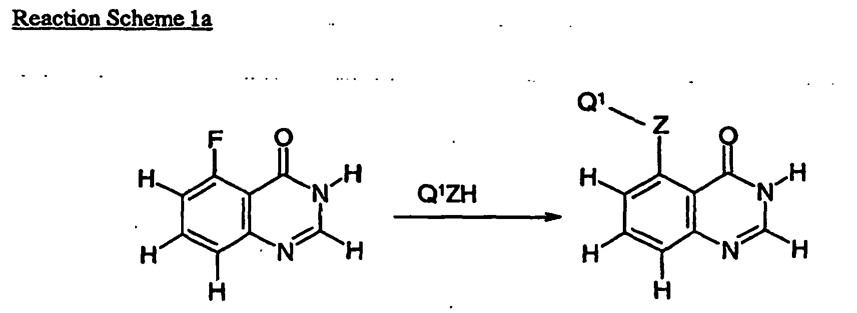 The reaction is conveniently performed in the presence of a suitable base. Suitable bases are as herein described under process (a), for example sodium hydride. The reaction is conveniently performed in a suitable inert solvent, for example N,N-dimethylacetamide. Reaction Scheme 1a is particularly suitable for the preparation of compounds of the formula III in which Z is O. The 5-fluoro-3,4-dihydraquinazoline starting material is commercially available or can be prepared using conventional methods, for example as described in J. Org. Chem. 1952, 17, 164-176.

Compounds of the Formula Q²NH₂ are commercially available compounds, or they are known in the literature, or can be prepared using conventional synthetic methods. For example when Q² is a group of the formula Ia the compound of the formula Q²NH₂ may be prepared in accordance with Reaction Scheme 3 or Reaction Scheme 4. 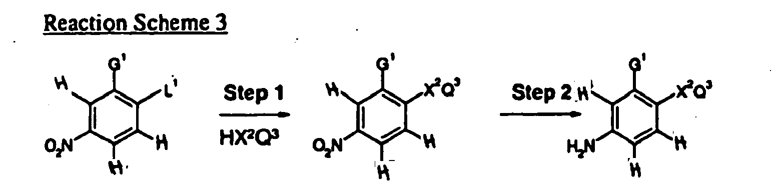 wherein X² is as hereinbefore defined, for example, NH, S, O or NHCH₂ and G¹, L¹ and Q³ are as hereinbefore defined, except any functional group is protected if necessary, and whereafter any protecting group that is present is removed by conventional means.

### Notes

Step 1. may be performed under analogous conditions to those used in process (a) described above. The compounds of the formula HX²Q³ are commercially available, or they are known in the literature, or can be prepared using well known processes in the art.

The reduction of the nitro group in step 2 may be carried out under standard conditions, for example by catalytic hydrogenation over a platinum/carbon, palladium/carbon or nickel catalyst, treatment with a metal such as iron, titanium chloride, tin II chloride or indium, or treatment with another suitable reducing agent such as sodium dithionite.

In an variation of process (a) the reduction of the nitro-compound in step 2 of reaction scheme 3 may be carried out as described above, followed directly with reaction with the compound of formula II in a telescoped process, thereby avoiding the need to isolate the compound of the formula Q²NH₂.

When Q² is a compound of the formula 1a in which X² is OCH₂ compounds of the formula Q²NH₂ may, for example, be prepared by reacting the starting nitro compound shown in Reaction Scheme 3 in which L¹ is OH with a compound of the formula Q³CH₂-halide (for example Q³CH₂-bromide). The nitro group may then be reduced to an amino group by using step 2 of the process in Reaction Scheme 3. Such compounds may also be prepared by reacting the starting nitro compound shown in Reaction Scheme 3 in which L¹ is halide with a compound of the formula Q³CH₂OH, followed by reduction of the nitro group as described above in Reaction Scheme 3. Compounds of the formula Q³CH₂OH are known or may be prepared using known methods, for example by reduction of the corresponding ester of the formula Q³COOR, wherein R is, for example (1-6C)alkyl, or benzyl, with a suitable reducing agent, for example sodium borohydride.

When Q² is a compound of the formula 1a in which X² is OCH₂ compounds of the formula Q²NH₂ may, for example, be prepared by coupling the starting nitro compound shown in Reaction Scheme 3 in which L¹ is OH with a compound of the formula Q³CH₂OH, conveniently in the presence of a suitable dehydrating agent. Suitable conditions for performing this reaction are analogous to those described below in relation to Process(b).

When Q² is a compound of the formula 1a in which X² is NHCH₂ compounds of the formula Q²NH₂ may, for example, be prepared according to Reaction Scheme 3a: 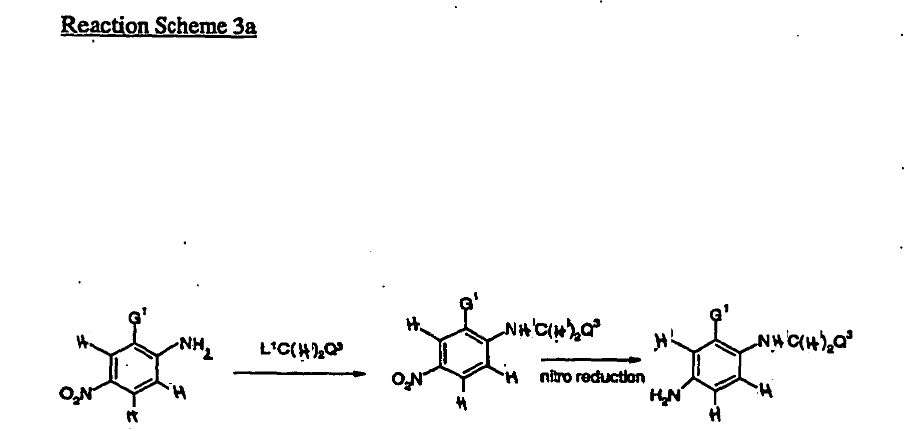 wherein G¹, L¹ and Q³ are as hereinbefore defined, except any functional group is protected if necessary, and whereafter any protecting group that is present is removed by conventional means. The first step of Reaction Scheme 3a may be performed under analogous conditions to those used in process (a) described above. The starting nitro compounds and the compounds of the formula Q³ NH₂ and Q³C(H)₂L¹ are commercially available, or they are known in the literature, or can be prepared using well known processes in the art. The reduction of the nitro group in step 2 may be carried out under analogous conditions to those described above for Reaction Scheme 3. 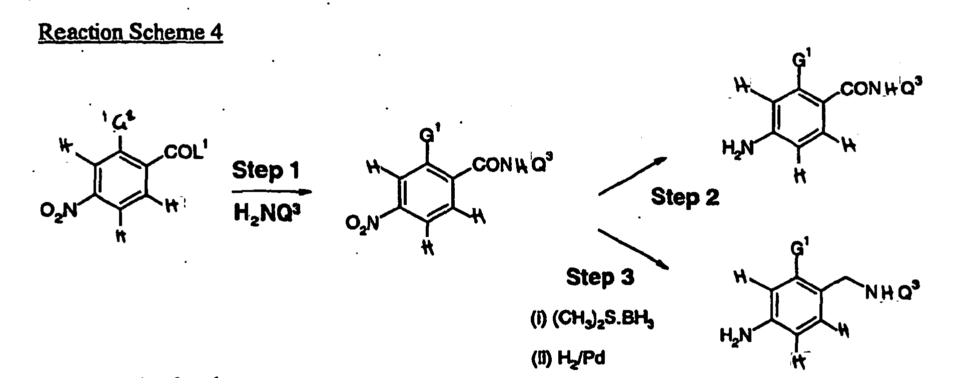 wherein G¹ and Q³ are as hereinbefore defined, except any functional group is protected if necessary, and whereafter any protecting group that is present is removed by conventional means, and L¹ is a suitable leaving group such as halide, for example chloro.

### Notes

### Suitable for the preparation of those compounds wherein X² is CO.

Step 1 may be carried out under analogous conditions to those used in process (a) described above.

The reduction of the nitro group in step 2 may be carried out as described above in Reaction Scheme 3.

When Q² is a compounds of the formula Ia in which X² is CO and Q³ is a nitrogen containing heterocyclyl group linked to X² by a nitrogen atom, the compound of the formula Q²NH₂ may be prepared by coupling the starting nitro compound shown in Reaction Scheme 3 in which L¹ is carboxy with a compound of the formula Q³H in the presence of a suitable coupling agent, for example O-(7-azabenzotriazol-1-yl)-N,N,N',N'-tetramethyluronium hexafluoro-phosphate (HATU). Suitable conditions for this reaction are analogous to those described in relation to process (j) below.

When Q² is a compound of the formula Ib and Id, the compound of the formula Q²NH₂ may be prepared, for example, by reacting an appropriate nitro-indole or nitro indazole with a compound of the formula Q⁴X³-halide, suitably in the presence of a base. The reaction is conveniently carried out in a suitable inert solvent, under analogous conditions to those described for Process (a). The nitro group on the resulting indole or indazole may then be reduced to an amino group using an analogous process to that described in step 2 in Reaction Scheme 3.
Process (b) For the production of those compounds of the Formula I wherein Z is an oxygen atom; the coupling, conveniently in the presence of a suitable dehydrating agent, of an alcohol of the Formula

Q¹-OH

wherein Q¹ has any of the meanings defined hereinbefore except that any functional group is protected if necessary with a quinazoline of the Formula VI 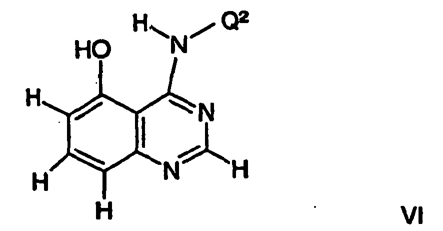 wherein Q² has any of the meanings defined hereinbefore except that any functional group is protected if necessary, whereafter any protecting group that is present is removed by conventional means.

A suitable dehydrating agent is, for example, a carbodiimide reagent such as dicyclohexylcarbodiimide or 1-(3-dimethylaminopropyl)-3-ethylcarbodiimide or a mixture of an azo compound such as diethyl or di-tert-butyl azodicarboxylate and a phosphine such as triphenylphosphine. The reaction is conveniently carried out in the presence of a suitable inert solvent or diluent, for example a halogenated solvent such as methylene chloride, chloroform or carbon tetrachloride and at a temperature in the range, for example, 0 to 150°C, preferably at or near ambient temperature.

The quinazoline of the Formula VI may be obtained by conventional procedures. For example, by cleavage of the group represented by X from the compound of the Formula VII 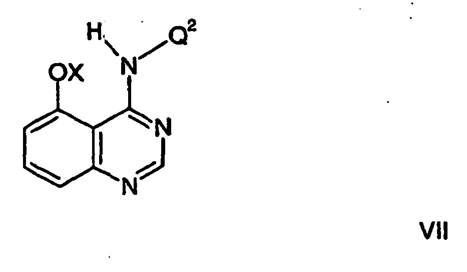 wherein X is as defined hereinbefore and Q² has any of the meanings defined hereinbefore except that any functional group is protected if necessary, whereafter any protecting group that is present is removed by conventional means.

The cleavage reaction is conveniently carried out as hereinbefore described in relation to step (1) in Reaction Scheme 1.

The compound of Formula VII may be prepared by for example reacting the compound of the Formula (IV) as hereinbefore defined, with a halogenating agent such as thionyl chloride, phosphoryl chloride or a mixture of carbon tetrachloride and triphenylphosphine. The resulting compound is then reacted with a compound of the Formula

Q²NH₂

wherein Q² has any of the meanings defined hereinbefore except that any functional group is protected if necessary, whereafter any protecting group that is present is removed by conventional means. The halogenation reaction may be performed under analogous conditions to those described above in relation to the reaction with the compound of the Formula III. The subsequent reaction with the compound of the Formula Q²NH₂ may be performed under analogous conditions to those described above in relation to the reaction with the compound of the Formula II.
Process(c) For the production of those compounds of the Formula I wherein Z is an oxygen atom, the reaction, conveniently in the presence of a suitable base, of an alcohol of the Formula

Q¹-OH

wherein Q¹ has any of the meanings defined hereinbefore except that any functional group is protected if necessary with a quinazoline of the Formula VIII 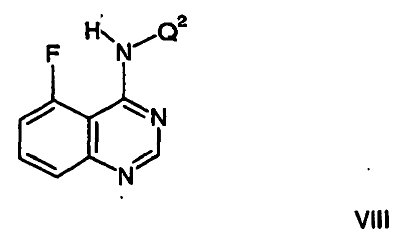 wherein Q² has any of the meanings defined hereinbefore except that any functional group is protected if necessary, whereafter any protecting group that is present is removed by conventional means.

A suitable base includes, for example a strong non-nucleophilic base such as an alkali metal hydride, for example sodium hydride, or an alkali metal amide, for example lithium di-isopropylamide (LDA).

The reaction is conveniently carried out in the presence of a suitable inert solvent or diluent, for example a halogenated solvent such as methylene chloride, chloroform or carbon tetrachloride, an ether such as tetrahydrofuran or 1,4-dioxan, an aromatic solvent such as toluene, or a dipolar aprotic solvent such as N,N-dimethylformamide, : N,N-dimethylacetamide, N,N-methylpyrrolidin-2-one or dimethylsulphoxide. The reaction is conveniently carried out at a temperature in the range, for example, 10 to 250°C, preferably in the range 40 to 150°C. Conveniently, this reaction may also be performed by heating the reactants in a sealed vessel using a suitable heating apparatus such as a microwave heater.

The quinazoline of the Formula VIII may be obtained by conventional procedures. For example, a quinazoline of the Formula IX 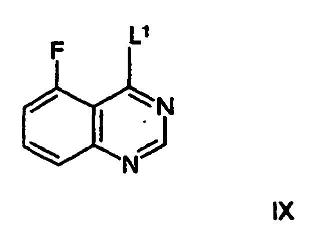 wherein L¹ is a displaceable group as defined hereinbefore (such as halogeno, for example chloro), may be reacted with a compound of the Formula

Q²NH₂

wherein Q² has any of the meanings defined hereinbefore except that any functional group is protected if necessary, whereafter any protecting group that is present is removed by conventional means. The reaction may be performed under analogous conditions to those described above under Process (a).

The quinazoline of Formula IX may be obtained using conventional methods, for example 5-fluoro-3,4-dihydroquinazolin-4-one may be reacted with a halogenating agent such as thionyl chloride, phosphoryl chloride or a mixture of carbon tetrachloride and triphenylphosphine whereafter any protecting group that is present is removed by conventional means, as described in relation to Process (a) above.
Process (d) For the production of those compounds of the Formula I wherein Q¹ or Q² contains a primary or secondary amino group, the cleavage of the corresponding compound of Formula I wherein Q¹ or Q² contains a protected primary or secondary amino group.

Suitable protecting groups for an amino group are, for example, any of the protecting groups disclosed hereinbefore for an amino group. Suitable methods for the cleavage of such amino protecting groups are also disclosed hereinbefore. In particular, a suitable protecting group is a lower alkoxycarbonyl group such as a tert-butoxycarbonyl group which may be cleaved under conventional reaction conditions such as under acid-catalysed hydrolysis, for example in the presence of trifluoroacetic acid. Process (e) For the production of those compounds of the Formula I wherein Q¹ or Q² contains a (1-6C)alkoxy or substituted (1-6C)alkoxy group or a (1-6C)alkylamino or substituted (1-6C)alkylamino group, the alkylation, conveniently in the presence of a suitable base as defined hereinbefore, of a quinazoline derivative of the formula I wherein Q¹ or Q² contains a hydroxy group or a primary or secondary amino group as appropriate.

A suitable alkylating agent is, for example, any agent known in the art for the alkylation of hydroxy to alkoxy or substituted alkoxy, or for the alkylation of amino to alkylamino or substituted alkylamino, for example an alkyl or substituted alkyl halide, for example a (1-6C)alkyl chloride, bromide or iodide or a-substituted (1-6C)alkyl chloride bromide or iodide, conveniently in the presence of a suitable base as defined hereinbefore, in a suitable inert solvent or diluent as defined hereinbefore and at a temperature in the range, for example, 10 to 140°C, conveniently at or near ambient temperature. An analogous procedure may be used to introduce optionally substituted (2-6C)alkenyloxy, (2-6C)alkenylamino, (2-6C)alkynyloxy or (2-6C)alkynylamino groups into Q¹ or Q². Examples of substituents that may be introduced to a compound of formula I using this process (e) include, for example the substitution of an NH group in a heterocyclyl represented by Q1-with an ethyl, allyl, 2-propynyl, 2-methoxyethyl or acetylmethyl, carbamoyl or methanesulphonyl group.
Process (f) For the production of those compounds of the Formula I wherein Q¹ contains an amino-hydroxy-disubstituted (1-6C)alkoxy group (such as 2-hydroxy-3-piperidinopropoxy, 2-hydroxy-3-methylaminopropoxy, 3-dimethylamino-2-hydroxypropoxy or 3-[N-(3-dimethylaminopropyl)-N-methylamino]-2-hydroxypropoxy), the reaction of a compound of the Formula I wherein Q¹ contains an epoxy-substituted (1-6C)alkoxy group with a heterocycle or an appropriate amine.

The reaction is conveniently carried out in the presence of a suitable inert diluent or carrier as defined hereinbefore and at a temperature in the range 10 to 150°C, preferably at or near ambient temperature.
Process (g) The reactions, conveniently in the presence of a suitable base as defined hereinbefore, of a quinazoline of the Formula X 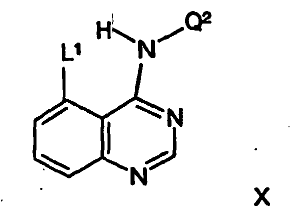 wherein L¹ is a displaceable group as defined hereinbefore and Q² has any of the meanings defined hereinbefore except that any functional group is protected if necessary, with a compound of the Formula

Q¹ZH

wherein Q¹ and Z have any of the meanings defined hereinbefore except that any functional group is protected if necessary, whereafter any protecting group that is present is removed by conventional means.

The reaction is conveniently carried out in the presence of a suitable base, such as an alkali metal hydride, for example sodium hydride.

The reaction is conveniently carried out in the presence of a suitable inert diluent or carrier as defined hereinbefore and at a temperature in the range 10 to 150°C, preferably at or near 125°C.

The compound of Formula X may be prepared using an analogous procedure to that described for the preparation of Formula VIII, except that the 5-fluoro atom is replaced by L¹.
Process (h) For the production of those compounds of the Formula I wherein Q¹ contains an amino-substituted (1-6C)alkoxy group (such as 3-piperidinopropoxy,
3-methylaminopropoxy or 3-dimethylaminopropoxy), the reaction of a compound of the Formula I wherein Q¹ contains a halogeno-substituted (1-6C)alkoxy group with an appropriate amine or a heterocycle containing a ring NH group.

The reaction is conveniently carried out in the presence of a suitable inert diluent or carrier as defined hereinbefore and at a temperature in the range 10 to 150°C, preferably at or near ambient temperature.
Process (i) For the production of those compounds of the Formula I wherein a heterocyclyl group in Q¹ or Q³ contains an S- or N-oxide the oxidation of a ring N or S atom in compound of the Formula (I). Suitable oxidizing agents include for example, a peracid (such as m-chlorbperbenzoic acid) or perphthalic acid. The reaction is conveniently carried out in a suitable inert solvent or diluent (such as dichloromethane) at a suitable temperature (such as-5 to 50°C).
Process (j) For the production of those compounds wherein Q² is a group of the formula 1a wherein
the group X²-Q³ is COQ³ and Q³ is a nitrogen linked heterocyclyl group, the coupling of the carboxy substituted quinazoline of the formula XI 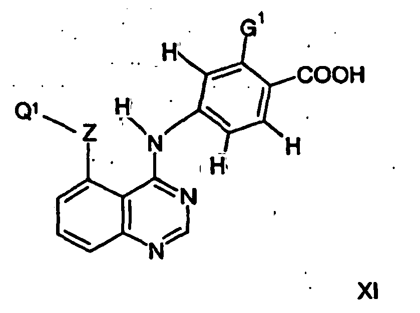 or a reactive derivative thereof, with Q³H (when Q³ is a nitrogen containing heterocyclyl group, for example hompiperidine) as appropriate, wherein Q¹, Q³, Z and G¹ are as hereinbefore defined except that any functional group is protected if necessary, whereafter any protecting group that is present is removed by conventional means. The coupling reaction is conveniently carried out in the presence of a suitable coupling agent, such as a carbodimide, or a suitable peptide coupling agent, for example O-(7-azabenzotdazol-1-yl)-N,N,N',N'-tetramethyluronium hexafluoro-phosphate. The coupling reaction is conveniently carried out in an inert solvent such as 1-methyl-2-pyrrolidinone, preferably in the presence of a suitable base, such as an organic amine, for example di-isopropylethylamine.

By 'reactive derivative' of a compound by the formula XI is meant a derivative of carboxylic acid of formula XI that will react with the amine to give the corresponding amide. Such reactive derivatives include, for example, an acid chloride of the compound of formula XI.

The compound of formula XI may be prepared using process (a) above by reacting a compound of the formula II with an appropriately protected 4-aminobenzoic acid (for example an alkyl or aryl 4-aminobenzoate), followed by removal of the carboxylic acid protecting group using a conventional deprotection technique for the removal of such groups (for example acid hydrolysis in HCl)
Process (k) For the production of those compounds wherein Q² is a group of the formula Ia wherein:
(i) X²Q³ is OCH₂Q³ and Q³ is aryl or heteroaryl, or
(ii) X²Q³ is OQ³ and Q³ is heteroaryl,
the reaction the reaction, optionally in the presence of a base, of a compound of formula I wherein Q² is a group of the formula Ia as defined hereinbefore except that the group X²Q³ in formula Ia is OH, with a compound of the formula L¹CH₂Q³ or L¹Q³ wherein L¹ is a displaceable group, and Q³ is as defined hereinbefore except that any functional group is protected if necessary, and whereafter any protecting group that is present is removed by conventional means. Suitable displaceable groups are, for example halogeno such as chloro, or alkanesulphonyloxy, such as mesyloxy. The reaction is conveniently carried out in an inert solvent such as or a dipolar aprotic solvent for example N,N-dimethylformamide, N,N-dimethylacetamide, N-methylpyrrolidin-2-one or dimethylsulphoxide, or acetonitrile. The reaction is conveniently carried out in the presence of a suitable base, as described in relation to process (a) above, such as an alkali metal carbonate, for example potassium carbonate. Generally the reaction is suitably performed at a temperature of from -10 to 120°C, conveniently at or near ambient temperature.

The starting material, the compound of formula I wherein Q² is a group of the formula Ia wherein X²Q³ is OH may be prepared using an analogous process to one of those described herein for the preparation of compounds of the Formula I. For example, Process (a) may be used wherein a compound of the Formula II as hereinbefore described is reacted with an appropriate 4-aminophenol. Compounds of the formula L¹CH₂Q³ or L¹Q³ are known or may be prepared using conventional procedures.
Process (l) For the production of those compounds of the formula I wherein Q¹ contains an (2-6C)alkanoylamino or substituted (2-6C)alkanoylamino, the acylation of a quinazoline derivative of the formula I wherein Q¹ contains an amino group. A suitable acylating agent is, for example, any agent known in the art for the acylation of amino to acylamino, for example an acyl halide, for example a (2-6C)alkanoyl chloride or bromide, conveniently in the presence of a suitable base, as defined hereinbefore, an alkanoic acid anhydride or mixed anhydride, for example a (2-6C)alkanoic acid anhydride such as acetic anhydride or the mixed anhydride formed by the reaction of an alkanoic acid and a (1-4C)alkoxycarbonyl halide, for example a (1-4C)alkoxycarbonyl chloride, in the presence of a suitable base as defined hereinbefore. In general the acylation is carried out in a suitable inert solvent or diluent as defined hereinbefore and at a temperature, in the range, for example, -30°C to 120°C, conveniently at or near ambient temperature.

An analogous process may be used to prepare compounds of the formula I containing an (1-6C)alkanesulphonylamino group or substituted alkanesulphonylamino group except the corresponding (1-6C)alkanesulphonylhalide or or substituted alkanesulphonylhalide (for example methanesulphonyl chloride) is used in place of the acylhalide.
Process (m): For the production of those compounds of the Formula I wherein Q² is an indole or indazole derivative of the formula Ib or Id as hereinbefore defined, the reaction, conveniently in the presence of a suitable base, of a compound of the formula Q⁴X³L¹, wherein L¹ is a displaceable group and Q⁴ and X³ have any of the meanings defined hereinbefore except that any functional group is protected if necessary, with a compound of the formula XII, 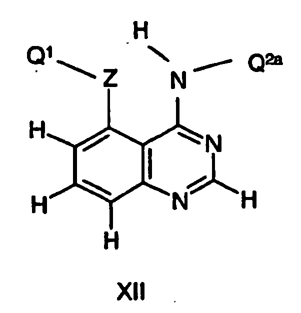 wherein Q¹ has any of the meanings defined hereinbefore except that any functional group is protected if necessary, and Q^{2a} is selected from a compound of the formula Ib' and Id' 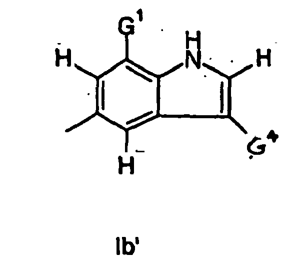 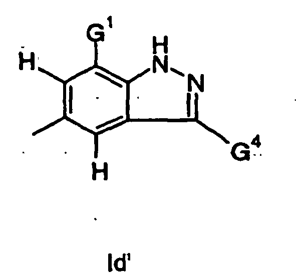 wherein G¹ and G⁴ have any of the meanings defined hereinbefore, except that any functional group is protected if necessary, whereafter any protecting group that is present is removed by conventional means.

Suitable displaceable groups represented by L¹ are as hereinbefore described herein in relation to process (a), such as halogeno, for example chloro.

The reaction is conveniently carried out in the presence of a base. Suitable bases are as hereinbefore described in relation to Process (a), for example sodium hydride.

The reaction is conveniently performed in a suitable inert solvent, for example or a dipolar aprotic solvent such as N,N-dimethylformamide, N,N-dimethylacetamide or N-methylpyrrolidin-2-one. The reaction is conveniently carried out at a temperature in the range, for example, 10 to 250°C, preferably in the range 20 to 80°C.

The starting material of the formula XII may be obtained using analogous processes to those described herein, for example using process (a) by reacting a quinazoline of the Formula II as hereinbefore described with the appropriate amino substituted indole or indazole derivative of the compounds of the formulae Ib' or Id'.

Compounds of the formula Q⁴X³L¹ are known or may be prepared using conventional procedures.
Process (n) For the production of those compounds of the Formula I wherein Q² is a group of the formula Ia wherein Q³ is aryl or heteroaryl and X² is S, the coupling of the compound of formula XVIII 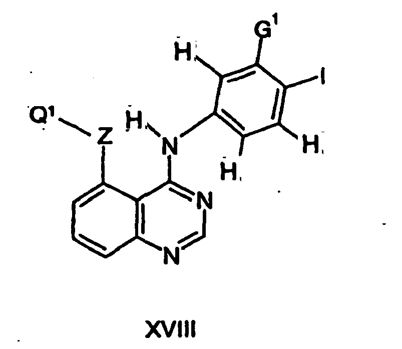 wherein Q¹, Z and G¹ have any of the meanings defined hereinbefore, except that any functional group is protected if necessary, with a compound of the formula Q³SH, wherein Q³ is aryl or heteroaryl as defined hereinbefore, except that any functional group is protected if necessary, in the presence of cuprous bromide, and a base, whereafter any protecting group that is present is removed by conventional means.

Suitable bases for the reaction include, for example organic bases such as diazabicyclo[5.4.0]undec-7-ene. The reaction is suitably performed under anhydrous conditions in an inert solvent, for example a hydrocarbon solvent such as toluene. The reaction is suitably performed at a temperature of from 80 to 150°C, for example from 90 to 130°C.

Compounds of the formula VIII may be prepared using analogous processes for the preparation of the compounds of the formula I described herein. For example a compound of formula XVIII may be prepared using an analogous process to Process (a) by reacting a compound of the formula II with an appropriate 4-iodoaniline.
Process (o) For the production of those compounds of the Formula I wherein Q¹ or Q² contains an (1-6C)alkylamino, substituted (1-6C)alkylamino group or a nitrogen linked heterocyclyl group (for example when Q¹ is 1-(4-methylpiperazin-1-yl)cyclohexan-4-yl), the reductive amination of an aldehyde or ketone group in a compound of formula 1, with a (1-6C)alkylamino, substituted (1-6C)alkylamino group or a heterocyclyl group containing an NH group in the presence of a suitable reducing agent. A suitable reducing agent is, for example, a hydride reducing agent, for example an alkali metal aluminium hydride such as lithium aluminium hydride, formic acid or, preferably, an alkali metal borohydride such as sodium borohydride, sodium cyanoborohydride, sodium triethylborohydride, sodium trimethoxyborohydride and sodium triacetoxyborohydride. The reaction is conveniently performed in a suitable inert solvent or diluent, for example tetrahydrofuran or dimethyl ether for the more powerful reducing agents such as lithium aluminium hydride, and, for example, ethylene chloride or a protic solvent such as methanol and ethanol for the less powerful reducing agents such as sodium triacetoxyborohydride and sodium cyanoborohydride. The reaction is conveniently performed at a temperature in the range, for example, 10 to 100°C, conveniently at or near ambient temperature.

An analogous reductive amination to that described above may be used to introduce an alkyl or substituted alkyl group onto a primary or secondary amine group in a compound of the formula I by reductive amination with a corresponding ketone in the presence of a suitable reducing agent. For example, for the production of those compounds of the Formula I wherein Q¹ or Q² contains a N-methyl group, the corresponding compound containing an NH group may be reacted with formaldehyde in the presence of a suitable reducing agent as described above.
Process (p) The conversion of one compound of the Formula I into another compound of the Formula L

It will be appreciated that certain of the various ring substituents in the compounds of the present invention may be introduced by standard aromatic substitution reactions or generated by conventional functional group modifications either prior to or immediately following the processes mentioned above (for example as in process (p)), and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halogeno group. Particular examples of modifications include the oxidation of alkylthio to alkylsulphinyl or alkylsulphonyl; the substitution of an NH group in Q¹ or Q² by the reaction with an optionally substituted alkyl halide, an optionally substituted alkenyl halide, an optionally substituted alykynyl halide or optionally substituted alkanoyl halide

When a pharmaceutically-acceptable salt of a quinazoline derivative of the Formula I is required, for example an acid-addition salt, it may be obtained by, for example, reaction of said quinazoline derivative with a suitable acid using a conventional procedure.

### Biological Assays

The inhibitory activities of compounds were assessed in non-cell based protein tyrosine kinase assays as well as in cell based proliferation assays before their *in vivo* activity was assessed in Xenograft studies.

### a) Protein Tyrosine Kinase phosphorylation Assays

This test measures the ability of a test compound to inhibit the phosphorylation of a tyrosine containing polypeptide substrate by an enzyme selected from the EGFR kinase, erbB2 kinase and erb4 kinase.

Recombinant intracellular fragments of EGFR, erbB2 and erbB4 (accession numbers X00588, X03363 and L07868 respectively) were cloned and expressed in the baculovirus/Sf21 system. Lysates were prepared from these cells by treatment with ice-cold lysis buffer (20mM N-2-hydroxyethylpiperizine-N'-2-ethanesulphonic acid (HEPES) pH7.5, 150mM NaCl, 10% glycerol, 1% Triton X-100, 1.5mM MgCl₂, 1mM ethylene glycol-bis(β-aminoethyl ether) N',N',N',N'-tetraacetic acid (EGTA), plus protease inhibitors and then cleared by centrifugation.

Constitutive kinase activity of these recombinant proteins was determined by their ability to phosphorylate a synthetic peptide (made up of a random co-polymer of Glutamic Acid, Alanine and Tyrosine in the ratio of 6:3:1). Specifically, Maxisorb^{™} 96-well immunoplates were coated with synthetic peptide (0.2µg of peptide in a 200µl phosphate buffered saline (PBS) solution and incubated at 4°C overnight). Plates were washed in 50mM HEPES pH 7.4 at room temperature to remove any excess unbound synthetic peptide. EGFR, erbB2 or erbB2 activities were assessed by incubation in peptide coated plates for 20 minutes at room temperature in 100mM HEPES pH 7.4 at room temperature, adenosine trisphosphate (ATP) at Km concentration for the respective enzyme, 10mM MnCl₂, 0.1mM Na₃VO₄ 0.2mM DL-dithiothreitol (DTT), 0.1% Triton X-100 with test compound in DMSO (final concentration of 2.5%). Reactions were terminated by the removal of the liquid components of the assay followed by washing of the plates with PBS-T (phosphate buffered saline with 0.5% Tween 20).

The immobilised phospho-peptide product of the reaction was detected by immunological methods. Firstly, plates were incubated for 90 minutes at room temperature with anti-phosphotyrosine primary antibodies that were raised in the mouse (4G10 from Upstate Biotechnology). Following extensive washing, plates were treated with Horseradish Peroxidase (HRP) conjugated sheep anti-mouse secondary antibody (NXA931 from Amersham) for 60 minutes at room temperature. After further washing, HRP activity in each well of the plate was measured colorimetrically using 22'-Azino-di-[3-ethylbenzthiazoline sulphonate (6)] diammonium salt crystals (ABTS^{™} from Roche) as a substrate.

Quantification of colour development and thus enzyme activity was achieved by the measurement of absorbance at 405nm on a Molecular Devices ThermoMax microplate reader. Kinase inhibition for a given compound was expressed as an IC₅₀ value. This was determined by calculation of the concentration of compound that was required to give 50% inhibition of phosphorylation in this assay. The range of phosphorylation was calculated from the positive (vehicle plus ATP) and negative (vehicle minus ATP) control values.

### b) KB cell proliferation assay

This assay measures the ability of a test compound to inhibit the proliferation of KB cells (human naso-pharangeal carcinoma obtained from the American Type Culture Collection (ATCC).

KB cells (human naso-pharangeal carcinoma obtained from the ATCC were cultured in Dulbecco's modified Eagle's medium (DMEM) containing 10% foetal calf serum, 2 mM glutamine and non-essential amino acids at 37°C in a 7.5% CO₂ air incubator. Cells were harvested from the stock flasks using Trypsin/ethylaminediaminetetraacetic acid (EDTA). Cell density was measured using a haemocytometer and viability was calculated using trypan blue solution before being seeded at a density of 1.25x10³ cells per well of a 96 well plate in DMEM containing 2.5% charcoal stripped serum, 1mM glutamine and non-essential amino acids at 37°C in 7.5% CO₂ and allowed to settle for 4 hours.

Following adhesion to the plate, the cells are treated with or without EGF (final concentration of 1ng/ml) and with or without compound at a range of concentrations in dimethylsulphoxide (DMSO) (1% final) before incubation for 4 days. Following the incubation period, cell numbers were determined by removal of the media by aspiration and incubating with 50µl of 3-(4,5-Dimethylthiazol-2-yl)-2,5-diphenyltetrazolium bromide (MTT) (stock 5mg/ml) for 2 hours. MTT solution was then removed by aspiration, allowed to air dry and the cells dissolved upon the addition of 100µl of DMSO.

Absorbance of this solubilised cells was read at 540nm to quantify cell biomass. Inhibition of proliferation was expressed as an IC₅₀ value. This was determined by calculation of the concentration of compound that was required to give 50% inhibition of proliferation. The range of proliferation was calculated from the positive (vehicle plus EGF) and negative (vehicle minus EGF) control values.

### c) H16N-2 cell proliferation assay

This assay measures the ability of a test compound to inhibit heregulin β or EGF driven proliferation of H16N-2 cells. These non-neoplastic eptihelial cells respond in a proliferative manner to stimulation with either EGF or heregulin β (Ram, G.R.and Ethier, S.P.(1996) Cell Growth and Differentiation, 7, 551-561) were isolated human mammary tissue (Band, V. and Sager, R. Tumour progression in breast cancer. In: J. S. Rhim and A. Dritschilo (eds.), Neoplastic Transformation in human Cell Culture, pp 169-178. Clifton, NJ: Humana Press, 1991) and were obtained from the Dana-Farber Cancer Institute, 44 Binney Street, Boston, Massachusetts 02115.

H16N-2 cells were routinely cultured in culture medium (a 1:1 mix of Gibco F12 and Ham's αMEM media containing 1 % foetal calf serum, 10mM HEPES, 1µg/ml Insulin, 12.5ng/ml EGF, 2.8µM Hydrocortisone, 2nM Estradiol 5µM Ascorbic Acid, 10µg/mll Transferrin, 0.1mM Phosphoethanolamine, 15nM Sodium Selenite, 2mM Glutamine, 10nM Tri-iodo-thrynoine, 35µg/ml Bovine pituitary Extract and 0.1mM Ethanolamine) at 37°C in a 7.5% CO₂ air incubator. Cells were harvested from the stock flasks using Trypsin/ethylaminediaminetetraacetic acid (EDTA). Cell density was measured using a haemocytometer and viability was calculated using trypan blue solution before being seeded at a density of 1.0x10³ cells per well of a 96 well plate in the above media at 37°C in 7.5% CO₂ and allowed to settle for 72 hours.

Following this, the cells were starved of serum for 24 hours upon the addition of starvation medium (a 1:1 mix of Gibco F12 and Ham's αMEM media containing, 10mM HEPES, 2nM Estradiol, 5µM Ascorbic Acid, 10µgg/ml Transferrin, 0.1mM Phosphoethanolamine, 15nM Sodium Selenite, 2mM Glutamine, and 0.1mM Ethanolamine) and incubated at 37°C in 7.5% CO₂. The cells were then treated with or without compound at a range of concentrations in dimethylsulphoxide (DMSO) (1% final) for two hours before the addition of exogenous ligand (at a final concentration of 100ng/ml of heregulin β or 5ng/ml of EGF) and incubation with both ligand and compound for 4 days at 37°C in 7.5% CO₂. Following the incubation period, cell numbers were determined by removal of the media by aspiration and incubating with 50µl of 3-(4,5-Dimethylthiazol-2-yl)-2, 5-diphenyltetrazolium bromide (MTT) (stock 5mg/ml) for 2 hours. MTT solution was then removed by aspiration, allowed to air dry and the cells dissolved upon the addition of 100µl of DMSO.

Absorbance of this solubilised cells was read at 540nm to quantify cell biomass. Inhibition of proliferation was expressed as an IC₅₀ value. This was determined by calculation of the concentration of compound that was required to give 50% inhibition of proliferation. The range of proliferation was calculated from the positive (vehicle plus ligand) and negative (vehicle minus ligand) control values.

### d) In vivo LoVo Xenograft assay

This assay measures the ability of a test compound to inhibit the growth of a LoVo tumour cell xenograft (colorectal adenocarcinoma obtained from the ATCC) in Female Swiss athymic mice (Alderley Park, *nu*/*nu* genotype).

Female Swiss athymic (*nu*/*nu* genotype) mice were bred and maintained in Alderley Park in negative pressure Isolators (PFI Systems Ltd.). Mice were housed in a barrier facility with 12hr light/dark cycles and provided with sterilised food and water *ad libitum.* All procedures were performed on mice of at least 8 weeks of age. LoVo tumour cell xenografts were established in the hind flank of donor mice by sub-cutaneous injections of 1x10⁷ freshly cultured cells in 100µl of serum free media per animal. On day 5 post-implant, mice were randomised into groups of 7 prior to the treatment with compound or vehicle control that was administered once daily at 0.1ml/kg body weight. Tumour volume was assessed twice weekly by bilateral Vernier calliper measurement, using the formula (length x width) x √(length x width) x (π/6), where length was the longest diameter across the tumour, and width was the corresponding perpendicular. Growth inhibition from start of treatment was calculated by comparison of the mean changes in tumour volume for the control and treated groups, and statistical significance between the two groups was evaluated using a Students *t* test.

### e) In vivo BT-474 Xenograft assay

This assay measures the ability of a test compound to inhibit the growth of a BT-474 tumour cell xenograft (human mammary carcinoma obtained from Dr Baselga, Laboratorio Recerca Oncologica, Paseo Vall D'Hebron 119-129, Barcelona 08035, Spain) in Female Swiss athymic mice (Alderley Park, *nu*/*nu* genotype) (Baselga, J. et al. (1998) Cancer Research, 58, 2825-2831).

Female Swiss athymic (*nu*/*nu* genotype) mice were bred and maintained in Alderley Park in negative pressure Isolators (PFI Systems Ltd.). Mice were housed in a barrier facility with 12hr light/dark cycles and provided with sterilised food and water *ad libitum.* All procedures were performed on mice of at least 8 weeks of age. BT-474 tumour cell xenografts were established in the hind flank of donor mice by sub-cutaneous injections of 1x10⁷ freshly cultured cells in 100µl of serum free media with 50% Matrigel per animal. On day 14 post-implant, mice were randomised into groups of 10 prior to the treatment with compound or vehicle control that was administered once daily at 0.1ml/kg body weight. Tumour volume was assessed twice weekly by bilateral Vernier calliper measurement, using the formula (length x width) x √(length x width) x (π/6), where length was the longest-diameter across the tumour, and width was the corresponding perpendicular. Growth inhibition from start of treatment was calculated by comparison of the mean changes in tumour volume for the control and treated groups, and statistical significance between the two groups was evaluated using a Students t test.

Although the pharmacological properties of the compounds of the Formula I vary with structural change as expected, in general activity possessed by compounds of the Formula I, may be demonstrated at the following concentrations or doses in one or more of the above tests (a), (b), (c), (d) and (e):-

| | |
|---|---|
| Test (a):- | IC₅₀ in the range, for example, 0.001 - 10 µM; |
| Test (b):- | IC₅₀ in the range, for example, 0.001 - 20 µM; |
| Test (c):- | IC₅₀ in the range, for example, 0.001 - 20 µM; |
| Test (d):- | activity in the range, for example, 1-200 mg/kg/day; |
| Test (e):- | activity in the range, for example, 1-200 mg/kg/day; |

No physiologically unacceptable toxicity was observed in Test (d) or (e) at the effective dose for compounds tested of the present invention. Accordingly no untoward toxicological effects are expected when a compound of Formula I, or a pharmaceutically-acceptable salt thereof, as defined hereinbefore is administered at the dosage ranges defined hereinafter.

According to a further aspect of the invention there is provided a pharmaceutical composition which comprises a quinazoline derivative of the Formula I, or a pharmaceutically-acceptable thereof, as defined hereinbefore in association with a pharmaceutically-acceptable diluent or carrier.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 0.5 g of active agent (more suitably from 0.5 to 100 mg, for example from 1 to 30 mg) compounded with an appropriate and convenient amount of excipients which may vary from about 5 to about 98 percent by weight of the total composition.

The size of the dose for therapeutic or prophylactic purposes of a compound of the Formula I will naturally vary according to the nature and severity of the conditions, the age and sex of the animal or patient and the route of administration, according to well known principles of medicine.

In using a compound of the Formula I for therapeutic or prophylactic purposes it will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of a compound of this invention.

We have found that the compounds of the present invention possess anti-proliferative properties such as anti-cancer properties that are believed to arise from their erbB family receptor tyrosine kinase inhibitory activity, particularly inhibition of the EGFR and/or erbB2 and/or erbB4 receptor tyrosine kinases, and especially the selective inhibition of erbB2 receptor tyrosine kinase. Accordingly the compounds of the present invention are expected to be useful in the treatment of disease or medical conditions mediated alone or in part by erbB receptor tyrosine kinases, i.e. the compounds may be used to produce a erbB receptor tyrosine kinase inhibitory effect in a warm-blooded animal in need of such treatment. Thus the compounds of the present invention may be useful in the treatment of malignant cells characterised by inhibition of one or more of the erbB family of receptor tyrosine kinases. Particularly the compounds of the invention may be used to produce an anti-proliferative and/or pro-agoptotic and/or anti-invasive effect mediated alone or in part by the inhibition of erbB receptor tyrosine kinases. Particularly, the compounds of the present invention are expected to be useful in the prevention or treatment of those tumours that are sensitive to inhibition of one or more of the erbB receptor tyrosine kinases, such as EGFR and/or erbB2 and/or erbB4 kinase that are involved in the signal transduction steps which drive proliferation and survival of these tumour cells. Accordingly the compounds of the present invention are expected to be useful in the treatment and/or prevention of a number of hyperproliferative disorders by providing an anti-proliferative effect. These disorders include, for example psoriasis, benign prostatic hyperplasia (BPH), atherosclerosis and restenosis and, in particular, erbB2 receptor tyrosine kinase driven tumours. Such benign or malignant tumours may affect any tissue and include non-solid tumours such as leukaemia, multiple myeloma or lymphoma, and also solid tumours, for example bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancers.

According to this aspect of the invention there is provided a compound of the formula I, or a pharmaceutically acceptable salt thereof, for use as a medicament.

Thus according to this aspect of the invention there is provided the use of a quinazoline derivative of the formula I, or a pharmaceutically-acceptable salt thereof, as defined hereinbefore in the manufacture of a medicament for use in the production of an anti-proliferative effect in a warm-blooded animal such as man.

A method for producing an anti-proliferative effect in a warm-blooded animal, such as man, in need of such treatement may comprise administering to said animal an effective amount of a quinazoline derivative of the formula I, or a pharmaceutically acceptable salt thereof, as hereinbefore defined.

According to a further aspect of the invention there is provided a compound of the formula I, or a pharmaceutically acceptable salt thereof, for use in the production of an anti-proliferative effect in a warm-blooded animal such as man.

According to a further aspect of the invention there is provided the use of a quinazoline derivative of the Formula I, or a pharmaceutically-acceptable salt thereof, as defined hereinbefore in the manufacture of a medicament for use in the prevention or treatment of those tumours which are sensitive to inhibition of erbB receptor tyrosine kinases, such as EGFR and/or erbB2 and/or erbB4, that are involved in the signal transduction steps which lead to the proliferation of tumour cells.

A method for the prevention or treatment of those tumours which are sensitive to inhibition of one or more of the erbB family of receptor tyrosine kinases, such as EGFR and/or erbB2 and/or erbB4, that are involved in the signal transduction steps which lead to the proliferation and/or survival of tumour cells in a warm-blooded animal, such as man, in need of such treatment, may comprise administering to said animal an effective amount of a quinazoline derivative of the Formula I, or a pharmaceutically-acceptable salt thereof, as defined hereinbefore.

According to a further aspect of the invention there is provided a compound of the formula I, or a pharmaceutically acceptable salt thereof, for use in the prevention or treatment of those tumours which are sensitive to inhibition of one or more of the erbB family of receptor tyrosine kinases, such as EGFR and/or erbB2 and/or erbB4, that are involved in the signal transduction steps which lead to the proliferation and/or survival of tumour cells.

According to a further aspect of the invention there is provided the use of a quinazoline derivative of the Formula I, or a pharmaceutically-acceptable salt thereof, as defined hereinbefore in the manufacture of a medicament for use in providing a EGFR and/or erbB2 and/or erbB4 kinase inhibitory effect.

A method for providing a EGFRand/or an erbB2 and/or an erbB4 kinases inhibitory effect in a warm-blooded animal, such as man, in need of such treatment may comprise administering to said animal an effective amount of a quinazoline derivative of the Formula I, or a pharmaceutically-acceptable salt thereof, as defined hereinbefore.

According to a further aspect of the invention there is provided a compound of the formula I, or a pharmaceutically acceptable salt thereof, for use in providing a EGFRand/or an erbB2 and/or an erbB4 kinase inhibitory effect.

According to a further aspect of the invention there is provided the use of a quinazoline derivative of the Formula I, or a pharmaceutically-acceptable salt thereof, as defined hereinbefore in the manufacture of a medicament for use in providing a selective erbB2 kinase inhibitory effect.

A method for providing a selective erbB2 kinase inhibitory effect in a warm-blooded animal, such as man, in need of such treatment, may comprise administering to said animal an effective amount of a quinazoline derivative of the Formula I, or a pharmaceutically-acceptable salt thereof, as defined hereinbefore.

According to a further aspect of the invention there is provided a compound of the formula I, or a pharmaceutically acceptable salt thereof, for use in providing a selective erbB2 kinase inhibitory effect.

By "a selective erbB2 kinase inhibitory effect" is meant that the quinazoline derivative of formula I is more potent against erbB2 receptor tyrosine kinase than it is against other kinase. In particular the quinazoline derivative of formula I is more potent against erbB2 receptor kinase than it is against EGFR tyrosine kinase. For example in a cellular assay (such as the H16N-2 assay described herein) the quinazoline derivative of formula I is at least 5 times, preferably at least 10 times more potent against erbB2 receptor tyrosine kinase driven proliferation than it is against EGFR tyrosine kinase driven proliferation, as determined from the relative IC₅₀ values

According to a further aspect of the present invention there is provided the use of a quinazoline derivative of the Formula I, or a pharmaceutically-acceptable salt thereof, as defined hereinbefore in the manufacture of a medicament for use in the treatment of a cancer selected from leukaemia, multiple myeloma, lymphoma, bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancer.

A method for treating a cancer selected from selected from leukaemia, multiple myeloma, lymphoma, bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancer in a warm-blooded animal, such as man, in need of such treatment, may comprise administering to said animal an effective amount of a quinazoline derivative of the Formula I, or a pharmaceutically-acceptable salt thereof, as defined hereinbefore.

According to a further aspect of the invention there is provided a compound of the formula I, or a pharmaceutically acceptable salt thereof, for use in the treatment of a cancer selected from leukaemia, multiple myeloma, lymphoma, bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancer.

The anti-proliferative treatment defined hereinbefore may be applied as a sole therapy or may involve, in addition to the quinazoline derivative of the invention, conventional surgery or radiotherapy or chemotherapy. Such chemotherapy may include one or more of the following categories of anti-tumour agents:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), antiandrogens (for example bicalutamide, flutamide, nilutamide and cyproterone acetate), LARH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5 α-reductase such as finasteride;
(iii) Agents which inhibit cancer cell invasion (for example metalloproteinase inhibitors like marimastat and inhibitors of urokinase plasminogen activator receptor function);
(iv) other inhibitors of growth factor function, for example such inhibitors include growth factor antibodies, growth factor receptor antibodies (for example the anti-erbb2 antibody trastuzumab [Herceptin^{™}] and the anti-erbb1 antibody cetuximab [C225], farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin^{™}], compounds such as those disclosed in International Patent Applications WO 97/22596, WO 97/30035, WO 97/32856 and WO 98/13354) and compounds that work by other mechanisms (for example linomide, inhibitors of integrin αvD3 function and angiostatin);
(vi) vascular damaging agents such as Combretastatin A4 and compounds disclosed in International Patent Applications WO 99/02166, WO00/40529, WO 00/41669, WO01/92224, WO02/04434 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

According to this aspect of the invention there is provided a pharmaceutical product comprising a quinazoline derivative of the formula I as defined hereinbefore and an additional anti-tumour agent as defined hereinbefore for the conjoint treatment of cancer.

Although the compounds of the Formula I are primarily of value as therapeutic agents for use in warm-blooded animals (including man), they are also useful whenever it is required to inhibit the effects of the erbB receptor tyrosine protein kinases. Thus, they are useful as pharmacological standards for use in the development of new biological tests for the evaluation of the effects of inhibitors of cell cycle activity in laboratory animals such as cats, dogs, rabbits, monkeys, rats and mice , and in the search for new pharmacological agents.

The invention will now be illustrated by the following non limiting examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulphate; evaporation of solvent was carried out using a rotary evaporator under reduced pressure (600-4000 Pascals; 4.5-30mmHg) with a bath temperature of up to 60°C;
(iii) chromatography means flash chromatography on silica gel; thin layer chromatography (TLC) was carried out on silica gel plates;
(iv) in general, the course of reactions was followed by TLC and / or analytical LC-MS, and reaction times are given for illustration only;
(v) final products had satisfactory proton nuclear magnetic resonance (NMR) spectra and/or mass spectral data;
(vi) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 300 MHz using perdeuterio dimethyl sulphoxide (DMSO-d₆) as solvent unless otherwise indicated; the following abbreviations have been used: s, singlet; d, doublet; t, triplet; q, quartet; m, multiplet; b, broad;
(viii) chemical symbols have their usual meanings; SI units and symbols are used;
(ix) solvent ratios are given in volume:volume (v/v) terms; and
(x) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺ which refers to the protonated mass ion; reference to M⁺ is to the mass ion generated by loss of an electron; and reference to M-H⁺ is to the mass ion generated by loss of a proton;
(xi) unless stated otherwise compounds containing an asymmetrically substituted carbon and/or sulphur atom have not been resolved;
(xii) where a synthesis is described as being analogous to that described in a previous example the amounts used are the millimolar ratio equivalents to those used in the previous example;
(xvi) the following abbreviations have been used:
   - THF: tetrahydrofuran;
   - DMF: *N,N*-dimethylformamide;
   - DMA: *N,N*-dimethylacetamide;
   - NMP: 1-methyl-2-pyrrolidinone;
   - DCM: dichloromethane;
   - DMSO: dimethylsulphoxide;
   - HATU: O-(7-Azabenzotriazol-1-yl)-N,N,N',N'-Tetramethyluronium Hexafluoro-Phosphate;
   - DCE: 1,2-dichloroethane;
   - *m*-CPBA: *Meta*-Chloroperbenzoic acid;
   - IPA: Isopropyl alcohol; and
   - ether: diethyl ether.
xvii) where a synthesis is described as leading to an acid addition salt (e.g. HCl salt), the stoichiometry of the salt was not determined. Unless otherwise stated, all NMR data is reported on free-base material, with isolated salts converted to the free-base form prior to characterisation by treating a solution of the salt in aqueous methanol with a base such as ammonium hydroxide or sodium bicarbonate thereby precipitating the free base, or by chromatography on silica using an eluant containing a base such as ammonia. Alternatively the free base may be obtained by an extraction method wherein the compound is partioned between an organic solvent and a basic aqueous medium. The free base is then isolated from the organic medium by, for example evaporation of the organic solvent.

The illustrative examples provided below are included for illustrative purposes only and do not form part of the present invention.

### Example 1

### 4-(1-Benzylindol-5-ylamino)-5-(1-methylpiperidin-4-yloxy)quinazoline hydrochloride

A solution of 4-chloro-5-(1-methylpiperidin-4-yloxy)quinazoline (0.15 g) **(reference example 2)** and 5-amino-1-benzylindole (0.12 g) in IPA (5 ml) **(reference example 7.1)** containing HCl in ether (1N, 0.54 ml) was heated at reflux for 1 hr. The solution was cooled and the resulting precipitate filtered to give the title compound as a yellow solid (0.115 g, 46%); NMR spectrum (DMSO-d6) 2.2 - 3.6 (m, 11H), 5.5 (s, 2H), 6.6 (s, 1H), 7.2 - 7.6 (m, 11H), 8.0 (m, 1H), 8.1 (m, 1H); Mass spectrum MH⁺ 464.

The procedure described above was repeated using the appropriate 4-chloroquinazoline and aniline. Thus were obtained the compounds described below:

### Example 1.1

### 4-(3-Chloro-4-phenoxyanilino)-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-phenoxyaniline **(reference example 8)** in 51% yield; NMR Spectrum (DMSO-d6) 1.90 (m, 2H), 2.17 (s, 3H), 2.20 (m, 4H), 2.65 (m, 2H), 4.78 (m, 1H), 6.94 (d, 2H), 7.10 (t, 1H), 7.19 (d, 1H), 7.24 (d, 1H), 7.36 (t, 3H), 7.59 (dd, 1H), 7.73 (t, 1H), 8.33 (d,1H); 8.56 (s, 1H), 10.19 (s, 1H); Mass Spectrum MH⁺ 461.

### Example 1.2

### 4-(3-Chloro-4-((3-fluorobenzyloxy)anilino))-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-(3-fluorobenzyloxy)aniline **(reference example 8.1)** in 70% yield; NMR Spectrum (DMSO-d6) 1.90 (m, 2H), 2.11 (m,2H), 2.17 (s, 3H), 2.26 (t, 2H), 2.61 (m, 2H), 4.77 (m, 1H), 5.23 (s, 2H), 7.11-7.34 (m, 6H), 7.39-7.53 (m, 2H), 7.69 (t, 1H), 8.11 (d, 1H), 8.49 (s, 1H), 10.03 (s, 1H); Mass Spectrum MH⁺ 493.

### Example 1.3

### 5-(1-Methylpiperidin-4-yloxy)-4-(4-phenoxyaniline)quinazoline hydrochloride

Obtained by reacting 4-chloro-5-(1-methylpiperidin-4-yloxy)quinazoline **(reference example 2)** with 4-phenoxyaniline in 41% yield; NMR spectrum (DMSO-d6) 2.2 - 2.5 (m, 4H), 2.7 (s, 3H), 3.1 (m, 2H), 3.5 (m, 2H), 5.1 (m, 2H), 7.1 (d, 2H), 7.2 (m, 3H), 7.4 (m, 2H), 7.6 (m, 2H), 7.7 (d, 1H), 7.8 (d, 1H), 8.0 (t, 1H), 8.8 (s, 1H); Mass spectrum MH⁺427.

### Example 1.4

### 5-(1-Methylpiperidin-4-yloxy)-4-(4-phenylthioaniline)quinazoline hydrochloride

Obtained by reacting 4-chloro-5-(1-methylpiperidin-4-yloxy)quinazoline **(reference example 2)** with 4-(phenylthio)aniline in 57% yield; NMR spectrum (DMSO-d6) 2.3 - 2.5 (m, 4H), 2.8 (s, 3H), 3.2 (m, 2H), 3.5 (m, 2H), 5.1 (m, 1H), 7.4 (m, 7H), 7.6 (m, 2H), 7.8 (d, 1H), 7.9 (d, 1H), 8.0 (t, 1H), 8.9 (s, 1H); Mass spectrum MH⁺ 443.

### Example 1.5

### 4-(1-Benzenesulphonylindol-5-ylamino)-5-(1-methylpiperidin-4-yloxy)quinazoline hydrochloride

Obtained by reacting 4-chloro-5-(1-methylpiperidin-4-yloxy)quinazoline **(reference example 2)** with 5-amino-1-benzenesulphonylindole **(reference example 7)** in 26% yield; NMR spectrum (DMSO-d6) 1.8 (m, 2H), 2.2 (s, 3H), 2.3 (m, 4H), 2.6 (m, 2H), 4.8 (m, 1H), 6.9 (d, 1H), 7.2 (d, 1H), 7.3 (d, 1H), 7.6 (m, 3H), 7.7 (m, 2H), 7.8 (d, 1H), 8.0 (m, 3H), 8.2 (s, 1H), 8.5 (s, 1H); Mass spectrum MH⁺ 514.

### Example 1.6

### 4-(3-Chloro-4-(3-pyridyloxy)anilino)-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-(pyridyl-3-oxy)aniline **(reference example 5)** in 17% yield; NMR spectrum (DMSO-d6, 373K) 2.2-2.5 (m, 2H), 2.5-2.8 (m, 2H), 2.8 (s, 3H), 2.8-3.6 (m, 4H), 5.0 (m, 1H), 7.2-7.3 (d, 1H), 7.3-7.35 (d, 1H), 7.35-7.5 (m, 3H), 7.7-7.75 (dd, 1H), 7.75-7.85 (t, 1H), 8.3 (d, 1H), 8.4 (d, 2H), 8.5 (s, 1H), 9.9 (bs, 1H), 10.3-10.5 (bs, 1H); Mass spectrum MH⁺ 462.

### Example 1.7

### 4-(3-Chloro4-(3-fluorophenoxy)anilino)-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-(3-fluorophenoxy)aniline **(reference example 5.1)** in 35% yield; NMR spectrum (DMSO-d6, 373K) 2.2 - 2.5 (m, 2H), 2.5 - 2.8 (m, 2H), 2.8 (s, 3H), 2.8 - 3.6 (m, 4H), 5.0 (m, 1H), 6.75 - 6.8 (dd, 1H), 6.8 - 6.85 (dt, 1H), 6.92 - 7.02 (td, 1H), 7.2 - 7.25 (d, 1H), 7.25 - 7.35 (d, 1H), 7.4 - 7.45 (m, 2H), 7.7 - 7.8 (dd, 1H), 7.8 - 7.85 (t, 1H), 8.3 (s, 1H), 8.6 (s, 1H), 9.9 - 10.1 (bs, 1H), 10.4 - 10.6 (bs, 1H); Mass spectrum MH⁺ 479.

### Example 1.8

### 3-Chloro-4-(2,3-difluorophenoxy)anillno)-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-(2,3-difluorophenoxy)aniline **(reference example 5.2)** in 67% yield; NMR spectrum (DMSO-d6, 373K) 2.3 - 2.4 (m, 2H), 2.5 - 2.6 (m, 2H),2.8 (s, 3H), 3.0 - 3.6 (m, 4H), 5.0 (m, 1H), 6.8 - 6.9 (m, 1H), 7.15 - 7.25 (m, 2H), 7.3 - 7.35 (d, 1H), 7.45 - 7.5 (d, 1H), 7.7 = 7.75 (dd, 1H). 7.75 - 7.85 (t, 1H), 8.32 - 8.35 (d, 1H), 8.6 (s, 1H), 10.0 (bs, 1H); Mass spectrum MH⁺ 497.

### Example 1.9

### 4-(3-Chloro-4-(2-pyrimidinyloxy)anilino)-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-(2-pyrimidinyloxy)aniline (ex Bionet Ltd) in 90% yield; NMR spectrum (DMSO-d6, 373K) 2.2 - 2.4 (m, 2H), 2.4 - 2.6 (m, 2H), 2.8 (s, 3H), 3.0 - 3.6 (m, 4H), 5.0 (m, 1H), 7.25 - 7.3 (t, 1H), 7.3 - 7.35 (d, 1H), 7.38 - 7.42 (d, 1H), 7.45 - 7.5 (d, 1H). 7.7 - 7.78 (dd,1H), 7.78 - 7.82 (t, 1H), 8.25 (d, 1H), 8.6 (s, 1H), 8.66 (s, 1H), 8.68 (s,1H,10.0 (bis, 1H); Mass spectrum MH⁺ 463.

### Illustrative Example 1.10

### 4-(3-Chloro-4-(2-thenoyl)anilino)-5-(1-methylpiperidin-4-yloxy)quinazoline hydrochloride

Obtained by reacting 4-chloro-5-(1-methylpiperidin4-yloxy)quinazoline **(reference example 2)** and 3-chloro-4-(2-thenoyl)aniline (prepared using the method of Example 15(6) of WO 96/15118) in 53% yield; NMR Spectrum (DMSO-d6) 2.2 - 2.6 (m, 4H), 2.8 (s, 3H), 3.0 - 3.6 (m, 4H), 5.0 - 5.1 (m, 1H), 7.25 (t, 1H), 7.3 - 7.4 (m, 1H), 7.45 (d, 1H), 7.55 (d, 1H), 7.6 (d, 1H), 7.75 - 7.85 (m, 2H), 8.2 (d, 1H), 8.4 (d, 1H), 8.65 (s, 1H); Mass Spectrum MH⁺ 477, 479.

### Example 1.11

### 4-(3-Chloro-4-((1-methyl-1H-imidazol-2yl-)methoxy)anilino-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-((1-methyl-1H-imidazol-2-yl)methoxy)aniline (prepared using the method of Example 15(24) of WO 96/15118) in 44% yield; NMR Spectrum (DMSO-d6) 2.2 - 2.6 (m, 4H), 2.8 (s, 3H), 3.1 - 3.6 (m, 4H), 3.75 (s, 3H), 4.9 - 5.0 (m, 1H), 5.25 (s, 2H), 6.9 (s, 1H), 7.2 (s, 1H), 7.25 (d, 1H), 7.35 (d, 1H), 7.4 (d, 1H), 7.55 (dd, 1H), 7.75 (t, 1H), 8.15 (d , 1H), 8.5 (s, 1H), 9.8 (bs, 1H); Mass Spectrum M-H⁺ 477.

### Example 1.12

### 4-(3-Chloro-4-(N-(2-pyridylmethyl)amino)anilino)-5-(1-methylpiperidin-4-yloxy)quinazoline hydrochloride

Obtained by reacting 4-chloro-5-(1-methylpiperidin-4-yloxy)quinazoline **(reference example 2)** and 3-Chloro-4-(N-2-pyridylmethyl)amino)aniline **(reference example 9.1)** in 46% yield; NMR Spectrum (DMSO-d6) 1.90 (m, 2H), 2.15 (m, 2H), 2.20 (s, 3H), 2.30 (m, 2H), 2.61 (m, 2H), 4.50 (d, 2H), 4.79 (tt, 1H), 6.23 (t, 1H), 6.63 (d, 1H), 7.19 (d, 1H), 7.23 - 7.35 (m, 3H), 7.36 (d, 1H), 7.68 (d, 1H), 7.77 (ddd, 1H), 7.96 (d, 1H), 8.44 (s, 1H), 8.57 (d, 1H), 9.90 (s, 1H); Mass Spectrum MH⁺ 475.

### Example 1.13

### 5-(1-Methylpiperidin-4-yloxy)-4-(3-methyl-4-[N-(2-pyridylmethyl)amino]anilino)quinazoline hydrochloride

Obtained by reacting 4-chloro-5-(1-methylpiperidin-4-yloxy)quinazoline **(reference example 2)** and 3-methyl-4-[N-(2-pyridylmethyl)amino]aniline **(reference example 5.3)** in 53% yield; NMR Spectrum (DMSO-d6) 2.27 (s, 3H), 2.30 (m, 2H), 2.50 (m, 2H), 2.75 (s, 3H), 3.15 (m, 2H), 3.55 (m, 2H), 4.52 (s, 2H), 5.10 (m, 1H), 6.43 (d, 1H), 7.27 (d, 1H), 7.32 (dd, 1H), 7.37-7.55 (m, 4H), 7.81 (dd, 1H), 7.91 (dd, 1H), 8.59 (d, 1H), 8.75 (s, 1H), 10.3 - 10.6 (d, 1H), 11.15 (bs, 1H); Mass Spectrum MH⁺ 455.

### Example 1.14

### 4-(3-Chloro-4-[N-methyl-N-(2-pyridyl)amino]anilino-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-[N-methyl-N-(2-pyridyl)amino]aniline **(reference example 5.4)** in 85% yield; NMR Spectrum (DMSO-d6) 2.25 (m, 2H), 2.40 (m, 2H), 2.79 (s, 3H), 3.20 (m, 2H), 3.40 (s, 3H), 3.57 (m, 2H), 5.00 (m, 1H), 6.24 (d, 1H), 6.66 (dd, 1H), 7.36 (d, 1H), 7.40 - 7.50 (m, 3H), 7.76 (d, 1H), 7.80 (dd, 1H), 8.15 (d, 1H), 8.38 (d, 1H), 8.64 (s, 1H), 9.85 - 10.25 (d, 1H), 10.60 - 10.90 (d, 1H); Mass Spectrum MH⁺ 475.

### Example 1.15

### 4-(3-Chloro-4-(2-pyridylamino)anilino)-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-(2-pyridylamino)aniline **(reference example 5.5)** in 89% yield; NMR Spectrum (DMSO-d6) 2.22 (m, 2H), 2.45 (m, 2H), 2.77 (s, 3H), 3.20 (m, 2H), 3.55 (m, 2H), 4.90-5.15 (m, 1H), 6.77 (dd, 1H), 6.97 (d, 1H), 7.37 (d, 1H), 7.41 (d, 1H), 7.53 (d, 1H), 7.60 (ddd, 1H), 7.80 (dd, 1H), 8.00 (d, 1H), 8.09 (d, 1H), 8.27 (d, 1H), 8.50 (s, 1H), 8.63 (s, 1H), 9.85 - 10.20 (d, 1H), 10.65 - 11.00 (d, 1H); Mass Spectrum MH⁺ 463.

### Example 1.16

### 5-(1-Methylpiperidin-4-yloxy)-4-(3-methyl-4-(2-pyridylamino)anilino)quinazoline hydrochloride

Obtained by reacting 4-chloro-5-(1-methylpiperidin-4-yloxy)quinazoline **(reference example 2)** and 3-methyl-4-(2-pyridylamino)aniline **(reference example 5.6)** in 86% yield; NMR Spectrum (DMSO-d6) 2.27 (m, 5H), 2.50 (m, 2H), 2.77 (s, 3H), 3.20 (m, 2H), 3.55 (m, 2H), 5.01 (m, 1H), 6.66 (dd, 1H), 6.72 (d, 1H), 7.31 (d, 1H), 7.38 (d, 1H), 7.52 (dd, 1H), 7.55 (d, 1H), 7.67 (d, 1H), 7.70 (s, 1H), 7.77 (dd, 1H), 8.03 (d, 1H), 8.26 (s, 1H), 8.54 (s,1H), 10.01 (s, 1H), 10.80 (s, 1H); Mass Spectrum MH⁺ 441.

### Example 1.17

### 4-(3-Methyl-4-[N-methyl-N-(2-pyridyl)amino]anilino-5-(1-methylpiperidin-4-yloxy)quinazoline hydrochloride

Obtained by reacting 4-chloro-5-(1-methylpiperidin-4-yloxy)quinazoline **(reference example 2)** and 3-methyl- 4-[N-methyl-N-(2-pyridyl)amino]aniline **(reference example 5.7)** in 73% yield; NMR Spectrum (DMSO-d6) 2.12 (s, 3H), 2.28 (m, 2H), 2.50 (m, 2H), 2.78 (s, 3H), 3.18 (m, 2H), 3.31 (s, 3H), 3.55 (m, 2H), 5.03 (m, 1H), 6.11 (d,1H), 6.61 (dd, 1H), 7.24 (d, 1H), 7.34 (d, 1H), 7.40 (dd, 1H), 7.41 (d, 1H), 7.79 (dd, 1H), 7.82 (s, 1H), 7.85 (d, 1H), 8.13 (d, 1H), 8.58 (s, 1H), 10.13 (bs, 1H), 10.82 (s, 1H); Mass Spectrum MH⁺ 455.6.

### Illustrative Example 1.18

### 4-(3-Chloro-4-((3-fluorophenylamino)methyl)anilino)-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-((3-fluorophenylamino)methyl)aniline **(reference example 10)** in 54% yield; NMR Spectrum (DMSO-d6) 1.93 (m, 2H), 2.15 (m, 2H), 2.19 (s, 3H), 2.27 (m, 2H), 2.66 (m, 2H), 4.33 (d, 2H), 4.80 (tt, 1H), 6.32 (dd, 1H), 6.36 (dd, 1H), 6.60 (t, 1H), 7.09 (ddd, 1H), 7.27 (d, 1H), 7.38 (d, 1H), 7.42 (d, 1H), 7.52 (dd, 1H), 7.75 (dd, 1H), 8.28 (d, 1H), 8.59 (s, 1H); Mass Spectrum MH⁺ 494.5.

### Example 1.19

### 4-(3-Chloro-4-(8-quinolylthio)anilino)-5-(1-methylpiperidin-4-yloxy)quinazoline hydrochloride

Obtained by reacting 4-chloro-5-(1-methylpiperidin-4-yloxy)quinazoline **(reference example 2)** and 3-chloro-4-(8-quinolylthio)aniline **(reference example 11)** in 61% yield; Mass spectrum MH⁺ 528.

### Example 2

### 4-(3-Chloro-4-((1-methyl-1H-imidazol-2-yl)thio)anilino)-5-(1-methylpiperidin-4-yloxy)quinazoline

To a solution of 4-chloro-5-(1-methylpiperidin-4-yloxy)quinazoline **(reference example 2)** (100 mg) and 3-chloro-4-((1-methyl-1*H*-imidazol-2-yl)thio)aniline (obtained as described in example 10 of WO 96/15118) (86 mg) in DMA (4 ml) was added 1.0 M HCl in diethyl ether (1.0 ml) and the resulting slurry heated at 80°C for 1 hour. The reaction mixture was then cooled to room temperature, diluted with acetone and the product filtered off. This solid was purified by column chromatography, eluting with DCM/methanol/880 NH₄OH (100/8/1), to give the title compound (116 mg, 67%); NMR spectrum (CDCl₃) 2.00 (m, 2H), 2.30 (m, 4H), 2.33 (s, 3H), 2.81 (m, 2H), 3.69 (s, 3H), 4.63 (m, 1H), 6.83 (d, 1H), 6.92 (d, 1H), 7.11 (d,1H), 7.22 (s, 1H), 7.40 (dd, 1H), 7.45 (d, 1H), 7.62 (t, 1H), 8.12 (d, 1H), 8.65 (s, 1H), 10.14 (s, 1H); Mass spectrum MH⁺ 481.

The procedure described above was repeated using the appropriate 4-chloroquinazoline and aniline. Thus were obtained the compounds described below:

### Example 2.1

### 4-(3-Chloro-4-(2-pyridyloxy)anilino)-5-(1-methylpiperidin-4-yloxy)quinazoline

Obtained by reacting 4-chloro-5-(1-methylpiperidin-4-yloxy)quinazoline **(reference example 2)** and 3-chloro-4-(2-pyridyloxy)aniline (obtained as described in example 15 (12) of WO 96/15118) in 65 % yield; NMR spectrum (DMSO-d6) 1.85 - 1.97 (m, 2H), 2.12 - 2.34 (m, 7H), 2.63 - 2.75 (m, 2H), 4.80 (m, 1H), 7.08 - 7.15 (m, 2H), 7.27 (d, 1H), 7.43 - 7.49 (m, 2H), 7.62 (dd, 1H), 7.75 (t, 1H), 7.85 - 7.90 (m, 1H), 7.99 - 8.12 (m, 1H), 8.28 (d, 1H), 8.58 (s, 1H), 10.22 (bs, 1H); Mass spectrum MH⁺ 462.

### Example 2.2

### 5-(1-Methylpiperidin-4-yloxy)-4-(3-methyl-4-(2-pyridylmethoxy)anilino)quinazoline

Obtained by reacting 4-chloro-5-(1-methylpiperidin-4-yloxy)quinazoline **(reference example 2)** and 3-methyl-4-(2-pyridylmethoxy)aniline (Example 13 of WO 96/15118) in 48% yield; NMR spectrum (DMSO-d6) 1.82 - 1.97 (m, 2H), 2.10 - 2.20 (m, 2H), 2.20 (s, 3H), 2.23 - 2.36 (m, 5H), 2.56 - 2.68 (m, 2H), 4.80 (m, 1H), 5.20 (s, 2H), 7.04 (s, 1H), 7.22 (d, 1H), 7.29 - 7.41 (m, 2H), 7.50 - 7.60 (m, 3H), 7.70 (t, 1H), 7.87 (t, 1H), 8.47 (s, 1H), 8.60 (m, 1H), 9.99 (s, 1H); Mass spectrum MH⁺ 456.

### Example 3

### 5-(1-tert-Butoxycarbonylpiperidin-4-yloxy)-4-(3-chloro-4-(3-fluorobenzyloxy)anilino)quinazoline

Di-*iso*-propylethylamine (0.234 g), 5-(1-*tert*-butyloxycarbonylpiperidin-4-yloxy)-4-chloroquinazoline (0.218 g) **(reference example 2.1)** and 3-chloro-4-(3-fluorobenzyloxy)aniline (0.162 g) **(reference example 8.1)** in DMA (10 ml) were heated at 90°C for 1.5 hours. The reaction was cooled, concentrated *in vacuo* and the residue purified by chromatography (using DCM to DCM-5% methanol as eluent) to give the title compound as a yellow solid (0.325 g, 94%); NMR spectrum (DMSO-d6) 1.40 (m, 9H), 1.79 (m, 2H), 2.14 - 2.22 (m, 2H), 3.15 (m, 2H), 3.84 (m, 2H), 4.94 (m, 1H), 5.26 (s, 2H), 7.17 (m, 1H), 7.22 - 7.37 (m, 5H), 7.43 - 7.54 (m, 2H), 7.73 (t, 1H), 8.12 (d, 1H), 8.51 (s, 1H), 9.96 (s, 1H); Mass spectrum MH⁺ 579.

The procedure described above was repeated using the appropriate 4-chloroquinazoline and aniline. Thus were obtained the compounds described below:

### Illustrative Example 3.1

### 4-(3-Chloro-4-(1,5-dimethylpyrazol-3-ylmethoxy)anilino)-5-(1-methylpiperidin-4-yloxy)quinazoline

Obtained by reacting 4-chloro-5-(1-methylpiperidin-4-yloxy)quinazoline **(reference example 2)** with 3-(2-chloro-4-aminophenoxymethyl)-1,5-dimethylpyrazole **(reference example 8.3)** in IPA in 3 1 % yield; NMR spectrum (DMSO-d6) 2.0 (m, 2H), 2.1 - 2.2 (m, 5H), 2.2 - 2.3 (m, 5H), 2.6 (m, 2H), 3.6 (s, 3H), 4.8 (m, 1H), 5.0 (s, 2H), 6.1 (s, 1H), 7.3 (d, 1H), 7.3 (m, 2H), 7.5 (dd, 1H), 7.7 (t, 1H), 8.1 (d, 1H), 8.5 (s, 1H), 10.0 (s, 1H); Mass spectrum MH⁺ 493.

### Illustrative Example 3.2

### 4-(3-Chloro-4-(1-methylpyrazol-3-ylmethoxy)anilino)-5-(1-methylpiperidin-4-yloxy)quinazoline

Obtained by reacting 4-chloro-5-(1-methylpiperidin-4-yloxy)quinazoline **(reference example 2)** with 3-(2-chloro-4-aminophenoxymethyl)-1-methylpyrazole **(reference example 8.4**) in IPA in 35% yield; NMR spectrum (DMSO-d6) 1.9 (m, 2H), 2.1 (m, 2H), 2.2 (s, 3H), 2.3 (m, 3H), 2.6 (m, 2H), 3.8 (s, 3H), 4.8 (m, 1H), 5.1 (s, 2H), 6.3 (d, 1H), 7.2 (d, 1H), 7.3 (m, 2H), 7.5 (m, 1H), 7.7 (m, 2H), 8.1 (d, 1H), 8.5 (s, 1H), 10.0 (bs, 1H); Mass spectrum NH⁺ 479.

### Example 3.3

### 4-(3-Chloro-4-((3-methylisoxazol-5-yl)methoxy)anilino)-5-(1-methylpiperidin-4-yloxy)quinazoline

Obtained by reacting 4-chloro-5-(1-methylpiperidin-4-yloxy)quinazoline **(reference example 2)** with 5-(2-chloro-4-aminophenoxymethyl)-3-methylisoxazole **(reference example 9.5)** in IPA in 63% yield; NMR spectrum (DMSO-d6) 1.9 (m, 2H), 2.1 (m, 2H), 2.2 (s, 3H), 2.2 (s, 3H), 2.26 (m, 2H), 2.6 (m, 2H), 3.8 (s, 3H), 4.8 (m, 1H), 5.3 (s, 2H), 6.5 (s, 1H), 7.2 (d, 1H), 7.3 (d, 2H), 7.5 (m, 1H), 7.7 (t, 1H), 8.1 (d, 1H), 8.5 (s, 1H), 10.0 (bs, 1H); Mass spectrum MH⁺ 480.

### Example 4

### 4-(4-(Azepan-1-ylcarbonyl)-3-chloroanilino)-5-(1-methylpiperidin-4-yloxy)quinazoline

Sodium hydride (60% dispersion in oil, 20 mg) was added to 4-hydroxy-1-methylpiperidine (46 mg) in DMA (0.5 ml) and the reaction stirred at 40°C for 30 minutes. 4-(4-(Azepan-1-ylcarbonyl)-3-chloroanilino)-5-fluoroquinazoline hydrochloride **(reference example 4)** (43.5 mg) was added and the reaction heated at 130°C for 3 hours. The reaction was quenched by addition of a few drops of water, then concentrated *in vacuo.* The residue was purified by chromatography (using DCM-2% methanolic ammonia to DCM-5% methanolic ammonia as eluent) to give the title compound as a white solid (66 mg, 67%); NMR spectrum (DMSO-d6) 1.55 (bs, 6H), 1.73 (bs, 2H), 1.88 - 1.97 (m, 2H), 2.14 - 2.22 (m, 5H), 2.26 (t, 2H), 2.64 - 2.74 (m, 2H), 3.23 (s, 2H), 3.58 (d, 2H), 4.80 (m, 1H), 7.27 (d, 1H), 7.37 (d, 1H), 7.39 (d, 1H), 7.64 (dd, 1H), 7.76 (t, 1H), 8.27 (d, 1H), 8.62 (s, 1H), 10.28 (s, 1H); Mass spectrum MH⁺ 494.

The procedure described above was repeated using the appropriate 5-fluoroquinazoline and alcohol. Thus was obtained the compound described below:

### Example 4.1

### 4-(1-(3-Fluorobenzyl)indazol-5-ylamino)-5-(1-methylpiperidin-4-yloxy)quinazoline

Obtained by reacting 4-(1-(3-fluorobenzyl)indazol-5-ylamino)-5-fluoroquinazoline hydrochloride **(reference example 4.1)** with 4-hydroxy-1-methylpiperidine in 41 % yield; NMR spectrum (DMSO-d6) 1.87 - 1.97 (m, 2H), 2.02 - 2.30 (m, 5H), 2.27 (m, 2H), 258 - 2.67 (m, 2H), 4.82 (m, 1H), 5.68 (s, 2H), 7.02 - 7.12 (m, 3H), 7.23 (d, 1H), 7.31 - 7.38 (m, 2H), 7.54 (dd, 1H), 7.68 - 7.77 (m, 2H), 8.15 (s, 1H), 8.37 (d, 1H), 8.48 (s, 1H), 10.19 (s, 1H); Mass spectrum MH⁺ 483.

### Example 4.2

### 4-(3-Chloro-4-(3-fluorobenzyloxy)anilino)-5-(tetrahydropyran-4-yloxy)quinazoline

Obtained by reacting 4-(3-chloro-4-(3-fluorobenzyloxy)anilino)-5-fluoroquinazoline **(reference example 33)** and tetrahydropyran-4-ol in 53% yield; NMR spectrum 1.80 - 1.95 (m, 2H), 2.20 (m, 2H), 3.55 (dt, 1H), 3.90 (dt, 1H), 4.95 (m, 1H), 5.24 (s, 2H), 7.16 (dt, 1H), 7.20 - 7.35 (m, 5H), 7.40 - 7.53 (m, 2H), 7.70 (t, 1H), 8.17 (d, 1H), 8.50 (s, 1H), 10.03 (bs, 1H); Mass Spectrum M⁺ 480.

### Example 4.3

### 4-(3-Chloro-4-(3-fluorobenzyloxy)anilino)-5-(1-methylpyrrolidin-3-yloxy)quinazoline

Obtained from 4-(3-chloro-4-(3-fluorobenzyloxy)anilino)-5-fluoroquinazoline **(reference example 33)** and 3-hydroxy-1-methylpyrrolidine in 50% yield; NMR spectrum 1.92 (m, 1H), 2.22 (m, 1H), 2.38 (m + s, 4H), 2.50 (m, 1H), 3.00 (dt, 1H), 3.17 (d, 1H), 5.23 (s, 2H), 5.32 (m, 1H), 7.15 (m, 2H), 7.23 - 7.37 (m, 4H), 7.43 (m, 1H), 7.70 (t, 1H), 7.77 (dd, 1H), 8.08 (d, 1H), 8.52 (s, 1H), 10.40 (bs, 1H); Mass Spectrum M⁺ 479.

### Example 4.4

### 4-(3-Chloro-4-(3-fluorobenzyloxy)anilino)-5-(tetrahydrofuran-3-yloxy)quinazoline

Obtained from 4-(3-chloro-4-(3-fluorobenzyloxy)anilino)-5-fluoroquinazoline **(reference example 33)** and 3-hydroxytetrahydrofuran in 64% yield; Mass Spectrum M⁺ 466.

### Example 5

### 4-(4-(2-Bromobenzyloxy)-3-chloroanilino)-5-(1-methylpiperidin-4-yloxy)quinazoline

Potassium carbonate (290 mg) was added to a mixture of 2-bromobenzylchloride (128 mg) and 4-(3-chloro-4-hydroxyanilino)-5-(1-methylpiperidin-4-yloxy)quinazoline hydrochloride **(reference example 4.2)** in DMF (15 ml). The mixture was stirred at room temperature overnight. The reaction mixture was then concentrated *in vacuo* and the residue purified by chromatography using 0-4% methanolic ammonia in DCM as eluent to afford the title compound as a pink solid (90 mg, 31%); NMR Spectrum (DMSO-d6) 1.90 (m, 2H), 2.11 (m, 2H), 2.17 (s, 3H), 2.26 (m, 2H), 2.61 (m, 2H), 4.78 (m, 1H), 5.21 (s, 2H), 7.20 - 7.34 (m, 4H), 7.42 7.54 (m, 2H), 7.61 - 7.73 (m, 3H), 8.13 (d, 1H), 8.50 (s, 1H), 10.05 (bs, 1H); Mass spectrum MH⁺ 555.

The procedure described above was repeated using the appropriate 4-hydroxyanilinoquinazoline and alkyl halide or mesylate. Thus were obtained the compounds described below:

### Example 5.1

### 4-(3-Chloro-4-(1,2,5-thiadiazol-3-ylmethoxy)anilino)-5-(1-methylpiperidin-4-yloxy)quinazoline

Obtained by reacting 4-(3-chloro-4-hydroxyanilino)-5-(1-methylpiperidin-4-yloxy)quinazoline hydrochloride **(reference example 4.2)** and 3-bromomethyl-1,7,5-thiadiazole (obtained as described in J. Heterocycl. Chem. 1984, 21, 1157-60); NMR Spectrum (DMSO-d6) 1.8 - 2.0 (m, 2H), 2.05 - 2.4 (m, 7H), 2.5 - 2.7 (m, 2H), 4.7 - 4.8 (m, 1H), 5.55 (s, 2H), 7.2 (d, 1H), 7.35 (d, 2H), 7.5 (d, 1H), 7.7 (t, 1H), 8.15 (s, 1H), 8.5 (s, 1H), 8.95 (s, 1H); Mass Spectrum MH⁺ 483.

### Example 5.2

### 4-(4-Benzyloxy-3-fluoroanilino)-5-(1-methylpiperidin-4-yloxy)quinazoline

Obtained by reacting 4-(3-fluoro-4-hydroxyanilino)-5-(1-methylpiperidin-4-yloxy)quinazoline hydrochloride **(reference example 4.3)** and benzyl chloride in 22% yield; Mass Spectrum MH⁺ 459.

### Example 5.3

### 4-(3-Fluoro-4-(2-fluorobenzyloxy)anilino)-5-(1-methylpiperidin-4-yloxy)quinazoline

Obtained by reacting 4-(3-fluoro-4-hydroxyanilino)-5-(1-methylpiperidin-4-yloxy)quinazoline hydrochloride **(reference example 4.3)** and 2-fluorobenzyl chloride in 37% yield; Mass Spectrum MH⁺ 477.

### Example 5.4

### 4-(4-(2,6-Difluorobenzyloxy)-3-fluoroanilino)-5-(1-methylpiperidin-4-yloxy)quinazoline

Obtained by reacting 4-(3-fluoro-4-hydroxyanilino)-5-(1-methylpiperidin-4-yloxy)quinazoline hydrochloride **(reference example 4.3)** and 2,6-difluorobenzyl chloride in 27% yield; Mass Spectrum M-H⁺ 493.

### Example 5.5

### 4-(4-(2-Cyanobenzyloxy)-3-fluoroanilino)-5-(1-methylpiperidin-4-yloxy)quinazoline

Obtained by reacting 4-(3-fluoro-4-hydroxyanilino)-5-(1-methylpiperidin-4-yloxy)quinazoline hydrochloride **(reference example 4.3)** and 2-cyanobenzyl chloride in 12% yield; Mass Spectrum MH⁺ 484.

### Example 5.6

### 4-(3-Fluoro-4-(2-pyridylmethoxylanilino)-5-(1-methylpiperidin-4-yloxy)quinazoline

Obtained by reacting 4-(3-fluoro-4-hydroxyanilino)-5-(1-methylpiperidin-4-yloxy)quinazoline hydrochloride **(reference example 4.3)** and 2-picolyl chloride hydrochloride in 11% yield; Mass Spectrum MH⁺ 460.

### Example 5.7

### 4-(3-Fluoro-4-(5-methylisoxazol-3-ylmethoxy)anilino)-5-(1-methylpiperidin-4-yloxy)quinazoline

Obtained by reacting 4-(3-fluoro-4-hydroxyanilino)-5-(1-methylpiperidin-4-yloxy)quinazoline hydrochloride **(reference example 4.3)** and 5-methyl-3-chloromethylisoxazole in 38% yield; Mass Spectrum MH⁺ 464.

### Example 5.8

### 4-(3-Chloro-4-(3,4-difluorobenzyloxy)anilino)-5-(1-methylpiperidin-4-yloxy)quinazoline

Obtained by reacting 4-(3-chloro-4-hydroxyanilino)-5-(1-methylpiperidin-4-yloxy)quinazoline hydrochloride **(reference example 4.2)** and 3,4-difluorobenzyl chloride in 53% yield; Mass Spectrum MH⁺ 511.

### Example 5.10

### 4-(3-Chloro-4-(isoxazol-3-ylmethoxy)anilino)-5-(1-methylpiperidin-4-yloxy)quinazoline

Obtained by reacting 4-(3-chloro-4-hydroxyanilino)-5-(1-methylpiperidin-4-yloxy)quinazoline hydrochloride **(reference example 4.2)** and 3-chloromethylisoxazole **(reference example 31)** in 71% yield; Mass Spectrum MH⁺ 466.

### Example 5.11

### 4-(3-Chloro-4-(5-methylisoxazol-3-ylmethoxy)anilino)-5-(tetrahydropyran-4-yloxy)quinazoline

Obtained by reacting 4-(3-chloro-4-hydroxyanilino)-5-(tetrahydropyran-4-yloxy)quinazoline **(reference example 42)** and 3-chloromethyl-5-methylisoxazole in 44% yield; NMR Spectrum (DMSO-d6) 1.8- 1.94 (m, 2H), 2.14 -2.25 (m, 2H), 2.41 (s, 3H), 3.54 (dt, 2H), 3.91 (dt, 2H), 4.91- 5.01 (m, 1H), 5.25 (s, 2H), 6.34 (s, 1H), 7.23 - 7.26 (m, 3H), 7.51 (dd, 1H), 7.72 (t, 1H), 8.16 (d, 1H), 8.51 (s, 1H), 10.04 (s, 1H); Mass spectrum MH⁺ 467.

### Illustrative Example 5.12

### 4-(3-Chloro-4-(2-pyrazinylmethoxy)anilino)-5-(tetrahydropyran-4-yloxy)quinazoline

Obtained by reacting 4-(3-chloro-4-hydroxyanilino)-5-(tetrahydropyran-4-yloxy)quinazoline **(reference example 42)** and 2-chloromethylpyrazine (obtained as described in Synthesis, 1984, 676) in 4% yield; NMR Spectrum (DMSO-d6) 1.80 -1.93 (m, 2H), 2.14 - 2.27 (m, 2H), 3.54 (t, 2H), 3.85 - 3.95 (m, 2H), 4.90 - 5.01 (m, 1H), 5.37 (s, 2H), 7.24 - 7.36 (m, 4H), 7.52 (dd, 1H), 7.72 (t, 1H), 8.18 (d ,1H), 8.51 (s, 1H), 8.62 - 8.69 (m, 1H), 8.84 (s, 1H), 10.05 (s, 1H); Mass spectrum MH⁺ 464.

### Example 5.13

### 4-(3-Chloro-4-(5-methylisoxazol-3-yl)anilino)-5-(tetrahydrofuran-3-yloxy)quinazoline

Obtained by reacting 4-(3-chloro-4-hydroxyanilino)-5-(tetrahydrofuran-3-yloxy)quinazoline **(reference example 42.1)** and 3-chloromethyl-5-methylisoxazole in 85% yield; NMR Spectrum (DMSO-d6) 2.12 - 2.23 (m, 1H), 2.28 - 2.41 (m, 1H), 2.41 (s, 3H), 3.79 - 3.99 (m, 3H), 4.19 (d, 1H), 5.24 (s, 2H), 5.46 (t, 1H), 6.33 (s, 1H), 7.19 (d, 1H), 7.29 (d, 1H), 7.35 (d, 1H), 7.57 (dd, 1H), 7.74 (t, 1H), 8.19 (d, 1H), 8.53 (s ,1H), 10.05 (s, 1H); Mass spectrum MH⁺ 453.

### Illustrative Example 5.14

### 4-(3-Chloro-4-(2-morpholinothiazol-4-ylmethoxy)anilino)-5-(1-methylpiperidin-4-yloxy)quinazoline

Obtained from 4-(4-chloromethylthiazol-2-yl)morpholine (obtained as described in Example 9 of US Patent US 5,455,351) and 4-(3-chloro-4-hydroxyanilino)-5-(1-methylpiperidin-4-yloxy)quinazoline **(reference example 4.2)** in 41 % yield; NMR spectrum (DMSO-d6) 1.85 -1.97 (m, 2H), 2.10 - 2.20 (m, 2H), 2.18 (s, 3H), 2.23 - 2.34 (m, 2H), 2.58 - 2.68 (m, 2H), 3.38 (m, 4H), 3.71 (m, 4H), 4.80 (m, 1H), 5.05 (s, 2H), 6.91 (s, 1H), 7.23 (d, 1H), 7.32 (d, 1H), 7.34 (d, 1H), 7.52 (dd, 1H), 7.73 (dd, 1H), 8.11 (d, 1H), 8.52 (s, 1H), 10.06 (s, 1H); Mass spectrum MH⁺ 567.

### Example 5.15

### 4-(4-Benzyloxy-3-methylanilino)-5-(1-methylpiperidin-4-yloxy)quinazoline

Obtained from benzyl chloride and 4-(3-methyl-4-hydroxyanilino)-5-(1-methylpiperidin-4-yloxy)quinazoline **(reference example 38)** in 48% yield; NMR spectrum (CDCl₃) 2.0 (m, 2H), 2.2 - 2.4 (m, 10H), 2.8 (m, 2H), 4.6 (m, 1H) 5.1 (s, 2H), 6.9 (t, 2H), 7.3 - 7.5 (m, 8H), 7.6 (t, 1H), 8.6 (s, 1H), 9.9 (s, 1H); Mass spectrum MH⁺ 455.

### Example 5.16

### 4-(4-(2-Fluorobenzyloxy)-3-methylanilino)-5-(1-methylpiperidin-4-yloxy)quinazoline

Obtained by reacting 4-(3-methyl-4-hydroxyanilino)-5-(1-methylpiperidin-4-yloxy)quinazoline **(reference example 38)** with 2-fluorobenzyl chloride in 74% yield; NMR spectrum (CDCl₃) 2.0 (m, 2H), 2.2 - 2.4 (m, 10H), 2.8 (m, 2H), 4.6 (m, 1H) 5.2 (s, 2H), 6.9 (d, 1H), 6.9 (d, 1H), 7.1 (t, 1H), 7.2 (t, 1H), 7.3 (m, 1H), 7.4 - 7.6 (m, 5H), 8.6 (s, 1H), 9.9 (s, 1H); Mass spectrum MH⁺ 473.

### Example 5.17

### 4-(4-(2,6-Difluorobenzyloxy)-3-methylanilino)-5-(1-methylpiperidin-4-yloxy)quinazoline

Obtained by reacting 4-(3-methyl-4-hydroxyanilino)-5-(1-methylpiperidin-4-yloxy)quinazoline **(reference example 38)** with 2,6-difluorobenzyl chloride in 72% yield; NMR spectrum (CDCl₃) 2.0 (m, 2H), 2.3 (m, 5H), 2.4 (m, 5H), 2.8 (m, 2H), 4.6 (m, 1H) 5.1 (s, 2H), 6.9 (m, 3H), 7.0 (d, 1H), 7.3 (m, 1H), 7.4 (m, 2H), 7.5 (dd, 1H), 7.6 (t, 1H), 8.6 (s, 1H), 9.9 (s, 1H); Mass spectrum MH⁺ 491.

### Example 5.18

### 4-(3-Methyl-4-(5-methylisoxazol-3-ylmethoxy)anilino)-5-(1-methylpiperidin-4-yloxy)quinazoline

Obtained by reacting 4-(3-methyl-4-hydroxyanilino)-5-(1-methylpiperidin-4-yloxy)quinazoline **(reference example 38)** with 3-chloromethyl-5-methylisoxazole in 70% yield; NMR spectrum (CDCl₃) 2.0 (m, 2H), 2.2 - 2.4 (m, 10H), 2.4 (s, 3H), 2.8 (m, 2H), 4.6 (m, 1H) 5.1 (s, 2H), 6.1 (s, 1H), 6.9 (m, 2H), 7.5 (m, 2H), 7.6 (t, 1H), 8.6 (s, 1H), 9.9 (s, 1H); Mass spectrum MH⁺ 460.

### Example 5.19

### 5-(1-Methylpiperidin-4-yloxy)-4-(3-methyl-4-(thiazol-4-ylmethoxy)anilino)quinazoline

Obtained by reacting 4-(3-methyl-4-hydroxyanilino)-5-(1-methylpiperidin-4-yloxy)quinazoline **(reference example 38)** with 4-chloromethylthiazole in 46% yield; NMR spectrum (CDCl₃) 2.0 (m, 2H), 2.2 - 2.4 (m, 10H), 2.8 (m, 2H), 4.6 (m, 1H) 5.3 (s, 2H), 6.9 (d, 1H), 6.9 (d, 1H), 7.4 - 7.6 (m, 5H), 8.6 (s, 1H), 8.8 (d, 1H), 9.9 (s, 1H); Mass spectrum MH⁺) 462.

### Example 5.20

### 4-(4-(2-Cyanobenzyloxy)-3-methylanilino)-5-(1-methylpiperidin-4-yloxy)quinazoline

Obtained by reacting 4-(3-methyl-4-hydroxyanilino)-5-(1-methylpiperidin-4-yloxy)quinazoline **(reference example 38)** with 2-chloromethyl benzonitrile in 33% yield; NMR spectrum (CDCl₃) 2.0 (m, 2H), 2.2 - 2.4 (m, 10H), 2.8 (m, 2H), 4.7 (m, 1H) 5.3 (s, 2H), 6.9 (m, 2H), 7.4 (m, 2H), 7.5 (m, 2H), 7.6 (m, 2H), 7.7 (m, 2H), 8.6 (s, 1H), 9.9 (s, 1H); Mass spectrum MH⁺ 480.

### Example 5.21

### 4-(4-(3-Fluorobenzyloxy)-3-methylanilino)-5-(1-methylpiperidin-4-yloxy)quinazoline

Obtained by reacting 4-(3-methyl-4-hydroxyanilino)-5-(1-methylpiperidin-4-yloxy)quinazoline **(reference example 38)** with 3-fluorobenzyl chloride in 43% yield; NMR spectrum (CDCl₃) 2.0 (m, 2H), 2.2 - 2.4 (m, 10H), 2.8 (m, 2H), 4.6 (m, 1H) 5.1 (s, 2H), 6.9 (m, 2H), 7.0 (m, 1H), 7.2 (m, 2H), 7.3 - 7.5 (m, 4H), 7.6 (t, 1H), 8.6 (s, 1H), 9.9 (s, 1H); Mass spectrum MH⁺ 473.

### Example 5.22

### 4-(3-Fluoro-4-(3-fluorobenzyloxy)anilino)-5-(1-methylpiperidin-4-yloxy)quinazoline

Obtained by reacting 3-fluorobenzyl chloride and 4-(3-fluoro-4-hydroxyanilino)-5-(1-methylpiperidin-4-yloxy)quinazoline **(reference example 4.3)** in 46% yield; NMR spectrum (DMSO-d6) 2.0 (m, 2H), 2.2 (m, 5H), 2.3 (m, 2H), 2.7 (m, 2H), 3.2 (m, 2H), 4.8 (m, 1H) 5.3 (s, 2H), 7.2 - 7.4 (m, 8H), 7.5 (m, 1H), 7.7 (m, 1H), 8.0 (m, 1H), 8.6 (s, 1H), 10.1 (s, 1H); Mass spectrum MH⁺ 477.

### Example 5.23

### 4-(3-Chloro-4-(2-methyloxazol-4-ylmethoxy)anilino)-5-(1-methylpiperidin-4-yloxy)quinazoline

Obtained from 4-(3-Chloro-4-hydroxyanilino)-5-(1-methylpipendin-4-yloxy)quinazoline **(reference example 4.2)** and methanesulphonic acid 2-methyloxazol-4-ylmethyl ester (reference example 48) in 111% yield; NMR Spectrum (DMSO-d6) 1.90 (m, 2H), 2.12 (m, 2H), 2.17 (s, 3H), 2.25 (m, 2H), 2.40 (s, 3H), 2.60 (m, 2H), 4.79 (m, 1H), 5.02 (s, 2H), 7.20 (d, 1H), 7.32 (d, 1H), 7.35 (d, 1H), 7.51 (dd, 1H), 7.70 (t, 1H), 8.05 (s, 1H), 8.10 (d, 1H), 8.50 (s, 1H), 10.04 (s, 1H); Mass spectrum M-H⁺ 480.

### Illustrative Example 5.24

### 4-(5-Chloro-2-fluoro-4-(2-pyridylmethoxy)anilino)-5-(1-methylpiperidin-4-yloxy)quinazoline

Obtained from 4-(5-chloro-2-fluoro-4-hydroxyanilino)-5-(1-methylpiperidin-4-yloxy)quinazoline **(reference example 4.7)** and 2-picolyl chloride hydrochloride in 28% yield; NMR spectrum (DMSO-d6, 373K),1.8 - 1.95 (m, 2H), 2.1- 2.2 (m, 2H), 2.2 (s, 3H), 2.2 - 2.3 (m, 2H), 2.7 - 2.8 (m, 2H), 4.75 - 4.85 (m, 1H), 5.3 (s, 2H), 7.25 (d, 1H), 7.3 - 7.4 (m, 3H), 7.6 (d, 1H), 7.7 - 7.8 (t, 1H), 7.85 - 7.95 (m, 1H), 8.6 (s, 1H), 8.6 - 8.7 (m, 2H), 10.0 (bs,1H); Mass Spectrum MH⁺ 495.

### Example 6

### 4-(3-Chloro-4-(decahydroquinolin-1-ylcarbonyl)anilino)-5-(1-methylpiperidin-4-yloxy)quinazoline

A suspension of 2-chloro-4-(5-(1-methylpiperidin-4-yloxy)quinazolin-4-ylamino)benzoic acid hydrochloride **(reference example 24)** (74.8 mg) in NMP (1.7 ml) was treated with a solution of HATU (76 mg) in NMP (1.7 ml) followed by a solution of di-*iso-*propylethylamine (43 mg) in NMP (1.7 ml) and the mixture stirred at room temperature for 30 minutes. To this was added decahydroquinoline (93 mg) and the mixture stirred at room temperature overnight. The solvent was removed *in vacuo* to give a gum that was treated with aqueous sodium hydrogen carbonate and extracted with DCM. The DCM solution was evaporated *in vacuo* to give an oil which was purified by column chromatography, eluting with DCM/methanol/880 NH₄OH (100/8/1), to give the title compound (37 mg, 42%); Mass spectrum MH⁺ 534.

The procedure described above was repeated using the appropriate amine and acid. Thus were obtained the compounds described below:

### Example 6.1

### 4-(3-Chloro-4-(decahydroisoquinolin-2-ylcarbonyl)anilino)-5-(1-methylpiperidin-4-yloxy)quinazoline

Obtained by reacting 2-chloro-4-(5-(1-methylpiperidin-4-yloxy)quinazolin-4-ylamino)benzoic acid hydrochloride **(reference example 24)** and decahydroisoquinoline in 35% yield; Mass spectrum MH⁺ 534.

### Example 6.2

### 4-(3-Chloro-4-(3-methylpiperidin-1-ylcarbonyl)anilino)-5-(1-methylpiperidin-4-yloxy)quinazoline

Obtained by reacting 2-chloro-4-(5-(1-methylpiperidin-4-yloxy)quinazolin-4-ylamino)benzoic acid hydrochloride **(reference example 24)** and 3-methylpiperidine in 59% yield; NMR spectrum (DMSO-d6, 373K) 0.90 (m, 3H), 1.25 (m, 1H), 1.50 (m, 1H), 1.68 (m, 2H), 1.84 (m, 1H), 2.00 (m, 2H), 2.20 (m, 2H), 2.25 (s, 3H), 2.35 (m, 2H), 2.75 (m, 3H), 2.95 (m, 3H), 4.82 (m, 1H), 7.27 (d, 1H), 7.37 (d, 1H), 7.43 (d, 1H), 7.70 (d, 1H), 7.78 (t, 1H), 8.23 (d, 1H), 8.64 (s, 1H), 10.25 (s, 1H); Mass spectrum MH⁺ 494.

### Example 6.3

### 4-(3-Chloro-4-(4-methylpiperidin-1-ylcarbonyl)anilino)-5-(1-methylpiperidin-4-yloxy)quinazoline

Obtained by reacting 2-chloro-4-(5-(1-methylpiperidin-4-yloxy)quinazolin-4-ylamino)benzoic acid hydrochloride **(reference example 24)** and 4-methylpiperidine in 71% yield; NMR spectrum (DMSO-d6, 373K) 1.00 (d, 3H), 1.16 (m, 2H), 1.70 (m, 3H), 2.00 (m, 2H), 2.20 (m, 2H), 2.28 (s, 3H), 2.36 (m, 2H), 2.75 (m, 2H), 2.95 (m, 4H), 4.82 (m, 1H), 7.28 (d, 1H), 7.39 (d, 1H), 7.42 (d, 1H), 7.70 (d, 1H), 7.78 (t, 1H), 8.21 (d, 1H), 8.65 (s, 1H), 10.24 (s, 1H); Mass spectrum MH⁺ 494.

### Example 6.4

### 4-(3-Ethynyl-4-(decahydroquinolin-1-ylcarbonyl)anilino)-5-(1-methylpiperidin-4-yloxy)quinazoline

Obtained by reacting 2-ethynyl-4-(5-(1-methylpiperidin-4-yloxy)quinazolin-4-ylamino)benzoic acid hydrochloride **(reference example 24.1)** and decahydroquinoline in 34% yield; Mass spectrum MH⁺ 524.

### Example 6.5

### 4-(3-Ethynyl-4-(homopiperidin-1-ylcarbonyl)anilino)-5-(1-methylpiperidin-4-yloxy)quinazoline

Obtained by reacting 2-ethynyl-4-(5-(1-methylpiperidin-4-yloxy)quinazolin-4-ylamino)benzoic acid hydrochloride **(reference example 24.1)** and homopiperidine in 51 % yield; Mass spectrum MH⁺ 484.

### Example 7

### 4-(3-Chloro-4-(3-fluorobenzyloxy)amilino)-5-(piperidin-4-yloxy)quinazoline

Trifluoroacetic acid (0.5 ml) was added to a solution of 5-(1-*tert-*butoxycarbonylpiperidine-4-yloxy)-4-(3-chloro-4-(3-fluorobenzyloxy)anilino)quinazoline (0.31 g) **(example 3)** in DCM (2 ml) and the solution stirred for 1 hour. The reaction was concentrated *in vacuo* and the residue triturated with *conc.* aq. ammonium hydroxide and filtered to give the title compound as a white solid (0.196 g, 87%); NMR spectrum (DMSO-d6) 1.76 - 1.92 (m, 2H), 2.24 - 2.36 (m, 2H), 2.77 - 2.90 (m, 2H), 3.15 - 3.28 (m, 2H), 4.65 - 4.77 (m, 1H), 5.16 (s, 2H), 6.90 - 7.08 (m, 3H), 7.18 - 7.29 (m, 2H), 7.32 - 7.66 (m, 4H), 7.92 (t, 1H), 8.63 (s, 1H), 10.05 (bs, 1H); Mass spectrum MH⁺ 479.

### Example 8

### 4-(3-Chloro-41(3-fluorobenzyloxy)anilino)-5-(1-propylpiperidin-4-yloxy)quinazoline

4-(3-Chloro-4-(3-fluorobenzyloxy)anilino)-5-(piperidin-4-yloxy)quinazoline (96 mg) **(example 7)**, propyl bromide (27 mg) and potassium carbonate (0.11 g) in DMA (2 ml) were stirred at room temperature overnight. The reaction was filtered and concentrated *in vacuo.* The residue was purified by chromatography (using DCM-5% methanol as eluent) to give the title compound as a white solid after trituration with ether (61 mg, 59%); NMR spectrum (CDCl₃) 0.92 (t, 3H), 1.45 1.61 (m, 2H), 1.92 - 2.06 (m, 2H), 2.20 - 2.39 (m, 6H), 2.79 - 2.92 (m, 2H), 4.64 (m, 1H), 5.16 (s, 2H), 6.87 - 7.06 (m, 3H), 7.17 - 7.29 (m, 2H), 7.31 - 7.41 (m, 1H), 7.44 (d, 1H), 7.52 (dd, 1H) 7.61 (t, 1H), 7.90 (d, 1H), 8.62 (s, 1H), 10.00 (bs, 1H); Mass spectrum MH⁺ 521.

The procedure described above was repeated using the appropriate amine and alkyl bromide or chloride. Thus were obtained the compounds described below:

### Example 8.1

### 4-(3-Chloro-4-(3-fluorobenzyloxy)anilino)-5-(1-allylpiperidin-4-yloxy)quinazoline

Obtained by reacting 4-(3-chloro-4-(3-fluorobenzyloxy)anilino)-5-(piperidin-4-yloxy)quinazoline **(example 7)** with allyl bromide in 75% yield; NMR spectrum (CDCl₃) 1.94 - 2.09 (m, 2H), 2.21 - 2.43 (m, 4H), 2.80 - 2.93 (m, 2H), 3.04 (d, 2H), 4.68 (m, 1H), 5.14 - 5.25 (m, 4H), 5.89 (m, 1H), 6.88 - 7.07 (m, 3H), 7.17 - 7.27, (m, 2H), 7.30 - 7.40 (m, 1H), 7.46 (d, 1H), 7.53 (dd, 1H), 7.61 (t, 1H), 7.90 (d, 1H), 8.62 (s, 1H), 9.98 (bs, 1H); Mass spectrum MH⁺ 519.

### Example 8.2

### 4-(3-Chloro-4-(3-fluorobenzyloxy)anilino)-5-(1-(2-propynyl)piperidin-4-yloxy)quinazoline

Obtained by reacting 4-(3-chloro-4-(3-fluorobenzyloxy)anilino)-5-(piperidin-4-yloxy)quinazoline **(example 7)** with 3-bromoprop-1-yne in 65% yield; NMR spectrum (CDCl₃) 1.97 - 2.12 (m, 2H), 2.18 (t, 1H), 2.25 - 2.34 (m, 2H), 2.50 - 2.62 (m, 2H), 2.86 - 2.97 (m, 2H), 3.39 (d, 2H), 4.68 (m, 1H), 5.15 (s, 2H), 6.90 - 7.07 (m, 3H), 7.19 - 7.29 (m, 2H), 7.31 - 7.40 (m, 1H), 7.46 (d, 1H), 7.51 (dd, 1H), 7.63 (t, 1H), 7.91 (d, 1H), 8.62 (s, 1H), 9.94 (bs, 1H); Mass spectrum MH⁺ 517.

### Example 8.3

### 4-(3-Chloro-4-(3-fluorobenzyloxy)anilino)-5-(1-(2-methoxyethyl)piperidin4-yloxy)quinazoline

Obtained by reacting 4-(3-chloro-4-(3-fluorobenzyloxy)anilino)-5-(piperidin-4-yloxy)quinazoline **(example 7)** with 2-bromoethyl methylether in 44% yield; NMR spectrum (CDCl₃) 1.96 - 2.10 (m, 2H), 2.23 - 2.33 (m, 2H), 2.35 - 2.47 (m, 2H), 2.64 (t, 2H), 2.87 - 2.97 (m, 2H), 3.36 (s, 3H), 3.52 (t, 2H), 4.66 (m, 1H), 5.16 (s, 2H), 6.88 - 7.08 (m, 3H), 7.18 - 7.29 (m, 2H), 7.31 - 7.40 (m, 1H), 7.44 (d, 1H), 7.52 (dd, 1H), 7.61 (t, 1H), 7.92 (d, 1H), 8.34 (s, 1H), 9.98 (bs, 1H); Mass spectrum MH⁺ 537.

### Example 8.4

### 2-(4-(4-(3-Chloro-4-(3-fluorobenzyloxy)anilino)quinazolin-5-yloxy)piperidin-1-yl)acetone.

Obtained by reacting 4-(3-chloro-4-(3-fluorobenzyloxy)anilino)-5-(piperidin-4-yloxy)quinazoline **(example 7)** with chloroacetone in 54 % yield; NMR spectrum (CDCl₃) 1.98 - 2.12 (m, 2H), 2.17, (s, 3H), 2.23 - 2.36 (m, 2H), 2.44 - 2.55 (m, 2H), 2.82 - 2.93 (m, 2H), 3.28 (s, 3H), 4.67 (m, 1H), 5.57 (s, 2H), 6.90 (d, 1H), 6.94 - 7.07 (m, 2H), 7.19 - 7.28 (m, 2H), 7.32 - 7.41 (m, 1H), 7.46 (d, 1H), 7.51 (dd, 1H), 7.61 (t, 1H), 7.92 (d, 1H), 8.63 (s, 1H), 9.96 (s, 1H); Mass spectrum MH⁺ 535.

### Example 8.5

### Methyl 2-(4-(4-(3-Chloro-4-(3-fluorobenzyloxy)anillno)quinazolin-5-yloxy)piperidin-1-yl)acetate

Obtained by reacting 4-(3-chloro-4-(3-fluorobenzyloxy)anilino)-5-(piperidin-4-yloxy)quinazoline **(example 7)** with methyl bromoacetate in 38% yield; NMR spectrum (CDCl₃) 2.00 - 2.15 (m, 2H), 2.24 - 2.35 (m, 2H), 2.54 - 2.65 (m, 2H), 2.91 - 3.02 (m, 2H), 3.31 (s, 2H), (s, 3H), 4.66 (m, 1H), 5.16 (s, 2H), 6.91 (d, 1H), 6.97 (d, 1H), 6.90 - 7.08 (m, 1H), 7.18 - 7.27 (m, 2H), 7.31 - 7.39 (m, 1H), 7.46 (d, 1H), 7.50 (dd, 1H), 7.62 (t, 1H), 7.93 (d, 1H), 8.63 (s; 1H), 9.96 (bs, 1H), Mass spectrum MH⁺ 551.

### Example 9

### 4-(3-Chloro-4-(3-fluorobenzyloxy)anilino)-5-(1-(methanesuphonyl)piperidin-4-yloxy)quinazoline

4-(3-Chloro-4-(3-fluorobenzyloxy)anilino)-5-(piperidin-4-yloxy)quinazoline (48 mg) **(example 7),** methanesulphonyl chloride (12.6 mg) and di-*iso*-propylethylamine (19.4 mg) in DCM (2 ml) were stirred at room temperature overnight. The reaction was concentrated *in vacuo.* The residue was purified by chromatography (using DCM to DCM-5% methanol as eluent) to give the title compound as a white solid (38 mg, 68%); NMR spectrum (DMSO-d6) 1.93 - 2.06 (m, 2H), 2.21 - 2.33 (m, 2H), 2.84 (s, 3H), 3.05 - 3.15 (m, 2H), 3.45 - 3.55 (m, 2H), 4.92 (m, 1H), 5.26 (s, 2H), 7.13 - 7.20 (m, 1H), 7.22 - 7.38 (m, 5H), 7.41 - 7.56 (m, 2H), 7.73 (t, 3H), 8.17 (s, 1H), 8.51 (s, 1H), 9.90 (bs, 1H); Mass spectrum MH⁺ 557.

The procedure described above was repeated using the appropriate amine and alkyl bromide or chloride. Thus was obtained the compound described below:

### Example 9.1

### 2-(4-(4-(3-Chloro-4-(3-fluorobenzyloxy)anilino)quinazolin-5-yloxy)piperidin-1-yl)acetamide

Obtained by reacting 4-(3-chloro-4-(3-fluorobenzyloxy)anilino)-5-(piperidin-4-yloxy)quinazoline **(example 7)** with 2-chloroacetamide in 75% yield; NMR spectrum (DMSO-d6) 1.90 - 2.02 (m, 2H), 2.15 - 2.25 (m, 2H), 2.37 - 2.46 (m, 2H), 2.72 - 2.80 (m, 2H), 2.89 (s, 2H), 4.81 (m, 1 H), 5.27 (s, 2H), 7.09 (bs, 1H), 7.16 (m, 1H), 7.22 7.36 (m, 6H), 7.42 - 7.52 (m, 2H), 7.72 (t, 1H), 8.17 (d, 1H), 8.52 (s, 1H), 10.10 (bs, 1H); Mass spectrum MH⁺ 536.

### Example 10

### 4-(1-(5-Methylisoxazol-3-ylmethyl)indol-5-ylamino)-5-(1-methylpiperidin-4-yloxy)quinazoline

Sodium hydride (60% dispersion in mineral oil, 25 mg) was added to a stirred solution of 4-(indol-5-ylamino)-5-(1-methylpiperidin-4-yloxy)quinazoline **(reference example 4.4)** (0.2 g) in DMF (2 ml). The solution was stirred at room temperature for 30 mins, then 5-methyl-3-isoxazolemethyl chloride (85 mg) added. The reaction was stirred for a further 3 hours, then poured into water. The resulting solid precipitate was filtered, dried and triturated with ether to give the title compound as a pale green solid (42 mg, 17%); NMR spectrum (DMSO-d6) 1.8 - 2.0 (m, 2H), 2.15 (s + m, 5H), 2.28 (m, 2H), 2.60 (m, 2H), 4.80 (m, 1H), 5.43 (s, 2H), 6.00 (s, 1H), 6.50 (d, 1H), 7.10 (d, 1H), 7.30 (m, 2H), 7.49 (m, 2H), 7.67 (t, 1H), 8.07 (dd, 1H), 8.42 (s, 1H), 10.10 (bs, 1H); Mass Spectrum MH⁺ 469.

The procedure described above was repeated using the appropriate indole and alkyl bromide or chloride. Thus were obtained the compounds described below:

### Example 10.1

### 4-(1-(2,6-Difluorobenzyl)indol-5-ylamino)-5-(1-methylpiperidin-4-yloxy)quinazoline

Obtained from 4-(indol-5-ylamino)-5-(1-methylpiperidin-4-yloxy)quinazoline **(reference example 4.4)** and 2,6-difluorobenzyl bromide in 89% yield; NMR spectrum (DMSO-d6) 1.80 -1.95 (m, 2H), 2.12 (s + m, 5H), 2.28 (m, 2H), 2.60 (m, 2H), 4.80 (m, 1H), 5.44 (s, 2H), 6.45 (d, 1H), 7.10 - 7.20 (m, 3H), 7.23 - 7.50 (m, 5H), 7.67 (t, 1H), 8.04 (d, 1H), 8.40 (s, 1H), 10.09 (bs, 1H); Mass Spectrum MH⁺ 500.

### Example 10.2

### 4-(1-(2-Cyanobenzyl)indol-5-ylamino)-5-(1-methylpiperidin-4-yloxy)quinazoline

Obtained from 4-(indol-5-ylamino)-5-(1-methylpiperidin-4-yloxy)quinazoline **(reference example 4.4)** and 2-cyanobenzyl bromide in 50% yield; NMR spectrum (DMSO-d6) 1.80 - 1.95 (m, 2H), 2.15 (s + m, 5H), 2.25 (m, 2H), 2.61 (m, 2H), 4.80 (m, 1H), 5.64 (s, 2H), 6.58 (d, 1H), 6.92 (d, 1H), 7.10 (d, 1H), 7.30 (m, 2H), 7.40 - 7.50 (m, 3H), 7.60'(dt, 1H), 7.68 (t, 1H), 7.90 (d, 1H), 8.13 (d, 1H), 8.42 (s, 1H), 10.11 (bs, 1H); Mass Spectrum MH⁺ 489.

### Example 10.3

### 5-(1-Methylpiperidin-4-yloxy)-4-(1-(2-pyridylmethyl)indol-5-ylamino)quinazoline

Obtained from 4-(indol-5-ylamino)-5-(1-methylpiperidin-4-yloxy)quinazoline **(reference example 4.4)** and 2-picolyl chloride hydrochloride in 42% yield; NMR spectrum (DMSO-d6) 1.80 - 1.95 (m, 2H), 2.15 (s + m, 5H), 2.25 (m, 2H), 2.62 (m, 2H), 4.80 (m, 1H), 5.50 (s, 2H), 6.52 (d, 1H), 6.98 (d, 1H), 7.10 (d, 1H), 7.25 (m, 3H), 7.42 (d, 1H), 7.52 (d, 1H), 7.69 (m, 2H), 7.90 (d, 1H), 8.08 (d, 1H), 8.41 (s, 1H), 8.53 (d, 1H), 10.10 (bs, 1H); Mass Spectrum MH⁺ 465.

### Example 10.4

### 5-(1-Methylpiperidin-4-yloxy)-4-(1-(thiazol-4-ylmethyl)indol-5-ylamino)quinazoline

Obtained from 4-(indol-5-ylamino)-5-(1-methylpiperidin-4-yloxy)quinazoline **(reference example 4.4)** and 4-chloromethylthiazole in 75% yield; NMR spectrum (DMSO-d6) 1.80 - 1.95 (m, 2H), 2.15 (s + m, 5H), 2.25 (m, 2H), 2.60 (m, 2H), 4.80 (m, 1H), 5.52 (s, 2H), 6.48 (d, 1H), 7.10 (d, 1H), 7.30 (m, 2H), 7.48 (m, 2H), 7.58 (d, 1H), 7.68 (t, 1H), 8.07 (d, 1H), 8.42 (s, 1H), 9.02 (d, 1H); Mass Spectrum MH⁺ 471.

### Example 10.5

### 4-(1-(4-Fluorobenzyl)indol-5-ylamino)-5-(1-methylpiperidin-4-yloxy)quinazoline

Obtained from 4-(indol-5-ylamino)-5-(1-methylpiperidin-4-yloxy)quinazoline **(reference example 4.4)** and 4-fluorobenzyl chloride in 78% yield; NMR spectrum (DMSO-d6) 1.80 - 1.95 (m, 2H), 2.15 (s + m, 5H), 2.25 (m, 2H), 2.60 (m, 2H), 4.80 (m, 1H), 5.40 (s, 2H), 6.50 (d, 1H), 7.10 - 7.30 (m, 7H), 7.48 (d, 1H), 7.53 (d, 1H), 7.69 (t, 1H), 8.08 (d, 1H), 8.42 (s, 1H), 10.10 (bs, 1H); Mass Spectrum MH⁺ 482.

### Example 10.6

### 4-(1-(2-Methoxybenzyl)indol-5-ylamino)-5-(1-methylpiperidin-4-yloxy)quinazoline

Obtained from 4-(indol-5-ylamino)-5-(1-methylpiperidin-4-yloxy)quinazoline **(reference example 4.4)** and 2-methoxybenzyl chloride in 20% yield; NMR spectrum (DMSO-d6) 1.80 - 1.95 (m, 2H), 2.15 (s + m, 5H), 2.25 (m, 2H), 2.60 (m, 2H), 3.84 (3H, s), 4.80 (m, 1H), 5.37 (s, 2H), 6.48 (d, 1H), 6.80 (m, 2H), 7.02 (d, 1H), 7.18 (d, 1H), 7.22 (m, 2H), 7.28 (d, 1H), 7.42 (m, 2H), 7.67 (t, 1H), 8.07 (d, 1H), 8.42 (s, 1H), 10.10 (bs, 1H); Mass Spectrum MH⁺ 494.

### Example 10.7

### 4-(1-(2-Chlorobenzyl)indol-5-ylamino)-5-(1-methylpiperidin-4-yloxy)quinazoline

Obtained from 4-(indol-5-ylamino)-5-(1-methylpiperidin-4-yloxy)quinazoline **(reference example 4.4)** and 2-chlorobenzyl chloride in 61% yield; NMR spectrum (DMSO-d6) 1.80 - 1.95 (m, 2H), 2.15 (s + m, 5H), 2.25 (m, 2H), 2.60 (m, 2H), 4.80 (m, 1H), 5.52 (s, 2H), 6.55 (d, 1H), 6.70 (d, 1H), 7.02 (d, 1H), 7.20 (m, 2H), 7.30 (m, 3H), 7.40 (d, 1H), 7.45 (m, 2H), 7.68 (t, 1H), 8.12 (d, 1H), 8.42 (s, 1H), 10.11 (bs, 1H); Mass Spectrum M⁺ 498.

### Example 10.8

### 4-(1-(2,5-Dimethylbenzyl)indol-5-ylamino)-5-(1-methylpiperidin-4-yloxy)quinazoline

Obtained from 4-(indol-5-ylamino)-5-(1-methylpiperidin-4-yloxy)quinazoline **(reference example 4.4)** and 2,5-dimethylbenzyl chloride in 75% yield; NMR spectrum (DMSO-d6) 1.80 - 1.95 (m, 2H), 2.10 (s, 3H), 2.15 (s + m, 5H), 2.23 (s, 3H), 2.25 (m, 2H), 2.60 (m, 2H), 4.80 (m, 1H), 5.37 (s, 2H), 6.51 (m, 2H), 6.97 (d, 1H), 7.05 (d, 1H), 7.20 (d,1H), 7.22 - 7.37 (m, 4H), 7.40 (d, 1H), 7.68 (t, 1H), 8.12 (d, 1H), 8.42 (s, 1H), 10.12 (bs, 1H); Mass Spectrum MH⁺ 492.

### Example 10.9

### 4-(1-(3-Chlorobenzyl)indol-5-ylamino)-5-(1-methylpiperidin-4-yloxy)quinazoline

Obtained from 4-(indol-5-ylamino)-5-(1-methylpiperidin-4-yloxy)quinazoline **(reference example 4.4)** and 3-chlorobenzyl chloride in 70% yield; NMR spectrum (DMSO-d6) 1.80 - 1.95 (m, 2H), 2.15 (s + m, 5H), 2.25 (m, 2H), 2.60 (m, 2H), 4.80 (m, 1H), 5.43 (s, 2H), 6.52 (d, 1H), 7.10 - 7.38 (m, 7H), 7.48 (d, 1H), 7.57 (d, 1H), 7.70 (t, 1H), 8.10 (d, 1H), 8.42 (s, 1H), 10.10 (bs, 1H); Mass Spectrum M⁺ 498.

### Example 10.10

### 5-(1-Methylpiperidin-4-yloxy)-4-(1-(2-methylthiazol-4-ylmethyl)indol-5-ylamino)quinazoline

Obtained from 4-(indol-5-ylamino)-5-(1-methylpiperidin-4-yloxy)quinazoline **(reference example 4.4**) and 4-chloromethyl-2-methylthiazole hydrochloride in 37% yield; NMR spectrum (DMSO-d6) 1.80 - 1.95 (m, 2H), 2.15 (s + m, 5H), 2.25 (m, 2H), 2.58 (s, 3H), 2.60 (m, 2H), 4.80 (m, 1H), 5.41 (s, 2H), 6.47 (d, 1H), 7.20 (d, 1H), 7.30 (m, 3H), 7.45 (d, 1H), 7.56 (d, 1H), 7.68 (t, 1H), 8.05 (d, 1H), 8.42 (s, 1H), 10.10 (bs, 1H); Mass Spectrum MH⁺ 485.

### Example 10.11

### 4-(1-(2-Fluorobenzyl)indol-5-ylamino)-5-(1-methylpiperidin-4-yloxy)quinazoline

Obtained from 4-(indol-5-ylamino)-5-(1-methylpiperidin-4-yloxy)quinazoline (reference example 4.4) and 2-fluorobenzyl chloride in 18% yield; Mass Spectrum MH⁺ 482.

### Example 10.12

### 4-(1-(3-Fluorobenzyl)indol-5-ylamino)-5.(1-methylpiperidin-4-yloxy)quinazoline

Obtained from 4-(indol-5-ylamino)-5-(1-methylpiperidin-4-yloxy)quinazoline **(reference example 4.4)** and 3-fluorobenzyl chloride in 33% yield; Mass Spectrum MH⁺ 482.

### Example 11

### 4-(4-Benzyloxy-3-ethynylanilino)-5-(1-methylpiperidin-4-yloxy)quinazoline

Sodium dithionite (1 g) was added to a solution of 4-benzyloxy-3-ethynyl-nitrobenzene **(reference example 28)** in ethanol (10 ml) and water (10 ml) and the solution heated at 80°C for 3 hours. The ethanol was removed *in vacuo* and the residue extracted with DCM. Combined organic extracts were dried and concentrated and the residue dissolved in IPA (0.5 ml). To this was added di-*iso*-propylethylamine (0.025 ml) and 4-chloro-5-(1-methylpiperidin-4-yloxy)quinazoline **(reference example 2)** and the solution heated at 90°C for 3 hours. The reaction was cooled and the resulting precipitate collected, washed with IPA and ether, and dried to give the title compound as a cream-coloured solid (3.5 mg, 2%); Mass Spectrum MH⁺ 465.

### Example 12

### 4-(3-Ethynyl 4-(2-fluorobenzyloxy)anilino)-5-(1-methylpiperidin-4-yloxy)quinazoline hydrochloride

A solution of 4-chloro-5-(1-methylpiperidin-4-yloxy)quinazoline **(reference example 2)** (70 mg) and 3-ethynyl-4-(2-fluorobenzyloxy)aniline **(reference example 30)** (70 mg) in IPA (2 ml) was heated at reflux for 2 hours. The solution was cooled and the resulting precipitate filtered, washed with IPA and ether to give the title compound as a yellow solid (0.115 g, 89%); Mass Spectrum MH⁺ 483.

The procedure described above was repeated using the appropriate chloroquinazoline and aniline. Thus were obtained the compounds described below:

### Example 12.1

### 4-(3-Ethynyl 4-(3-fluorobenzyloxy)anilino)-5-(1-methylpiperidin-4-yloxy)quinazoline hydrochloride

Obtained by reacting 4-chloro-5-(1-methylpiperidin-4-yloxy)quinazoline **(reference example 2)** with 3-ethynyl-4-(3-fluorobenzyloxy)aniline **(reference example 30.1)** in 80% yield; Mass Spectrum MH⁺ 483.

### Example 12.2

### 4-(3-Ethynyl 4-(2,6-difluorobenzyloxy)anilino)-5-(1-methylpiperidin-4-yloxy)quinazoline hydrochloride

Obtained by reacting 4-chloro-5-(1-methylpiperidin-4-yloxy)quinazoline (reference example 2) with 4-(2,6-difluorobenzyloxy)-3-ethynylaniline **(reference example 30.2)** in 73% yield; Mass Spectrum MH⁺ 502.

### Example 12.3

### 4-(3-Ethynyl 4-(5-methylisoxazol-3-ylmethoxy)anilino)-5-(1-methylpiperidin-4-yloxy)quinazoline hydrochloride

Obtained by reacting 4-chloro-5-(1-methylpiperidin-4-yloxy)quinazoline **(reference example 2)** with 3-ethynyl-4-(5-methylisoxazol-3-ylmethoxy)aniline **(reference example 30.4)** in 61 % yield; Mass Spectrum MH⁺ 471.

### Example 12.4

### 4-(3-Ethynyl 4-(thiazol-4-ylmethoxy)anilino)-5-(1-methylpiperidin-4-yloxy)quinazoline hydrochloride

Obtained by reacting 4-chloro-5-(1-methylpiperidin-4-yloxy)quinazoline **(reference example 2)** with 3-ethynyl-4-(thiazol-4-ylmethoxy)aniline (reference example 30.3) in 20% yield; Mass Spectrum MH⁺ 473.

### Example 12.5

### 4-(3-Chloro-4-(2-pyrimidinylmethoxy)anilino)-5-(1-methylpiperidin-4-yloxy)quinazoline hydrochloride

Obtained by reacting 4-chloro-5-(1-methylpiperidin-4-yloxy)quinazoline **(reference example 2)** with 3-chloro-4-(2-pyrimidinylmethoxy)aniline (reference example 9.2) in 40% yield; NMR spectrum (DMSO-d6) 1.90 (m, 2H), 2.15 (m, 2H), 2.20 (s, 3H), 2.25 (m, 2H), 2.60 (m, 2H), 4.75 (m, 1H), 5.40 (s, 2H), 7.15 (d, 1H), 7.20 (d, 2H), 7.30 (d, 1H), 7.45 (dd, 1H), 7.47 (t, 1H), 7.70 (t, 1H), 8.10 (d, 1H), 8.50 (s, 1H), 8.85 (d, 2H), 10.0 (s, 1H); Mass spectrum M-H⁺ 477.

### Illustrative Example 12.6

### 4-(4-(2-Aminothiazol-4-ylmethoxy)-3-chloroanilino)-5-(1-methylpiperidin-4-yloxy)quinazoline hydrochloride

Obtained from 4-chloro-5-(1-methylpiperidin-4-yloxy)quinazoline **(reference example 2)** and 4-(2-aminothiazol-4-ylmethoxy)-3-chloroaniline (reference example 9.3) in 75% yield; NMR spectrum (DMSO-d6) 1.84 - 1.95 (m, 2H), 2.07 - 2.17 (m, 2H), 2.17 (s, 3H), 2:22 - 2.32 (m, 2H), 2.57 - 2.67 (m, 2H), 4.78 (m, 1H). 4.94 (s, 2H), 6.59 (s, 1H), 6.95 (s, 2H), 7.21 (d, 1H). 7.29 (d, 1H), 7.32 (d, 1H), 7.49 (dd, 1H), 7.70 (dd, 1H), 8.08 (d, 1H), 8.49 (s, 1H), 10.03 (s, 1H); Mass spectrum M-H⁺ 497.

### Example 12.7

### 4-(3-Fluoro-4-(1-methyl-1H-imidazol-2-ylthio)anilino)-5-(1-methylpiperidin-4-yloxy)quinazoline hydrochloride

Obtained from 4-chloro-5-(1-methylpiperidin-4-yloxy)quinazoline **(reference example 2)** and 3-fluoro-4-(1-methyl-1*H*-imidazol-2-ylthio)aniline **(reference example 6.2)** in 31% yield; NMR spectrum (DMSO-d6, 373K) 1.9 - 2.0 (m, 2H), 2.1- 2.2 (m, 2H), 2.2 (s, 3H), 2.25 - 2.35 (m, 2H), 2.6 - 2.7 (m, 2H), 3.7 (s, 3H), 4.7 - 4.8 (m, 1H), 7.0 (s, 1H), 7.1 - 7.15 (t, 1H), 7.2 (d, 1H), 7.3 (s, 1H), 7.35 - 7.42 (dd, 1H), 7.7 - 7.8 (t, 1H), 8.0 - 8.1 (dd, 1H), 8.55 (s, 1H), 10.2 (bs, 1H); Mass Spectrum MH⁺ 465.

### Example 12.8

### 4-(3-Fluoro-4-(1-methyl-1H-1,3,4-triazol-2-ylthio)anilino)-5-(1-methylpiperidin-4-yloxy)quinazoline hydrochloride

Obtained from 4-chloro-5-(1-methylpiperidin-4-yloxy)quinazoline **(reference example 2)** and 3-fluoro-4-(1-methyl-1*H*-1,3,4-triazol-2-ylthio)aniline (reference example 6.3) in 16% yield; NMR spectrum (DMSO-d6, 373K) 1.9 - 2.0 (m, 2H), 2.1 - 2.2 (m, 2H), 2.2 (s, 3H), 2.25 - 2.35 (m, 2H), 2.6 - 2.7 (m, 2H), 3.6 (s, 3H), 4.7 - 4.8 (m, 1H), 7.2 (d, 1H), 7.35 - 7.5 (m, 3H), 7.7 - 7.8 (t, 1H), 8.1 - 8.2 (d, 1H), 8.5 - 8.6 (d, 2H), 10.2 (bs, 1H); Mass Spectrum MH⁺ 466.

### Example 12.9

### 4-(3-Chloro-4-(2-pyridylthio)anilino)-5-(1-methylpiperidin-4-yloxy)quinazoline hydrochloride

Obtained from 4-chloro-5-(1-methylpiperidin-4-yloxy)quinazoline (**reference example 2**) and 3-Chloro-4-(2-pyridylthio)aniline (**reference example 6.4**) in 11% yield; NMR spectrum (DMSO-d6, 373K) 1.9- 2.1 (m, 2H), 2.15 - 2.3 (m, 2H), 2.3 (s, 3H), 2.3 - 2.45 (m, 2H), 2.7 - 2.85 (m, 2H), 4.75 - 4.9 (m, 1H), 7.0 - 7.1 (d, 1H), 7.15 - 7.25 (dd, 1H), 7.25 - 7.35 (d, 1H), 7.4 - 7.5 (d, 1H), 7.6 - 7.7 (m, 1H), 7.7 - 7.85 (m, 3H), 8.3 - 8.45 (m, 2H), 8.6 (s, 1H), 10.3 (bs, 1H); Mass Spectrum MH⁺ 478.

### Example 12.10

### 4-(3-Chloro-4-(2-pyrimidinylthio)anilino)-5-(1-methylpiperidin-4-yloxy)quinazoline hydrochloride

Obtained from 4-chloro-5-(1-methylpiperidin-4-yloxy)quinazoline (**reference example 2**) and 3-chloro-4-(2-pyrimidinylthio)aniline (**reference example 6.5**) in 6% yield; NMR spectrum (DMSO-d6, 373K) 1.9 - 2.0 (m, 2H), 2.2 - 2.25 (m, 2H), 2.25 (s, 3H), 2.3 -, 2.4 (m, 2H), 2.7 - 2.8 (m, 2H), 4.75 - 4.85 (m, 1H), 7.2 - 7.25 (t, 1H), 7.25 - 7.3 (d, 1H), 7.4 - 7.45 (d, 1H), 7.7 - 7.75 (m, 1H), 7.75 - 7.85 (d, 2H), 8.45 (s, 1H), 8.6 (d, 2H), 8.65 (s, 1H), 10.3 (bs, 1H); Mass Spectrum MH⁺ 479.

### Example 12.11

### 4-(3-Chloro-4-(1H-imidazol-2-ylthio)anilino)-5-(1-methylpiperidin-4-yloxy)quinazoline hydrochloride

Obtained from 4-chloro-5-(1-methylpiperidin-4-yloxy)quinazoline (**reference example 2**) and 3-chloro-4-(1*H*-imidazol-2-ylthio)aniline (**reference example 9.6**) in 74% yield; NMR spectrum (DMSO-d6, 373K) 1.8 - 1.9 (m, 2H), 2.1 - 2.25 (m, 2H), 2.15 (s, 3H), 2.6 - 2.7 (m, 2H) 4.7 - 4.8. (m, 1H), 6.7 - 6.8 (d, 1H), 7.1 (bs, 1H), 7.2 - 7.25 (d, 1H), 7.3 - 7.4 (d, 1H), 7.4 - 7.5 (dd, 1H), 7.7 - 7.8 (t, 1H), 8.35 (d, 1H), 8.55 (s, 1H), 10 - 10.2 (bs, 1H), 12.8 - 12.9 (bs, 1H); Mass spectrum MH⁺ 467.

### Example 12.12

### 4-(3-Fluoro-4-(1H-imidazol-2-ylthio)anilino)-5-(1-methylpiperidin-4-yloxy)quinazoline hydrochloride

Obtained from 4-chloro-5-(1-methylpiperidin-4-yloxy)quinazoline (reference example 2) and 3-fluoro-4-(1*H* imidazol-2-ylthio)aniline (**reference example 8.5**) in 33% yield; NMR spectrum (DMSO-d6, 373K) 1.9 - 2.0 (m, 2H), 2.15 - 2.25 (m, 2H), 2.25 (s, 3H), 2.6 - 2.7 (m, 2H) 4.7 - 4.8. (m, 1H), 7.1 (s, 1H), 7.2 - 7.3 (m, 1H), 7.4 - 7.5 (d, 1H), 7.7 - 7.8 (t, 1H), 8.1-8.2 (d, 1H), 8.6 (s, 1H), 10.2-10.5 (bs, 1H), 12.0 - 12.8 (bs, 1H); Mass spectrum MH⁺ 451.

### Example 12.13

### 4-(3-Chloro-4-(2-thiazolylthio)anilino)-5-(1-methylpiperidin-4-ylosy)quinazoline hydrochloride

Obtained from 4-chloro-5-(1-methylpiperidin-4-yloxy)quinazoline (reference example 2) and 3-chloro-4-(2-thiazolylthio)aniline (**reference example 9.7**) in 29% yield; NMR spectrum (DMSO-d6, 373K) 1.9 - 2.0 (m, 2H), 2.15 - 2.25 (m, 2H), 2.25 (s, 3H), 2.3 - 2.4 (m, 2H), 2.7 - 2.8 (m, 2H), 4.75 - 4.85 (m, 1H), 7.2 - 7.5 (d, 1H), 7.4 - 7.45 (d, 1H), 7.7 (d, 1H), 7.8 (s, 1H, 7,8 - 7,9 (m, 2H), 8.4 (s, 1H), 8.65 (s; 1H), 10.4 (bs, 1H); Mass spectrum MH⁺ 482.

### Illustrative Example 13

### 4-(3-Chloro-4-(2-pyrazinylmethoxy)anilino)-5-(1-methylpiperidin-4-yloxy)quinazoline

A suspension of 2-methylpyrazine (940 mg); *N*-chlorosuccinimide (1.34 g) and benzoyl peroxide (70%, 71 mg) in carbon tetrachloride (50 ml) was heated at reflux for 24 hours. The reaction was cooled to 0°C in an ice bath and then filtered through diatomaceous earth. To the crude solution of 2-chloromethylpyrazine in carbon tetrachloride was added potassium carbonate (138 mg), 4-(3-chloro-4-hydroxyanilino)-5-(1-methylpiperidin-4-yloxy)quinazoline (**reference example 4.2**) (90 mg) and *cis*-dicyclohexano-18-crown-6 (5 mg), and the reaction mixture heated at 80°C. After 16 hours, the reaction mixture was concentrated *in vacuo* and the resulting residue was partitioned between DCM and water. The combined organic extracts were dried and concentrated and the residue purified by chromatography (0-3% 7N methanolic ammonia in DCM) to yield the title compound as a pale pink solid (46 mg; 41%); NMR spectrum (DMSO-d6) 1.90 (m, 2H), 2.15 (m, 2H), 2.20 (s, 3H), 2.25 (m, 2H), 2.60 (m, 2H), 4.75 (m, 1H), 5.35 (s, 2H), 7.20 (d, 1H), 7.35 (d, 2H), 7.50 (dd, 1H), 7.70 (t, 1H), 8.15 (d, 1H), 8.50 (s, 1H), 8.65 (dd, 2H), 8.85 (s, 1H); Mass spectrum M-H⁺ 477.

The procedure described above was repeated using the appropriate methylheterocycle. Thus was obtained the compound described below:

### Example 13.1

### 4-(3-Chloro-4-(4-pyrimidinylmethoxy)anilino)-5-(1-methylpiperidin-4-yloxy)quinazoline

Obtained from 4-(3-chloro-4-hydroxyanilino)-5-(1-methylpiperidin-4-yloxy)quinazoline (**reference example 4.2**) and 4-methylpyrimidine in 12% yield; NMR spectrum (DMSO-d6) 1.95 (m, 2H), 2.15 (m, 2H), 2.25 (s, 3H), 2.40 (m, 2H), 2.75 (m, 2H), 4.80 (m, 1H), 5.35 (s, 2H), 7.25 (t, 1H), 7.35 (d, 2H), 7.50 (dd, 1H), 7.65 (dd, 1H), 7.70 (t, 1H), 8.15 (d, 1H), 8.50 (s, 1H), 8.85 (d, 2H), 9.20 (d, 1H), 10.0 (s, 1H); Mass spectrum M-H⁺ 477.

### Example 14

### 4-(3-Chloro-4-(2-pyridylmethoxy)anilino)-5-(1-methylpiperidin-4-yloxy)quinazoline

Potassium carbonate (43.2 g) was added to a solution of 4-(3-chloro-4-hydroxyanilino)-5-(1-methylpiperidin-4-yloxy)quinazoline (30 g) (**reference example 4.2**), 2-picolyl chloride hydrochloride (13.8 g) and 18-crown-6 (1 g) in acetonitrile (1000 ml) and the reaction heated at reflux for 3 hours. The reaction was filtered whilst hot, and allowed to cool. The resulting crystalline solid was filtered and recrystallised from acetonitrile to give the title compound as beige needles (19.36 g, 52%); NMR sprectrum (DMSO-d6) 1.80 -1.95 (m, 2H), 2.10 -2.20 (s + m, 5H), 2.22 (m, 2H), 2.60 (m, 2H), 4.75 (m, 1H), 5.24 (s, 2H), 7.18 (d, 1H), 7.23 (d, 1H), 7.33 (m, 2H), 7.45 (dd, 1H), 7.54 (d, 1H), 7.66 (t, 1H), 7.83 (dt, 1H), 8.12 (d, 1H), 8.47 (s, 1H), 8.57 (d, 1H), 10.02 (bs, 1H); Mass spectrum M⁺ 476.

The procedure described above was repeated using the appropriate halomethyl compound. Thus were obtained the compounds described below:

### Illustrative Example 14.1

### 4-(3-Chloro-4-(imidazo[1,2-a]pyridin-2-ylmethoxy)anilino)-5-(1-methylpiperidin-4-yloxy)quinazoline

Obtained from 4-(3-choro-4-hydroxyanilino)-5-(1-methylpiperidin-4-yloxy)quinazoline (**reference example 4.2**) and 2-chloromethylimidazo[1,2-*a*]pyridine in 28% yield; NMR spectrum (DMSO-d6) 1.85 - 1.97 (m, 2H), 2.10 - 2.20 (m, 2H), 2.17 (s, 3H), 2.23 - 2.34 (m, 2H), 2.56 - 2.69 (m, 2H), 4.80 (m, 110, 5.32 (s, 2H), 6.89 (ddd, 1H), 7.23 (d, 1H), 7.24 (ddd, 1H), 7.33 (d, 1H), 7.41 (d, 1H), 7.51 (dd, 1H), 7.54 (dd, 1H), 7.72 (dd, 1H), 8.02 (s, 1H), 8.11 (d, 1H), 8.50 (s, 1H), 8.55 (dd, 1H), 10.06 (s, 1H); Mass spectrum MH⁺ 515.

### Illustrative Example 14.2

### 4-(4-(Benzo[d]isoxazol-3-ylmethoxy)-3-chloroanilino)-5-(1-methylpiperidin-4-yloxy)quinazoline

Obtained from 3-bromomethylbenzo[d]isoaazole (prepared as in Chim. Ther. 1972, 7(2),127-132) and 4-(3-chloro-4-hydroxyanilino-5-(1-methylpiperidin-yloxy)quinazoline (**reference example 4.2**) in 36% yield; NMR spectrum (DMSO-d6) 1.86 -1.97 (m, 2H), 2.08 - 2.22 (m, 2H), 2.18 (s, 3H), 2.23 - 2.33 (m, 2H), 2.57 - 2.68 (m, 2H), 4.79 (m, 1H), 5.73 (s, 2H), 7.23 (d, 1H), 7.34 (d, 1H), 7.42 (d, 1H), 7.47 (dd, 1H), 7.55 (dd, 1H), 7.71 (ddd, 1H), 7.73 (dd, 1H), 7.80 (d, 1H), 8.03 (d, 1H), 8.15 (d, 1H), 8.52 (s, 1H), 10.07 (s, 1H); Mass spectrum MH⁺ 516.

### Example 15

### 4-[3-Chloro-4-(2-pyrimidinyloxy)anilino]-5-(tetrahydrofuran-3-yloxy)quinazoline

A mixture of 4-(3-chloro-4-hydroxyanilino)-5-(tetrahydrofuran-3-yloxy)quinazoline (70 mg) (**reference example 42.1**), 2-chloropyrimidine (25 mg), potassium carbonate (275 mg) and 1,4,7,10,13,16-hexaoxacyclooctadecane (5 mg) in acetonitrile (8 ml) was heated at reflux for 30 hours. Water (30 ml) was added and the mixture was extracted with DCM (30 ml). The extracts were dried and concentrated *in vacuo*. The residue was purified by chromatography using 0-2% methanol-DCM as eluent to afford the title compound as a solid (15 mg, 17%); NMR Spectrum (DMSO-d6) 2.15 - 2.24 (m, 1H), 2.30 - 2.42 (m, 1H), 3.8 - 3.94 (m, 2H), 3.98 (q, 1H), 4.23 (d, 1H), 5.46 (t, 1H), 7.23 (d, 1H), 7.28 (t, 1H), 7.39 (d, 1H), 7.41 (d, 1H), 7.70 (dd, 1H), 7.77 (t, 1H), 8.31 (d,1H), 8:60 (s, 1H), 8.65 (d, 2H), 10.21 (s, 1H); Mass spectrum MH⁺ 436.

### Illustrative Example 16

### 4-(3-Chloro-4-(1,2,4-oxadiazol-3-ylmethoxy)anilino)-5-(1-methylpiperidin-4-yloxy)quinazoline

2-(2-Chloro-4-(5-(1-methylpiperidin-4-yloxy)quinazolin-4-ylamino)phenoxy)-*N-*hydroxyacetamidine (200 mg) (**reference example 35**) was dissolved in formic acid (4 ml). Trimethyl orthoformate (4 ml) was added, and the mixture stirred at ambient temperature for 1 hour. The mixture was evaporated, and the residue purified by chromatography, using 0 to 1.5% (7:1 methanol / conc. ammonia (aq)) in DCM as eluent to give the title compound as a white crystalline solid (157 mg, 77%); NMR spectrum (DMSO-d6) 1.83 - 1.96 (m, 2H), 2.07 - 2.20 (m, 2H), 2.17 (s, 3H), 2.22 - 2.32 (m, 2H), 2.57 - 2.67 (m, 2H), 4.78 (m, 1H), 5.45 (s, 2H), 7.22 (d, 1H), 7.32 (d, 1H), 7.33 (d, 1H), 7.52 (dd, 1H), 7.71 (dd, 1H), 8.12 (d, 1H), 8.51 (s, 1H), 9.67 (s, 1H), 10.05 (s, 1H); Mass spectrum MH⁺ 467.

The procedure described above was repeated using the appropriate hydroxyacetamidine and orthoester. Thus was obtained the compound described below:

### Illustrative Example 16.1

### 4-(3-Chloro-4-(5-methyl-1,2,4-oxadiazol-3-ylmethoxy)anilino-5-(1-methylpiperidin-4-yloxy)quinazoline

Obtained from 2-(2-chloro-4-(5-(1-methylpiperidin-4-yloxy)quinazolin-4-ylamino)phenoxy)-*N*-hydroxyacetamidine (**reference Example 35**) and trimethyl orthoacetate in 60% yield; NMR spectrum (DMSO-d6) 1.83 -1.97 (m, 2H), 2.08 - 2.18 (m, 2H), 2.17 (s, 3H), 2.21 - 2.31 (m, 2H), 2.57 - 2.67 (m, 2H), 2.61 (s, 3H), 4.78 (m, 1H), 5.34 (s, 2H), 7.21 (d, 1H), 7.31 (d, 1H), 7.32 (d, 1H), 7.52 (dd, 1H), 7.71 (dd, 1H), 8.11 (d, 1H), 8.50 (s, 1H), 10.05 (s, 1H); Mass spectrum MH⁺ 467.

### Illustrative Example 17

### 4-(4-(5-Amino-1,3,4-oxadtazol-2-ylmethoxy)-3-chloroanilino)-5-(1-methylpiperidin-4-yloxy)quinazoline

Potassium hydrogen carbonate (10 mg) was dissolved in a mixture of ethanol (2 ml) and water (4 ml) 2-[2-Chloro-4-(5-(1-methylpiperidin-4-yloxy)quinazolin-4-ylamino)phenoxy]acetic acid hydrazide (**reference example 37**) (40 mg) was added. The suspension was briefly sonicated, then cooled to 0°C. Cyanogen bromide (3M in DCM, 31 µl) was added dropwise. The mixture was allowed to warm to ambient temperature overnight with stirring. The resulting yellow solution was evaporated, and the residue purified by chromatography, using 0 to 4% (7:1 methanol / conc. ammonia (aq)) in DCM as eluent to give the title compound as a white crystalline solid (14 mg, 33%); NMR spectrum (DMSO-d6) 1.87 -1.97 (m, 2H), 2.08 - 2.20 (m, 2H), 2.17 (s, 3H), 2.20 - 2.34 (m, 2H), 2.58 - 2.69 (m,2H), 4.79 (m, 1H), 5.26 (s, 2H), 7.20 (s, 2H), 7.24 (d, 1H), 7.34 (d, 1H), 7.36 (d, 1H), 7.56 (d, 1H), 7.72 (dd, 1H), 8.13 (d, 1H), 8.52 (s, 1H), 10.09 (s, 1H); Mass spectrum MH⁺ 482.

### Example 18

### 4-(3-Chloro-4-12-pyridylmethoxy)anilino-5-(tetrahydropyran-4-yloxy)quinazoline

Phosphorus oxychloride (0.21 ml) was added dropwise to a solution of 3,4-dihydro-5-(tetrahydropyran-4-yloxy)quinazolin-4-one (**reference example 1.2**) (57 mg) and di-*iso-*propylethylamine (0.045 ml) in anhydrous 1,2-dichloroethane (5 ml) at 0°C. The mixture was heated at 80°C for 3 hours and then concentrated *in vacuo.* The residue was azeotroped with toluene (5 ml), di-*iso*-propylethylamine (0.5 ml) was added and the mixture was again concentrated *in vacuo.* The residue was dissolved in IPA (1 ml) and 3-chloro-4-(2-pyridylmethoxy)aniline (obtained as described in PCT Int. Appl. WO 9615118) (100 mg) was added. The resulting mixture was heated at 80°C for 12 hours and then concentrated *in vacuo.* The residue was purified by chromatography, using 0-5% methanol - DCM as eluent, to give the title compound as a solid (48 mg, 43%); NMR Spectrum (DMSO-d6) 1.80 - 1.95 (m, 2H), 2.13 - 2.27 (m, 2H), 3.54 (t, 2H), 3.84 - 3.98 (m, 2H), 4.89 - 5.00 (m, 1H), 5.28 (s, 2H), 7.21 - 7.39 (m, 4H), 7.49 (d, 1H), 7.56 (d, 1H), 7.71 (t, 1H), 7.85 (t, 1H), 8.15 - 8.20 (m, 1H), 8.50 (s, 1H), 8.54 - 8.61 (m, 1H), 10.03 (s, 1H); Mass spectrum MH⁺ 463.

The procedure described above was repeated using the appropriate 3,4-dihydroquinazolin-4-one and aniline. Thus were obtained the compounds described below:

### Example 18.1

### 4-(1-(3-Fluorobenzyl)indazol-5-ylamino)-5-(tetrahydropyran-4-yloxy)quinazoline

Obtained by reacting 3,4-dihydro-5-(tetrahydropyran-4-yloxy)quinazolin-4-one (**reference example 1.2**) and 5-amino-1-(3-fluorobenzyl)indazole (obtained as described in PCT Int. Appl. WO 9802438) in 19% yield; NMR Spectrum (DMSO-d6) 1.80 - 1.95 (m, 2H), 2.16 - 2.27 (m, 2H), 3.55 (dt, 2H), 3.91 (dt, 2H), 4.93 - 5.03 (m, 1H), 5.69 (s, 2H), 7.02 - 7.12 (m, 3H), 7.26 (d, 1H), 7.30 - 7.38 (m, 2H), 7.54 (dd, 1H), 7.71 (t, 2H), 8.14 (s, 1H), 8.39 (d , 1H), 8.48 (s, 1H), 10.16 (s,1H); Mass spectrum MH⁺ 470.

### Example 18.2

### 4-(3-Chloro-4-((1H-imidazol-2-ylthio)anilino)-5-(tetrahydropyran-4-yloxy)quinazoline

Obtained by reacting 3,4-dihydro-5-(tetrahydropyran-4-yloxy)quinazolin-4-one (**reference example 1.2**) and 3-chloro-4-(1*H*-imidazol-2-ylthio)aniline (**reference example 9.6**) in 10% yield; NMR Spectrum (DMSO-d6) 1.78 - 1.93 (m, 2H), 2.12 - 2.24 (m, 2H), 3.52 (dt, 2H), 3.89 (dt, 2H), 4.88 - 4.99 (m, 1H), 6.75 (d, 1H), 7.26 (bs, 1H), 7.28 (d, 1H), 7.35 - 7.44 (m, 3H), 7.73 (t, 1H), 8.40 (d, 1H), 8.57 (s, 1H), 10.18 (s, 1H), 12.86 (s, 1H); Mass spectrum M-H⁺ 452.

### Example 18.3

### 4-(3-Chloro-4-(2-pyridylmethoxy)anilino)-5-(tetrahydrofuran-3-yloxy)quinazoline

Obtained by reacting 3,4-dihydro-5-(tetrahydrofuran-3-yloxy)quinazolin-4-one (**reference example 1.3**) and 3-chloro-4-(2-pyridylmethoxy)aniline (obtained as described in PCT Int. Appl. WO 9615118) in 15% yield; NMR Spectrum (CDCl₃) 2.23 - 2.45 (m, 2H), 3.94 - 4.04 (m, 2H), 4.10 (q, 1H), 4.26 (d, 1H), 5.25 (t, 1H), 5.28 (s, 2H), 6.84 (d, 1H), 6.99 (d, 1H), 7.22 (dd, 1H), 7.44 - 7.54 (m, 2H), 7.59 - 7.66 (m, 2H), 7.74 (dt, 1H), 8.11 (d, 1H), 8,60 (d, 1H), 8.64 (s, 1H), 9.90 (s, 1H); Mass spectrum MH⁺ 449.

### Illustrative Example 19

### 4-(3-Chloro-4-(1-cyanomethyl-1H-imidazol-2-ylthio)anilino)-5-(1-methylpiperidin-4-yloxy)quinazoline

To a stirred solution of 4-(3-chloro-4-(1*H*-imidazol-2-ylthio)anilino)-5-(1-methylpiperidin-4-yloxy)quinazoline (**example 12.11**) (460 mg) in acetonitrile (30 ml) was added potassium carbonate (1.0 g), chloroacetonitrile (80 mg) and *cis*-dicyclohexano-18-crown-6 (20 mg) and the solution stirred and heated at reflux for 18 hours. The solution was cooled, filtered and evaporated, and the residue purified by chromatography using ethyl acetate and then ammonia/methanol/DCM (30ml of 2.3N ammonia in methanol, 970ml of DCM) as eluent. Evaporation of the relevant fractions and trituration with ether yielded the title compound as a white solid (160 mg, 31%); NMR spectrum (DMSO-d6, 373K) 1.9 - 2:0 (m, 2H), 2.15 - 2.25 (m, 2H), 2.25 (s, 3H), 2-3 - 2.4 (m, 2H), 2.7 - 2.8 (m, 2H), 4.75 - 4.85 (m, 1H), 5.4 (s, 2H), 7.0 - 7.05 (d, 1H), 7.2 - 7.25 (t, 1H), 7.4 - 7.45 (d, 1H), 7,5 - 7.6 (dd, 1H), 7.6 (s, 1H), 7.7 - 7.8 (t, 1H), 8.3 (d, 1H), 8.6 ((s, 1H), 10.1 (bs, 1H); Mass spectrum MH⁺ 504.

The procedure described above was repeated using the appropriate imidazole and alkyl halide. Thus were obtained the compounds described below:

### Illustrative Example 19.1

### 4-(4-(1-Carbamoylmethyl-1H-imidazol-2-ylthio)-3-chloroanilino)-5-(1-methylpiperidin-4-yloxy)quinazoline

Obtained by reacting 4-(3-chloro-4-(1*H*-imidazol-2-ylthio)anilino)-5-(1-methylpiperidin-4-yloxy)quinazoline (**example 12.11**) with 2-chloroacetamide in 34% yield; NMR spectrum (DMSO-d6, 373K) 1.9 - 2.0 (m, 2H), 2.15 - 2.25 (m, 2H), 2.25 (s, 3H), 2.3 - 2.4 (m, 2H), 2.7 - 2.8 (m, 2H), 4.75 (s, 2H), 4.75 - 4.85 (m, 1H), 7.0 - 7.05 (d, 1H), 7.0 - 7.2 (bs, 2H), 7.2 (s, 1H), 7.2 -7.25 (d, 1H), 7.4 - 7.45 (d, 1H), 7,5 - 7.55 (dd, 1H), 7.7 - 7.8 (t, 1H), 8.25 (d, 1H), 8.6 (s, 1H), 10.1 (bs, 1H); Mass spectrum MH⁺ 522.

### Illustrative Example 19.2

### 4-(3-Chloro-4-(1-(2-methoxyethyl)-1H-imidazol-2-ylthio)anilino)-5-(1-methylpiperidin-4-yloxy)quinazoline

Obtained by reacting 4-(3-chloro-4-(1*H*-imidazol-2-ylthio)anilino)-5-(1-methylpiperidin-4-yloxy)quinazoline (**example 12.11**) with 2-bromoethylmethylether in 61% yield; NMR spectrum (DMSO-d6, 373K) 1.9 - 2.0 (m, 2H), 2.15 - 2.25 (m, 2H), 2.25 (s, 3H), 2.3 - 2.45 (m, 2H), 2.7 - 2.8 (m, 2H), 3.2 (s, 3H), 3.6 - 3.65 (t, 2H), 4.2 - 4.25 (t, 2H), 4.75 - 4.85 (m, 1H), 6.9 - 6.95 (d, 1H), 7.1 (s, 1H), 7.2 - 7.25 (d, 1H), 7.4 - 7.45 (d, 1H), 7.45 (s, 1H), 7.5 - 7.55 (dd, 1H), 7.7 - 7.8 (t, 1H), 8.2 (s, 1H), 8.55 (s, 1H), 10.1 (bs, 1H); Mass spectrum MH⁺ 525.

### Illustrative Example 19.3

### 4-(3-Chloro-4-(1-(N,N-diethylcarbamoylmethyl)-1H-imidazol-2-ylthio)anilino)-5-(1-methylpiperidin-4-yloxy)quinazoline

Obtained by reacting 4-(3-chloro-4-(1*H*-imidazol-2-ylthio)anilino)-5-(1-methylpipeddin-4-yloxy)quinazoline (**example 12.11**) with 2-chloro-N,N-diethylacetamide in 28% yield; NMR spectrum (DNMO-d6, 373K) 1.0 - 1.25 (bs, 6H), 1.9 - 2.0 (m, 2H), 2.15 - 2.25 (m, 2H), 2.25 (s, 3H), 2.3 - 2.45 (m, 2H), 2.7 - 2.8 (m, 2H), 3.3 - 3.4 (q, 4H), 4.75 - 4.85 (m, 1H), 5.0 (s, 1H), 6.95 - 7.0 (d, 1H), 7.15 (s, 1H), 7.2 - 7.25 (d, 1H), 7.3 - 7.35 (d, 1H), 7.35 (s, 1H), 7.45 - 7.5 (dd, 1H), 7.7 - 7.8 (t, 1H), 8.2 (s, 1H), 8.55 (s, 1H), 10.1(bs, 1H); Mass spectrum MH⁺ 578.

### Illustrative Example 19.4

### 4-(4-(1-tert-Butoxycarbonylmethyl -1H-imidazol-2-ylthio)-3-chloroanilino)-5-(1-methylpiperidin-4-yloxyl)quinazoline

Obtained by reacting 4-(3-chloro-4-(1*H*-imidazol-2-ylthio)anilino)-5-(1-methylpiperidin-4-yloxy)quinazoline (**example 12.11**) with tert-butyl 2-bromo-acetatein 44% yield; Mass spectrum MH⁺ 581.

### Illustrative Example 19.5

### 4-(3-Chloro-4-(1-difluoromethyl-1H-imidazol-2-ylthio)anilino)-5-(1-methylpiperidin-4-yloxy)quinazoline

Obtained by reacting 4-(3-chloro-4-(1*H*-imidazol-2-ylthio)anilino)-5-(1-methylpiperidin-4-yloxy)quinazoline (**example 12.11**) with ethyl difluorobromoacetate in 34% yield; NMR spectrum (DMSO-d6, 373K) 1.95 - 2.05 (m, 2H), 2.15 - 2.25 (m, 2H), 2.3 (s, 3H), 2.3 - 2.45 (m, 2H), 2.7 - 2.85 (m, 2H), 4.75 - 4.85 (m, 1H), 7.15 - 7.2 (d, 1H), 7.2 - 7.3 (d, 1H), 7.3 (s, 1H), 7.4 - 7.45 (d, 1H), 7.6 - 7.65 (dd, 1H), 7.7 - 8.0 (q,1H), 7.7 - 7.8 (t, 1H), 8.3 (s, 1H). 8.65 (s, 1H), 10.0-10.2 (bs, 1H); Mass spectrum MH⁺ 517.

### Illustrative Example 19.6

### 4-(4-(1-Cyanomethyl-1H-imidazol-2-ylthio)-3-fluoroanilino)-5-(1-methylpiperidin-4-yloxy)quinazoline

Obtained by reacting 4-(3-fluoro-4-(1*H*-imidazol-2-ylthio)anilino)-5-(1-methylpiperidin-4-yloxy)quinazoline **(example 12.12)** with chloroacetonitrile in 40% yield; NMR spectrum (DMSO-d6, 373K) 1.9 - 2.0 (m, 2H), 2.15 - 2.25 (m, 2H), 2.2 (s, 3H), 2.25 - 2.35 (m, 2H), 2.6 - 2.7 (m, 2H), 4.7 - 4.8 (m, 1H), 5.35 (s, 2H), 7.1 (s, 1H), 7.2 - 7.25 (d, 1H), 7.25 - 7.3 (t, 1H), 7.4 - 7.42 (d. 1H), 7.42 - 7.45 (dd, 1H), 7.5 (s, 1H), 7.7 - 7.8 (t, 1H), 8.0-8.1 (dd, 1H), 8.6 (s, 1H), 10.2 (bs, 1H); Mass spectrum MH⁺ 490.

### Illustrative Example 20

### 4-(4-(1-Carboxymethyl-1H-imidazol-2-ylthio)-3-chloroanilino)-5-(1-methylpiperidin-4-yloxy)quinazoline dihydrochloride.

A solution of 4-(3-chloro-4-(1-*tert*-butoxycarbonylmethyl-1*H*-imidazol-2-ylthio)anilino-5-(1-methylpiperidin-4-yloxy)quinazoline **(example 19.4)** (100 mg) in dioxane (20 ml) was treated with an ethereal solution of hydrogen chloride (3 ml, 1M), and heated at reflux for 4 hours. The solution was cooled to give a precipitate that was filtered and washed with ether to give the title compound as a white solid (98 mg, 95%); NMR spectrum (DMSO-d6, 373K) 2.3 - 2.5 (m, 2H), 2.5 - 2.6 (m, 2H), 2.8 (s, 3H), 3.1- 3.4 (bs, 2H), 3.4 - 3.7 (bs, 2H), 4.9 (s, 2H), 5.0 - 5.2 (m, 1H), 7.15 - 7.2 (d, 1H), 7.3 (s, 1H), 7.45 - 7.5 (d,1H), 7.6 (s, 1H), 7.6 - 7.7 (m, 1H), 7.9 - 8.0 (t, 1H), 8.2 - 8.3 (bs, 1H), 8.8 (s, 1H), 10.2-10.5 (bs, 1H), 10.9-11.5 (bs, 1H); Mass spectrum MH⁺ 523.

### Example 21

### 5-(1-Methylpiperidin-4-yloxy)-4-(4-(thiazol-ylthio)anilino)quinazoline

To a solution of 4-(4-iodoanilino)-5-(1-methylpiperidin-4-yloxy)quinazoline **(reference example 4.6)** (229 mg) in anhydrous and degassed toluene (3 ml) in a pressure vial was added 2-mercaptothiazole (114 mg), cuprous bromide (15 mg) and 1,8-diazabicyclo[5,4,0]undec-7-ene (152 mg). The solution was purged with nitrogen and the vial capped and then heated at 118°C for 18 hours. The contents of the vessel were purified by chromatography using ammonia/methanol/DCM (30 ml of 2.3N ammonia in methanol, 970 ml of DCM) as eluent to give the title compound as a solid (195 mg, 87%); NMR spectrum (DMSO-d6, 373K) 1.5-1.7 (m, 2H), 2.1 - 2.2 (m, 1H), 2.2 (s, 3H), 2.2 - 2.4 (m, 1H), 2.6 - 2.65 (m, 1H), 2.7 - 2.8 (m, 1H), 3.4 - 3.5 (t, 1H), 3.5 - 3.6 (d,1H), 4.7 - 4.9 (1H), 7.25 - 7.3 (d, 1H), 7.35 - 7.4 (d, 1H), 7.6 (s, 1H), 7.65 - 7.8 (m, 4H), 7.95 - 8.0 (d, 2H), 8.6 (s, 1H), 10.3 10.35 (bs, 1H); Mass spectrum MH⁺ 450.

### Illustrative Example 22

### 5-(1-Methylpiperidin-4-yloxy)-4-(4-(2-thiazolylsulphonyl)anilino)quinazoline

To a solution of 4-chloro-5-(1-methylpiperidin4-yloxy)quinazoline **(reference example 2)** (80 mg) and 4-thiazol-2-ylsulphonylaniline **(reference example 8.6)** (140 mg) in anhydrous THF was added sodium hydride (100 mg, 40% dispersion in oil). The solution was stirred and heated at reflux overnight. The reaction was concentrated and the residue purified by chromatograph using ethyl acetate and then a mixture of 3% 2.3N ammonia in methanol in DCM as eluent to give the title compound as a white solid (40 mg, 28%); NMR spectrum (DMSO-d6, 373K) 1.9 - 2.0 (m, 2H), 2.15 - 2.25 (m, 2H), 2.25 (s, 3H), 2.3 - 2.4 (m, 2H), 2.7 - 2.8 (m, 2H), 4.75 - 4.85 (m, 1H), 7.2 - 7.3 (d, 1H), 7.4 - 7.5 (d, 1H), 7.7 - 7.8 (t, 1H), 8.0 - 8.1 (m, 3H), 8.1 - 8.2 (m, 3H), 8.65 (s, 1H), 10.4 (bs, 1H); Mass spectrum MH⁺ 482.

### Illustrative Example 23

### 4-(3-Chloro-4-(3-fluorobenzyloxy)aniline)-5-(1-(4-methylpiperazin-1-yl)cyclohex-4-yloxy)quinazoline

Sodium triacetoxyborohydride (0.43 g) was added to a solution of 4-(3-chloro-(3-fluorobenzyloxy)aniline)-5-(1-oxo-cyclohex-4-yloxy)quinazoline acetate **(reference example 47)** (0.1 g) and 1-methylpiperazine (0.34 ml) in DCE (25 ml) and the reaction stirred for 16 hours. The solution was concentrated *in vacuo* and the residue partitioned between DCM and saturated aqueous sodium hydrogen carbonate solution. Combined organic extracts were dried and concentrated and the residue purified by chromatography using DCM - 5% ammonia (7N) in methanol as eluent to give the title compound as a white solid (24 mg, 23%); Mass Spectrum M⁺ 576.

### Illustrative Example 24

### 4-(3-Chloro-4-(1,2,3-thiadiazol-4-ylmethoxy)anilino)-5-(1-methylpiperidin-4-yloxy)quinazoline

4-Hydroxymethyl-1,2,3-thiadiazole (obtained as described in Example 1 of European Patent Appl. EP 0326640) (81 mg) was dissolved in DCM (5 ml). Di-*iso*-propylethylamine (122 µl) was added, and the solution cooled to 0°C. Methanesulphonyl chloride (54 µl) was added dropwise; the solution was allowed to warm to ambient temperature, and was stirred for a further 2 hours. The solvent was evaporated, and the residue dissolved in DMA (10 ml). Potassium carbonate (552 mg) was added, followed by 4-(3-chloro-4-hydroxyanilino)-5-(1-methylpiperidin-4-yloxy)quinazoline **(reference example 4.2)** (150 mg). The mixture was briefly sonicated, then stirred at ambient temperature for 16 hours. The solvent was evaporated, and the residue partitioned between DCM and water. Combined organic extracts were filtered through a silicone-treated filter paper and evaporated. The residue was purified by chromatography, using 0 to 2% (7:1 methanol /*c*. ammonia (aq)) in DCM as eluent. Evaporation of the appropriate fractions followed by crystallisation of the residue from ethyl acetate *iso*-hexane gave the title compound as a white crystalline solid (50 mg, 26%);.NMR Spectrum (DMSO-d6) 1.88 -1.98 (m,2H), 2.12 - 2.22 (m, 2H), 2.19 (s, 3H), 2.25 - 2.33 (m, 2H), 2.61- 2.69 (m, 2H), 4.81 (m, 1H), 5.72 (s, 2H), 7.24 (d, 1H), 7.35 (d, 1H), 7.45 (d, 1H), 7.57 (dd, 1H), 7.74 (dd, 1H), 8.15 (d, 1H), 8.54 (s, 1H), 9.32 (s. 1H), 10.09 (s, 1H); Mass Spectrum MH⁺ 483.4.

### Illustrative Example 25

### 4-(3-Chloro-4-(2-pyridylmethoxy)anilino)-5-(2-piperidinoethoxy)quinazoline

4-(3-Chloro-4-(2-pyridylmethoxy)anilino)-5-fluoroquinazoline **(reference example 4.8)** (76 mg) and 2-piperidinolethanol (30 mg) were dissolved in 1,4-dioxane, (5 ml) in a 10my pressure vessel and sodium hydride (40 mg, 60% dispersion in oil) added. The vessel was sealed and heated at 150°C for 20 minutes, using the "Discover^{™} microwave synthesis system" (CEM Microwave technology Ltd). The reaction was cooled and pressure released and vessel uncapped. The solution purified by chromatography using 2.3 M ammonia/methanol in DCM (3:97) as eluent to give the title compound as a white solid after trituration with ether (25 mg, 26%); NMR spectrum (DMSO-d6, 373K) 1.3 - 1.4 (m, 2H), 1.4 -1.5 (m, 4H), 2.4 - 2.5 (m, 4H), 2.8 - 2.85 (t, 2H), 4.35 - 4.45 (t, 2H), 5.3 (s, 2H), 7.1 (d, 1H), 7.2 (d, 1H), 7.3 - 7.35 (m, 1H), 7.35 (d, 1H), 7.55 (d, 1H), 7.65 7.75 (m, 2H), 7.8 - 7.9 (m, 1H), 7.95 (s, 1H), 8.5 (s, 1H), 8.6 (d, 1H), 10.15 - 10.2 (bs, 1H); Mass Spectrum MH⁺ 491.

The procedure described above was repeated using the appropriate alcohol and the 5-fluoroquinazoline. Thus were obtained the compounds described below:

### Illustrative Example 25.1

### 4-(3-Chloro-4-(2-pyridylmethoxy)anilino)-5-(1-methylpiperidin-2-ylmethoxy)quinazoline

Obtained by reacting (1-methylpiperidin-2-yl)methanol with 4-(3-chloro-4- (2-pyridylmethoxy)anilino)-5-fluoroquinazoline **(reference example 4.8)** in 23% yield; NMR spectrum (DMSO-d6, 373K) 1.3 -1.5 (m, 2H), 1.5 - -1.6(m, 1H), 1.6 -1.7 (m, 1H), 1.7 - 1.8 (m, 2H), 2.2 - 2.3 (m,1H), 2.35 (s, 3H), 4.2 (d, 1H), 4.5 (d, 1H), 5.3 (s, 2H), 7.1 (d, 1H), 7.3 (d, 1H), 7.3 (m, 1H), 7.35 (d, 1H), 7.5 (d, 1H), 7.7 - 7.8 (t, 1H), 7.85 - 7.95 (m, 2H), 8.0 (s. 1H), 8.5 (s, 1H), 8.6 (d, 1H), 10.3 -10.4 (bs, 1H); Mass Spectrum MH⁺ 491.

### Illustrative Example 25.2

### 4-(3-Chloro-4-(2-pyridylmethoxy)anilino-5-(2-azepan-1-ylethoxy)quinazoline

Obtained by reacting 2-azepan-1-ylethanol with 4-(3-chloro-4-(2-pyridylmethoxy)anilino)-5-fluoroquinazoline **(reference example 4.8)** in 46% yield; NMR spectrum (DMSO-d6, 373K) 1.4 -1.5 (m, 6H).1.5 - 1.6 (m, 2H). 2.7 - 2.8 (m, 2H), 3.05 - 3.1 (t, 2H), 4.35 - 4.4 (t, 2H), 5.3 (s, 2H, 7.1 (d, 1H), 7.2 (d, 1H), 7.3 - 7.35 (m, 1H), 7.35 (d, 1H), 7.6 (d, 1H), 7.7 (dd, 1H), 7.75 (t, 1H), 7.8 - 7.9 (t, 1H), 7.95 (s, 1H), 8.5 (s, 1H), 8.6 (d, 1H), 10.2 (bs, 1H); Mass Spectrum MH⁺ 505.

### Illustrative Example 25.3

### 4-(3-Chloro-4-(-2-pyridylmethoxy))anilino-5-(2-morpholinoethoxy)quinazoline

Obtained by reacting 2-morpholinoethanol with 4-(3-chloro-4-(2-pyridylmethoxyanilino)-5-fluoroquinazoline **(reference example 4.8)** in 42% yield; NMR spectrum (DMSO-d6, 373K) 2.8 - 2.9 (m, 6H), 3.3 - 3.4 (m, 4H), 4.4 (t, 2H), 5.3 (s; 2H), 7.1 (d, 1H), 7.25 (d, 1H), 7.3 - 7.35 (m, 1H), 7.35 (d, 1H), 7.6 (d, 1H), 7.7 (dd, 1H), 7.75 (t, 1H), 7.8-7.9 (t,1H), 7.95 (s,1H), 8.5 (s, 1H), 8.6 (d, 1H), 10.2 (bs, 1H); Mass Spectrum MH⁺ 493.

### Illustrative Example 25.4

### 4-(3-Chloro-4-(2-pyridylmethoxy)anilino-5-(2-pyrrolidinoethoxy)quinazoline

Obtained by reacting 2-pyrrolidinoethanol with 4-(3-chlo-4- (2-pyridylmethoxy)anilino)-5-fluoroquinazoline **(reference example 4.8)** in 44% yield; NMR spectrum (DMSO-d6,373K) 1.6 - 1.7 (m, 4H), 2.6 - 2.7 (m, 4H), 3.0 (t, 2H), 4.4 (t, 2H), 5.3 (s. 2H), 7.1 (d, 1H), 7.2 (d, 1H), 7.3 - 7.35 (m, 1H), 7.35 (d, 1H), 7.6 (d, 1H), 7.65 - 7.75 (m, 2H), 7.8 - 7.9 (t, 1H), 7.95 (s, 1H), 8.5 (s, 1H), 8.6 (d, 1H), 10.3 (bs, 1H); Mass Spectrum MH⁺ 475 .

### Illustrative Example 25.5

### 4-(3-Chloro-4-(2-pyridylmethoxy)anilino-5-(3-morpholinopropoxy)quinazoline

Obtained by reacting 3-morpholinopropanol with 4-(3-chloro4-(2-pyridylmethoxy)anilino)-5-fluoroquinazoline **(reference example 4.8)** in 15% yield; NMR spectrum (DMSO-d6, 373K) 2.1- 2.2 (m, 2H), 2.3 - 2.4 (m, 4H), 2.5 - 2.6 (m, 2H), 3.5 - 3.6 (m, 4H), 4.4 (t, 2H), 5.3 (s, 2H), 7.1 (d, 1H), 7.25 (d, 1H), 7.3 -7.35 (m, 1H). 7.35 (d; 1H), 7.5 - 7.6(m, 2H), 7.75 (t, 1H), 7.8 - 7.9 (t, 1H), 8.05 (s, 1H), 8.5 (s, 1H), 8.6 (d, 1H), 9.9 (bs, 1H); Mass Spectrum MH⁺ 507.

### Illustrative Example 25.6

### 4-(3-Chloro-4-(2-pyridylmethoxy)anilino-5-(3-(4-methylpiperazin-1-yl)propoxy)quinazoline

Obtained by reacting 3-(4-methylpiperazin-1-yl)propanol with 4-(3-chloro-4-( 2-pyridylmethoxyanilino)-5-fluoroquinazoline **(reference example 4.8)** in 13% yield; NMR spectrum (DMSO-d6, 373K) 2.1- 2.2 (m, 2H), 2.2 (s, 3H). 2.25 - 2.35 (m, 4H), 2.35 - 2.45 (m, 4H), 2.5 - 2.6 (m, 2H), 4.4 - 4.45 (t, 4H), 5.3 (s, 2H), 7.1 (d, 1H), 7.25 (d, 1H), 7.3 - 7.35 (m, 1H), 7.35 (d, 1H), 7.55 - 7.65 (m, 2H), 7.7 7.75 (t. 1H), 7.8 - 7.85 (t, 1H), 8.05 (d, 1H), 8.5 (s, 1H), 8.6 (d, 1H), 9.9 (bs, 1H); Mass Spectrum MH⁺ 520.

### Illustrative Example 25.7

### 4-(3-Chloro-4-(3-fluorobenzyloxy)anilino)-5-(1-methylpiperidin-2-ylmethoxy)quinazoline

Obtained by reacting (1-methylpiperidin-2-yl)methanol with 4-(3-chloro-4-(3-fluorobenzyloxy)anilino)-5-fluoroquinazoline **(reference example 33)** in 17% yield; NMR spectrum (DMSO-d6, 373K) 1.3 -1.6 (m, 4H), 1.6 - 1.8 (m, 2H), 2.2 - 2.3 (dt, 1H), 2.35 (s, 3H), 2.4 - 2.6 (m,2H), 4.2 (dd, 1H), 4.5 (dd, 1H), 5.25 (s, 1H), 7.1 (d, 1H), 7.1- 7.15 (m, 1H), 7.3 (m,1H), 7.25 - 7.4 (m, 3H), 7.4 - 7.5 (m, 1H), 7.7 - 7.8 (t, 1H), 7.9 - 7.95 (dd, 1H), 8.0 (d, 1H), 8.5 (s. 1H), 10.4 (bs, 1H); Mass Spectrum MH⁺ 508.

### Illustrative Example 25.8

### 4-(3-Chloro-4-(3-fluorobenzyloxy)anilino)-5-(2-pyrrolidinoethoxy)quinazoline

Obtained by reacting 2-pyrrolidinoethanol with 4-(3-chloro-4-(3-fluorobenzyloxy)anilino-5-fluoroquinazoline **(reference example 33)** in 22% yield; NMR spectrum (DMSO-d6, 373K) 1.6 -1.7 (m, 4H), 2.6 - 2.7 (m, 4H), 3.0 - 3.1 (m, 2H), 4.4 - 4.5 (m, 2H), 5.3 (s, 2H), 7.1-7.2 (m, 2H), 7.3 (d, 1H), 7.3 - 7.4 (m, 3H), 7.45 - 7.5 (m, 1H), 7.7 - 7.8 (m, 2H), 8.0 (d, 1H), 8.5 (s. 1H) 10.3 (bs, 1H); Mass Spectrum MH⁺ 493.

### Illustrative Example 25.9

### 4-(3-Chloro-4-(3-fluorobenzyloxy)anilino)-5-(3-morpholinopropoxy)quinazoline

Obtained by reacting 3-morpholinopropanol with 4-(3-chloro-4-(3-fluorobenzyloxy)anilino)-5-fluoroquinazoline **(reference example 33)** in 22% yield; NMR spectrum (DMSO-d6, 373K) 2.1- 2.2 (m, 2H), 2.4 - 2.45 (m, 4H), 2.5 - 2.6 (m, 2H), 3.55 - 3.6 . (m, 4H), 4.4 - 4.45 (t, 2H), 5.3 (s, 2H), 7.1 - 7.2 (m, 2H), 7.3 (d, 1H), 7.3 - 7:4 (m, 3H), 7.45 - 7.5 (m. 1H), 7.6 - 7.7 (m, 1H), 7.8 - 7.9 (t, 1H), 8.0 (d, 1H), 8.55 (s, 1H) 10.0 (bs, 1H); Mass Spectrum MH⁺ 524.

### Illustrative Example 25.10

### 4-(3-Chloro-4-(3-fluorobenzyloxy)anilino)-5-[2-(1-methylpyrrolidin-2-yl)ethoxy]quinazoline

Obtained by reacting 2-(1-methylpyrolidin-2-yl)ethanol with 4-(3-chloro-4-(3-fluorobenzyloxy)anilino)-5-fluoroquinazoline **(reference example 33)** in 22% yield; Mass Spectrum MH⁺ 508.

### Illustrative Example 25.11

### 4-(3-Chloro-4-(3-fluorobenzyloxy)anilino)-5-(2-morpholinoethoxy)quinazoline

Obtained by reacting 2-morpholinoethanol with 4-(3-chloro-4-(3-fluorobenzyloxy)anilino)-5-fluoroquinazoline **(reference example 33)** in 25% yield; NMR spectrum (DMSO-d6, 373K) 2.5 - 2.6 (m, 4H), 2.9 - 3.0 (m, 2H), 3.5 - 3.6 (m, 4H), 4.4 - 4.5 (m, 2H), 5.3 (s, 2H), 7.1 - 7.2 (m, 2H), 7.3 (d, 1H). 7.3 - 7.35 (m, 3H), 7.35 - 7.4 (m, 1H). 7.7 - 7.8 (m, 2H), 8.0 (d, 1H), 8.5 (s, 1H), 10.2 (bs, 1H); Mass Spectrum MH⁺ 510.

### Illustrative Example 25.12

### 4-(3-Chloro-4-(3-fluorobenzyloxy)anilino)-5-(3-(4-methylpiperazin-1 yl)propoxy)quinazoline

Obtained by reacting 3-(4-methylpiperazin-1-yl)propanol with 4-(3-chloro-4-(3-fluorobenzyloxy)anilino)-5-fluoroquinazoline **(reference example 33)** in 13% yield; Mass Spectrum MH⁺ 537.

### Example 26

The compounds in Table 1 were prepared as follows:
A stock solution of 4-(3-chloro-4-hydroxyanilino)-5-(1-methylpiperidin-4-yloxy)quinazoline **(reference example 4.2)** (335 mg) in DMF (7.92 ml) was prepared, and 100 µl aliquots transferred to a 96 well plate. Using a resin loader, potassium carbonate (300mg) was distributed evenly across the plate. An appropriate benzyl or heteroarylmethyl chloride (0.11 mM) were dissolved in DMF (1 ml) and 100 µl of each solution transferred to the plate. The plate was agitated at 25°C for 16 hours. To each well was added DMF (100 µl) and trisamine scavenger resin (726 mg) by using the resin loader to distribute evenly across the wells. The plate was agitated for 3 hours at 25°C. Each well was filtered to remove resin and inorganic material and the remaining filtrates were concentrated *in vacuo.* DMSO (550µl) was added to each well and aliquots of 50 µl were then taken from each well for LCMS purity determination. LCMS purity was determined on a Phenomenex Synergi column (reverse phase silica, 50 x 2 mm, flow rate 1.1 ml/minute), eluting with acetonitrile-water containing formic acid (0.05%) on a gradient from 5-95% over 4.5 minutes, with UV detection at 254 nm. There was thus obtained the compound shown in bold in Table 1.

**Table 1**

| In Table 1 EG refers to Example, RT refers to the LCMS retention time (minutes) | | | |
|---|---|---|---|
| **EG** | **Compound** | ***M*-H⁺** | **RT** |
| 26.1 | **4-(3-Chloro-4-(2,6-dichlorobenzyloxy)anilino)-5-(1-methylpiperidin-4-yloxy)quinazoline** Obtained by reaction with 2,6-dichlorobenzyl chloride | 542 | 1.40 |
| 26.2 | **4-(3-Chloro-4-(4-fluorobenzyloxy)anilino)-5-(1-methylpiperidin-4-yloxy)quinazoline** Obtained by reaction with 4-fluorobenzyl chloride | 492 | 1.30 |
| 26.3 | **4-(3-Chloro-4-(3-nitrobenzyloxy)anilino)-5-(1-methylpiperidin-4-yloxy)quinazoline** Obtained by reaction with 3-nitrobenzyl chloride | 519 | 1.29 |
| 26.4 | **4-(3-Chloro-4-(3-pyridylmethoxy)anilino)-5-(1-methylpiperidin-4-yloxy)quinazoline** Obtained by reaction with 3-picolyl chloride | 475 | 0.81 |
| 26.5 | **4-(4-(Benzo(1,3)dioxol-5-ylmethoxy)-3-chloroanilino)-5-(1-methylpiperidin-4-yloxy)quinazoline** Obtained by reaction with benzo(1,3)dioxol-5-ylmethyl chloride | 518 | 1.26 |
| 26.6 | **4-(3-Chloro-4-(2-methoxybenzyloxy)anilino)-5-(1-methylpiperidin-4-yloxy)quinazoline** Obtained by reaction with 2-methoxybenzyl chloride | 504 | 1.31 |
| 26.7 | **4-(3-Chloro-4-(5-methylisoxazol-3-ylmethoxy)anilino)-5-(1-methylpiperidin-4-yloxy)quinazoline** Obtained by reaction with 5-methylisoxazol-3-ylmethyl chloride | 479 | 1.05 |
| 26.8 | **4-(3-Chloro-4-(2-chlorobenzyloxy)anilino)-5-(1-methylpiperidin-4-yloxy)quinazoline** Obtained by reaction with 2-chlorobenzyl chloride | 508 | 1.37 |
| 26.9 | **4-(3-Chloro-4-(2-chloro-6-fluorobenzyloxy)anilino)-5-(1-methylpiperidin4-yloxy)quinazoline** Obtained by reaction with 2-chloro-6-fluorobenzyl chloride | 526 | 1.34 |
| 26.10 | **4-(3-Chloro-4-(2,5-dimethylbenzyloxy)anilino)-5-(1-methylpiperidin-4-yloxy)quinazoline** Obtained by reaction with 2, 5-dimethylbenzyl chloride | 503 (*M*⁺) | 1.45 |
| 26.11 | **4-(3-Chloro4-(3-methoxybenzyloxy)anilino)-5-(1-methylpiperidin-4-yloxy)quinazoline** Obtained by reaction with 3-methoxybenzyl chloride | 504 | 1.26 |
| 26.12 | **4-(3-Chloro-4-(2-nitrobenzyloxy)anilino)-5-(1-methylpiperidin-4-yloxy)quinazoline** Obtained by reaction with 2-nitrobenzyl chloride | 519 | 1.27 |
| 26.13 | **4-(3-Chloro-4-(4-pyridylmethoxy)anilino)-5-(1-methylpiperidin-4-yloxy)quinazoline** Obtained by reaction with 4-picolyl chloride | 474 | 0.74 |
| 26.14 | **4-(3-Chloro-4-(2,6-difluorobenzyloxy)anilino)-5-(1-methylpiperidin-4-yloxy)quinazoline** Obtained by reaction with 2,6-difluorobenzyl chloride | 509 | 1.27 |
| 26.15 | **4-(3-Chloro-4-(2-fluorobenzyloxy)anilino)-5-(1-methylpiperidin-4-yloxy)quinazoline** Obtained by reaction with 2-fluorobenzyl chloride | 492 | 1.28 |
| 26.16 | **4-(3-Chloro-4-(3-chlorobenzyloxy)anilino)-5-(1-methylpiperidin-4-yloxy)quinazoline** Obtained by reaction with 3-chlorobenzyl chloride | 508 | 1.39 |
| 26.17 | **4-(3-Chloro-4-(3-methylbenzyloxy)anilino)-5-(1-methylpiperidin-4-yloxy)quinazoline** Obtained by reaction with 3-methylbenzyl chloride | 488 | 1.37 |
| 26.18 | **4-(3-Chloro-4-(5-chlorothiophen-2-ylmethoxy)anilino)-5-(1-methylpiperidin-4-yloxy)quinazoline** Obtained by reaction with 5-chlorothiophen-2-ylmethyl chloride | 514 | 1.39 |
| 26.19 | **4-(3-Chloro-4-(2-cyanobenzyloxy)anilino)-5-(1-methylpiperidin-4-yloxy)quinazoline** Obtained by reaction with 2-cyanobenzyl chloride | 500 (*M*⁺) | 1.20 |
| 26.20 | **4-(3-Chloro-4-(2-methylthiazol-4-ylmethoxy)anilino)-5-(1-methylpiperidin-4-yloxy)quinazoline** Obtained by reaction with 2-methylthiazol-4-ylmethyl chloride | 495 | 1.06 |
| 26.21 | **4-(3-Chloro-4-(4-methyl-2-nitrobenzyloxy)anilino)-5-(1-methylpiperidin-4-yloxy)quinazoline** Obtained by reaction with 4-methyl-2-nitrobenzyl chloride | 533 | 1.37 |
| 26.22 | **4-(3-Chloro-4-(thiazol-4-ylmethoxy)anilino)-5-(1-methylpiperidin-4-yloxy)quinazoline** Obtained by reaction with 4-chloromethylthiazole | 481 | 0.97 |
| 26.23 | **4-(3-Chloro-4-(6-chloropyrid-3-ylmethoxy)anilino)-5-(1-methylpiperidin-4-yloxy)quinazoline** Obtained by reaction with 6-chloropyrid-3-ylmethyl chloride | 509 | 1.13 |

### Example 27

### Pharmaceutical composition

The following illustrates a representative pharmaceutical dosage forms of the invention as defined herein (the active ingredient being termed "Compound X"), for therapeutic or prophylactic use in humans:

| (a) | Tablet I | mg/tablet |
|---|---|---|
| | Compound X | 100 |
| | LactosePh.Eur | 182.75 |
| | Croscarmellose sodium | 12.0 |
| | Maize starch paste (5% w/v paste) | 2.25 |
| | Magnesium stearate | 3.0 |

| (b) | Injection I | (50 mg/ml) |
|---|---|---|
| | Compound X | 5.0% w/v |
| | 1M Sodium hydroxide solution | 15.0% v/v |
| | 0.1M Hydrochloric acid (to adjust pH to 7.6) | |
| | Polyethylene glycol 400 | 4.5% w/v |
| | Water for injection to 100%. | |

The above formulations may be obtained by conventional procedures well known in the pharmaceutical art. For example the tablet may be prepared by blending the components together and compressing the mixture into a tablet.

### Starting Materials

The starting materials used in the examples were prepared as follows.

### Reference Example 1

### 3,4-Dihydro-5-(1-methylpiperidin-4-yloxy)quinazolin-4-one

Sodium hydride (4.1 g, 60%) was added in portions to 4-hydroxy-1-methylpiperidine (10.7 g) in DMA (125 ml). The reaction was stirred at room temperature for 15 minutes, 50°C for 15 minutes then allowed to cool to room temperature. 5-Fluoro-3,4-dihydroquinazolin-4-one (5.1 g) was added in a single portion, and the mixture heated at 80°C for 2 hours. The reaction was cooled, concentrated *in vacuo* and the residue dissolved in methanol. DOWEX H⁺ ion exchange resin (75 g) was added and the mixture was stirred at room temperature for 1 hour. The mixture was then filtered and the resin washed with methanol. The resin was then suspended in ammonia (7N solution in methanol) and this mixture was stirred at room temperature for 1 hour. The mixture was then filtered, and the filtrate was concentrated *in vacuo* to give the title compound as a white solid after trituration with ether (7.3 g, 91%); NMR spectrum (DMSO-d6) 1.72 (m, 2H), 1.88 (m, 2H), 2.15 (s, 3H), 2.19 (m, 2H), 2.63 (m, 2H), 4.46 (m, 1H), 7.00 (d, 1H), 7.14 (d, 1H), 7.61 (t, 1H), 7.91 (s, 1H), 11.75 (bs, 1H).

The procedure described above was repeated using the appropriate alcohol. Thus was obtained the compound described below:

### Reference Example 1.1

### 1-(tert-Butoxycarbonylpiperidin-4-yloxy)-3,4-dihydroquinazolin-4-one

Obtained from 1-(*tert*-butoxycarbonyl-4-hydroxypiperidine in 87% yield; NMR spectrum (DMSO-d6) 1.39 (s, 9H), 1.6 - 1.87 (m, 4H), 3.32 - 3.43 (m, 2H), 3.47 - 3.60 (m, 2H), 4.75 (m, 1H), 7.08 (d, 1H), 7.17 (d, 1H), 7.64 (t, 1H) 8.84 (s, 1H), 11.80 (bs, 1H); Mass spectrum MH⁺ 346.

### Reference Example 1.2

### 3,4-Dihydro-5-(tetrahydropyran-4-yloxy)quinazolin-4-one

Obtained from tetrahydropyran-4-ol in 27% yield; NMR Spectrum (DMSO-d6) 1.61 - 1.78 (m, 2H), 1.84 - 2.00 (m, 2H), 3.42 - 3.56 (m, 2H), 3.85 - 3.97 (m, 2H), 4.65 - 4.78 (m, 1H), 7.04 (d, 1H), 7.18 (d, 1H), 7.61 (t, 1H), 7.93 (s, 1H); Mass spectrum MH⁺ 247.

### Reference Example 1.3

### 3,4-Dihydro-5-(tetrahydrofuran-3-yloxy)quinazolin-4-one

Obtained from tetrahydrofuran-3-ol in 71% yield; NMR Spectrum (DMSO-d6) 1.98 - 2.08 (m, 1H), 2.16 - 2.26 (m, 1H), 3.75 - 3.98 (m, 4H), 5.02 - 5.10 (m, 1H), 6.96 (d, 1H), 7.18 (d, 1H), 7.61 (t, 1H), 7.93 (s, 1H), 11.8 (s, 1H); Mass spectrum MH⁺ 233.

### Reference Example 1.4

### 3,4-Dihydro-5-(1,4-dioxaspiro[4.5]-dec-8-yloxy)quinazolin-4-one

Obtained from 1, 4-dioxaspiro[4.5]decan-8-ol **(reference example 46)** in 45 yield; NMR Spectrum (DMSO-d6) 1.48 (m, 2H), 1.70 - 2.00 (m, 6H), 3.85 (s, 4H), 4.63 (m, 1H), 7.03 (d, 1H), 7.14 (d, 1H), 7.60 (t, 1H), 7.90 (s, 1H), 11.74 (bs, 1H); Mass Spectrum M-H⁺ 301.

### Reference Example 2

### 4-Chloro-5-(1-methylpiperidin-4-yloxy)quinazoline

Phosphorus oxychloride (3.59 ml) was added to a solution of 5-(1-methylpiperidin-4-yloxy)-3,4-dihydroquinazolin-4-one (1.0 g) **(Reference Example 1)** and di-*iso-*propylethylamine (2.01 ml) in DCM (70 ml), and the resulting solution heated at reflux for 16 hours. The reaction was cooled and concentrated *in vacuo* and the residue dissolved in ethyl acetate and cooled to 0°C. Cold saturated aqueous sodium hydrogen carbonate solution was added and the two-phase mixture stirred at 0°C for 15 minutes. The organic layer was separated, dried and concentrated *in vacuo* to yield the title compound as a yellow solid (0.665 g, 62%); NMR spectrum (CDCl₃) 2.10 (m, 2H), 2.23 (m, 2H), 2.42 (s, 3H), 2.60 (m, 2H), 2.84 (m, 2H), 4.73 (m, 1H), 7.04 (d, 1H), 7.62 (d, 1H), 7.81 (t, 1H), 8.93 (s, 1H); Mass spectrum M⁺ 278.

The procedure described above was repeated using the appropriate 3,4-dihydroquinazolin-4-one. Thus was obtained the compound described below:

### Reference Example 2.1

### 5-(1-tert-Butloxycarbonylpiperidin-4-yloxy)-4-chloroquinazoline

Obtained from 5-(1-*tert*-butoxycarbonylpiperidin-4-yloxy)-3,4-dihydroquinazoline **(reference example 1.1)** in 66% yield; NMR spectrum (DMSO-d6) 1.38 (s, 9H), 1.58 - 1.90 (m, 4H), 3.30 - 3.60 (m, 4H), 4.82 (m, 1H), 7.14 - 7.28 (m, 2H), 7.74 (t, 1H), 8.33 (s, 1H).

### Reference Example 3

### 4-Chloro-5-fluoroquinazoline hydrochloride

To a suspension of 5-fluoro-3,4-dihydroquinazolin-4-one (0.5 g) in thionyl chloride (5 ml) was added DMF (0.2 ml). The mixture was heated at reflux under an atmosphere of nitrogen for 3 hours. The mixture was evaporated *in vacuo,* the residue re-suspended in dry toluene, and evaporated again. The residue was dried *in vacuo* to give the title compound as a pale yellow solid (634 mg, 95%), which was used without further manipulation.

### Reference Example 4

### 4-(4-(Azepan-1-ylcarbonyl)-3-chloroanilino)-5-fluoroquinazoline hydrochloride

To a solution of 4-(azepan-1-ylcarbonyl)-3-chloroaniline **(reference example 11.1)** (126.4 mg) in IPA (3 ml) was added 4-chloro-5-fluoroquinazoline **(reference example 3)** (131 mg). The mixture was heated at reflux, under an atmosphere of nitrogen, for 1 hour producing a precipitate. The mixture was cooled to room temperature and the product filtered off, washed with IPA, diethyl ether and dried *in vacuo* to yield the title compound (199 mg, 100%); NMR spectrum (DMSO-d6) 1.54 (bs, 6H), 1.72 (bs, 2H), 3.21 (bs, 2H), 3.58 (bs, 2H), 7.46 (d, 1H), 7.62 - 7.72 (m, 2H), 7.81 (d, 1H), 7.91 (s, 1H), 8.07 (m, 1H), 8.89 (s,1H); Mass spectrum MH⁺ 399.

The procedure described above was repeated using the appropriate aniline and 4-chloroquinazoline. Thus was obtained the compound described below:

### Reference Example 4.1

### 4-(1-(3-Fluorobenzyl)indazol-5-ylamino)-5-fluoroquinazoline hydrochloride

Obtained from 4-chloro-5-fluoroquinazoline hydrochloride **(reference example 3)** and 5-amino-1-(3-fluorobenzyl)indazole (obtained as described in PCT Int. Appl. WO9802438) in 66% yield; NMR spectrum (DMSO-d6) 5.74 (s, 2H), 7.00 - 7.15 (m, 3H), 7.36 (m, 1H), 7.54 (dd, 1H), 7.67 (dd, 1H), 7.77 (d, 1H), 7.82 (d, 1H), 7.97 (s, 1H), 8.06 (m, 1H), 8.22 (s, 1H), 8.77, (s, 1H); Mass spectrum MH⁺ 383.

### Reference Example 4.2

### 4-(3-Chloro-4-hydroxyanilino)-5-(1-methylpiperidin-4-yloxy)quinazoline

Obtained by reacting 4-chloro-5-(1-methylpiperidin-4-yloxy)quinazoline **(reference example 2)** and 3-chloro-4-hydroxyaniline in 60% yield; NMR spectrum (DMSO-d6) 1.9 (m, 2H), 2.1 (m, 5H), 2.3 (m, 2H), 2.6 (m, 2H), 4.8 (m, 1H), 7.0 (d, 1H), 7.2 (d, 1H), 7.3 (m, 2H), 7.7 (m, 1H), 8.0 (d, 1H), 8.4 (s, 1H), 10.0 (s, 1H); Mass spectrum MH⁺385.

### Reference Example 4.3

### 4-(3-Fluoro-4-hydroxyanilino)-5-(1-methylpiperidin-4-yloxy)quinazoline hydrochloride

Obtained by reacting 4-chloro-5-(1-methylpiperidin-4-yloxy)quinazoline **(reference example 2)** and 3-fluoro-4-hydroxyaniline in 87% yield; Mass Spectrum MH⁺ 369.

### Reference Example 4.4

### 4-(Indol-5-ylamino)-5-(1-methylpiperidin-4-yloxy)quinazoline

Obtained by reacting 4-chloro-5-(1-methylpiperidin-4-yloxy)quinazoline **(reference example 2)** and 5-aminoindole in 55% yield; NMR spectrum (DMSO-d6) 1.80 - 2.00 (m, 4H), 2.10 - 2.20 (m + s, 5H), 2.27 (m, 2H), 2.62 (m, 2H), 4.80 (m, 1H), 6.42 (s, 1H), 7.19 (d, 1H), 7.28 (m, 2H), 7.35 (m, 1H), 7.40 (d, 1H), 7.67 (t, 1H), 8.06 (s, 1H), 8.42 (s, 1H); Mass Spectrum MH⁺ 374.

### Reference Example 4.5

### 2-[2-Chloro-4-(5-(1-methylpiperidin-4-yloxy)quinazolin-4-ylamino)phenoxy]acetonitrile hydrochloride

Obtained from 2-(4-amino-2-chlorophenoxy)acetonitrile **(reference example 9.4)** and ; 4-chloro-5-(1-methylpiperidin-4-yloxy)quinazoline **(reference example 2)** in 96 % yield; NMR spectrum (DMSO-d6) 2.10 - 2.32 (m, 2H), 2.32 - 2.50 (m, 2H), 2.76 (s, 3H), 3.03 - 3.25 (m, 2H), 3.40 - 3.60 (m, 2H), 4.98 (m, 1H), 5.28 (s, 2H), 7.28 (d, 1H), 7.33 (d, 1H), 7.38 (d, 1H), 7.65 (d, 1H), 7.76 (dd, 1H), 8.21 (d, 1H), 8.55 (s, 1H), 9.95 (bs, 1H); Mass Spectrum MH⁺ 424.

### Reference Example 4.6

### 4-(4-Iodoanilino)-5-(1-methylpiperidin-4-yloxy)quinazoline

Obtained from 4-iodoaniline and -chloro-5-(1-methylpiperidin-4-yloxy)quinazoline **(reference example 2)** in 67% yield; NMR spectrum (DMSO-d6).1.8 - 2.0 (m, 2H), 2.1 - 2.3 (m, 4H), 2.17 (s, 3H), 2.6 - 2.7 (m, 2H), 4.7 - 4.85 (m, 1H), 7.2 - 7.25 (d, 1H), 7.35 - 7.4 (d, 1H), 7.6 - 7.8 (m, 5H), 8.5 (s, 1H), 10.15 - 10.2 (bs, 1H); Mass spectrum MH⁺ 461.

### Reference Example 4.7

### 4-(5-Chloro-2-fluoro-4-hydroxyanilino)-5-(1-methylpiperidin-4-yloxy)quinazoline

Obtained from 4-chloro-5-(1-methylpiperidin-4-yloxy)quinazoline **(reference example 2)** and 4-amino-2-chloro-5-fluorophenol **(reference example 8.7)** in 83% yield; Mass Spectrum MH⁺ 495.

### Reference Example 4.8

### 4-(3-Chloro-4-(2-pyridylmethoxy)anilino)-5-fluoroquinazoline

Obtained from 4-chloro-5-fluoroquinazoline hydrochloride **(reference example 3)** and 3-chloro-4-(2-pyridylmethoxy)aniline (obtained as described in PCT Int. Appl. (1996) WO 9615118) in 78% yield; Mass spectrum MH⁺ 381.

### Reference Example 5

### 3-Chloro-4-(3-pyridyloxy)aniline

Titanium (III) chloride (10 wt. % in 20-30% aq. HCl, 6.5 ml) was added to a solution of 3-chloro-1-nitro-4-(3-pyridyloxy)benzene (0.25 g) **(reference example 14)** in acetone (18 ml) and water (3.6 ml) containing ammonium acetate (1.6 g) and the solution stirred overnight at room temperature. The reaction was concentrated *in vacuo* and the residue partitioned -between ethyl acetate and 880 ammonia solution. Combined organic extracts were dried and concentrated to give the title compound (0.15 g, 68%); Mass spectrum MH⁺ 221.

The procedure described above was repeated using the appropriate nitro compound. Thus were obtained the compounds described below:

### Reference-Example 5.1

### 3-Chloro-4-(3-fluorophenoxy)aniline

Obtained from 3-chloro-4-(3-fluorophenoxy)-1-nitrobenzene **(reference example 14.1)** in 65% yield; Mass spectrum MH⁺ 238.

### Reference Example 5.2

### 3-Chloro-4-(2,3-difluorophenoxy)aniline

Obtained from 3-chloro-4-(2,3-difluorophenoxy)-1-nitrobenzene **(reference example 14.2)** in 62% yield; Mass spectrum MH⁺ 256.

### Reference Example 5.3

### 3-Methyl-4-(N-(2-pyridylmethyl)amino)aniline

Obtained from 3-methyl-4-(N-(2-pyridylmethyl)amino)nitrobenzene **(reference example 15.1)** in 71% yield; NMR Spectrum (DMSO-d6) 2.07 (s, 3H), 4.13 (s, 2H), 4.17 (d, 2H), 4.88 (t, 1H), 6.14 (d, 1H), 6.21 (dd, 1H), 6.36 (dd, 1H), 7.20 (dd, 1H), 7.32 (d, 1H), 7.68 (ddd, 1H), 8.50 (d, 1H); Mass spectrum MH⁺ 214.

### Reference Example 5.4

### 3-Chloro-4-[N-methyl-N-(2-pyridyl)amino]aniline

Obtained from 3-chloro-4-[N-methyl-N-(2-pyridyl)amino]nitrobenzene **(reference example 16)** in 95% yield; NMR Spectrum (DMSO-d6) 3.23 (s, 3H), 5.50 (s, 2H), 6.04 (d, 1H), 6.57 (dd, 1H), 6.59 (dd, 1H), 6.74 (d, 1H), 7.01 (d, 1H), 7.35 (ddd, 1H), 8.08 (d, 1H); Mass spectrum MH⁺ 236.

### Reference Example 5.5

### 3-Chloro-4-(2-pyridyl)amino)aniline

Obtained from 3-chloro-4-(2-pyridylamino)nitrobenzene **(reference example 16.1)** in 60% yield; NMR Spectrum (DMSO-d6) 5.23 (s, 2H), 6.44 (d, 1H), 6.51 (dd, 1H), 6.58 (dd, 1H), 6.68 (dd, 1H), 7.16 (d, 1H), 7.42 (ddd, 1H), 7.92 (s,1H), 7.97 (d, 1H); Mass spectrum MH⁺ 222.

### Reference Example 5.6

### 3-Methyl-4-(2-pyridylamino)aniline

Obtained from 3-methyl-4-(2-pyridylamino)nitrobenzene **(reference example 16.2)** in 96% yield; NMR Spectrum (DMSO-d6) 2.01 (s, 3H), 4.88 (s, 2H), 6.28 (d, 1H), 6.39 (dd, 1H), 6.46 (d, 1H), 6.51 (dd, 1H), 6.87 (d, 1H), 7.37 (ddd, 1H), 7.73 (s, 1H), 7.95 (d, 1H); Mass spectrum MH⁺ 200.

### Reference Example 5.7

### 3-Methyl-4-[N-methyl-N-(2-pyridyl)amino]aniline

Obtained from 3-methyl-4-[N-methyl-N-(2-pyridyl)amino]nitrobenzene **(reference example 16.3)** in 89% yield; NMR Spectrum (DMSO-d6) 1.90 (s, 3H), 3.22 (s, 3H), 5.09 (s, 2H), 5.96 (d, 1H), 6.46 (dd, 1H), 6.48 (dd, 1H), 6.50 (d, 1H), 6.80 (d, 1H), 7.30 (ddd,1H), 8.08 (d, 1H); Mass spectrum MH⁺ 214.

### Reference Example 6

### 5-Amino-3-chloro-1-(2-pyridylmethyl)indole

A solution of 3-chloro-5-nitro-1-(2-pyridylmethyl)indole **(reference example 13)** (2.5 g) in ethanol (130 ml) was stirred at room temperature. Sodium dithionite (7.6 g) in water (18 ml) was added, and the mixture was heated to 50°C for 5 hours, then cooled to room temperature. The ethanol was removed *in vacuo,* and the residue was partitioned between DCM and water. The DCM layer was separated, dried over sodium sulphate, then concentrated *in vacuo* to give the crude material, which was purified by column chromatography using 50% DCM in hexane then neat DCM to give the title compound as an orange solid (488 mg, 23%); NMR Spectrum (CDCl₃) 3.53 (s, 2H), 5.27 (s, 2H), 6.61 (dd, 1H), 6.68 (d, 1H), 6.86 (d, 1H), 7.01 (d, 1H), 7.04 (s, 1H), 7.11 (dd, 1H), 7.47 (dt, 1H), 8.55 (m, 1H); Mass Spectrum MH⁺ 258.

The procedure described above was repeated using the appropriate aryl nitro compound. Thus were obtained the compounds described below:

### Reference Example 6.1

### 5-Amino-3-chloro-1-(2-pyridylmethyl)indazole

Obtained from 3-chloro-5-nitro-1-(2-pyridylmethyl)indazole **(reference example 13.1)** in 24% yield; NMR Spectrum (CDCl₃) 3.3 (bs, 2H), 6.65 (dd, 1H), 6.77 (m, 1H), 6.84 7.02 (m; 5H), 7.24 (m, 1H).

### Reference Example 6.2

### 3-Fluoro-4-(1-methyl-1H-imidazol-2-ylthio)aniline

Obtained from 3-fluoro-4-(1-methyl-1*H*-imidazol-2-ylthio)nitrobenzene **(reference example 43)** in 86% yield; Mass spectrum MH⁺ 224.

### Reference Example 6.3

### 3-Fluoro-4-(1-methyl-1H-1,3,4-triazol-2-ylthio)aniline

Obtained from 3-fluoro-4-(1-methyl-1*H*-3,4-triazol-2-ylthio)nitrobenzene **(reference example 43.1)** in 14% yield; Mass spectrum MH⁺ 225.

### Reference Example 6.4

### 3-Chloro-4-(2-pyridylthio)aniline

Obtained from 3-chloro-4-(2-pyridylthio)nitrobenzene **(reference example 44)** in 21% yield; Mass spectrum MH⁺ 237.

### Reference Example 6.5

### 3-Chloro-4-(2-pyrimidinylthio)aniline

Obtained from 3-chloro-4-(2-pyrimidinylthio)nitrobenzene **(reference example 44.1)** in 27%; Mass spectrum MH⁺ 238.

### Reference Example 7

### 5-Amino-1-benzenesulphonylindole

1-Benzenesulphonyl-5-nitroindole **(reference example 26)** (0.3 g) and 10% Pd/C (30 mg) in ethyl acetate / ethanol (9:1, 20 ml) were stirred under a hydrogen atmosphere for 2 hours. The reaction was filtered and concentrated *in vacuo* to give the title compound as a beige solid (0.18 g, 67%); NMR spectrum (DMSO-d6) 4.9 (bs, 2H), 6.6 (m, 3H), 7.6 (m, 5H), 7.8 (d, 2H); Mass spectrum MH⁺273.

The procedure described above was repeated using the appropriate nitro aryl compound. Thus was obtained the compound described below:

### Reference Example 7.1

### 5-Amino-1-benzylindole

Obtained from 1-benzyl-5-nitroindole **(reference example 26.1)** in 61% yield; NMR spectrum (DMSO-d6) 4.5 (bs, 2H), 5.2 (s, 2H), 6.2 (d, 1H), 6.4 (dd, 1H), 6.6 (s, 1H), 7.1 (d, 1H), 7.2 (d, 2H), 7.3 (m, 4H); Mass spectrum MH⁺223.

### Reference Example 8

### 3-Chloro-4-phenoxyaniline

To a solution of 2-chloro-4-nitro-1-phenoxybenzene **(reference example 14.3)** (1.76 g) in ethyl acetate / ethanol (105 ml, 9.5:1) was added 10% Pt/C (135 mg). The resulting solution was subjected to a hydrogen atmosphere for 18 hours at room temperature. The catalyst was then filtered off and the solvent concentrated *in vacuo* to give a yellow oil which was purified by flash chromatography eluting with 10 - 15% ethyl acetate / iso-hexane to give the title compound as a clear oil (1.15 g, 74%); NMR Spectrum (DMSO-d6) 5.28 (bs, 2H), 6.54 (dd, 1H), 6.71 (d, 1H), 6.78 (d, 2H), 6.88 (d, 1H), 6.98 (tt, 1H), 7.28 (t, 2H); Mass Spectrum MH⁺ 220.

The procedure described above was repeated using the appropriate nitro compound. Thus were obtained the compounds described below:

### Reference Example 8.1

### 3-Chloro-4-(3-fluorobenzyloxy)aniline

Obtained from 2-chloro-1-(3-fluorobenzyloxy)-4-nitrobenzene **(reference example 18)** in 75% yield; NMR Spectrum (DMSO-d6) 4.91 (s, 2H), 5.01 (s, 2H), 6.45 (dd, 1H), 6.63 (s, 1H), 6.89 (d, 1H), 7.12 (t, 1H), 7.24 (t, 2H), 7.40 (m, 1H); Mass Spectrum MH⁺ 252.

### Reference Example 8.2

### tert-Butyl 4-amino-2-chlorobenzoate

Obtained from *tert*-butyl 2-chloro-4-nitrobenzoate **(reference example 20.1)** in 75% yield; NMR Spectrum (CDCl₃) 1.58 (s, 9H), 3.99 (s, 2H), 6.51 (dd, 1H), 6.66 (d, 1H), 7.68 (d, 1H); Mass Spectrum M⁺ 227.

### Reference Example 8.3

### 3-(2-Chloro-4-aminophenoxymethyl)-1,5-dimethylpyrazole

Obtained from 3-(2-chloro-4-nitrophenoxymethyl)-1,5-dimethylpyrazole **(reference example 40)** in 99% yield; NMR data (DMSO-d6) 2.3 (s, 3H), 3.7 (s, 3H), 4.9 (s, 2H), 5.2 (bs, 2H), 6.1 (s, 1H), 6.5 (m, 1H), 6.7 (d, 1H), 7.0 (d, 1H), 8.6 (bs, 1H); Mass spectrum MH⁺ 252.

### Reference Example 8.4

### 3-(2-Chloro-4-aminophenoxymethyl)-1-methylpyrazole

Obtained from 3-(2-chloro-4-nitrophenoxymethyl)-1-methylpyrazole **(reference example 40.1)** in 87% yield; NMR data (DMSO-d6) 3.8 (s, 3H), 4.9 (s, 4H), 6.3 (d, 1H), 6.4 (m, 1H), 6.6 (d, 1H), 6.9 (d, 1H); Mass spectrum MH⁺252.

### Reference Example 8.5

### 3-Fluoro-4-(1H-imidazol-2-ylthio)aniline

Obtained from 3-fluoro-4-(1*H*-imidazol-2-ylthio)nitrobenzene **(reference example 44.3)** in 98% yield.

### Reference Example 8.6

### 4-(Thiazol-2-ylsulphonyl)aniline

Obtained from 4-(thiazol-2-ylsulphonyl)nitrobenzene **(reference example 45)** in 79% yield; Mass spectrum MH⁺ 240.

### Reference Example 8.7

### 4-Amino-2-chloro-5-fluorophenol

Obtained from 2-chloro-5-fluoro-4-nitrophenol **(reference example 49)** in 100% yield; NMR spectrum (CDCl₃) 4.2 - 4.8 (bs, 2H), 6.6 - 6.7 (d, 1H), 6.75 - 6.8 (d, 1H), 9.3 (s, 1H).

### Reference Example 9

### tert-Butyl 4-amino-2-ethynylbenzoate

To a stirred suspension of *tert*-butyl 2-ethynyl-4-nitrobenzoate **(reference example 22)** (8.76 g) in a mixture of acetic acid (110 ml) and water (11 ml) was added iron (reduced by hydrogen, 8.63 g) in portions, with cooling in a cold water bath. The mixture was stirred at room temperature for 3 hours, diluted with ethyl acetate and filtered. The filtrate was evaporated *in vacuo,* the residue treated with aqueous sodium hydrogen carbonate and extracted with DCM. The organic extract was dried and evaporated *in vacuo* to give a brown solid. This was purified by column chromatography using DCM / ethyl acetate (97/3 to 95/5) as eluent to give the title compound (7.30 g, 95%); NMR spectrum (CDCl₃) 1.59 (s, 9H), 3.30 (s, 1H), 3.97 (s, 2H), 6.60 (dd, 1H), 6.83 (d, 1H), 7.77 (d, 1H).

The procedure described above was repeated using the appropriate nitro compound. Thus were obtained the compounds described below:

### Reference Example 9.1

### 3-Chloro-4-(2-pyridylmethyl)amino)aniline

Obtained from 3-chloro-4-(2-pyridylmethyl)amino)nitrobenzene **(reference example 15)** in 58% yield; NMR Spectrum (DMSO-d6) 4.34 (d, 2H), 4.54 (s, 2H), 5.36 (t, 1H), 6.37 (m, 2H), 6.60 (s, 1H), 7.26 (dd, 1H), 7.33 (d, 1H), 7.73 (dd, 1H), 8.54 (d, 1H); Mass spectrum MH⁺ 236.

### Reference Example 9.2

### 3-Chloro-4-(2-pyrimidinylmethoxy)aniline

Obtained from 3-chloro-4-(2-pyrimidinylmethoxy)nitrobenzene **(reference example 14.4)** in 93% yield; Mass spectrum M-H⁺ 266.

### Reference Example 9.3

### 4-(2-Aminothiazol-4-ylmethoxy)-3-chloroaniline

Obtained from 4-(2-aminothiazol-4-ylmethoxy)-3-chloronitrobenzene **(reference example 34)** (80 mg) in 85% yield; NMR spectrum (DMSO-d6) 4.74 (s, 2H), 4.89 (bs, 2H), 6.46 (dd, 1H), 6.51 (s, 1H), 6.63 (d, 1H), 6.91 (d, 1H), 6.92 (s, 2H); Mass spectrum MH⁺ 256.

### Reference Example 9.4

### 2-(4-Amino-2-chlorophenoxy)acetonitrile

Obtained from 2-(2-chloro-4-nitrophenoxy)acetonitrile **(reference example 18.6)** in 96% yield; NMR spectrum (DMSO-d6) 4.99 (s, 2H), 5.11 (bs, 2H), 6.49 (dd, 1H), 6.64 (d, 1H), 6.98 (d, 1H).

### Reference Example 9.5

### 5-(2-Chloro-4-aminophenoxymethyl)-3-methylisoxazole

Obtained from 5-(2-chloro-4-nitrophenoxymethyl)-3-methylisoxazole **(reference example 40.2)** in 8% yield; NMR spectrum (DMSO-d6) 2.2 (s, 3H), 5.0 (s, 2H), 5.1 (s, 2H), 6.4 (s, 1H), 6.5 (m, 1H), 6.6 (d, 1H), 6.9 (d, 1H); Mass spectrum MH⁺239.

### Reference Example 9.6

### 3-Chloro-4-(1H-imidazol-2-ylthio)aniline

Obtained from 3-chloro-4-(1*H*-imidazol-2-ylthio)nitrobenzene **(reference example 44.2)** in 76% yield.

### Reference Example 9.7

### 3-Chloro-4-(2-thiazolylthio)aniline

Obtained from 3-chloro-4-(2-thiazolylthio)nitrobenzene **(reference example 44.5)** in 56% yield; Mass spectrum MH⁺ 243.

### Reference Example 10

### 3-Chloro-4-((3-fluorophenylamino)methyl)aniline

3-Chloro-4-((3-fluorophenylamino)methyl)nitrobenzene **(reference example 19)** (50 mg) was dissolved in ethanol (2 ml). Saturated ammonium chloride (0.2 ml) was added, and the mixture heated to reflux. Indium metal powder (100 mesh, 143 mg) was added, and the mixture stirred at reflux for 1 hour. The mixture was cooled to room temperature, made basic with concentrated aqueous ammonia solution, and filtered through a bed of diatomaceous earth. The diatomaceous earth was washed with a 1:1 mixture of DCM and methanol containing 1 % concentrated aqueous ammonia solution (20 ml). The combined filtrates were evaporated, and the residue partitioned between water and DCM. The organic layer was evaporated, and the residue purified by chromatography, using 15% ethyl acetate in *iso*-hexane as eluent to give the title compound as an orange oil (20 mg, 45%); NMR spectrum (DMSO-d6) 4.10 (d, 2H), 5.15 (s, 2H), 6.20 - 6.30 (m, 3H), 6.37 (d, 1H), 6.45 (dd, 1H), 6.60 (d, 1H), 6.95 - 7.05 (m, 2H).

### Reference Example 11

### 3-Chloro-4-(8-quinolylthio)aniline

To 3-chloro-1-nitro-4-(8-quinolylthio)benzene **(reference example 17)** (26 g) was added ethanol (5.0 ml) and tin (II) chloride (2.20 g). This was heated to 80°C for 15 minutes, then allowed to cool overnight. The mixture was made basic with 880 ammonia solution, and the product extracted into ethyl acetate (3 x 15 ml), after centrifuging. The solvent was removed *in vacuo* to yield the title compound (0.203 mg, 86%); Mass spectrum MH⁺ 286.

The procedure described above was repeated using the appropriate nitro compound. Thus was obtained the compound described below:

### Reference Example 11.1

### 4-(Azepan-1-ylcarbonyl)-3-chloroaniline

Obtained from 1-(2-chloro-4-nitrobenzoyl)azepane **(reference example 25)** in 73% yield; NMR spectrum (CDCl₃) 1.48 - 1.74 (m, 6H), 1.74 - 1.92 (m, 2H), 3.23 - 3.33 (m, 2H), 3.35 - 3.84 (m, 2H), 3.85 (s, 2H), 6.54 (dd, 1H), 6.68 (d, 1H), 7.02 (d, 1H); Mass spectrum MH⁺ 253.

### Reference Example 12

### 3-Chloro-5-nitroindole

*N*-Chlorosuccinimide (1.65 g) was added in portions to a solution of 5-nitroindole (2.00 g) in DMF (20 ml). The resulting solution was stirred at room temperature for 18 hours. The pale brown solution was poured into water (200 ml) to give a yellow precipitate which was filtered, washed with water and dried *in vacuo* to give the title compound as a yellow solid (2.40 g, 99%). Mass spectrum M-H⁺ 195.

### Reference Example 13

### 3-Chloro-5-nitro-1-(2-pyridylmethyl)indole

Picolyl chloride hydrochloride (3.26 g) was added to a stirred mixture of 3-chloro-5-nitroindole **(reference example 12)** (1.97 g) and potassium carbonate (13.8 g) in DMF (50 ml). The mixture was heated to 50°C and stirred for 2 hours, after which time the solvent was removed *in vacuo.* The residue was dissolved in DCM, then washed with water, and dried over sodium sulphate to give the product as a yellow solid (2.53 g, 88%); NMR Spectrum (CDCl₃) 5.43 (s, 2H), 6.90 (d, 1H), 7.23 (dd, 1H), 7.35 (s, 1H), 7.37 (d, 1H), 7.62 (dt, 1H), 8.12 (dd, 1H), 8.60 (m, 2H).

The procedure described above was repeated using the appropriate indazole. Thus was obtained the compound described below:

### Reference Examples 13.1

### 3-Chloro-5-nitro-1-(2-pyridylmethyl)indazole

Obtained from 3-chloro-5-nitroindazole in 74% yield; NMR Spectrum (CDCl₃) 5.32 (s, 2H), 6.80 (d, 1H), 6.89 (d, 1H), 7.01 (dt, 1H), 7.28 (m, 3H), 8.12 (dd, 1H), 8.61 (d, 1H).

### Reference Example 14

### 3-Chloro-1-nitro-4-(3-pyridyloxy)benzene

To a stirred solution of 3-hydroxypyridine (1.05 g) in DMA (25 ml) under a nitrogen atmosphere was added sodium hydride (0.44 g, 60% suspension in oil). The solution was stirred at ambient temperature for two hours. To this solution was added 3-chloro-4-fluoronitrobenzene (1.75 g) and the solution was stirred and heated at 90°C for four hours. The solution was cooled and poured into water (120 ml) and extracted with ethyl acetate. The combined organic extracts were washed with water, brine, and dried with anhydrous magnesium sulphate. Evaporation of the solvent gave a solid, which was recrystallised from a mixture of ethyl acetate / *iso*-hexane to give the title compound as a yellow solid (2.15 g, 86%); NMR spectrum (CDCl₃) 6.95-6.98 (dd,1H), 7.4 (d, 2H), 8.1 (dd, 1H), 8.4 (d, 1H), 8.5 - 8.7 (d, 2H); Mass spectrum MH⁺ 251.

The procedure described above was repeated using 3-chloro-4-fluoronitrobenzene and the appropriate phenol or alcohol. Thus were obtained the compounds described below:

### Reference Example 14.1

### 3-Chloro-4-(3-fluorophenoxy)nitrobenzene

Obtained from 3-chloro-4-fluoronitrobenzene and 3-fluorophenol in 43% yield; NMR spectrum (CDCl₃) 6.8-6.9 (m, 2H), 6.95-7.0 (m, 2H), 7.3-7.45 (m, 1H), 8.1 (dd, 1H), 8.4 (d, 1H); Mass spectrum M-H⁺ 266.

### Reference Example 14.2

### 3-Chloro-4-(2,3-difluorophenoxy)nitrobenzene

Obtained from 3-chloro-4-fluoronitrobenzene and 2,3-difluorophenol in 55% yield; NMR spectrum (CDCl₃); 6.8 - 6.9 (dd, 1H), 6.9 - 7.0 (m, 1H), 7.1 - 7.2 (m, 2H), 8.0 - 8.1 (dd, 1H), 8.4 (t, 1H); Mass spectrum M-H⁺ 284.

### Reference Example 14.3

### 3-Chloro-4-phenoxynitrobenzene

Obtained from 3-chloro-4-fluoronitrobenzene and phenol in 42% yield; NMR Spectrum (DMSO-d6) 7.02 (d, 1H), 7.18 (dt, 2H), 7.30 (tt, 1H), 7.49 (t, 2H), 8.16 (dd, 1H), 8.44 (d, 1H); Mass Spectrum M-H⁺ 248.

### Reference Example 14.4

### 3-Chloro-4-(pyrimidin-2-ylmethoxy)nitrobenzene

Obtained from 3-chloro-4-fluoronitrobenzene and pyrimidine-2-methanol **(reference example 27)** in 36% yield; NMR spectrum (DMSO-d6) 5.60 (s, 2H), 7.30 (d, 1H), 7.47 (t,1H), 8.14 (dd,1H), 8.31 (d, 1H), 8.82 (d, 1H); Mass spectrum M-H⁺ 266.

### Reference Example 15

### 3-Chloro-4-(2-pyridyl)amino)nitrobenzene

A mixture of 3-chloro-4-fluoronitrobenzene (1.76 g), 2-(aminomethyl)pyridine (1.03 ml) and di-*iso*-propylethylamine (1.74 ml) was dissolved in acetonitrile (60 ml) and heated overnight at reflux. The mixture was cooled to room temperature and the resulting solid was collected by filtration and washed with diethyl ether, giving the title compound as a yellow solid (2.21 g, 84%); NMR spectrum (DMSO-d6) 4.66 (d, 2H), 6.73 (d, 1H), 7.30 (dd, 1H), 7.33 (dd, 1H), 7.59 (t, 1H), 7.78 (ddd, 1H), 8.00 (dd, 1H), 8.19 (d, 1H), 8.56 (d, 1H); Mass spectrum MH⁺ 266.

The procedure described above was repeated using the appropriate amine and fluoronitro compound. Thus was obtained the compound described below:

### Reference Example 15.1

### 3-Methyl-4-(2-pyridylmethyl)amino)nitrobenzene

Obtained from 4-fluoro-3-methylnitrobenzene and 2-(aminomethyl)pyridine in 6% yield; NMR spectrum (DMSO-d6) 2.26 (s, 3H), 4.58 (d, 2H), 6.48 (d, 1H), 7.17 (t, 1H), 7.28 (dd, 1H), 7.31 (d, 1H), 7.76 (ddd, 1H), 7.88 (dd, 1H), 7.92 (d, 1H), 8.55 (d, 1H); Mass spectrum MH⁺ 244.

### Reference Example 16

### 3-Chloro-4-[N-methyl-N-(2-pyridyl)amino]nitrobenzene

Sodium hydride (60% dispersion, 200 mg) was suspended in DMA (25 ml), and 2-(methylamino)pyridine (513 µl) was added under a nitrogen atmosphere at room temperature. The mixture was stirred for 20 minutes at room temperature. 3-Chloro-4-fluoronitrobenzene (878 mg) was added as a solution in DMA (5 ml), causing a dark red solution to form: The mixture was stirred overnight at room temperature, quenched by the careful addition of water, acidified with acetic acid, and the pH adjusted to pH 8 with concentrated aqueous ammonia solution. The mixture was poured into water (200 ml), and extracted with DCM (3 x 100ml). The combined extractions were filtered through a phase separating filter paper, and evaporated to give a brown oil, which was purified by chromatography, usin 15% ethyl acetate in *iso-*hexane as eluent to give the title compound as a yellow solid (226mg, 17%); NMR spectrum (DMSO-d6) 3.17 (s, 3H), 6.57 (d, 1H), 6.78 (dd, 1H), 7.57 (dd, 1H), 7.70 (d, 1H), 8.11 (d, 1H), 8.24 (dd, 1H), 8.37 (d, 1H); Mass spectrum MH⁺ 266.

The procedure described above was repeated using the appropriate fluoronitrobenzene and aminopyridine. Thus were obtained the compounds described below:

### Reference Example 16.1

### 3-Chloro-4-(2-pyridylamino)nitrobenzene

Obtained from 3-chloro-4-fluoronitrobenzene and 2-aminopyridine in 16% yield; NMR spectrum (DMSO-d6) 7.04 (dd, 1H), 7.36 (d, 1H), 7.76 (ddd, 1H), 8.16 (dd, 1H), 8.30 (d, 1H), 8.31 (d, 1H), 8.61 (d, 1H), 9.01 (s, 1H); Mass spectrum MH⁺ 252.

### Reference Example 16.2

### 3-Methyl-4 4-(2-pyridylamino)nitrobenzene

Obtained from 4-fluoro-3-methylnitrobenzene and 2-aminopyridine in 36% yield; NMR spectrum (DMSO-d6) 2.39 (s, 3H), 6.92 (dd, 1H), 7.19 (d, 1H), 7.68 (ddd, 1H), 8.00 (dd, 1H), 8.06 (d,1H), 8.21 (d, 1H), 8.31 (d, 1H), 8.51 (s, 1H); Mass spectrum MH⁺230.

### Reference Example 16.3

### 3-Methyl-4-[N-methyl-N-(2-pyridyl)amino]nitrobenzene

Obtained from 4-fluoro-3-methylnitrobenzene and 2-(methylamino)pyridine in 39% yield; NMR spectrum (DMSO-d6) 2.12 (s, 3H), 3.32 (s, 3H), 6.36 (d, 1H), 6.71 (dd, 1H), 7.48 (d, 1H), 7.49 (ddd, 1H), 8.10 (m, 2H), 8.20 (d, 1H); Mass spectrum MH⁺244.

### Reference Example 17

### 3-Chloro-1-nitro-4-(8-quinolylthio)benzene

8-Quinolinethiol (0.32 g) was dissolved in DMF (4 ml) under argon. To this was added sodium bis(trimethylsilyl)amine (2.2 ml, 1.0M in THF) and the reaction stirred at room temperature for 1 hour. To this was added a solution of 3-chloro-4-fluoronitrobenzene (0.35 g) in DMF (0.5 ml), and stirring continued at room temperature overnight. The solution was then poured into water (20 ml) and stirred for 30 minutes. The resulting solid was filtered off, washed with water (5 ml) then with a 1:1 mix of ethyl acetate / *iso*-hexane (6 ml) and dried *in vacuo* to yield the title compound (0.26 g, 41 %); Mass spectrum MH⁺ 317.

### Reference Example 18

### 2-Chloro-1-(3-fluorobenzyloxy)-4-nitrobenzene

To a solution of 2-chloro-4-nitrophenol (20.0 g) in acetone (400 ml) was added potassium carbonate (47.76 g) followed by the dropwise addition of 3-fluorobenzylbromide (32.67 g) over 15 minutes. The reaction mixture was then stirred at room temperature for 16 hours and filtered to remove insoluble material. The solvent was then concentrated *in vacuo* and the solid remaining was purified by flash chromatography, using 30 - 80% DCM / *iso-*hexane as eluent to give the title compound (30.96 g, 95%); NMR Spectrum (DMSO-d6) 5.39 (s, 2H), 7.18 (t, 1H), 7.30 (m, 2H), 7.45 (m, 2H), 8.23 (dd, 1H), 8.32 (d, 1H); Mass Spectrum M-H⁺ 280.

The procedure described above was repeated using the appropriate phenol and alkyl chloride or bromide. Thus were obtained the compounds described below:

### Reference Example 18.1

### 4-(2-Fluorobenzyloxy)-3-iodo-nitrobenzene

Obtained from 4-hydroxy-3-iodo-nitrobenzene (obtained as described in J. Org. Chem. 1990,55,5287-5291) and 2-fluorobenzyl bromide (0.39 g) in 85% yield; Mass Spectrum M-H⁺ 372.

### Reference Example 18.2

### 4-(3-Fluorobenzyloxy)-3-iodo-nitrobenzene

Obtained by reacting 4-hydroxy-3-iodo-nitrobenzene and 3-fluorobenzyl bromide in 99% yield; Mass Spectrum M-H⁺ 372.

### Reference Example 18:3

### 4-(2,6-Difluorobenzyloxy)-3-iodo-nitrobenzene

Obtained by reacting 4-hydroxy-3-iodo-nitrobenzene and 2, 6-difluorobenzyl bromide in 100% yield; Mass Spectrum M-H⁺ 390.

### Reference Example 18.4

### 3-Iodo-4-(4-thiazolylmethoxy)-nitrobenzene

Obtained by reacting 4-hydroxy-3-iodo-nitrobenzene and 4-chloromethylthiazole hydrochloride in 100% yield; Mass Spectrum MH⁺ 363.

### Reference Example 18.5

### 3-Iodo-4-(5-methylisoxazol-3-ylmethoxy)-nitrobenzene

Obtained by reacting 4-hydroxy-3-iodo-nitrobenzene and 3-chloromethyl-5-methylisoxazole in 74% yield; Mass Spectrum M-H⁺ 359.

### Reference Example 18.6

### 2-(2-Chloro-4-nitrophenoxy)acetonitrile

Obtained by reacting 2-chloro-4-nitrophenol and chloroacetonitrile in 39% yield; NMR spectrum (CDCl₃) 4.96 (s, 2H), 7.15 (d, 1H), 8.22 (dd, 1H), 8.36 (d, 1H); Mass Spectrum M-H⁺ 211.

### Reference Example 19

### 3-Chloro-4-((3-fluorophenylamino)methyl)nitrobenzene

2-Chloro-4-nitrobenzyl bromide (501 mg) was dissolved in acetonitrile (30 ml). Di-*iso*-propylethylamine (348 µl) and 3-fluoroaniline (192 µl) were added, and the mixture heated at reflux overnight. The solvent was evaporated, and the residue purified by chromatography, using 15 to 25% ethyl acetate in *iso*-hexane as eluent to give the title compound as a brown oil (470 mg, 84%); NMR spectrum (DMSO-d6) 4.42 (d, 2H), 6.25 - 6.38 (m, 3H), 6.74 (t, 1H), 7.05 (dd, 1H), 7.59 (d, 1H), 8.17 (dd, 1H), 8.38 (d, 1H); Mass spectrum MH⁺ 283.

### Reference Example 20

### tert-Butyl 2-bromo-4-nitrobenzoate

A solution of 2-bromo-4-nitrobenzoic acid (1.66 g) in THF (8 ml) was stirred in a nitrogen atmosphere, carbonyl diimidazole (1.37 g) added and the mixture stirred at 60°C for 2 hours. After cooling to 40°C, *tert*-butanol (0.98 g) and DBU (1.03 g) were added and stirring at 40°C continued overnight. Ether was added to the cooled solution and the solution washed with 10% aqueous HCl, water, aqueous sodium hydrogen carbonate and brine. The ether solution was dried over MgSO₄, filtered and evaporated *in vacuo* to give an oil, which was purified by chromatography using DCM as eluent to give the title compound (1.41 g, 69%); NMR spectrum (DMSO-d6) 1.56 (s, 9H), 7.87 (d, 1H), 8.27 (dd, 1H), 8.45 (d, 1H).

The procedure described above was repeated using the appropriate benzoic acid. Thus was obtained the compound described below:

### Reference Example 20.1

### tert-Butyl 2-chloro-4-nitrobenzoate

Obtained in 81% yield; NMR spectrum (CDCl₃) 1.63 (s, 9H), 7.84 (d, 1H), 8.13 (dd, 1H), 8.29 (d, 1H); Mass spectrum M⁺ 257.

### Reference Example 21

### tert-Butyl 4-nitro-2-(trimethylsilylethynyl)benzoate

A solution of *tert*-butyl 2-bromo-4-nitrobenzoate **(reference example 20)** (10.8 g) in triethylamine (85 ml) was stirred under a nitrogen atmosphere, (trimethylsilyl)acetylene (4.27 g) was added followed by *bis*(triphenylphosphine)palladium (II) chloride (0.34 g) and the mixture stirred at 80°C for 3 hours. The mixture was filtered, the residue washed with ethyl acetate and the combined washings and filtrate evaporated *in vacuo,* The resulting oil was dissolved in ethyl acetate and heated briefly at reflux with decolourising charcoal. The charcoal was removed by filtration and the solvent removed *in vacuo* to give a brown oil. This was purified by column chromatography, eluting with *iso*-hexane / ethyl acetate (95/5) to give the title compound (10.70 g, 94%); NMR spectrum (CDCl₃) 0.21 (s, 9H), 1.57 (s, 9H), 7.80 (d, 1H), 8.07 (dd, 1H), 8.29 (d, 1H).

### Reference Example 22

### tert-Butyl 2-ethynyl-4-nitrobenzoate

A solution of *tert*-butyl 4-nitro-2-(trimethylsilylethynyl)benzoate **(reference example 21)** (12.54 g) in methanol (250 ml) was treated with potassium carbonate (25.0 g) and stirred for 15 minutes. The mixture was poured into water, extracted with ethyl acetate, the extracts dried, filtered and evaporated *in vacuo* to give the crude product. This was purified by chromatography, using *iso*-hexane / ethyl acetate (9/1) to give the title compound (8.77 g, 90%); NMR spectrum (CDCl₃) 1.63 (s, 9H), 3.49 (s, 1M, 7.99 (d, 1H), 8.18 (dd, 1H), 8.40 (d, 1H).

### Reference Example 23

### tert-Butyl 2-chloro-4-(5-(1-methylpiperidin-4-yloxy)quinazolin-4-ylamino)benzoate

To a solution of 4-chloro-5-(1-methylpiperidin-4-yloxy)quinazoline (**reference example 2)** (1.2 g) and *tert*-butyl 4-amino-2-chlorobenzoate **(reference example 8.2)** (0.98 g) in DMA (60 ml) was added HCl in dioxane (4.0M, 3.0 ml) and the mixture heated at 80°C for 1 hour. The reaction was cooled, diluted with acetone and the resulting precipitate filtered off and dried. The crude product was purified by chromatography, using DCM / methanol 880 NH₄OH (100/8/1) as eluent, to give the title compound (1.04 g, 52%); NMR spectrum (DMSO-d6) 1.54 (s, 9H), 1.94 (m, 2H), 2.18 (s, 3H), 2.25 (m, 4H), 2.65 (m, 2H), 4.78 (m, 1H), 7.27 (d, 1H), 7.38 (d, 1H), 7.65 (dd, 1H), 7.78 (m, 2H), 8.34 (d, 1H), 8.64 (s, 1H), 10.36 (s, 1H); Mass spectrum MH⁺ 469.

The procedure described above was repeated using the appropriate aniline and chloroquinazoline. Thus was obtained the compound described below:

### Reference Example 23.1

### tert-Butyl 2-ethynyl-4-(5-(1-methylpiperidin-4-yloxy)quinazolin-4-ylamino)benzoate hydrochloride

Obtained from 4-chloro-5-(1-methylpiperidin-4-yloxy)quinazoline **(reference example 2)** and *tert*-butyl 4-amino-2-ethynylbenzoate **(reference example 9)** in 57% yield; NMR spectrum (CDCl₃) 1.62 (s, 9H), 2.07 (m, 2H), 2.31 (m, 4H), 2.35 (s, 3H), 2.84 (m, 2H), 3.39 (s, 1H). 4.65 (m, 1H), 6.95 (d, 1H), 7.49 (d, 1H), 7.64 (t, 1H), 7.91 (dd, 1H), 7.96 (d, 1H), 8.08 (d, 1H), 8.71 (s, 1H), 10.29 (1, 1H); Mass spectrum MH⁺ 459.

### Reference Example 24

### 2-Chloro-4-(5-(1-methylpiperidin-4-yloxy)quinazolin-4-ylamino)benzoic acid hydrochloride

A solution of *tert*-butyl 2-chloro-4-(5-(1-methylpiperidin-4-yloxy)quinazolin-4-ylamino)benzoate **(reference example 23)** (1.04 g) in 2N HCl (50 ml) was stirred at 50°C for 2 hours and allowed to cool to room temperature. The mixture was diluted with acetone and the resulting precipitate filtered and dried to give the title compound (1.00g, 100%); Mass spectrum MH⁺ 413.

The procedure described above was repeated using the appropriate *tert*-butyl ester. Thus was obtained the compound described below:

### Reference Example 24.1

### 2-Ethynyl-4-(5-(1-methylpiperidin-4-yloxy)quinazolin-4-ylamino)benzoic acid hydrochloride

Obtained from *tert*-butyl 2-ethynyl-4-(5-(1-methylpiperidin-4-yloxy)quinazolin-4-ylamino)benzoate hydrochloride **(reference example 23.1)** in 91% yield; Mass spectrum MH⁺ 403.

### Reference Example 25

### 1-(2-Chloro-4-nitrobenzoyl)azepane

To a solution of 2-chloro-4-nitrobenzoic acid (4.02 g) and triethylamine (2.20 g) in DCM (150 ml) was added O-(7-azabenztriazol-1-yl)-N,N,N',N'-tetramethyluronium hexafluorophosphate (8.36 g). This mixture was stirred at room temperature under an atmosphere of nitrogen for 3 hours. Hexamethyleneimine (2.18 g) was added and this mixture stirred at room temperature for 2 hours. The solvent was removed *in vacuo* and the residue purified by chromatography using 0 - 10% ethyl acetate in DCM as eluent to give the title compound as a colourless oil which crystallised upon standing (5.09 g, 90%); NMR spectrum (CDCl₃) 1.53 -1.77 (m, 6H), 1.79-1.93 (m, 2H), 3.15 - 3.27 (m, 2H), 3.60 - 3.84 (m, 2H), 7.48 (d,1H), 8.18 (dd, 1H), 8.30 (d, 1H); Mass spectrum MH⁺ 283.

### Reference Example 26

### 1-Benzenesulphonyl-5-nitroindole

5-Nitroindole (2 g) was added to sodium hydride (1.98 g) in THF at 0°C over 15 minutes to give a red solution. Once effervescence had subsided, benzene sulphonyl chloride (1.73 ml) was added dropwise over 5 minutes to give an orange suspension. After 30 minutes a further amount of benzene sulphonyl chloride (0.47 ml) was added to give a yellow suspension. The THF was removed *in vacuo* and water was added to give a yellow solid. This was filtered and dried *in vacuo* to afford the title compound as a yellow solid (4.33g, > 100%); NMR spectrum (DMSO-d6) 7.0 (d, 1H), 7.6 (m, 2H), 7.7 (m, 1H), 8.0 (m, 3H), 8.2 (m, 2H), 8.6 (d, 1H).

The procedure described above was repeated using the appropriate alkylating agent. Thus was obtained the compound described below:

### Reference Example 26.1

### 1-Benzyl-5-nitroindole

Obtained in > 100% yield; NMR spectrum (DMSO-d6) 5.5 (s, 2H), 6.8 (d, 1H), 7.2 (m, 5H), 7.7 (d, 1H), 7.8 (d, 1H), 8.0 (dd, 1H), 8.6 (d, 1H); Mass spectrum M-H⁺ 251.

### Reference Example 27

### Pyrimidine-2-methanol

Sodium borohydride (0.28 g) was added in a single portion to a stirred solution of methyl pyrimidine-2-carboxylate (1 g) in ethanol (50 ml). The reaction was stirred for 4 hours, then water (4 ml) added and the reaction stirred overnight. The resulting solid was filtered and the filtrate concentrated. The residue was purified by chromatography using DCM - 10% methanol as eluent to give the title compound as a colourless liquid (0.28 g, 35%); NMR spectrum (CDCl₃) 3.74 (bs, 1H), 4.83 (d, 1H), 7.22 (dt, 1H), (8.75 (d, 2H); Mass Spectrum MH⁺111.

The procedure described above was repeated using the appropriate ester. Thus was obtained the compound described below:

### Reference Example 27.1

### 3-Hydroxymethylisoxazole

Obtained from 3-ethoxycarbonylisoxazole **(reference example 41)** in 93% yield; NMR spectrum (CDCl₃) 2.50 (bs, 1H), 4.80 (s, 2H), 6.42 (d, 1H), 8.37 (d, 1H).

### Reference Example 27.2

### 5-(Hydroxymethyl)-3-methylisoxazole

Obtained from 3-methylisoxazole-5-carboxylic acid methyl ester **(reference example 41)** in 73% yield; NMR spectrum (DMSO-d6) 2.2 (s, 3H), 4.9 (s, 2H), 5.5 (t, 1H), 6.2 (s, 1H). **Reference Example 28**

### 4-Benzyloxy-3-ethynyl-nitrobenzene

Benzyl bromide (0.22 ml) was added to 4-hydroxy-3-iodo-nitrobenzene (0.5 g) and potassium carbonate (0.86 g) in DMF (10 ml) and the reaction stirred overnight. To the resulting suspension was added trimethylsilylacetylene (0.77 ml), copper(I)iodide (7 mg), *bis*(triphenylphosphine)-dichloropalladium (26 mg) and triethylamine (2 ml) under nitrogen and the mixture stirred at room temperature for 1 hour. Methanol was added to the reaction, and stirring was continued for a further hour, before concentration *in vacuo.* The residue was purified by chromatography using DCM - 25% *iso*-hexane as eluent to give the title compound as a yellow solid (0.1 g, 21%); NMR spectrum (DMSO-d6) 4.47 (s, 1H), 5.35 (s, 2H), 7.30 - 7.50 (m, 6H), 8.23 (m, 2H); Mass Spectrum M-H⁺ 252.

### Reference Example 29

### 4-(2-fluorobenzyloxy)-3-(trimethylsilylethynyl)-nitrobenzene

To a solution of 4-(2-fluorobenzyloxy)-3-iodo-nitrobenzene (0.49 g) **(reference example 18.1)** in acetonitrile (10 ml) was added trimethylsilylacetylene (0.54 ml), copper(I)iodide (5 mg), *bis*(triphenylphosphine)-dichloropalladium (18 mg) and triethylamine (10 ml) under nitrogen and the mixture stirred at room temperature for 4 hours. The reaction was concentrated *in vacuo* and the residue purified by chromatography using DCM as eluent to give the title compound as a yellow solid (0.33 g, 73%); Mass Spectrum M-H⁺ 342.

The procedure described above was repeated using the appropriate iodobenzene. Thus were obtained the compounds described below:

### Reference Example 29.1

### 4-(3-Fluorobenzyloxy)-3-(trimethylsilylethynyl)-nitrobenzene

Obtained from 4-(3-fluorobenzyloxy)-3-iodo-nitrobenzene **(reference example 18.2);** Mass Spectrum M-H⁺ 342.

### Reference Example 29.2

### 4-(2,6-Difluorobenzyloxy)-3-(trimethylsilylethynyl)-nitrobenzene

Obtained from 4-(2, 6-difluorobenzyloxy)-3-iodo-nitrobenzene **(reference example 18.3);** Mass Spectrum M-H⁺ 360.

### Reference Example 29.3

### 4-(4-Thiazolylmethoxy)-3-(trimethylsilylethynyl)-nitrobenzene

Obtained from 3-iodo-4-(thiazol-4-ylmethyloxy)-nitrobenzene **(reference example 18.4)** in 60% yield; Mass Spectrum MH⁺ 333.

### Reference Example 29.4

### 4-(5-methylisoxazol-3-ylmethoxy)-3-(trimethylsilylethynyl)-nitrobenzene

Obtained from 3-iodo-4-(5-methylisoxazol-3-ylmethyloxy)-nitrobenzene (**reference example 18.5)** in 86% yield; Mass Spectrum MH⁺ 333.

### Reference Example 30

### 3-Ethynyl 4-(2-fluorobenzyloxy)aniline

A solution of 4-(2-fluorobenzyloxy)-3-(trimethylsilylethynyl)nitrobenzene (310 mg) (**reference example 29)** and 10% Pt/C in ethyl acetate / ethanol (9:1, 10 ml) was stirred under an atmosphere of hydrogen for 20 mins. The catalyst was removed by filtration and the solution concentrated *in vacuo* to give a green solid. This was dissolved in methanol (100 ml) and DCM (50 ml), potassium carbonate (0.375 g) added and the solution stirred for 30 mins. The reaction was filtered and concentrated *in vacuo.* The residue was purified by chromatography using DCM as eluent to give the title compound as a yellow oil (0.13 g, 62%, 2 steps); Mass Spectrum MH⁺ 283.

The procedure described above was repeated using the appropriate nitrobenzene. Thus were obtained the compounds described below:

### Reference Example 30.1

### 3-Ethynyl 4-(3-fluorobenzyloxy)aniline

Obtained from 4-(3-fluorobenzyloxy)-3-(trimethylsilylethynyl)nitrobenzene (**reference example 29.1);** Mass Spectrum MH⁺ 283.

### Reference Example 30.2

### 4-(2,6-Difluorobenzyloxy)-3-ethynylaniline

Obtained from 4-(2, 6-difluorobenzyloxy)-3-(trimethylsilylethynyl)nitrobenzene **(reference example 29.2);** Mass Spectrum MH⁺ 301.

### Reference Example 30.3

### 3-Ethynyl 4-(4-thiazolylmethoxy)aniline

Obtained from 4-(thiazol-4-ylmethyloxy)-3-(trimethylsilylethynyl)nitrobenzene **(reference example 29.3);** Mass Spectrum MH⁺ 231.

### Reference Example 30.4

### 3-Ethynyl 4-(5-methylisoxazol-3-ylmethoxy)aniline

Obtained from 4-(5-methylisoxazol-3-ylmethoxy)-3-(trimethylsilylethynyl)nitrobenzene **(reference example 29.4;** Mass Spectrum MH⁺ 229.

### Reference Example 31

### 3-Chloromethylisoxazole

Thionyl chloride (5 ml) was added to 3-hydroxymethylisoxazole **(reference example 27.1)** (0.5 g), followed by DMF (0.1 ml). The reaction was heated at 100°C for 3 hours and then concentrated *in vacuo.* The residue was partitioned between DCM and saturated aqueous sodium hydrogen carbonate solution (T < 5°C). Combined organic extracts were washed with water, dried and concentrated to give the title compound as a brown oil (0.163 g, 28%); NMR spectrum (CDCl₃) 4.63 (s, 2H), 6.45 (d, 1H), 8.40 (m, 1H).

### Reference Example 32

### 3-Ethoxycarbonylisoxazole

Triethylamine (2.21 ml) in ether (13 ml) was added dropwise to a solution of carboethoxychloraldoxime (2 g) and vinyl acetate (11.4 g) in ether (27 ml) heated at reflux. The solution was heated for a further hour then cooled and extracted with water. Organic extracts were dried and concentrated and the resulting oil was heated at 180°C for 30 mins. The reaction was partitioned between DCM and water. Combined organic extracts were dried and concentrated to give the title compound as a pale brown oil (1.64 g, 88%); NMR spectrum (CDCl₃) 1.43 (t, 3H), 4.46 (q,2H), 6.79 (d, 1H), 8.52 (d,1H).

### Reference Example 33

### 4-(3-Chloro-4-(3-fluorobenzvloxv)anilino)-5-fluoroquinazoline

3-Chloro-4-(3-fluorobenzyloxy)aniline **(reference example 8.1)** (1.65 g), 4-chloro-5-fluoroquinazoline **(reference example 3)** (1.20 g) and di-*iso*-propylethylamine (1.15 ml) in IPA (50 ml) were heated at reflux for 1 hr. The resulting precipitate was isolated by filtration, and washed with IPA and ether to give the title compound as a grey solid (2.02 g, 77%); NMR spectrum (DMSO-d6) 5.24 (s, 2H), 7.15 (dt, 1H), 7.23 (d, 1H), 7.30 (m, 1H), 7.36 - 7.50 (m, 2H), 7.55 - 7.63 (m, 2H), 7.78 - 7.92 (m, 2H), 8.55 (s, 1H), 9.10 (d, 1H); Mass Spectrum MH⁺ 398.

### Reference Example 34

### 4-(2-Aminothiazol-4-ylmethoxy)-3-chloronitrobenzene

Sodium hydride (60% dispersion in mineral oil, 200mg) was suspended in DMA (20 ml), and 2-chloro-4-nitrophenol (1.74 g) was added. The mixture was stirred at ambient temperature for 1 hour. Tetra-*n*-butylammonium iodide (20 mg) was added. 2-Amino-4-(chloromethyl)thiazole hydrochloride (185 mg) was added dropwise over 1 hour as a solution in DMA (20 ml). The solution was stirred overnight at ambient temperature. The solvent was evaporated, and the residue partitioned between saturated sodium carbonate solution and DCM. Combined organic extracts were dried and concentrated and the residue purified by chromatography using ethyl acetate as eluent to give the title compound as a yellow solid (85 mg, 30%); NMR spectrum (DMSO-d6) 5.11 (s, 2H), 6.66 (s, 1H), 6.99 (b s, 2H), 7.49 (d, 1H), 8.19 (dd, 1H), 8.29 (d, 1H); Mass Spectrum MH⁺ 286.

### Reference Example 35

### 2-(2-Chloro-4-(5-(1-methylpiperidin-4-yloxy)quinazolin-4-ylamino)phenoxy)-N-hydroxyacetamidine

Hydroxylamine hydrochloride (121 mg) was dissolved in a mixture of ethanol (4 ml) and water (16 ml). Potassium carbonate (131 mg) was added. 2-[2-Chloro-4-(5-(1-methylpiperidin-4-yloxy)quinazolin-4-ylamino)phenoxy]acetonitrile hydrochloride **(reference example 4.5)** (200 mg) was added, the mixture briefly sonicated, then heated to 90°C for 1 hour. The colourless solution was cooled to ambient temperature and the resulting solid filtered, washed with water and diethyl ether and dried *in vacuo* to give the title compound as a white crystalline solid (163 mg, 82%); NMR spectrum (DMSO-d6) 1.83 - 1.96 (m, 2H), 2.08 - 2.18 (m, 2H), 2.17 (s, 3H), 2.22 - 2.32 (m, 2H), 2.56 - 2.66 (m, 2H), 4.48 (s, 2H), 4.78 (m, 1H), 5.58 (s, 2H), 7.21 (d, 1H), 7.27 (d, 1H), 7.31 (d, 1H), 7.49 (dd, 1H), 7.70 (dd, 1H), 8.09 (d, 1H), 8.50 (s, 1H), 9.31 (s, 1H), 10.03 (s, 1H); Mass Spectrum MH⁺ 457.

### Reference Example 36

### Ethyl 2-[2-Chloro-4-(5-(1-methylpiperidin-4-yloxy)quinazolin-4-ylamino)phenoxy]acetate

4-(3-Chloro-4-hydroxyanilino)-5-(1-methylpiperidin-4-yloxy)quinazoline **(reference example 4.2)** (384 mg) and potassium carbonate (552mg) were suspended in acetonitrile. The suspension was briefly sonicated, and ethyl bromoacetate (128 µl) was added. The mixture was heated at 80°C for 90 minutes, then cooled to ambient temperature. The solid was removed by filtration, and the filtrate evaporated. The residue was purified by chromatography, using 0 to 1.5% (7:1 methanol / conc. ammonia (aq)) in DCM as eluent to give the title compound as a white crystalline solid (375mg, 80%); NMR spectrum (DMSO-d6) 1.21 (t, 3H), 1.83 - 1.97 (m, 2H), 2.07 - 2.17 (m, 2H), 2.17 (s, 3H), 2.21 - 2.32 (m, 2H), 2.56 - 2.66 (m, 2H), 4.17 (q, 2H), 4.79 (m, 1H), 4.90 (s, 2H), 7.10 (d, 1H), 7.21 (d, 1H), 7.32 (d, 1H), 7.47 (dd, 1H), 7.71 (dd, 1H), 8.11 (d, 1H), 8.50 (s, 1H), 10.04 (s, 1H); Mass Spectrum MH⁺ 471.

### Reference Example 37

### 2-[2-Chloro-4-(5-(1-methylpiperidin-4-yloxy)quinazolin-4-ylamino)phenoxy]acetic acid hydrazide

Ethyl 2-[2-chloro-4-(5-(1-methyl-piperidin-4-yloxy)quinazolin-4-ylamino)phenoxy]acetate **(reference example 36)** (150 mg) was dissolved in ethanol (15 ml). Pyridine (2 drops) and hydrazine monohydrate (150 µl) were added, and the mixture heated at reflux for 16 hours. The solvent was evaporated and the residue purified by chromatography, using 0 to 3% (7:1 methanol / conc. ammonia (aq)) in DCM as eluent to give the title compound as a white crystalline solid (77 mg, 53%); NMR spectrum (DMSO-d6) 1.83 - 1.97 (m, 2H), 2.08 - 2.18 (m, 2H), 2.17 (s, 3H), 2.22 - 2.32 (m, 2H), 2.56 - 2.67 (m, 2H), 4.34 (bs, 2H), 4.58 (s, 2H), 4.78 (s, 1H), 7.11 (d, 1H), 7.21 (d, 1H), 7.32 (d, 1H), 7.48 (dd, 1H), 7.71 (dd, 1H), 8.10 (d, 1H), 8.50 (s, 1H), 9.20 (bs, 1H), 10.04 (s, 1H): Mass Spectrum MH⁺ 471.

### Reference Example 38

### 4-(3-Methyl-4-hydroxyanilino)-5-(1-methylpiperidin-4-yloxy)quinazoline

A solution of hydrochloric acid in dioxane (4M, 2 ml) was added to a mixture of 4-chloro-5-(1-methylpiperidin-4-yloxy)quinazoline **(reference example 2)** (2.78 g) and 4-amino-2-methylphenol (1.35 g) in THF (150 ml). The resulting suspension was heated at reflux for 3 hours, then allowed to cool to room temperature. Filtration gave the crude product as a yellow solid, which was partitioned between aqueous sodium hydrogen carbonate solution and DCM. Combined organic extracts were dried and concentrated to give the crude product, which was purified by trituration under cold methanol to give the title compound as a white solid (2.88 g, 79%); NMR spectrum (CDCl₃) 2.0 (m, 2H), 2.2 (m, 2H), 2.3 (s, 3H), 2.3 (s, 3H), 2.4 (m, 2H), 2.8 (m, 2H), 4.7 (m, 1H) 6.7 (d, 1H), 6.9 (d, 1H), 7.3 (dd, 1H), 7.4 (d, 1H), 7.4 (d, 1H), 7.6 (t, 1H), 8.6 (s, 1H), 9.9 (s, 1H); Mass spectrum MH⁺ 365.

### Reference Example 39

### 1,5-Dimethyl-3-hydroxymethylpyrazole

A solution of lithium aluminium hydride (1M solution in THF, 17.8 ml) was added to a solution of 1,5-dimethylpyrazole-3-carboxylic acid (500 mg) in THF (20 ml) at room temperature. The mixture was heated at reflux for 4 hours, then cooled in an ice bath. Ethyl acetate (25 ml) was added dropwise to destroy the excess lithium aluminium hydride (heat evolved), and resultant mixture was then poured into aqueous hydrochloric acid (1M, 50 ml). The product was extracted into ethyl acetate, dried and concentrated *in vacuo* to give the title compound as a pale brown oil (230 mg, 51%); NMR spectrum (DMSO-d6) 2.2 (s, 3H), 3.6 (s, 3H), 4.3 (s, 2H), 5.9 (s, 1H).

The procedure described above was repeated using the appropriate acid or ester. Thus was obtained the compound described below:

### Reference Example 39.1

### 1-Methyl-3-hydroxymethylpyrazole

Obtained from 1-methylpyrazole-3-carboxylic acid in 38% yield; NMR spectrum (DMSO-d6) 3.8 (s, 3H), 4.4 (s, 2H), 6.1 (d, 1H), 7.5 (d, 1H).

### Reference Example 40

### 3-(2-Chloro-4-nitrophenoxymethyl)-1,5-dimethylpyrazole

A solution di-*tert*-butylazodicarboxylate (419 mg) in DCM (2 ml) was added to a solution of 2-chloro-4-nitrophenol (211 mg), triphenylphosphine (478 mg), and 1,5-dimethyl-3-hydroxymethylpyrazole **(reference example 39)** (230mg) in DCM (8 ml), which was stirred and cooled in an ice bath, under a nitrogen atmosphere. The mixture was then allowed to warm to room temperature, and stirred for 3 hours. The reaction mixture was concentrated *in vacuo,* and purified by chromatography, using 0-5% methanol in DCM as eluent. This gave the title compound (230 mg, 67%); Mass spectrum MH⁺ 282.

The procedure described above was repeated using the appropriate phenol and alcohol. Thus was obtained the compound described below:

### Reference Example 40.1

### 3-(2-Chloro-4-nitrophenoxymethyl)-1-methylpyrazole

Obtained by reacting 2-chloro-4-nitrophenol and 1-methyl-3-hydroxymethylpyrazole **(reference example 39.1)** in 49% yield; NMR spectrum (DMSO-d6) 3.8 (s, 3H), 5.3 (s, 2H), 6.3 (d, 1H), 7.5 (d, 1H), 7.7 (d, 1H), 8.2 (m, 1H), 8.3 (m, 1H); Mass spectrum MH⁺268.

### Reference Example 40.2

### 5-(2-Chloro-4-nitrophenoxymethyl)-3-methylisoxazole

Obtained by reacting 2-chloro-4-nitrophenol (251 mg) and 5-(hydroxymethyl)-3-methylisoxazole **(reference example 27.2)** in 100% yield; NMR spectrum (DMSO-d6) 2.24 (s, 3H), 5.5 (s, 2H), 6.5 (s, 1H), 7.5 (d, 1H), 8.2 (m, 1H), 8.3 (m, 1H); Mass spectrum MH⁺267.

### Reference Example 41

### 3-Methylisoxazole-5-carboxylic acid methyl ester

Potassium carbonate (1.19 g) was added portionwise to a cooled solution (0°C) of 3-methylisoxazole-5-carboxylic acid (500 mg) in DMF (25 ml). After 5 minutes at 0°C, dimethyl sulphate (0.37 ml) was added dropwise. The reaction mixture was stirred at room temperature for 1 hour, then concentrated *in vacuo.* Water was added, and the product was extracted into DCM. The combined organics were dried, then concentrated *in vacuo* to give the title compound as a white solid (424 mg, 76%); NMR spectrum (DMSO-d6) 2.3 (s, 3H), 3.9 (s, 3H), 7.1 (s,1H).

### Reference Example 42

### 4-(3-Chloro-4-hydroxyanilino)-5-(tetrahydropyran-4-yloxy)quinazoline

Phosphorus oxychloride (2.2 ml) was added dropwise to a solution of 3,4-dihydro-5-(tetrahydropyran-4-yloxy)quinazolin-4-one **(reference example 1.2)** (623 mg) and di-*iso-*propylethylamine (1.36 ml) in anhydrous 1, 2-dichloroethane (25 ml) at 0°C. The mixture was heated at 80°C for 3 hours and then concentrated *in vacuo.* The residue was azeotroped with toluene (20 ml), di-*iso*-propylethylamine (5 ml) was added and the mixture was again concentrated *in vacuo.* The residue was dissolved in IPA (10 ml) and 4-amino-2-chlorophenol (475 mg) was added. The resulting mixture was heated at 80°C for 12 hours and then concentrated *in vacuo.* The residue was purified by chromatography, using 0-5% methanol - DCM as eluent, to give the title compound as a solid (338 mg, 36%); NMR Spectrum (DMSO-d6) 1.79 -1.95 (m, 2H), 2.15 - 2.26 (m, 2H), 3.51 - 3.60 (m, 2H), 3.84 - 3.96 (m, 2H), 4.92 - 5.01 (m, 1H), 7.00 (d, 1H), 7.23 (d, 1H), 7.29 - 7.38 (m, 2H), 7.68 (t, 1H), 8.02 (d, 1H), 8.46 (s, 1H), 9.98 (s, 1H); Mass spectrum MH⁺ 372.

The procedure described above was repeated using the appropriate 3,4-dihydroquinazolin-4-one and aniline. Thus were obtained the compounds described below:

### Reference Example 42.1

### 4-(3-Chloro-4-hydroxyanilino)-5-(tetrahydrofuran-3-yloxy)quinazoline

Obtained from 3,4-dihydro-5-(tetrahydrofuran-3-yloxy)quinazolin-4-one **(reference example 1.3)** and 4-amino-2-chlorophenol in 48% yield; NMR Spectrum (DMSO-d6) 2.12 - 2.21 (m, 1H), 2.17 - 2.41 (m, 1H), 3.78 - 4.00 (m, 3H), 4.18 (d, 1H), 5.40 - 5.44 (m, 1H), 6.98 (d, 1H), 7.18 (d, 1H), 7.36 (d, 1H), 7.40 (dd, 1H), 7.72 (t, 1H), 8.03 (d, 1H), 8.48 (s, 1H), 9.96 (s, 1H); Mass spectrum MH⁺ 358.

### Reference Example 42.2

### 4-(3-Chloro-4-(3-fluorobenzyloxy)aniline)-5-(1,4-dioxaspiro[4.5]dec-8-yloxy)quinazoline

Obtained from 3,4-dihydro-5-(1, 4-dioxaspiro[4.5]dec-8-yloxy)quinazolin-4-one **(reference example 1.4)** and 3-chloro-4-(3-fluorobenzyloxy)aniline**(reference example 8.1)** in 89% yield; NMR Spectrum (DMSO-d6) 1.70 (m, 4H), 1.96 (m, 2H), 2.12 (m, 2H), 3.88 (s, 4H), 4.87 (m, 1H), 5.24 (s, 2H), 7.17 (dt, 1H). 7.20 - 7.35 (m, 6H), 7.45 (m, 2H), 7.73 (t, 1H), 8.10 (d, 1H), 8.50 (s, 1H), 10.00 (bs, 1H); Mass Spectrum M⁺ 536.

### Reference Example 43

### 3-Fluoro-4-(1-methyl-1H-imidazol-2-ylthio)nitrobenzene

To a stirred solution of 2-mercapto-1-methyl-1*H*- imidazole (1.14 g), in DMF (20 ml), was added sodium hydride (0.44 g) in small portions and the reaction stirred at ambient temperature until effervescence ceased. To this was added a solution of 3,4-difluoronitrobenzene (1.59 g) in DMF (10 ml), and the solution stirred at 80°C for 4 hours. The reaction was poured into water (150 ml) and organic material extracted into ethyl acetate (150 ml). The organic layer was washed successively with water (3 x 150ml), brine (150 ml) and dried. Evaporation of the solvent gave an oil which was purified by chromatography using ethyl acetate and then 10% methanol / ethyl acetate as eluent to give title compound as a solid (1.8 g, 70%); NMR spectrum (DMSO-d6) 2.5 (s, 3H), 6.7 - 6.9 (t, 1H), 7.2 (t, 1H), 7.6 (s, 1H), 7.95 - 8.05 (dd, 1H), 8.15 - 8.25 (dd, 1H); Mass spectrum MH⁺ 254.

The procedure described above was repeated using the appropriate thiol. Thus was obtained the compound described below:

### Reference Example 43.1

### 3-Fluoro-4-(1-methyl-1H-1,3,4-triazol-2-ylthio)nitrobenzene

Obtained by reacting 3,4-difluoronitrobenzene with 1-methyl-2-mercaptotriazole in 39% yield; NMR spectrum (CDCl₃ 3.7 (s, 3H), 7.3 - 7.4 (t, 1H), 7.9 - 8.0 (m, 1H), 8.36 (s, 1H); Mass spectrum MH⁺ 255.

### Reference Example 44

### 3-Chloro-4-(2-pyridylthio)nitrobenzene

To a stirred solution of 2-mercaptopyridine (1.11 g) in acetonitrile (25 ml) was added potassium carbonate (2.76 g), 3 chloro-4-fluoronitrobenzene (1.75 g) and *cis*-dicyclohexano-18-crown-6 (10 mg) and the solution was stirred and heated at reflux for 6 hours. The solution was filtered and evaporated and the residue purified by chromatography using *iso*-hexane / ethyl acetate (2: 1) to ethyl acetate as eluent to give a solid which was recrystallised from ethyl acetate / *iso*-hexane to give the title compound as a yellow solid (1.5 g, 58%); NMR spectrum (CDCl₃) 7.2 - 7.3(m, 2H), 7.4 - 7.48 (d, 1H), 7.5 - 7.55 (d, 1H), 7.65 - 7.75 (dt, 1H), 8.0 - 8.1 (dd, 1H), 8.3 (d, 1H), 8.6 (m, 1H); Mass spectrum MH⁺ 267.

The procedure described above was repeated using the appropriate thiol and fluoronitrobenzene. Thus was obtained the compound described below:

### Reference Example 44.1

### 3-Chloro-4-(2-pyrimidinylthio)nitrobenzene

Obtained by reacting 3-chloro-4-fluoro-nitrobenzene with 2-mercaptopyrimidine in 75% yield; NMR spectrum (CDCl₃) 7.02 - 7.1 (t, 1H), 7.94 - 8.0 (d, 1H), 8.1 - 8.2 (dd, 1H), 8.4 (d, 1H), 8.5 (d, 2H); Mass spectrum MH⁺268.

### Reference Example 44.2

### 3-Chloro-4-(1H-imidazol-2-ylthio)nitrobenzene

Obtained by reacting 3-chloro-4-fluoro-nitrobenzene with 2-mercaptoimidazole in 58% yield; Mass spectrum MH⁺ 255.

### Reference Example 44.3

### 3-Fluoro-4-(1H-imidazol-2-ylthio)nitrobenzene

Obtained by reacting 3,4-difluoronitrobenzene and 2-mercaptoimidazole in 38% yield; Mass spectrum MH⁺ 240.

### Reference Example 44.4

### 4-(2-Thiazolylthio)nitrobenzene

Obtained by reacting 4-fluoronitrobenzene and 2-mercaptothiazole in 77% yield; NMR spectrum (CDCl₃) 7.5 (d, 1H), 7.5 - 7.6 (d, 2H), 7.9 (d, 1H), 8.1 - 8.2 (d, 2H).

### Reference Example 44.5

### 3-Chloro-4-(2-thiazolylthio)nitrobenzene

Obtained by reacting 3-chloro-4-fluoronitrobenzene and 2-mercaptothiazole in 65% yield; NMR spectrum (CDCl₃ 7.25 (d, 1H), 7.6 (d, 1H), 8.0 (d, 1H), 8.0 - 8.05 (d, 1H), 8.15 (d, 1H); Mass spectrum MH⁺ 273.

### Reference Example 45

### 4-(2-Thiazolylsulphonyl)nitrobenzene

To a solution of 4-(2-thiazolylthio)nitrobenzene **(reference example 44.4)** (238 mg) in DCM (50 ml) was added 3-chloroperbenzoic acid (500 mg) and the reaction stirred for 18 hours. The reaction was evaporated and the residue dissolved in ethyl acetate (100 ml) and washed with sodium hydroxide solution (2 x 50ml, 2N), water (2 x 50ml), brine (50 ml) and dried. Concentration of the solvent gave the title compound as a white solid (200 mg, 69%); NMR spectrum (CDCl₃) 7.75 (d, 1H), 8.0 (d, 1H), 8.3 - 8.4 (d, 2H), 8.4 - 8.5 (d, 2H).

### Reference Example 46

### 1,4-Dioxaspiro[4.5]decan-8-ol

Sodium borohydride (1.61 g) was added to a solution of 1, 4-dioxaspiro[4.5]decan-8-one (6.65 g) in ethanol (100 ml) and the reaction stirred for 4 hours. Water (10 ml) was added and the reaction stirred for 16 hours. The resulting solid was filtered and the filtrate concentrated *in vacuo.* The residue was purified by chromatography using *iso*-hexane - 30% ethyl acetate as eluent to give the title compound as a colourless oil (5.49 g, 82%); NMR Spectrum (CDCl₃) 1.50 - 1.95 (m, 8H), 3.80 (m, 1H), 3.95 (s, 4H); Mass Spectrum MH⁺ 159.

### Reference Example 47

### 4-(3-Chloro-4-(3-fluorobenzyloxy)anilino)-5-(1-oxo-cyclohex-4-yloxy)quinazoline acetate

A solution of 4-(3-chloro-4-(3-fluorobenzyloxy)anilino)-5-(1,4-dioxaspiro[4.5]dec-8-yloxy)quinazoline **(reference example 42.2)** (2.25 g) in acetic acid (40 ml) and water (10 ml) was heated at 90°C for 18 hours, then concentrated *in vacuo.* The residue was triturated with ether to give the title compound as a white solid (1.98 g, 85%); Mass Spectrum M⁺ 492.

### Reference Example 48

### Methanesulphonic acid 2-methyloxazol-4-ylmethyl ester

To a solution of (2-methyloxazol-4-yl)methanol (obtained as described in J. Chem. Soc., Perkin Trans. 1, 2000, 2415 - 2428) (112 mg) in DCM (10 ml) was added triethylamine (275 µl). The solution was cooled to 0°C and methane sulphonyl chloride (85 µl) was added dropwise. The reaction mixture was allowed to warm up to ambient temperature and left stirring for nine hours. The mixture was concentrated *in vacuo* to give the title compound which was used without further purification (103mg; 40%); Mass Spectrum M-H⁺ 192.

### Reference Example 49

### 2-Chloro-5-fluoro-4-nitrophenol

2-Chloro-5-fluorophenol (1.46 g) was stirred in ethanol and ferric nitrate monohydrate (4.04 g) added slowly. The solution turned blue and was heated at 50°C for 5 hours. The solution was cooled and evaporated to ¼ volume and 1N hydrochloric acid (100 ml) and water (100 ml) added and the solution extracted with ethyl acetate, washed with water, brine, dried and evaporated to give an oil which was purified by chromatography, using *iso*-hexane / ethyl acetate (3:1) as eluent to give the title compound as a solid; NMR spectrum (CDCl₃) 6.2-6.4 (bs, 1H), 6.9 - 6.95 (d, 1H), 8.2 (d, 1H).

## Claims

1. A quinazoline derivative of the Formula I 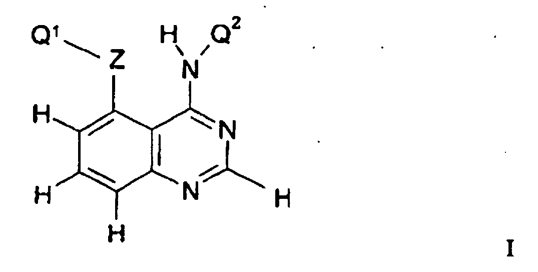 wherein:
the Q¹-Z- group is selected from cyclopentyloxy, cyclohexyloxy, tetrahydrofuran-3-yloxy, tetrahydropyran-3-yloxy, tetrahydropyran-4-yloxy, tetrahydrothiopyran-3-yloxy, tetrahydrothiopyran-4-yloxy, 1,1-dioxotetrahydrothiopyran-3-yloxy, 1,1-dioxotetrahydrothiopyran-4-yloxy, 1-oxotetrahydrothiopyran-3-yloxy, 1-oxotetrahydrothiopyran-4-yloxy, tetrahydrothien-3-yloxy, 1,1-dioxotetrahydrothien-3-yloxy, 1-oxotetrahydrothien-3-yloxy, pyrrolidin-3-yloxy, pyrrolidin-2-yloxy, piperidin-3-yloxy, piperidin-4-yloxy, homopiperidin-3-yloxy, homopiperidin-4-yloxy and azetidin-3-yloxy,
and wherein any azetidinyl, pyrrolidinyl, piperidinyl or homopiperidinyl group within the Q¹-Z- group is optionally N- substituted by a substituent T¹ selected from (1-4C)alkyl, (2-4C)alkenyl, (2-4C)alkynyl, (2-4C)alkanoyl, (1-4C)alkoxycarbonyl, carbamoyl, N-(1-4C)alkylcarbamoyl, N,N-di-(1-4C)alkylcarbamoyl and (1-4C)alkylsulphonyl,
and wherein adjacent carbon atoms in any (2-4C)alkylene chain within the N-substituent T¹ are optionally separated by the insertion into the chain of a group selected from O, NH and CO,
and wherein any CH₂ or CH₃ group within the N- substituent T¹ optionally bears on each said CH₂ or CH₃ group a substituent selected from hydroxy, amino, methylamino, di-methylamino, ethylamino, diethylamino, carbamoyl, N-methylcarbamoyl, N,N-dimethylcarbamoyl, acetyl, methoxycarbonyl and ethoxycarbonyl,
and wherein any heterocyclyl group within the Q¹-Z- group optionally bears 1 or 2 oxo substituents;
Q² is selected from a group of formula Ia, Ib and Id 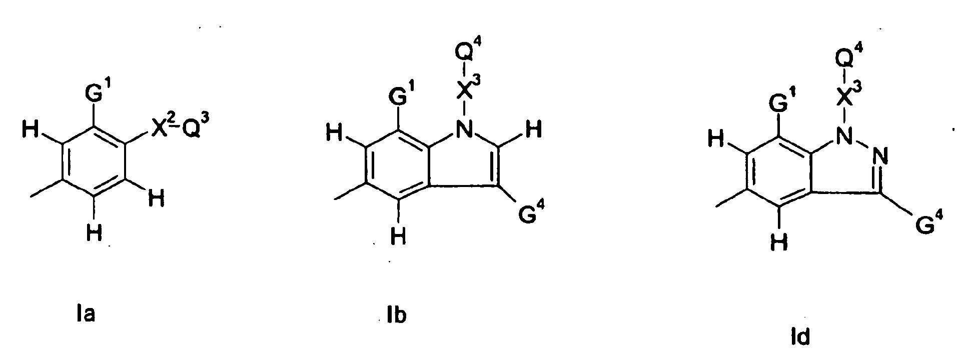
wherein G¹ is selected from hydrogen, fluoro, chloro, bromo, cyano, (1-4C)alkyl and (2-3C)alkynyl,
G⁴ is selected from hydrogen, fluoro, chloro, bromo, cyano, (1-4C)alkyl and ethynyl,
X² is selected from O, S, NH, N(CH₃), OCH₂, CO and NHCH₂,
and Q³ is a group selected from phenyl, 2-furyl, 2- or 3-thienyl, 2-,4-or 5-oxazolyl, 3-,4- or 5-isoxazolyl, 2-,4-or 5-1H-imidazolyl, 2-,4-or 5-thiazolyl, 3- or 4-(1H-1,2,4-triazolyl), 3- or 5-(1,2,5-thiadiazolyl), 2- 3- or 4-pyridyl, 2-, 4- or 5-pyrimidinyl, 1,3-benzodioxol-4-yl, 1,3-benzodioxol-5-yl, 2-, 3-, 4-, 5-, 6-, 7- or 8-quinolinyl, 1-, 3-, 4-, 5-, 6-, 7- or 8-isoquinolinyl and 2-, 3-, 4-, 5-, 6-, 7- or 8-quinazolinyl, which group is optionally substituted with one or two substituents, which may be the same or different, selected from nitro, fluoro, chloro, bromo, (1-4C)alkyl, trifluoromethyl, (1-4C)alkoxy, (2-3C)alkynyl and cyano,
or X² is CO and Q³ is a group selected from pyrrolidin-1yl, piperidino, homopiperidino, morpholino, piperazin-1-yl, homopiperazin-1-yl, decahydroquinolin-1-yl, and decahydroisoquinolin-2-yl, which group optionally bears 1 or 2 substituents which may be the same or different selected from fluoro, chloro, bromo, cyano, hydroxy and (1-4C)alkyl,
and any heterocyclyl group represented by Q³ optionally bears 1 or 2 oxo substituents,
X³ is selected from SO₂ and CH₂,
Q⁴ is a group selected from phenyl, 2-furyl, 3-furyl, 2-(1,3-oxazolyl), 4-(1,3-oxazolyl), 3-, 4- or 5-isoxazolyl, 2-imidazolyl, 4-imidazolyl, 2-, 3-or 4-pyridyl, 2-, 4-or 5- pyrimidinyl, 1,2,4-triazol-3-yl, 2-thienyl, 3-thienyl, 2-(1,3-thiazolyl), 4-(1,3-thiazolyl), 3-, 4- or 5- isothiazolyl and 1,2,5-thiadiazol-3-yl group which group optionally bears 1 or 2 substituents, which may be the same or different, selected from nitro, fluoro, chloro, bromo, cyano, (1-4C)alkyl, (1-4C)alkoxy, trifluoromethyl and (2-3C)alkynyl;
or a pharmaceutically acceptable salt thereof.

2. A quinazoline derivative according to claim 1, wherein the Q¹-Z- group is selected from cyclopentyloxy, tetrahydrofuran-3-yloxy, tetrahydropyran-4-yloxy, tetrahydrothiopyran-4-yloxy, 1,1-dioxotetrahydrothiopyran-4-yloxy, 1-oxotetrahydrothiopyran-4-yloxy; tetrahydrothien-3-yloxy, 1,1-dioxotetrahydrothien-3-yloxy, 1-oxotetrahydrothien-3-yloxy, pyrrolidin-3-yloxy, pyrrolidin-2-yloxy, piperidin-3-yloxy, piperidin-4-yloxy, homopiperidin-3-yloxy, homopiperidin-4-yloxy and azetidin-3-yloxy,
and wherein the azetidinyl, pyrrolidinyl, piperidinyl or homopiperidinyl group within the Q¹-Z- group is optionally N- substituted by a substituent selected from methyl, ethyl, n-propyl, iso-propyl, n-butyl, iso-butyl, tert-butyl, allyl, 2-propynyl, acetyl, propionyl, methoxycarbonyl, ethoxycarbonyl, propoxycarbonyl, tert-butoxycarbonyl, methylsulphonyl, ethylsulphonyl, 2-methoxyethyl, carbamoylmethyl, N-methylcarbamoylmethyl, N,N-di-methylcarbamoylmethyl, 2-carbamoylethyl, 2-(N-methylcarbamoyl)ethyl, 2-(N,N-di-methylcarbamoyl)ethyl, acetylmethyl, 2-acetylethyl, methoxycarbonylmethyl and 2-methoxycarbonylethyl,
and wherein any heterocyclyl group within the Q¹-Z- group optionally bears 1 or 2 oxo substituents;
or a pharmaceutically acceptable salt thereof.

3. A quinazoline derivative according to claim 1, wherein the Q¹-Z- group is piperidin-4-yloxy which group is optionally N-substituted by a substituent selected from methyl, ethyl, n-propyl, iso-propyl, allyl, 2-propynyl, methoxycarbonyl, ethoxycarbonyl, propoxycarbonyl, tert-butoxycarbonyl, methylsulphonyl, 2-methoxyethyl, carbamoylmethyl, N-methylcarbamoylmethyl, N,N-di-methylcarbamoylmethyl, acetylmethyl, 2-acetylethyl, methoxycarbonylmethyl and 2-methoxycarbonylethyl,
and wherein any heterocyclyl group within the Q¹-Z- group optionally bears 1 or 2 oxo substituents;
or a pharmaceutically acceptable salt thereof.

4. A quinazoline derivative according to claim 1, wherein Q² is a group of the formula Ia wherein
G¹ is selected from fluoro, chloro, bromo and ethynyl,
X² is CO and Q³ is selected from piperidino, homopiperidino, decahydroquinolin-1-yl, and decahydroisoquinolin-2-yl,
and wherein Q³ optionally bears 1 or 2 substituents selected from fluoro, chloro, bromo, cyano, hydroxy, methyl and ethyl,
and wherein Q³ optionally bears 1 or 2 oxo substituents;
or a pharmaceutically acceptable salt thereof.

5. A quinazoline derivative according to claim 1, wherein Q² is a group of the formula Ib wherein G¹ is hydrogen,
G⁴ is selected from hydrogen, fluoro, chloro, bromo, cyano, methyl and ethynyl,
X³ is selected from SO₂ and CH₂, and Q⁴ is selected from phenyl and 2-, 3- or 4-pyridyl which optionally bears 1 or 2 substituents, which may be the same or different, selected from fluoro, chloro, bromo, nitro, cyano and methyl;
or a pharmaceutically acceptable salt thereof.

6. A quinazoline derivative according to claim 1, wherein Q² is a group of the formula Id wherein G¹ is hydrogen,
G⁴ is selected from hydrogen, fluoro, chloro, bromo, cyano, methyl and ethynyl,
X³ is selected from SO₂ and CH₂, and Q⁴ is selected from phenyl and 2-, 3- or 4-pyridyl which optionally bears 1 or 2 substituents, which may be the same or different, selected from fluoro, chloro, bromo, nitro, cyano and methyl;
or a pharmaceutically acceptable salt thereof.

7. A quinazoline derivative according to claim 1, wherein the Q¹-Z- group is selected from tetrahydrofuran-3-yloxy, tetrahydropyran-3-yloxy and tetrahydropyran-4-yloxy,
and wherein Q¹ optionally bears an oxo substituent;
or a pharmaceutically acceptable salt thereof.

8. A quinazoline derivative according to claim 1, wherein the Q¹-Z- group is piperidin-4-yloxy optionally substituted at the 1 position by a substituent selected from methyl, allyl, 2-methoxyethyl and carbamoylmethyl,
and wherein the piperidin-4-yl group optionally bears an oxo substituent;
or a pharmaceutically acceptable salt thereof.

9. A quinazoline derivative according to claim 1, wherein the Q¹-Z- group is 1-methylpiperidin-4-yloxy;
or a pharmaceutically acceptable salt thereof.

10. A quinazoline derivative according to claim 1, wherein Q² is a group of the formula Ia wherein
G¹ is chloro,
X² is OCH₂,
Q³ is phenyl optionally substituted by 1 or 2 substituents selected from fluoro and cyano;
or a pharmaceutically acceptable salt thereof.

11. A quinazoline derivative according to claim 10, wherein Q³ is selected from phenyl, 2-fluorophenyl, 3-fluorophenyl, 2-cyanophenyl, 3-cyanophenyl, 2,5-difluorophenyl and 2,6-difluorophenyl;
or a pharmaceutically acceptable salt thereof.

12. A quinazoline derivative according to claim 1, wherein Q² is a group of the formula Id wherein G¹ and G⁴ are hydrogen, X³ is CH₂ and Q⁴ is phenyl optionally substituted by fluoro or chloro,
or a pharmaceutically acceptable salt thereof.

13. A quinazoline derivative according to claim 12, wherein Q⁴ is 3-fluorophenyl;
or a pharmaceutically acceptable salt thereof.

14. A quinazoline derivative according to claim 1 selected from:
4-(3-Chloro-4-phenoxyanilino)-5-(1-methylpiperidin-4-yloxy)quinazoline;
4-(3-Chloro-4-((3-fluorobenzyloxy)anilino))-5-(1-methylpiperidin-4-yloxy)quinazoline;
4-(1-(3-Fluorobenzyl)indazol-5-ylamino)-5-(1-methylpiperidin-4-yloxy)quinazoline;
4-(3-Chloro-4-((decahydroquinolin-1-yl)carbonyl)anilino)-5-(1-methylpiperidin-4-yloxy)quinazoline;
4-(3-Chloro-4-((decahydroisoquinolin-2-yl)carbonyl)anilino)-5-(1-methylpiperidin-4-yloxy)quinazoline;
4-(3-Chloro-4-((3-methylpiperidin-1-yl)carbonyl)anilino)-5-(1-methylpiperidin-4-yloxy)quinazoline;
4-(3-Ethynyl-4-((decahydroquinolin-1-yl)carbonyl)anilino)-5-(1-methylpiperidin-4-yloxy)quinazoline;
4-(3-Chloro-4-(5-methylisoxazol-3-ylmethoxy)anilino)-5-(1-methylpiperidin-4-yloxy)quinazoline;
4-(3-Chloro-4-(2, 6-difluorobenzyloxy)anilino)-5-(1-methylpiperidin-4-yloxy)quinazoline;
4-(3-chloro-4-(2-fluorobenzyloxy)anilino)-5-(1-methylpiperidin-4-yloxy)quinazoline;
4-(3-Chloro-4-(2-cyanobenzyloxy)anilino)- 5-(1-methylpiperidin-4-yloxy)quinazoline; and
4-(3-Chloro-4-(thiazol-4-ylmethoxy)anilino)-5-(1-methylpiperidin-4-yloxy)quinazoline;
or a pharmaceutically acceptable acid addition salt thereof.

15. A quinazoline derivative according to claim 1 selected from:
4-(1-Benzylindol-5-ylamino)-5-(1-methylpiperidin-4-yloxy)quinazoline;
4-(1-Benzenesulphonylindol-5-ylamino)-5-(1-methylpiperidin-4-yloxy)quinazoline;
4-(1-(2-Cyanobenzyl)indol-5-ylamino)-5-(1-methylpiperidin-4-yloxy)quinazoline;
4-(1-(2-Methoxybenzyl)indol-5-ylamino)-5-(1-methylpiperidin-4-yloxy)quinazoline;
4-(1-(2-Chlorobenzyl)indol-5-ylamino)-5-(1-methylpiperidin-4-yloxy)quinazoline;
4-(1-(2,5-Dimethylbenzyl)indol-5-ylamino)-5-(1-methylpiperidin-4-yloxy)quinazoline;
4-(1-(2-Fluorobenzyl)indol-5-ylamino)-5-(1-methylpiperidin-4-yloxy)quinazoline; and
4-(1-(3-Fluorobenzyl)indol-5-ylamino)-5-(1-methylpiperidin-4-yloxy)quinazoline
or a pharmaceutically acceptable acid addition salt thereof.

16. A quinazoline derivative according to claim 1 selected from:
4-(3-Chloro-4-(3-fluorophenoxy)anilino)-5-(1-methylpiperidin-4-yloxy)quinazoline; and
4-(3-Chloro-4-((1-methyl-*1H*-imidazol-2-yl)thio)anilino)-5-(1-methylpiperidin-4-yloxy)quinazoline;
or a pharmaceutically acceptable acid addition salt thereof.

17. A quinazoline derivative according to claim 1 selected from:
4-(3-Chloro-4-(3-fluorobenzyloxy)anilino)-5-(tetrahydropyran-4-yloxy)quinazoline;
4-(4-Benzyloxy-3-methylanilino)-5-(1-methylpiperidin-4-yloxy)quinazoline;
4-(3-Methyl-4-(5-methylisoxazol-3-ylmethoxy)anilino)-5-(1-methylpiperidin-4-yloxy)quinazoline;
4-(4-(3-Fluorobenzyloxy)-3-methylanilino)-5-(1-methylpiperidin-4-yloxy)quinazoline;
4-(3-Ethynyl 4-(3-fluorobenzyloxy)anilino)-5-(1-methylpiperidin-4-yloxy)quinazoline;
4-(3-Ethynyl 4-(2,6-difluorobenzyloxy)anilino)-5-(1-methylpiperidin-4-yloxy)quinazoline; and
4-(3-Chloro-4-(pyridin-2-ylmethoxy)anilino)-5-(1-methylpiperidin-4-yloxy)quinazoline;
or a pharmaceutically acceptable acid addition salt thereof.

18. A process for the preparation of a quinazoline derivative according to claim 1, or a pharmaceutically acceptable salt thereof, which comprises:
(a) the reaction of a quinazoline of the Formula II 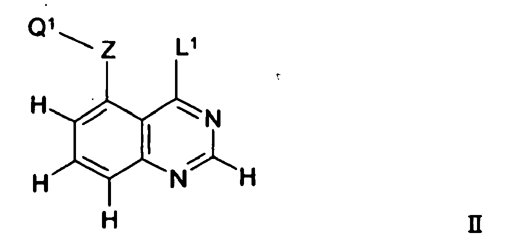 wherein L¹ is a displaceable group and Q¹ and Z have any of the meanings defined in claim 1 except that any functional group is protected if necessary, with an compound of the Formula
Q²NH₂
wherein Q² has any of the meanings defined in claim 1 except that any functional group is protected if necessary, whereafter any protecting group that is present is removed by conventional means; or
(b) the coupling of an alcohol of the Formula
Q¹-OH
wherein Q¹ has any of the meanings defined in claim 1 (and Z is O) except that any functional group is protected if necessary with a quinazoline of the Formula VI 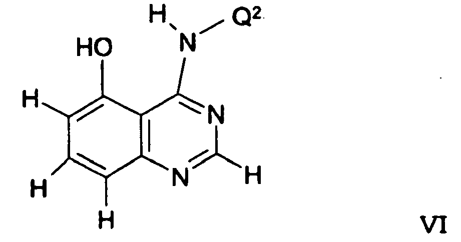 wherein Q² has any of the meanings defined in claim 1 except that any functional group is protected if necessary, whereafter any protecting group that is present is removed by conventional means; or
(c) the reaction of an alcohol of the Formula
Q¹-OH
wherein Q¹ has any of the meanings defined in claim 1 (and Z is O) except that any functional group is protected if necessary with a quinazoline of the Formula VIII 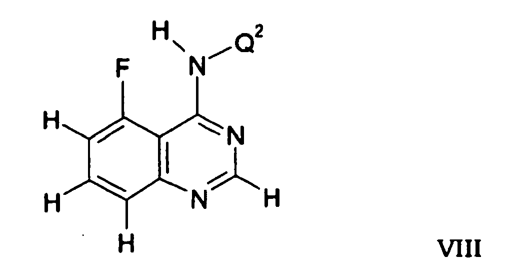 wherein Q² has any of the meanings defined in claim 1 except that any functional group is protected if necessary, whereafter any protecting group that is present is removed by conventional means; or
(d) for the production of those compounds of the Formula I wherein Q¹ or Q² contains a primary or secondary amino group, the cleavage of the corresponding compound of Formula I wherein Q¹ or Q² contains a protected primary or secondary amino group; or
(e) for the production of those compounds of the Formula I wherein Q¹ or Q² contains a (1-6C)alkoxy or substituted (1-6C)alkoxy group or a (1-6C)alkylamino or substituted (1-6C)alkylamino group, the alkylation of a quinazoline derivative of the formula I wherein Q¹ or Q² contains a hydroxy group or a primary or secondary amino group as appropriate; or
(f) for the production of those compounds of the Formula I wherein Q¹ contains an amino-hydroxy-disubstituted (1-6C)alkoxy group, the reaction of a compound of the Formula I wherein Q¹ contains an epoxy-substituted (1-6C)alkoxy group with a heterocycle or an appropriate amine; or
(g) the reaction of a quinazoline of the Formula X 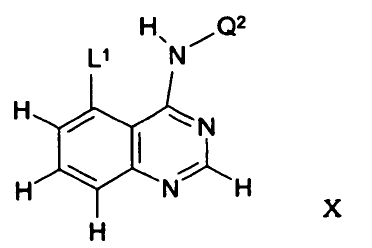 wherein L¹ is a displaceable group and Q² has any of the meanings defined in claim 1 except that any functional group is protected if necessary, with a compound of the Formula
Q¹ZH
wherein Q¹ and Z have any of the meanings defined in claim 1 except that any functional group is protected if necessary, whereafter any protecting group that is present is removed by conventional means; or
(h) for the production of those compounds of the Formula I wherein Q¹ contains an amino-substituted (1-6C)alkoxy group, the reaction of a compound of the Formula I wherein Q¹ contains a halogeno-substituted (1-6C)alkoxy group with an appropriate amine or a heterocycle containing a ring NH group; or
(i) for the production of those compounds of the Formula I wherein a heterocyclyl group in Q¹ or Q³ contains an S- or N-oxide the oxidation of a ring N or S atom in a compound of the formula (I); or
(j) for the production of those compounds of the Formula I wherein the group X²-Q³ is COQ³ and Q³ is a nitrogen linked heterocyclyl group as defined in claim 1, the coupling of the carboxy substituted quinazoline of the formula XI: 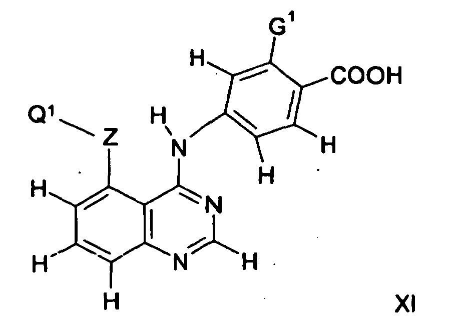 or a reactive derivative thereof, with Q³H, wherein Q¹, Q³, Z and G¹ are as defined in claim 1 except that any functional group is protected if necessary, whereafter any protecting group that is present is removed by conventional means; or
(k) for the production of those compounds wherein Q² is a group of the Formula Ia wherein:
(i) X²Q³ is OCH₂Q³ and Q³ is aryl or heteroaryl as defined in claim 1, or
(ii) X²Q³ is OQ³ and Q³ is heteroaryl as defined in claim 1, the reaction, optionally in the presence of a base, of a compound of formula I as defined in claim 1 wherein Q² is a group of the formula Ia as defined in claim 1 except that the group X²Q³ in formula Ia is OH, with a compound of the formula L¹CH₂Q³ or L¹Q³,
wherein L¹ is a displaceable group, and Q³ is as defined in claim 1 except any functional group is protected if necessary, and whereafter any protecting group that is present is removed by conventional means; or
(l) for the production of those compounds of the formula I wherein Q¹ contains an (2-6C)alkanoylamino or substituted (2-6C)alkanoylamino group, the acylation of a quinazoline derivative of the formula I wherein Q¹ contains an amino group; or
(m) for the production of those compounds of the Formula I wherein Q² is an indole or indazole derivative of the formula Ib or Id as defined in claim 1, the reaction of a compound of the formula Q⁴X³L¹, wherein L¹ is a displaceable group and Q⁴ and X³ are as defined in claim 1, except that any functional group is protected if necessary, with a compound of the formula XII, 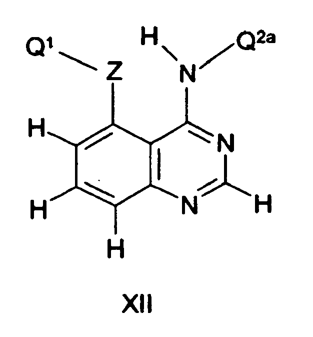 wherein Q¹-Z- is as defined in claim 1, except that any functional group is protected if necessary, and Q^{2a} is selected from a compound of the formula Ib' and Id' 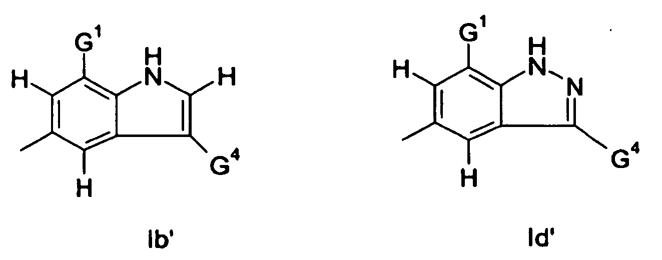 wherein G¹ and G⁴ have any of the meanings defined in claim 1, except that any functional group is protected if necessary, whereafter any protecting group that is present is removed by conventional means; or
(n) for the production of those compounds of the Formula I wherein Q² is a group of the formula la wherein Q³ is aryl or heteroaryl as defined in claim 1 and X² is S, the coupling of the compound of formula XVIII 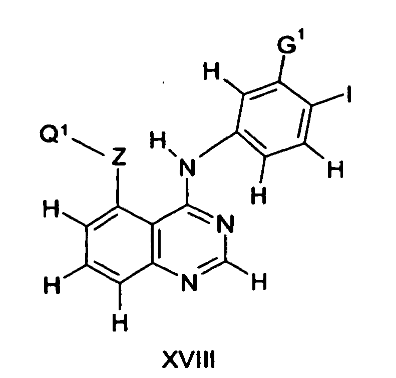 wherein Q¹, Z and G¹ have any of the meanings defined in claim 1, except that any functional group is protected if necessary, with a compound of the formula Q³SH, wherein Q³ is aryl or heteroaryl as defined in claim 1, except that any functional group is protected if necessary, in the presence of cuprous bromide, and a base, whereafter any protecting group that is present is removed by conventional means;
(o) for the production of those compounds of the Formula I wherein Q¹ or Q² contains an (1-6C)alkylamino, substituted (1-6C)alkylamino group or a nitrogen linked heterocyclyl group, the reductive amination of an aldehyde or ketone group in a compound of formula 1, with a (1-6C)alkylamino, substituted (1-6C)alkylamino group or a heterocyclyl group containing an NH group in the presence of a suitable reducing agent; or
(p) the conversion of one compound of the Formula I into another compound of the Formula I;
and when a pharmaceutically acceptable salt of a quinazoline derivative of formula I is required it may be obtained using a conventional procedure.

19. A pharmaceutical composition which comprises a quinazoline derivative of the Formula I, or a pharmaceutically acceptable thereof, as defined in claim 1 in association with a pharmaceutically acceptable diluent or carrier.

20. A quinazoline derivative of the Formula I, or a pharmaceutically acceptable salt thereof, as defined in claim 1 for use in a method of treatment of the human or animal body by therapy.

21. The use of a quinazoline derivative of the Formula I, or a pharmaceutically acceptable salt thereof, as defined in claim 1 in the manufacture of a medicament for use in the prevention or treatment of tumours which are sensitive to the inhibition of one or more erbB receptor tyrosine kinases.

22. The use of a quinazoline derivative of the Formula I, or a pharmaceutically acceptable salt thereof, as defined in claim 1 in the manufacture of a medicament for use in the treatment of a cancer selected from leukaemia, multiple myeloma, lymphoma, bile duct, bone, bladder, brain/CNS, breast, colorectal, endometrial, gastric, head and neck, hepatic, lung, neuronal, oesophageal, ovarian, pancreatic, prostate, renal, skin, testicular, thyroid, uterine and vulval cancer.

## Patentansprüche

1. Chinazolinderivat der Formel I 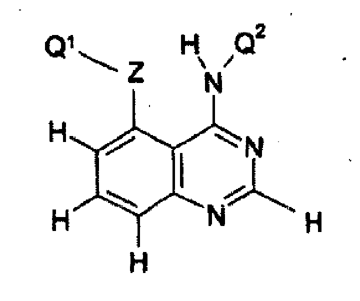 worin:
die Gruppe Q¹-Z- unter Cyclopentyloxy, Cyclohexyloxy, Tetrahydrofuran-3-yloxy, Tetrahydropyran-3-yloxy, Tetrahydropyran-4-yloxy, Tetrahydrothiopyran-3-yloxy, Tetrahydrothiopyran-4-yloxy, 1,1-Dioxotetrahydrothiopyran-3-yloxy, 1,1-Dioxotetrahydrothiopyran-4-yloxy, 1-Oxotetrahydrothiopyran-3-yloxy, 1-Oxotetrahydrothiopyran-4-yloxy, Tetrahydrothien-3-yloxy, 1,1-Dioxotetrahydrothien-3-yloxy, 1-Oxotetrahydrothien-3-yloxy, Pyrrolidin-3-yloxy, Pyrrolidin-2-yloxy, Piperidin-3-yloxy, Piperidin-4-yloxy, Homopiperidin-3-yloxy, Homopiperidin-4-yloxy und Azetidin-3-yloxy ausgewählt ist,
und worin jede Azetidinyl-, Pyrrolidinyl-, Piperidinyl- oder Homopiperidinylgruppe in der Gruppe Q¹-2- gegebenenfalls durch einen unter C₁₋₄-Alkyl, C₂₋₄-Alkenyl, C₂₋₄-Alkinyl, C₂₋₄-Alkanoyl, C₁₋₄-Alkoxycarbonyl, Carbamoyl, N-C₁₋₄-Alkylcarbamoyl, N,N-Di-C₁₋₄-alkylcarbamonyl und C₁₋₄-Alkylsulfonyl ausgewählten Substituenten T¹ N-substituiert ist,
und worin benachbarte Kohlenstoffatome in jeder C₂₋₄-Alkylenkette im N-Substituenten T¹ gegebenenfalls durch Einschub einer unter O, NH und CO ausgewählten Gruppe in die Kette getrennt sind,
und worin jede CH₂- oder CH₃-Gruppe im N-Substituenten T¹ gegebenenfalls an jeder CH₂- oder CH₃-Gruppe einen unter Hydroxy, Amino, Methylamino, Dimethylamino, Ethylamino, Diethylamino, Carbamoyl, N-Methylcarbamoyl, N,N-Dimethylcarbamoyl, Acetyl, Methoxycarbonyl und Ethoxycarbonyl ausgewählten Substituenten trägt,
und worin jede Heterocyclylgruppe in der Gruppe Q¹-Z- gegebenenfalls 1 oder 2 Oxosubstituenten trägt; Q² unter einer Gruppe der Formel Ia, Ib und Id 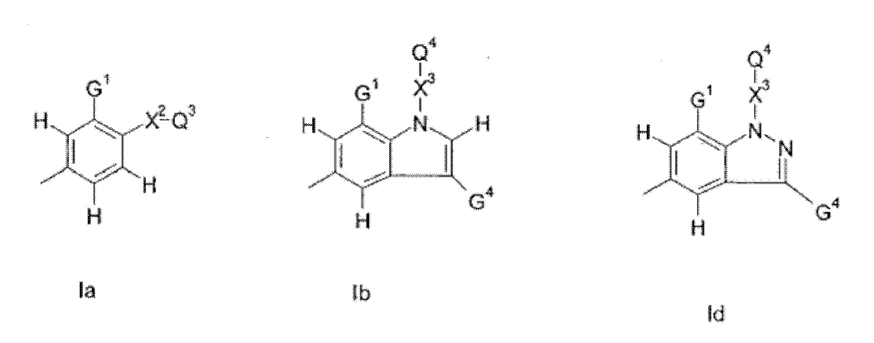
worin G¹ unter Wasserstoff, Fluor, Chlor, Brom, Cyano, C₁₋₄-Alkyl und C₂₋₃-Alkinyl ausgewählt ist,
G⁴ unter Wasserstoff, Fluor, Chlor, Brom, Cyano, C₁₋₄-Alkyl und Ethinyl ausgewählt ist,
X² unter O, S, NH, N(CH₃), OCH₂, CO und NHCH₂ ausgewählt ist,
und Q³ für eine unter Phenyl, 2-Furyl, 2- oder 3-Thienyl, 2-, 4- oder 5-Oxazolyl, 3-, 4- oder 5-Isoxazolyl, 2-, 4- oder 5-1H-Imidazolyl, 2-, 4-oder 5-Thiazolyl, 3- oder 4-(1H-1,2,4-Triazolyl), 3- oder 5-(1,2,5-Thiadiazolyl), 2-, 3- oder 4-Pyridyl, 2-, 4- oder 5-Pyrimidinyl, 1,3-Benzodioxol-4-yl, 1,3-Benzodioxol-5-yl, 2-, 3-, 4-, 5-, 6-, 7- oder 8-Chinolinyl, 1-, 3-, 4-, 5-, 6-, 7-, oder 8-Isochinolinyl und 2-, 3-, 4-, 5-, 6-, 7- oder 8-Chinazolinyl und ausgewählte Gruppe steht, welche gegebenenfalls durch einen oder zwei Substituenten, die gleich oder verschieden sein können und unter Nitro, Fluor, Chlor, Brom, C₁₋₄-Alkyl, Trifluormethyl, C₁₋₄-Alkoxy, C₂₋₃-Alkinyl und Cyano ausgewählt sind, substituiert ist,
oder X² für CO steht und Q³ für eine unter Pyrrolidin-1-yl, Piperidino, Homopiperidino, Morpholino, Piperazin-1-yl, Homopiperazin-1-yl, Decahydrochinolin-1-yl und Decahydroisochinolin-2-yl ausgewählte Gruppe steht, welche gegebenenfalls 1 oder 2 Substituenten trägt, die gleich oder verschieden sein können und unter Fluor, Chlor, Brom, Cyano, Hydroxy und C₁₋₄-Alkyl ausgewählt sind,
und jede durch Q³ wiedergegebene Heterocyclylgruppe gegebenenfalls 1 oder 2 Oxosubstituenten trägt, X³ unter SO₂ und CH₂ ausgewählt ist,
Q⁴ für eine unter Phenyl, 2-Furyl, 3-Furyl, 2-(1,3-Oxazolyl), 4-(1,3-Oxazolyl), 3-, 4- oder 5-Isoxazolyl, 2-Imidazolyl, 4-Imidazolyl, 2-, 3-oder 4-Pyridyl, 2-, 4- oder 5-Pyrimidinyl, 1,2,4-Triazol-3-yl, 2-Thienyl, 3-Thienyl, 2-(1,3-Thiazolyl), 4-(1,3-Thiazolyl), 3-, 4- oder 5-Isothiazolyl und 1,2,5-Thiadiazol-3-yl ausgewählte Gruppe steht, welche gegebenenfalls 1 oder 2 Substituenten trägt, die gleich oder verschieden sein können und unter Nitro, Fluor, Chlor, Brom, Cyano, C₁₋₄-Alkyl, C₁₋₄-Alkoxy, Trifluormethyl und C₂₋₃-Alkinyl ausgewählt sind,
ausgewählt ist;
oder ein pharmazeutisch annehmbares Salz davon.

2. Chinazolinderivat nach Anspruch 1, worin die Gruppe Q¹-Z- unter Cyclopentyloxy, Tetrahydrofuran-3-yloxy, Tetrahydropyran-4-yloxy, Tetrahydrothiopyran-4-yloxy, 1,1-Dioxotetrahydrothiopyran-4-yloxy, 1-Oxotetrahydrothiopyran-4-yloxy, Tetrahydrothien-3-yloxy, 1,1-Dioxotetrahydrothien-3-yloxy, 1-Oxotetrahydrothien-3-yloxy, Pyrrolidin-3-yloxy, Pyrrolidin-2-yloxy, Piperidin-3-yloxy, Piperidin-4-yloxy, Homopiperidin-3-yloxy, Homopiperidin-4-yloxy und Azetidin-3-yloxy ausgewählt ist,
und worin die Azetidinyl-, Pyrrolidinyl-, Piperidinyl- oder Homopiperidinylgruppe in der Gruppe Q¹-Z- gegebenenfalls durch einen unter Methyl, Ethyl, n-Propyl, Isopropyl, n-Butyl, Isobutyl, tert-Butyl, Allyl, 2-Propinyl, Acetyl, Propionyl, Methoxycarbonyl, Ethoxycarbonyl, Propoxycarbonyl, tert-Butoxycarbonyl, Methylsulfonyl, Ethylsulfonyl, 2-Methoxyethyl, Carbamoylmethyl, N-Methylcarbamoylmethyl, N,N-Dimethylcarbamoylmethyl, 2-Carbamoylethyl, 2-(N-Methylcarbamoyl)ethyl, 2-(N,N-Dimethylcarbamoyl)ethyl, Acetylmethyl, 2-Acetylethyl, Methoxycarbonylmethyl und 2-Methoxycarbonylethyl ausgewählten Substituenten N-substituiert ist, und worin jede Heterocyclylgruppe in der Gruppe Q¹-Z- gegebenenfalls 1 oder 2 Oxosubstituenten trägt; oder ein pharmazeutisch annehmbares Salz davon.

3. Chinazolinderivat nach Anspruch 1, worin die Gruppe Q¹-Z- für Piperidin-4-yloxy steht, das gegebenenfalls durch einen unter Methyl, Ethyl, n-Propyl, Isopropyl, Allyl, 2-Propinyl, Methoxycarbonyl, Ethoxycarbonyl, Propoxycarbonyl, tert-Butoxycarbonyl, Methylsulfonyl, 2-Methoxyethyl, Carbamoylmethyl, N-Methylcarbamoylmethyl, N,N-Dimethylcarbamoylmethyl, Acetylmethyl, 2-Acetylethyl, Methoxycarbonylmethyl und 2-Methoxycarbonylethyl ausgewählten Substituenten N-substituiert ist,
und worin jede Heterocyclylgruppe in der Gruppe Q¹-Z- gegebenenfalls 1 oder 2 Oxosubstituenten trägt; oder ein pharmazeutisch annehmbares Salz davon.

4. Chinazolinderivat nach Anspruch 1, worin Q² für eine Gruppe der Formel Ia, worin
G¹ unter Fluor, Chlor, Brom und Ethinyl ausgewählt ist,
X² für CO steht und Q³ unter Piperidino, Homopiperidino, Decahydrochinolin-1-yl und Decahydroisochinolin-2-yl ausgewählt ist,
und worin Q³ gegebenenfalls 1 oder 2 unter Fluor, Chlor, Brom, Cyano, Hydroxy, Methyl und Ethyl ausgewählte Substituenten trägt,
und worin Q³ gegebenenfalls 1 oder 2 Oxosubstituenten trägt,
steht;
oder ein pharmazeutisch annehmbares Salz davon.

5. Chinazolinderivat nach Anspruch 1, worin Q² für eine Gruppe der Formel Ib, worin
G¹ für Wasserstoff steht,
G⁴ unter Wasserstoff, Fluor, Chlor, Brom, Cyano, Methyl und Ethinyl ausgewählt ist,
X³ unter SO₂ und CH₂ ausgewählt ist und Q⁴ unter Phenyl und 2-, 3- oder 4-Pyridyl, das gegebenenfalls 1 oder 2 Substituenten trägt, die gleich oder verschieden sein können und unter Fluor, Chlor, Brom, Nitro, Cyano und Methyl ausgewählt sind, ausgewählt ist,
steht;
oder ein pharmazeutisch annehmbares Salz davon.

6. Chinazolinderivat nach Anspruch 1, worin Q² für eine Gruppe der Formel Id, worin
G¹ für Wasserstoff steht,
G⁴ unter Wasserstoff, Fluor, Chlor, Brom, Cyano, Methyl und Ethinyl ausgewählt ist,
X³ unter SO₂ und CH₂ ausgewählt ist und Q⁴ unter Phenyl und 2-, 3- oder 4-Pyridyl, das gegebenenfalls 1 oder 2 Substituenten trägt, die gleich oder verschieden sein können und unter Fluor, Chlor, Brom, Nitro, Cyano und Methyl ausgewählt sind, ausgewählt ist,
steht;
oder ein pharmazeutisch annehmbares Salz davon.

7. Chinazolinderivat nach Anspruch 1, worin die Gruppe Q¹-2- unter Tetrahydrofuran-3-yloxy, Tetrahydropyran-3-yloxy und Tetrahydropyran-4-yloxy ausgewählt ist,
und worin Q¹ gegebenenfalls einen Oxosubstituenten trägt;
oder ein pharmazeutisch annehmbares Salz davon.

8. Chinazolinderivat nach Anspruch 1, worin die Gruppe Q¹-Z- für Piperidin-4-yloxy, das gegebenenfalls in der 1-Position durch einen unter Methyl, Allyl, 2-Methoxyethyl und Carbamoylmethyl ausgewählten Substituenten substituiert ist,
steht,
und worin die Piperidin-4-ylgruppe gegebenenfalls einen Oxosubstituenten trägt;
oder ein pharmazeutisch annehmbares Salz davon.

9. Chinazolinderivat nach Anspruch 1, worin die Gruppe Q¹-2- für 1-Methylpiperidin-4-yloxy steht; oder ein pharmazeutisch annehmbares Salz davon.

10. Chinazolinderivat nach Anspruch 1, worin Q² für eine Gruppe der Formel Ia, worin
G¹ für Chlor steht,
X² für OCH₂ steht,
Q³ für Phenyl, das gegebenenfalls durch 1 oder 2 unter Fluor und Cyano ausgewählte Substituenten substituiert ist, steht,
steht;
oder ein pharmazeutisch annehmbares Salz davon.

11. Chinazolinderivat nach Anspruch 10, worin Q³ unter Phenyl, 2-Fluorphenyl, 3-Fluorphenyl, 2-Cyanophenyl, 3-Cyanophenyl, 2,5-Difluorphenyl und 2,6-Difluorphenyl ausgewählt ist;
oder ein pharmazeutisch annehmbares Salz davon.

12. Chinazolinderivat nach Anspruch 1, worin Q² für eine Gruppe der Formel Id, worin G¹ und G⁴ für Wasserstoff stehen, X³ für CH₂ steht und Q⁴ für Phenyl, das gegebenenfalls durch Fluor oder Chlor substituiert ist, steht, steht;
oder ein pharmazeutisch annehmbares Salz davon.

13. Chinazolinderivat nach Anspruch 12, worin Q⁴ für 3-Fluorphenyl steht;
oder ein pharmazeutisch annehmbares Salz davon.

14. Chinazolinderivat nach Anspruch 1, ausgewählt unter:
4-(3-Chlor-4-phenoxyanilino)-5-(1-methylpiperidin-4-yloxy)chinazolin;
4-(3-Chlor-4-((3-fluorbenzyloxy)anilino))-5-(1-methylpiperidin-4-yloxy)chinazolin;
4-(1-(3-Fluorbenzyl) indazol-5-ylamino)-5-(1-methylpiperidin-4-yloxy)chinazolin;
4-(3-Chlor-4-((decahydrochinolin-1-yl)carbonyl)-anilino)-5-(1-methylpiperidin-4-yloxy)chinazolin;
4-(3-Chlor-4-((decahydroisochinolin-2-yl)-carbonyl) anilino)-5-(1-methylpiperidin-4-yloxy)-chinazolin;
4-(3-Chlor-4-((3-methylpiperidin-1-yl)carbonyl)-anilino)-5-(1-methylpiperidin-4-yloxy)chinazolin;
4-(3-Ethinyl-4-((decahydrochinolin-1-yl)carbonyl)-anilino)-5-(1-methylpiperidin-4-yloxy)chinazolin;
4-(3-Chlor-4-(5-methylisoxazol-3-ylmethoxy)-anilino)-5-(1-methylpiperidin-4-yloxy)chinazolin;
4-(3-Chlor-4-(2,6-difluorbenzyloxy)anilino)-5-(1-methylpiperidin-4-yloxy)chinazolin;
4-(3-Chlor-4-(2-fluorbenzyloxy)anilino)-5-(1-methylpiperidin-4-yloxy)chinazolin;
4-(3-Chlor-4-(2-cyanobenzyloxy)anilino)-5-(1-methylpiperidin-4-yloxy)chinazolin und
4-(3-Chlor-4-(thiazol-4-ylmethoxy)anilino)-5-(1-methylpiperidin-4-yloxy)chinazolin;
oder ein pharmazeutisch annehmbares Säureadditionssalz davon.

15. Chinazolinderivat nach Anspruch 1, ausgewählt unter:
4-(1-Benzylindol-5-ylamino)-5-(1-methylpiperidin-4-yloxy)chinazolin;
4-(1-Benzolsulfonylindol-5-ylamino)-5-(1-methylpiperidin-4-yloxy)chinazolin;
4-(1-(2-Cyanobenzyl)indol-5-ylamino)-5-(1-methylpiperidin-4-yloxy)chinazolin;
4-(1-(2-Methoxybenzyl)indol-5-ylamino)-5-(1-methylpiperidin-4-yloxy)chinazolin;
4-(1-(2-Chlorbenzyl)indol-5-ylamino)-5-(1-methylpiperidin-4-yloxy)chinazolin;
4-(1-(2,5-Dimethylbenzyl)indol-5-ylamino)-5-(1-methylpiperidin-4-yloxy)chinazolin;
4-(1-(2-Fluorbenzyl)indol-5-ylamino)-5-(1-methylpiperidin-4-yloxy)chinazolin und
4-(1-(3-Fluorbenzyl)indol-5-ylamino)-5-(1-methylpiperidin-4-yloxy)chinazolin;
oder ein pharmazeutisch annehmbares Säureadditionssalz davon.

16. Chinazolinderivat nach Anspruch 1, ausgewählt unter:
4-(3-Chlor-4-(3-fluorphenoxy)anilino)-5-(1-methylpiperidin-4-yloxy)chinazolin und
4-(3-Chlor-4-((1-methyl-1H-imidazol-2-yl)thio)-anilino)-5-(1-methylpiperidin-4-yloxy)chinazolin;
oder ein pharmazeutisch annehmbares Säureadditionssalz davon.

17. Chinazolinderivat nach Anspruch 1, ausgewählt unter:
4-(3-Chlor-4-(3-fluorbenzyloxy)anilino)-5-(tetrahydropyran-4-yloxy)chinazolin;
4-(4-Benzyloxy-3-methylanilino)-5-(1-methylpiperidin-4-yloxy)chinazolin;
4-(3-Methyl-4-(5-methylisoxazol-3-ylmethoxy)-anilino)-5-(1-methylpiperidin-4-yloxy)chinazolin;
4-(4-(3-Fluorbenzyloxy)-3-methylanilino)-5-(1-methylpiperidin-4-yloxy)chinazolin;
4-(3-Ethinyl-4-(3-fluorbenzyloxy)anilino)-5-(1-methylpiperidin-4-yloxy)chinazolin;
4-(3-Ethinyl-4-(2,6-difluorbenzyloxy)anilino)-5-(1-methylpiperidin-4-yloxy)chinazolin und
4-(3-Chlor-4-(pyridin-2-ylmethoxy)anilino)-5-(1-methylpiperidin-4-yloxy)chinazolin;
oder ein pharmazeutisch annehmbares Säureadditionssalz davon.

18. Verfahren zur Herstellung eines Chinazolinderivats nach Anspruch 1 oder eines pharmazeutisch annehmbaren Salzes davon, bei dem man:
(a) ein Chinazolin der Formel II 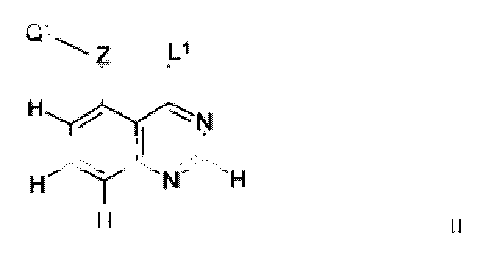 worin L¹ für eine austauschbare Gruppe steht und Q¹ und Z eine der in Anspruch 1 definierten Bedeutungen besitzen, wobei jedoch jede funktionelle Gruppe gegebenenfalls geschützt ist, mit einer Verbindung der Formel
Q²NH₂
worin Q² eine der in Anspruch 1 definierten Bedeutungen besitzt, wobei jedoch jede funktionelle Gruppe gegebenenfalls geschützt ist, umsetzt und danach jede vorhandene Schutzgruppe mit herkömmlichen Mitteln abspaltet; oder
(b) einen Alkohol der Formel
Q¹-OH
worin Q¹ eine der in Anspruch 1 definierten Bedeutungen besitzt (und Z für O steht), wobei jedoch jede funktionelle Gruppe gegebenenfalls geschützt ist, mit einem Chinazolin der Formel VI 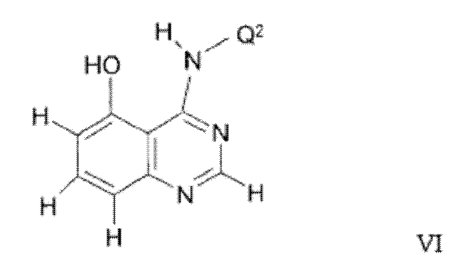 worin Q² eine der in Anspruch 1 definierten Bedeutungen besitzt, wobei jedoch jede funktionelle Gruppe gegebenenfalls geschützt ist, kuppelt und danach jede vorhandene Schutzgruppe mit herkömmlichen Mitteln abspaltet; oder
(c) einen Alkohol der Formel
Q¹-OH
worin Q¹ eine der in Anspruch 1 definierten Bedeutungen besitzt (und Z für 0 steht), wobei jedoch jede funktionelle Gruppe gegebenenfalls geschützt ist, mit einem Chinazolin der Formel VIII 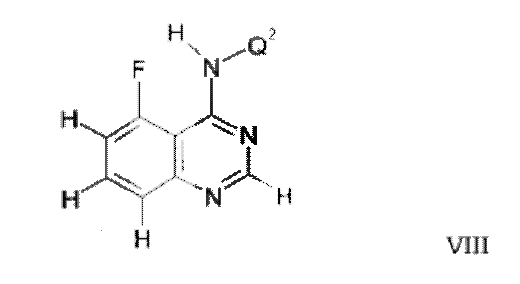 worin Q² eine der in Anspruch 1 definierten Bedeutungen besitzt, wobei jedoch jede funktionelle Gruppe gegebenenfalls geschützt ist, umsetzt und danach jede vorhandene Schutzgruppe mit herkömmlichen Mitteln abspaltet; oder
(d) zur Herstellung derjenigen Verbindungen der Formel I, worin Q¹ oder Q² eine primäre oder sekundäre Aminogruppe enthält, die entsprechende Verbindung der Formel (I), worin Q¹ oder Q² eine geschützte primäre oder sekundäre Aminogruppe enthält, spaltet; oder
(e) zur Herstellung derjenigen Verbindungen der Formel I, worin Q¹ oder Q² eine C₁₋₆-Alkoxy- oder substituierte C₁₋₆-Alkoxygruppe oder eine C₁₋₆-Alkylamino- oder substituierte C₁₋₆-Alkylaminogruppe enthält, ein Chinazolinderivat der Formel I, worin Q¹ oder Q² eine Hydroxygruppe bzw. eine primäre oder sekundäre Aminogruppe enthält, alkyliert; oder
(f) zur Herstellung derjenigen Verbindungen der Formel I, worin Q¹ eine amino-hydroxydisubstituierte C₁₋₆-Alkoxygruppe enthält, eine Verbindung der Formel I, worin Q¹ eine epoxysubstituierte C₁₋₆-Alkoxygruppe enthält, mit einem Heterocyclus oder einem geeigneten Amin umsetzt; oder
(g) ein Chinazolin der Formel X 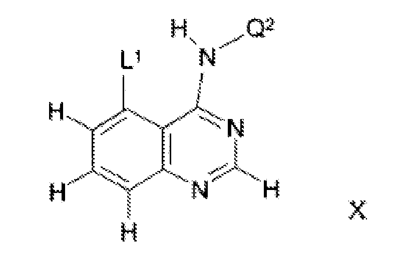 worin L¹ für eine austauschbare Gruppe steht und Q² eine der in Anspruch 1 definierten Bedeutungen besitzt, wobei jedoch jede funktionelle Gruppe gegebenenfalls geschützt ist, mit einer Verbindung der Formel
Q¹ZH
worin Q¹ und Z eine der in Anspruch 1 definierten Bedeutungen besitzen, wobei jedoch jede funktionelle Gruppe gegebenenfalls geschützt ist, umsetzt und danach jede vorhandene Schutzgruppe mit herkömmlichen Mitteln abspaltet; oder
(h) zur Herstellung derjenigen Verbindungen der Formel I, worin Q¹ eine aminosubstituierte C₁₋₆-Alkoxygruppe enthält, eine Verbindung der Formel I, worin Q¹ eine halogensubstituierte C₁₋₆-Alkoxygruppe enthält, mit einem geeigneten Amin oder einem Heterocyclus mit einer Ring-NH-Gruppe umsetzt; oder
(i) zur Herstellung derjenigen Verbindungen der Formel I, worin eine Heterocyclylgruppe in Q¹ oder Q³ ein S- oder N-Oxid enthält, ein Ring-N-Atom oder Ring-S-Atom in einer Verbindung der Formel (I) oxidiert; oder
(j) zur Herstellung derjenigen Verbindungen der Formel I, worin die Gruppe X²-Q³ für COQ³ steht und Q³ für eine über Stickstoff gebundene Heterocyclylgruppe gemäß Anspruch 1 steht, das carboxysubstituierte Chinazolin der Formel XI: 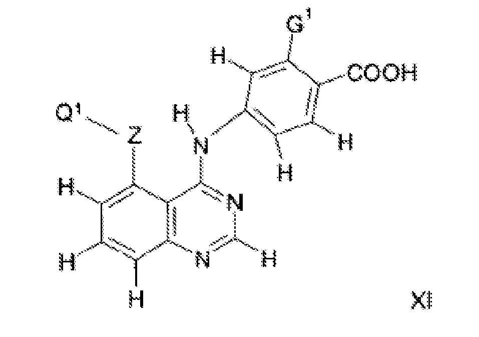 oder ein reaktives Derivat davon mit Q³H kuppelt, worin Q¹, Q³, Z und G¹ eine der in Anspruch 1 definierten Bedeutungen besitzen, wobei jedoch jede funktionelle Gruppe gegebenenfalls geschützt ist, und danach jede vorhandene Schutzgruppe mit herkömmlichen Mitteln abspaltet; oder
(k) zur Herstellung derjenigen Verbindungen, worin Q² für eine Gruppe der Formel Ia, worin:
(i) X²Q³ für OCH₂Q³ steht und Q³ für Aryl oder Heteroaryl gemäß Anspruch 1 steht, oder
(ii) X²Q³ für OQ³ steht und Q³ für Heteroaryl gemäß Anspruch 1 steht,
steht, eine Verbindung der Formel I gemäß Anspruch 1, worin Q² für eine Gruppe der Formel Ia gemäß Anspruch 1 steht, wobei jedoch die Gruppe X²Q³ in Formel Ia für OH steht, mit einer Verbindung der Formel L¹CH₂Q³ oder L¹Q³ umsetzt, gegebenenfalls in Gegenwart einer Base,
worin L¹ für eine austauschbare Gruppe steht und Q³ die in Anspruch 1 angegebene Bedeutung besitzt, wobei jedoch jede funktionelle Gruppe gegebenenfalls geschützt ist, und danach jede vorhandene Schutzgruppe mit herkömmlichen Mitteln abspaltet; oder
(l) zur Herstellung derjenigen Verbindungen der Formel I, worin Q¹ eine C₂₋₆-Alkanoylamino- oder substituierte C₂₋₆-Alkanoylaminogruppe enthält, ein Chinazolinderivat der Formel I, worin Q¹ eine Aminogruppe enthält, acyliert; oder
(m) zur Herstellung derjenigen Verbindungen der Formel I, worin Q² für ein Indol- oder Indazolderivat der Formel Ib oder Id gemäß Anspruch 1 steht, eine Verbindung der Formel Q⁴X³L¹, worin L¹ für eine austauschbare Gruppe steht und Q⁴ und X³ die in Anspruch 1 angegebene Bedeutung besitzen, wobei jedoch jede funktionelle Gruppe gegebenenfalls geschützt ist, mit einer Verbindung der Formel XII 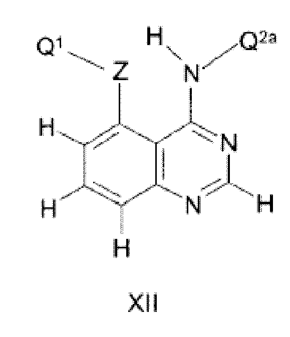 worin Q¹-2- die in Anspruch 1 angegebene Bedeutung besitzt, wobei jedoch jede funktionelle Gruppe gegebenenfalls geschützt ist, und Q^{2a} unter einer Verbindung der Formel Ib' und Id' 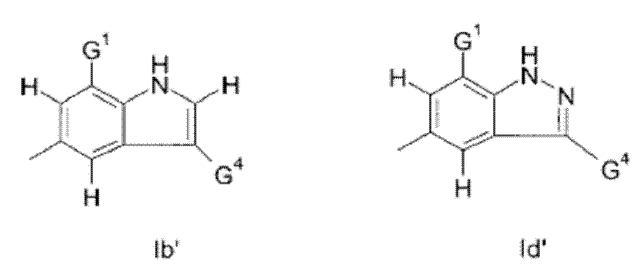 worin G¹ und G⁴ eine der in Anspruch 1 angegebenen Bedeutungen besitzen, wobei jedoch jede funktionelle Gruppe gegebenenfalls geschützt ist, ausgewählt ist, umsetzt und danach jede vorhandene Schutzgruppe mit herkömmlichen Mitteln abspaltet; oder
(n) zur Herstellung derjenigen Verbindungen der Formel I, worin Q² für eine Gruppe der Formel Ia,
worin Q³ für Aryl oder Heteroaryl gemäß Anspruch 1 steht und X² für S steht, steht, eine Verbindung der Formel XVIII 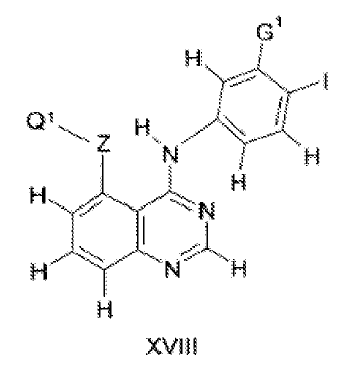 worin Q¹, Z und G¹ eine der in Anspruch 1 angegebenen Bedeutungen besitzen, wobei jedoch jede funktionelle Gruppe gegebenenfalls geschützt ist, in Gegenwart von Kupfer(I)-bromid und einer Base mit einer Verbindung der Formel Q³SH, worin Q³ für Aryl oder Heteroaryl gemäß Anspruch 1 steht, wobei jedoch jede funktionelle Gruppe gegebenenfalls geschützt ist, kuppelt und danach jede vorhandene Schutzgruppe mit herkömmlichen Mitteln abspaltet; oder
(o) zur Herstellung derjenigen Verbindungen der Formel I, worin Q¹ oder Q² eine C₁₋₆-Alkylamino- oder substituierte C₁₋₆-Alkylaminogruppe oder eine über Stickstoff gebundene Heterocyclylgruppe enthält, eine Aldehyd- oder Ketongruppe in einer Verbindung der Formel 1 mit einer C₁-₆-Alkylamino- oder substitiuerten C₁₋₆-Alkylaminogruppe oder einer Heterocyclylgruppe mit einer NH-Gruppe in Gegenwart eines geeigneten Reduktionsmittels reduktiv aminiert; oder
(p) eine Verbindung der Formel I in eine andere Verbindung der Formel I umwandelt;
und ein gegebenenfalls gewünschtes pharmazeutisch annehmbares Salz eines Chinazolinderivats der Formel I nach einer herkömmlichen Vorgehensweise erhältlich ist.

19. Pharmazeutische Zusammensetzung, die ein Chinazolinderivat der Formel I oder ein pharmazeutisch annehmbares Salz davon nach Anspruch 1 zusammen mit einem pharmazeutisch annehmbaren Verdünnungsmittel oder Träger enthält.

20. Chinazolinderivat der Formel I oder ein pharmazeutisch annehmbares Salz davon nach Anspruch 1 zur Verwendung bei einem Verfahren zur Behandlung des menschlichen oder tierischen Körpers durch Therapie.

21. Verwendung eines Chinazolinderivats der Formel I oder eines pharmazeutisch annehmbaren Salzes davon nach Anspruch 1 bei der Herstellung eines Arzneimittels zur Verwendung bei der Prävention oder Behandlung von Tumoren, die gegenüber der Inhibierung einer oder mehrerer erbB-Rezeptor-Tyrosinkinasen empfindlich sind.

22. Verwendung eines Chinazolinderivats der Formel I oder eines pharmazeutisch annehmbaren Salzes davon nach Anspruch 1 bei der Herstellung eines Arzneimittels zur Verwendung bei der Behandlung einer unter Leukämie, multiplem Myelom, Lymphom, Gallengangs-, Knochen-, Blasen-, Hirn/ZNS-, Brust-, Kolorektal-, Endometrial-, Magen-, Kopf- und Hals-, Leber-, Lungen-, Nerven-, Speiseröhren-, Eierstock-, Bauchspeicheldrüsen-, Prostata-, Nieren-, Haut-, Hoden-, Schilddrüsen-, Gebärmutter- und Vulvakrebs ausgewählten Krebserkrankung.

## Revendications

1. Dérivé de la quinazoline de formule I 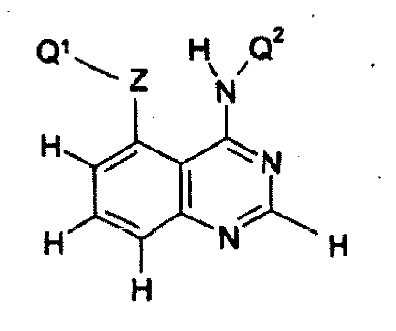 dans laquelle :
le groupement Q¹-Z- est choisi parmi cyclopentyloxy, cyclohexyloxy, tétrahydrofuran-3-yloxy, tétrahydropyran-3-yloxy, tétrahydropyran-4-yloxy, tétrahydrothiopyran-3-yloxy, tétrahydrothiopyran-4-yloxy, 1,1-dioxotétrahydrothiopyran-3-yloxy, 1,1-dioxotétrahydrothiopyran-4-yloxy, 1-oxotétrahydrothiopyran-3-yloxy, 1-oxotétrahydrothiopyran-4-yloxy, tétrahydrothién-3-yloxy, 1,1-dioxotétrahydrothién-3-yloxy, 1-oxotétrahydrothién-3-yloxy, pyrrolidin-3-yloxy, pyrrolidin-2-yloxy, pipéridin-3-yloxy, pipéridin-4-yloxy, homopipéridin-3-yloxy, homopipéridin-4-yloxy et azétidin-3-yloxy,
et où un groupement azétidinyle, pyrrolidinyle, pipéridinyle ou homopipéridinyle quelconque dans le groupement Q¹-Z- est éventuellement N-substitué par un substituant T¹ choisi parmi (1-4C)alkyle, (2-4C)alcényle, (2-4C)alcynyle, (2-4C)alcanoyle, (1-4C)alcoxycarbonyle, carbamoyle, N-(1-4C)alkylcarbamoyle, N,N-di-(1-4C)alkylcarbamoyle et (1-4C)alkylsulfonyle,
et où les atomes de carbone adjacents dans une chaîne (2-4C)alkylène quelconque dans le N-substituant T¹ sont éventuellement séparés par l'insertion dans la chaîne d'un groupement choisi parmi O, NH et CO,
et où un groupement CH₂ ou CH₃ quelconque dans le N-substituant T¹ porte éventuellement sur chaque dit groupement CH₂ ou CH₃ un substituant choisi parmi hydroxy, amino, méthylamino, di-méthylamino, éthylamino, diéthylamino, carbamoyle, N-méthylcarbamoyle, N,N-diméthylcarbamoyle, acétyle, méthoxycarbonyle et éthoxycarbonyle,
et où un groupement hétérocyclyle quelconque dans le groupement Q¹-2- porte éventuellement 1 ou 2 substituants oxo ;
Q² est choisi parmi un groupe de formule Ia, Ib et Id 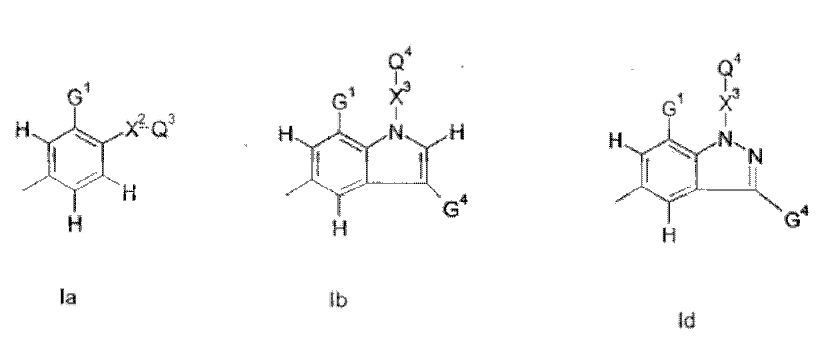
dans lesquelles G¹ est choisi parmi hydrogène, fluoro, chloro, bromo, cyano, (1-4C)alkyle et (2-3C)alcynyle,
G⁴ est choisi parmi hydrogène, fluoro, chloro, bromo, cyano, (1-4C)alkyle et éthynyle,
X² est choisi parmi 0, S, NH, N(CH₃), OCH₂, CO et NHCH₂,
et Q³ est un groupement choisi parmi phényle, 2-furyle, 2- ou 3-thiényle, 2-, 4- ou 5-oxazolyle, 3-, 4- ou 5-isoxazolyle, 2-, 4- ou 5-1H-imidazolyle, 2-, 4- ou 5-thiazolyle, 3- ou 4-(1H-1,2,4-triazolyle), 3- ou 5-(1,2,5-thiadiazolyle), 2- 3- ou 4-pyridyle, 2-, 4- ou 5-pyrimidinyle, 1,3-benzodioxol-4-yle, 1,3-benzodioxol-5-yle, 2-, 3-, 4-, 5-, 6-, 7- ou 8-quinoléinyle, 1-, 3-, 4-, 5-, 6-, 7- ou 8-isoquinoléinyle et 2-, 3-, 4-, 5-, 6-, 7- ou 8-quinazolinyle, lequel groupement est éventuellement substitué par un ou deux substituants, qui peuvent être identiques ou différents, choisis parmi nitro, fluoro, chloro, bromo, (1-4C)alkyle, trifluorométhyle, (1-4C)alcoxy, (2-3C)alcynyle et cyano,
ou X² est CO et Q³ est un groupement choisi parmi pyrrolidin-1-yle, pipéridino, homopipéridino, morpholino, pipérazin-1-yle, homopipérazin-1-yle, décahydroquinoléin-1-yle, et décahydroisoquinoléin-2-yle, lequel groupement porte éventuellement 1 ou 2 substituants qui peuvent être identiques ou différents choisis parmi fluoro, chloro, bromo, cyano, hydroxy et (1-4C)alkyle,
et un groupement hétérocyclyle quelconque représenté par Q³ porte éventuellement 1 ou 2 substituants oxo,
X³ est choisi parmi SO₂ et CH₂,
Q⁴ est un groupement choisi parmi un groupement phényle, 2-furyle, 3-furyle, 2-(1,3-oxazolyle), 4-(1,3-oxazolyle), 3-, 4- ou 5-isoxazolyle, 2-imidazolyle, 4-imidazolyle, 2-, 3- ou 4-pyridyle, 2-, 4- ou 5-pyrimidinyle, 1,2,4-triazol-3-yle, 2-thiényle, 3-thiényle, 2-(1,3-thiazolyle), 4-(1,3-thiazolyle), 3-, 4- ou 5-isothiazolyle et 1,2,5-thiadiazol-3-yle, lequel groupement porte éventuellement 1 ou 2 substituants, qui peuvent être identiques ou différents, choisis parmi nitro, fluoro, chloro, bromo, cyano, (1-4C)alkyle, (1-4C)alcoxy, trifluorométhyle et (2-3C)alcynyle ;
ou un sel pharmaceutiquement acceptable de celui-ci .

2. Dérivé de la quinazoline selon la revendication 1, **caractérisé en ce que** le groupement Q¹-Z- est choisi parmi cyclopentyloxy, tétrahydrofuran-3-yloxy, tétrahydropyran-4-yloxy, tétrahydrothiopyran-4-yloxy, 1,1-dioxotétrahydrothiopyran-4-yloxy, 1-oxotétrahydrothiopyran-4-yloxy, tétrahydrothién-3-yloxy, 1,1-dioxotétrahydrothién-3-yloxy, 1-oxotétrahydrothién-3-yloxy, pyrrolidin-3-yloxy, pyrrolidin-2-yloxy, pipéridin-3-yloxy, pipéridin-4-yloxy, homopipéridin-3-yloxy, homopipéridin-4-yloxy et azétidin-3-yloxy,
et où le groupement azétidinyle, pyrrolidinyle, pipéridinyle ou homopipéridinyle dans le groupement Q¹-Z- est éventuellement N-substitué par un substituant choisi parmi méthyle, éthyle, n-propyle, iso-propyle, n-butyle, iso-butyle, tert-butyle, allyle, 2-propynyle, acétyle, propionyle, méthoxycarbonyle, éthoxycarbonyle, propoxycarbonyle, tert-butoxycarbonyle, méthylsulfonyle, éthylsulfonyle, 2-méthoxyéthyle, carbamoylméthyle, N-méthylcarbamoylméthyle, N,N-di-méthylcarbamoylméthyle, 2-carbamoyléthyle, 2-(N-méthylcarbamoyl)éthyle, 2-(N,N-diméthylcarbamoyl)éthyle, acétylméthyle, 2-acétyléthyle, méthoxycarbonylméthyle et 2-méthoxycarbonyléthyle,
et où un groupement hétérocyclyle quelconque dans le groupement Q¹-Z- porte éventuellement 1 ou 2 substituants oxo ;
ou un sel pharmaceutiquement acceptable de celui-ci.

3. Dérivé de la quinazoline selon la revendication 1, **caractérisé en ce que** le groupement Q¹-Z- est pipéridin-4-yloxy, lequel groupement est éventuellement N-substitué par un substituant choisi parmi méthyle, éthyle, n-propyle, iso-propyle, allyle, 2-propynyle, méthoxycarbonyle, éthoxycarbonyle, propoxycarbonyle, tert-butoxycarbonyle, méthylsulfonyle, 2-méthoxyéthyle, carbamoylméthyle, N-méthylcarbamoylméthyle, N,N-di-méthylcarbamoylméthyle, acétylméthyle, 2-acétyléthyle, méthoxycarbonylméthyle et 2-méthoxycarbonyléthyle,
et où un groupement hétérocyclyle quelconque dans le groupement Q¹-Z- porte éventuellement 1 ou 2 substituants oxo ;
ou un sel pharmaceutiquement acceptable de celui-ci.

4. Dérivé de la quinazoline selon la revendication 1, **caractérisé en ce que** Q² est un groupement de formule Ia dans laquelle
G¹ est choisi parmi fluoro, chloro, bromo et éthynyle,
X² est CO et Q³ est choisi parmi pipéridino, homopipéridino, décahydroquinoléin-1-yle, et décahydroisoquinoléin-2-yle,
et où Q³ porte éventuellement 1 ou 2 substituants choisis parmi fluoro, chloro, bromo, cyano, hydroxy, méthyle et éthyle,
et où Q³ porte éventuellement 1 ou 2 substituants oxo ;
ou un sel pharmaceutiquement acceptable de celui-ci.

5. Dérivé de la quinazoline selon la revendication 1, **caractérisé en ce que** Q² est un groupement de formule Ib dans laquelle G¹ est hydrogène,
G⁴ est choisi parmi hydrogène, fluoro, chloro, bromo, cyano, méthyle et éthynyle,
X³ est choisi parmi SO₂ et CH₂, et Q⁴ est choisi parmi phényle et 2-, 3- ou 4-pyridyle qui porte éventuellement 1 ou 2 substituants, qui peuvent être identiques ou différents, choisis parmi fluoro, chloro, bromo, nitro, cyano et méthyle ;
ou un sel pharmaceutiquement acceptable de celui-ci.

6. Dérivé de la quinazoline selon la revendication 1, **caractérisé en ce que** Q² est un groupement de formule Id dans laquelle G¹ est hydrogène,
G⁴ est choisi parmi hydrogène, fluoro, chloro, bromo, cyano, méthyle et éthynyle,
X³ est choisi parmi SO₂ et CH₂, et Q⁴ est choisi parmi phényle et 2-, 3- ou 4-pyridyle qui porte éventuellement 1 ou 2 substituants, qui peuvent être identiques ou différents, choisis parmi fluoro, chloro, bromo, nitro, cyano et méthyle ;
ou un sel pharmaceutiquement acceptable de celui-ci.

7. Dérivé de la quinazoline selon la revendication 1, **caractérisé en ce que** le groupement Q¹-Z- est choisi parmi tétrahydrofuran-3-yloxy, tétrahydropyran-3-yloxy et tétrahydropyran-4-yloxy,
et où Q¹ porte éventuellement un substituant oxo ;
ou un sel pharmaceutiquement acceptable de celui-ci.

8. Dérivé de la quinazoline selon la revendication 1, **caractérisé en ce que** le groupement Q¹-2- est pipéridin-4-yloxy éventuellement substitué en position 1 par un substituant choisi parmi méthyle, allyle, 2-méthoxyéthyle et carbamoylméthyle,
et où le groupement pipéridin-4-yle porte éventuellement un substituant oxo ;
ou un sel pharmaceutiquement acceptable de celui-ci.

9. Dérivé de la quinazoline selon la revendication 1, **caractérisé en ce que** le groupement Q¹-Z- est un méthylpipéridin-4-yloxy ;
ou un sel pharmaceutiquement acceptable de celui-ci.

10. Dérivé de la quinazoline selon la revendication 1, **caractérisé en ce** Q² est un groupement de formule Ia dans laquelle
G¹ est chloro,
X² est OCH₂,
Q³ est phényle éventuellement substitué par 1 ou 2 substituants choisis parmi fluoro et cyano ;
ou un sel pharmaceutiquement acceptable de celui-ci.

11. Dérivé de la quinazoline selon la revendication 10, **caractérisé en ce que** Q³ est choisi parmi phényle, 2-fluorophényle, 3-fluorophényle, 2-cyanophényle, 3-cyanophényle, 2,5-difluorophényle et 2,6-difluorophényle ;
ou un sel pharmaceutiquement acceptable de celui-ci .

12. Dérivé de la quinazoline selon la revendication 1, **caractérisé en ce que** Q² est un groupement de formule Id dans laquelle G¹ et G⁴ sont hydrogène, X³ est CH₂ et Q⁴ est phényle éventuellement substitué par fluoro ou chloro,
ou un sel pharmaceutiquement acceptable de celui-ci.

13. Dérivé de la quinazoline selon la revendication 12, **caractérisé en ce que** Q⁴ est 3-fluorophényle ;
ou un sel pharmaceutiquement acceptable de celui-ci.

14. Dérivé de la quinazoline selon la revendication 1, choisi parmi :
la 4-(3-chloro-4-phénoxyanilino)-5-(1-méthylpipéridin-4-yloxy)quinazoline ;
la 4-(3-chloro-4-((3-fluorobenzyloxy)anilino))-5-(1-méthylpipéridin-4-yloxy)quinazoline ;
la 4-(1-(3-fluorobenzyl)indazol-5-ylamino)-5-(1-méthylpipéridin-4-yloxy)quinazoline ;
la 4-(3-chloro-4-((décahydroquinoléin-1-yl)carbonyl)aniline)-5-(1-méthylpipéridin-4-yloxy)quinazoline ;
la 4-(3-chloro-4-((décahydroisoquinoléin-2-yl)carbonyl)aniline)-5-(1-méthylpipéridin-4-yloxy)quinazoline ;
la 4-(3-chloro-4-((3-méthylpipéridin-1-yl)carbonyl)aniline)-5-(1-méthylpipéridin-4-yloxy)quinazoline ;
la 4-(3-éthynyl-4-((décahydroquinoléin-1-yl)carbonyl)aniline)-5-(1-méthylpipéridin-4-yloxy)quinazoline ;
la 4-(3-chloro-4-(5-méthylisoxazol-3-ylméthoxy)aniline)-5-(1-méthylpipéridin-4-yloxy)quinazoline ;
la 4-(3-chloro-4-(2,6-difluorobenzyloxy)anilino)-5-(1-méthylpipéridin-4-yloxy)quinazoline ;
la 4-(3-chloro-4-(2-fluorobenzyloxy)anilino)-5-(1-méthylpipéridin-4-yloxy)quinazoline ;
la 4-(3-chloro-4-(2-cyanobenzyloxy)anilino)-5-(1-méthylpipéridin-4-yloxy)quinazoline ;
et
la 4-(3-chloro-4-(thiazol-4-ylméthoxy)anilino)-5-(1-méthylpipéridin-4-yloxy)quinazoline ;
ou un sel d'addition d'acide pharmaceutiquement acceptable de celui-ci.

15. Dérivé de la quinazoline selon la revendication 1, choisi parmi :
la 4-(1-benzylindol-5-ylamino)-5-(1-méthylpipéridin-4-yloxy)quinazoline ;
la 4-(1-benzènesulfonylindol-5-ylamino)-5-(1-méthylpipéridin-4-yloxy)quinazoline ;
la 4-(1-(2-cyanobenzyl)indol-5-ylamino)-5-(1-méthylpipéridin-4-yloxy)quinazoline ;
la 4-(1-(2-méthoxybenzyl)indol-5-ylamino)-5-(1-méthylpipéridin-4-yloxy)quinazoline ;
la 4-(1-(2-chlorobenzyl)indol-5-ylamino)-5-(1-méthylpipéridin-4-yloxy)quinazoline ;
la 4-(1-(2,5-diméthylbenzyl)indol-5-ylamino)-5-(1-méthylpipéridin-4-yloxy)quinazoline ;
la 4-(1-(2-fluorobenzyl)indol-5-ylamino)-5-(1-méthylpipéridin-4-yloxy)quinazoline ;
et
la 4-(1-(3-fluorobenzyl)indol-5-ylamino)-5-(1-méthylpipéridin-4-yloxy)quinazoline
ou un sel d'addition d'acide pharmaceutiquement acceptable de celui-ci.

16. Dérivé de la quinazoline selon la revendication 1, choisi parmi :
la 4-(3-chloro-4-(3-fluorophénoxy)anilino)-5-(1-méthylpipéridin-4-yloxy)quinazoline ;
et
la 4-(3-chloro-4-((1-méthyl-1*H*-imidazol-2-yl)thio)anilino)-5-(1-méthylpipéridin-4-yloxy)quinazoline ;
ou un sel d'addition d'acide pharmaceutiquement acceptable de celui-ci.

17. Dérivé de la quinazoline selon la revendication 1, choisi parmi :
la 4-(3-chloro-4-(3-fluorobenzyloxy)anilino)-5-(tétrahydropyran-4-yloxy)quinazoline ;
la 4-(4-benzyloxy-3-méthylanilino)-5-(1-méthylpipéridin-4-yloxy)quinazoline ;
la 4-(3-méthyl-4-(5-méthylisoxazol-3-ylméthoxy)anilino)-5-(1-méthylpipéridin-4-yloxy)quinazoline ;
la 4-(4-(3-fluorobenzyloxy)-3-méthylanilino)-5-(1-méthylpipéridin-4-yloxy)quinazoline ;
la 4-(3-éthynyl 4-(3-fluorobenzyloxy)anilino)-5-(1-méthylpipéridin-4-yloxy)quinazoline ;
la 4-(3-éthynyl 4-(2,6-difluorobenzyloxy)anilino)-5-(1-méthylpipéridin-4-yloxy)quinazoline ; et
la 4-(3-chloro-4-(pyridin-2-ylméthoxy)anilino)-5-(1-méthylpipéridin-4-yloxy)quinazoline ;
ou un sel d'addition d'acide pharmaceutiquement acceptable de celui-ci.

18. Procédé de préparation d'un dérivé de la quinazoline selon la revendication 1, ou d'un sel pharmaceutiquement acceptable de celui-ci, **caractérisé en ce qu'**il comprend :
(a) la réaction d'une quinazoline de formule II 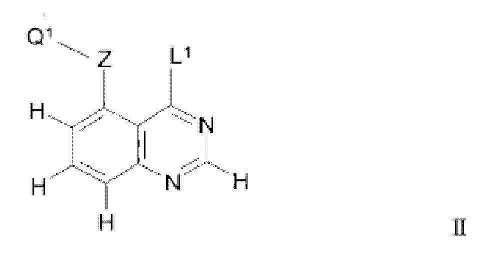 dans laquelle L¹ est un groupement déplaçable et Q¹ et Z ont l'une quelconque des significations définies dans la revendication 1 sauf qu'une fonction quelconque est protégée le cas échéant, avec un composé de formule
Q²NH₂
dans laquelle Q² a l'une quelconque des significations définies dans la revendication 1 sauf qu'une fonction quelconque est protégée le cas échéant, puis un groupement protecteur quelconque qui est présent est enlevé par des moyens classiques ; ou
(b) le couplage d'un alcool de formule
Q¹-OH
dans laquelle Q¹ a l'une quelconque des significations définies dans la revendication 1 (et Z est 0) sauf qu'une fonction quelconque est protégée le cas échéant, avec une quinazoline de formule VI 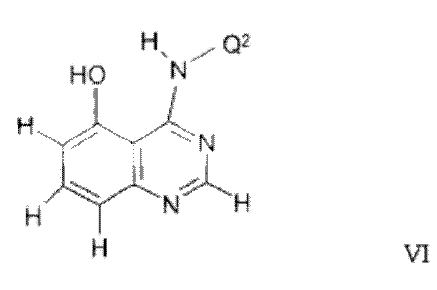 dans laquelle Q² a l'une quelconque des significations définies dans la revendication 1 sauf qu'une fonction quelconque est protégée le cas échéant, puis un groupement protecteur quelconque qui est présent est enlevé par des moyens classiques ; ou
(c) la réaction d'un alcool de formule
Q¹-OH
dans laquelle Q¹ a l'une quelconque des significations définies dans la revendication 1 (et Z est 0) sauf qu'une fonction quelconque est protégée le cas échéant, avec une quinazoline de formule VIII 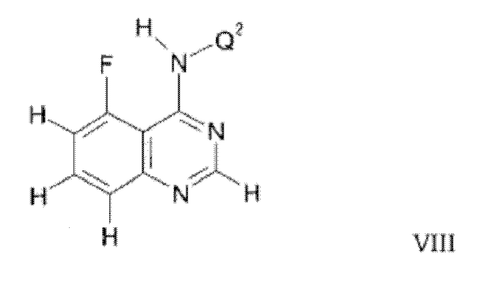 dans laquelle Q² a l'une quelconque des significations définies dans la revendication 1 sauf qu'une fonction quelconque est protégée le cas échéant, puis un groupement protecteur quelconque qui est présent est enlevé par des moyens classiques ; ou
(d) pour la production des composés de formule I, dans laquelle Q¹ ou Q² contient un groupement amino primaire ou secondaire, le clivage du composé correspondant de formule I dans laquelle Q¹ ou Q² contient un groupement amino primaire ou secondaire protégé ; ou
(e) pour la production des composés de formule I dans laquelle Q¹ ou Q² contient un groupement (1-6C)alcoxy ou (1-6C)alcoxy substitué ou un groupement (1-6C) alkylamino ou (1-6C)alkylamino substitué, l'alkylation d'un dérivé de la quinazoline de formule I dans laquelle Q¹ ou Q² contient un groupement hydroxy ou un groupement amino primaire ou secondaire comme approprié ; ou
(f) pour la production des composés de formule I dans laquelle Q¹ contient un groupement (1-6C)alcoxy amino-hydroxy-disubstitué, la réaction d'un composé de formule I dans laquelle Q¹ contient un groupement (1-6C)alcoxy époxy-substitué avec un hétérocycle ou une amine appropriée ; ou
(g) la réaction d'une quinazoline de formule X 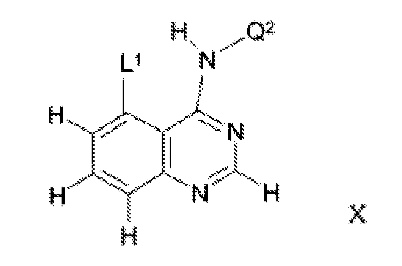 dans laquelle L¹ est un groupement déplaçable et Q² a l'une quelconque des significations définies dans la revendication 1 sauf qu'une fonction quelconque est protégée le cas échéant, avec un composé de formule
Q¹ZH
dans laquelle Q¹ et Z ont l'une quelconque des significations définies dans la revendication 1 sauf qu'une fonction quelconque est protégée le cas échéant, puis un groupement protecteur quelconque qui est présent est enlevé par des moyens classiques ; ou
(h) pour la production des composés de formule I dans laquelle Q¹ contient un groupement (1-6C)alcoxy amino-substitué, la réaction d'un composé de formule I dans laquelle Q¹ contient un groupement (1-6C)alcoxy halogéno-substitué, avec une amine appropriée ou un hétérocycle contenant un groupement NH du cycle ; ou
(i) pour la production des composés de formule I dans laquelle un groupement hétérocyclyle dans Q¹ ou Q³ contient un S- ou N-oxyde, l'oxydation d'un atome de N ou de S du cycle dans un composé de formule (I) ; ou
(j) pour la production des composés de formule I dans laquelle le groupement X²-Q³ est COQ³ et Q³ est un groupement hétérocyclyle lié par azote tel que défini dans la revendication 1, le couplage de la quinazoline carboxy-substituée de formule XI : 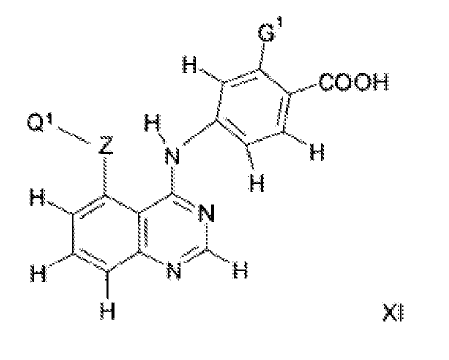 ou d' un dérivé réactif de celle-ci, avec Q³H, où Q¹, Q³, Z et G¹ sont tels que définis dans la revendication 1 sauf qu'une fonction quelconque est protégé le cas échéant, puis un groupement protecteur quelconque qui est présent est enlevé par des moyens classiques ; ou
(k) pour la production des composés dans lesquels Q² est un groupement de formule Ia dans laquelle :
(i) X²Q³ est OCH₂Q³ et Q³ est aryle ou hétéroaryle tel que défini dans la revendication 1, ou
(ii) X²Q³ est OQ³ et Q³ est hétéroaryle tel que défini dans la revendication 1,
la réaction, éventuellement en présence d'une base, d'un composé de formule I tel que défini dans la revendication 1 dans laquelle Q² est un groupement de formule Ia tel que défini dans la revendication 1 sauf que le groupement X²Q³ dans la formule Ia est OH, avec un composé de formule L¹CH₂Q³ ou L¹Q³,
dans lesquelles L¹ est un groupement déplaçable, et Q³ est tel que défini dans la revendication 1 sauf qu'une fonction quelconque est protégée le cas échéant, puis un groupement protecteur quelconque qui est présent est enlevé par des moyens classiques ; ou
(l) pour la production des composés de formule I dans laquelle Q¹ contient un groupement (2-6C)alcanoylamino ou (2-6C)alcanoylamino substitué, l'acylation d'un dérivé de la quinazoline de formule I dans laquelle Q¹ contient un groupement amino ; ou
(m) pour la production des composés de formule I dans laquelle Q² est un dérivé de l'indole ou de l'indazole de formule Ib ou Id telles que définies dans la revendication 1, la réaction d'un composé de formule Q⁴X³L¹, dans laquelle L¹ est un groupement déplaçable et Q⁴ et X³ sont tels que définis dans la revendication 1, sauf qu'une fonction quelconque est protégée le cas échéant, avec un composé de formule XII, 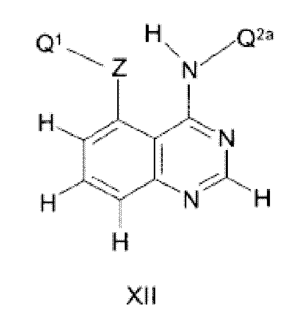 dans laquelle Q¹-Z- est tel que défini dans la revendication 1, sauf qu'une fonction quelconque est protégée le cas échéant, et Q^{2a} est choisi parmi un composé de formule Ib' et Id' 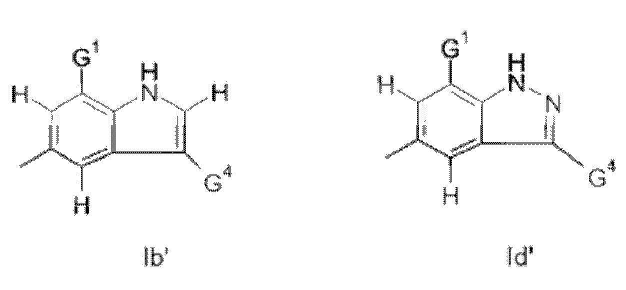 dans lesquelles G¹ et G⁴ ont l'une quelconque des significations définies dans la revendication 1, sauf qu'une fonction quelconque est protégée le cas échéant, puis un groupement protecteur quelconque qui est présent est enlevé par des moyens classiques ; ou
(n) pour la production des composés de formule I dans laquelle Q² est un groupement de formule Ia dans laquelle Q³ est aryle ou hétéroaryle tels que définis dans la revendication 1 et X² est S, le couplage du composé de formule XVIII 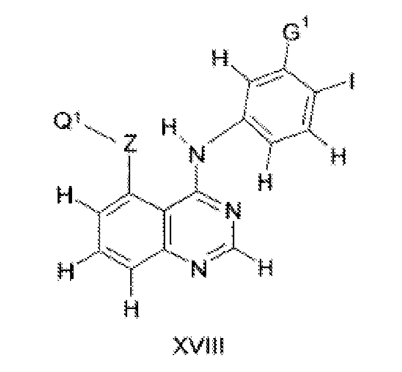 dans laquelle Q¹, Z et G¹ ont l'une quelconque des significations définies dans la revendication 1, sauf qu'une fonction quelconque est protégée le cas échéant, avec un composé de formule Q³SH, où Q³ est aryle ou hétéroaryle tel que défini dans la revendication 1, sauf qu'une fonction quelconque est protégée le cas échéant, en présence de bromure cuivreux, et d'une base, puis un groupement protecteur quelconque qui est présent est enlevé par des moyens classiques ;
(o) pour la production des composés de formule I dans laquelle Q¹ ou Q² contient un groupement (1-6C)alkylamino, (1-6C)alkylamino substitué ou un groupement hétérocyclyle lié par l'azote, l'amination réductrice d'un groupement aldéhyde ou cétone dans un composé de formule I, avec un groupement (1-6C)alkylamino, (1-6C)alkylamino substitué ou un groupement hétérocyclyle contenant un groupement NH en présence d'un agent de réduction convenable ; ou
(p) la transformation d'un composé de formule I en un autre composé de formule 1 ;
et lorsqu'on souhaite un sel pharmaceutiquement acceptable d'un dérivé de la quinazoline de formule I, il peut être obtenu en utilisant un mode opératoire classique.

19. Composition pharmaceutique, **caractérisée en ce qu'**elle comprend un dérivé de la quinazoline de formule I, ou un sel pharmaceutiquement acceptable de celui-ci, telle que définie dans la revendication 1, en association avec un diluant ou un support pharmaceutiquement acceptable.

20. Dérivé de la quinazoline de formule I, ou un sel pharmaceutiquement acceptable de celui-ci, telle que définie dans la revendication 1, destiné à être utilisé dans une méthode de traitement de l'organisme humain ou animal par thérapie.

21. Utilisation d'un dérivé de la quinazoline de formule I, ou d'un sel pharmaceutiquement acceptable de celui-ci, telle que définie dans la revendication 1, dans la fabrication d'un médicament destiné à être utilisé dans la prévention ou le traitement de tumeurs qui sont sensibles à l'inhibition d'une ou plusieurs récepteurs tyrosine kinases erB.

22. Utilisation d'un dérivé de quinazoline de formule I, ou d'un sel pharmaceutiquement acceptable de celui-ci, tel que défini dans la revendication 1, pour la fabrication d'un médicament destiné à être utilisé pour le traitement d'un cancer choisi parmi une leucémie, un myélome multiple, un lymphome, un cancer de la voie biliaire, osseux, de la vessie, du cerveau/SNC, du sein, colorectal, endométrial, gastrique, de la tête et du cou, hépatique, du poumon, neuronal, oesophagien, ovarien, pancréatique, de la prostate, rénal, de la peau, testiculaire, de la thyroïde, de l'utérus et de la vulve.
